(19) Europäisches Patentamt / European Patent Office / Office européen des brevets

(11) **EP 3 234 128 B1**

(12) **EUROPEAN PATENT SPECIFICATION**

(45) Date of publication and mention of the grant of the patent:
**18.06.2025 Bulletin 2025/25**

(21) Application number: **15868702.0**

(22) Date of filing: **15.12.2015**

(51) International Patent Classification (IPC):
**C07H 21/04** (2006.01) **C12Q 1/6869** (2018.01)
**C12N 15/01** (2006.01)

(52) Cooperative Patent Classification (CPC):
**C12Q 1/6869; C07H 21/04; C12N 15/01;**
C12Q 2535/122; C12Q 2545/107

(86) International application number:
**PCT/AU2015/050797**

(87) International publication number:
**WO 2016/094947 (23.06.2016 Gazette 2016/25)**

(54) **SEQUENCING CONTROLS**

SEQUENZSTEUERUNGEN

TÉMOINS DE SÉQUENÇAGE

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 16.12.2014 AU 2014905092
24.09.2015 AU 2015903892

(43) Date of publication of application:
25.10.2017 Bulletin 2017/43

(60) Divisional application:
25175130.1

(73) Proprietor: Garvan Institute of Medical Research
Darlinghurst, NSW 2010 (AU)

(72) Inventor: MERCER, Timothy
Darlinghurst, New South Wales 2010 (AU)

(74) Representative: Mewburn Ellis LLP
Aurora Building
Counterslip
Bristol BS1 6BX (GB)

(56) References cited:
WO-A2-03/016550

• **LUKAS PAUL: "Spike-in RNA Variants: Design, Production and Application", 17 August 2014 (2014-08-17), XP002780199, Retrieved from the Internet <URL:https://de.slideshare.net/ERCC-Workshop/20140710-3-lpaulercc20workshop> [retrieved on 20180416]**
• **CARLSON CHRISTOPHER S ET AL: "Using synthetic templates to design an unbiased multiplex PCR assay", NATURE COMMUNICATIONS, vol. 4, 2013, pages 2680, XP008165824, ISSN: 2041-1723, [retrieved on 20131025], DOI: 10.1038/NCOMMS3680**
• **SIMON A HARDWICK ET AL: "Spliced synthetic genes as internal controls in RNA sequencing experiments", NATURE METHODS, vol. 13, no. 9, September 2016 (2016-09-01), pages 792 - 798, XP055466160, ISSN: 1548-7091, DOI: 10.1038/nmeth.3958**
• **IRA W DEVESON ET AL: "Representing genetic variation with synthetic DNA standards", NATURE METHODS, vol. 13, no. 9, September 2016 (2016-09-01), pages 784 - 791, XP055466163, ISSN: 1548-7091, DOI: 10.1038/nmeth.3957**

- "Standard Reference Material® 2374', Certificate of Analysis", 2013, pages 1, Retrieved from the Internet <URL:www-s.nist.gov/srmors/ certificates/ 2374.pdf?CFID=38635724&CFTOKEN=233- de0287151462d-AEO162D6- A0D9-8E6C-2FF1298DAOCBFE53> [retrieved on 20160219]
- "ERCC RNA Spike-In Control Mixes", USER GUIDE, 2012, pages 1 - 5, 10, 13-14 and 17, Retrieved from the Internet <URL:https://tools. thermofisher.com/content/sfs/manuals/ cms_086340.pdt> [retrieved on 20160219]
- HWANG, B. ET AL.: "Quantitative oligonucleotide microarray data analysis with an artificial standard probe strategy", BIOSENSORS AND BIOELECTRONICS, vol. 23, no. 11, 2008, pages 1738 - 1744, XP022625065, doi:10.1016/ j.bios.2008.01.024
- LOVEN, J. ET AL.: "Revisiting global gene expression analysis", CELL, vol. 151, no. 3, 2012, pages 476 - 82, XP028952803, doi:10.1016/ j.cell.2012.10.012
- JIANG, L. ET AL.: "Synthetic spike-in standards for RNA-seq experiments", GENOME RESEARCH, vol. 21, no. 9, 2011, pages 1543 - 1551, XP055152443, doi:10.1101/gr.121095.111
- ZOOK, J. ET AL.: "Synthetic spike-in standards improve run-specific systematic error analysis for DNA and RNA sequencing", PLOS ONE, vol. 7, no. 7, 2012, pages 3 - 7
- NATURE METHODS, vol. 11, no. 2, 2014, pages 163 - 166, XP002733949
- HAYNES, R. ET AL.: "Standard reference material 2366 for measurement of human cytomegalovirus DNA", THE JOURNAL OF MOLECULAR DIAGNOSTICS, vol. 15, no. 2, 2013, pages 177 - 185, XP055454787
- WONG, G. ET AL.: "Multiple-complete-digest restriction fragment mapping: generating sequence-ready maps for large-scale DNA sequencing", PROCEEDINGS OF THE NATIONAL ACADEMY OF SCIENCES, vol. 94, no. 10, 1997, pages 5225 - 5230, XP055454791

## Description

## Technical Field

The present disclosure generally relates to sequencing controls (or "standards"), which can be used to calibrate a wide variety of sequencing methods. For example, the sequencing controls disclosed herein can be used to calibrate a wide variety of high throughput sequencing methods (for example, those referred to as next generation sequencing methods). The present disclosure also generally relates to the use of the sequencing controls in a wide variety of applications including, for example, in the calibration of a wide variety of sequencing methods.

## Background

Next-generation sequencing (NGS) technologies (exemplified by services and products provided by companies such as Illumina, Nanopore, PacBio, Ion Torrent, Roche 454 Pyrosequencing (see, e.g., Bentley, D.R. *et al.,* 2008; Clarke, J. *et al.,* 2009; Ronaghi, M. *et al.,* 1998; Eid, J. *et al.,* 2009; Rothberg, J.M. *et al.,* 2011) and others) enable the high-throughput, massively parallel sequencing of nucleic acid molecules. These technologies have the capacity to determine the nucleotide base sequence of millions of RNA and DNA molecules within a single sample. Furthermore, the rate at which individual RNA or DNA sequences are determined is proportional to the relative abundance of that individual RNA or DNA sequence within the sample. Therefore, NGS can also be used to determine the quantity of one or more nucleic acid sequences within a sample.

NGS is widely used to determine the sequence and/or measure the quantities of nucleic acids found within samples taken from natural sources, such as animals, plants, microorganisms, or the diverse population of microbes within an environmental sample (Edwards, R.A. *et al.,* 2006). These uses include the determination of an organism's full genome sequence (see, e.g., Bentley, D.R. *et al.,* 2008), the determination of the sequence and abundance of messenger RNA present within a sample (see, e.g., Mortazavi, A. *et al.,* 2008), or the sequencing and measurement of a range of cellular features, such as epigenetic modifications (see, e.g., Bernstein, B.E. *et al.,* 2005), protein binding sites (see, e.g., Johnson, D.S., *et al.,* 2007), and three-dimensional DNA structure (see, e.g., Lieberman-Aiden, E. *et al.,* 2009), and other features.

The millions of individual RNA or DNA sequences determined by NGS can be merged by *de novo* assembly into longer sequences (called contigs) or matched to a known reference sequence. *De novo* assembly of DNA sequences can be used to assemble an organism's genome; *de novo* assembly of RNA sequences can indicate gene sequence, length and isoforms. The matching or alignment of DNA sequences to a reference genome can identify the location of genetic differences or variation between individuals. The location of matches between DNA sequences and the reference genome can indicate locations of epigenetic features, such as histone modifications, or protein binding sites. Alignment of RNA sequences to a reference genome can indicate the existence of intron sequences that are excised during the process of gene splicing.

In some instances, during the operation of such sequencing methods, nucleic acids of known quantities or sequences, termed standards, have been added (or "spiked-in") to a natural sample of nucleic acids. The resultant combined mixture may then be analysed using a range of genetic technologies (such as NGS technologies), including microarray technologies, quantitative polymerase chain reaction methods, and others. The quantities or sequences of the sample nucleic acids can be compared to the known quantities or sequences of the added nucleic acid standards, in order to provide a reference scale that can be used to measure and determine the quantities or sequences of a natural sample of nucleic acids.

Currently used RNA and DNA standards are derived from natural sources. For example, a DNA sequence extracted from the NA12878 cell line originally derived from a Caucasian female human has been extensively characterized and has been used to assess the performance of analytical tools to identify genetic variation (Zook, J.M. *et al.,* 2014). Ribonucleic-acid standards (known as ERCC Spike-Ins) containing sequences derived from the archaea *Methanocaldococcus jannaschii* were developed for microarrays and qRT-PCR technologies (Baker, S.C. *et al.,* 2005; Consortium, E.R.C., 2005) and have been used with RNA sequencing (Jiang, L. *et al.,* 2011).

However, the disadvantage of nucleic acid standards that have been derived from natural sources is that they often cannot be added directly to samples because they share homologous sequences with the nucleic acid sequences of interest in the sample. The use of nucleic acid standards that have been derived from natural sources results in a failure to be able to distinguish the standards from the homologous sequences of interest that are present in the sample. Accordingly, the value of such standards as a tool to calibrate the sequencing methods applied to the sample of interest is limited and there remains a need for alternative and improved sequencing controls.

Lukas Paul, "Spike-in RNA variants: Design, Production and Application", ERCC 2.0 Workshop, 17 August 2014, https://de.slideshare.net/ERCC-Workshop/20140710-3-1paulercc20workshop describes spike-in RNA variants simulating transcript variants in which retrieved sequences are inverted.

# EP 3 234 128 B1

**Summary**

The invention is defined by the scope of the appended claims.

The present disclosure describes artificial sequencing controls and methods using such artificial sequencing controls. The term "controls" is used herein interchangeably with the term "standards". Thus, the present disclosure provides novel, artificial sequencing standards.

In one aspect, the present disclosure provides a method of calibrating a polynucleotide sequencing process. The method comprises making an artificial polynucleotide sequence, wherein the artificial polynucleotide sequence comprises at least 100 nucleotides, wherein any 100 contiguous nucleotides of the artificial polynucleotide sequence have less than 100% sequence identity with any known naturally occurring genomic sequence of the same length, and wherein the artificial polynucleotide sequence is a DNA sequence. Making the artificial polynucleotide sequence comprises retrieving a natural nucleotide sequence, and reversing the natural nucleotide sequence, wherein the reversed sequence has the same nucleotide bases in the same order as the natural nucleotide sequence, wherein the order of the nucleotide bases is in the 5' to 3' direction in the natural nucleotide sequence and in the 3' to 5' direction in the reversed sequence. The method further comprises adding the artificial polynucleotide sequence to a sample comprising a target polynucleotide sequence to be determined, determining the sequence of the target polynucleotide; determining the sequence of the one or more artificial polynucleotide sequence; comparing the sequence determined to an original sequence of the artificial polynucleotide sequence; determining the accuracy of the sequence determination based on the comparing; and calibrating the sequence determination using the determined accuracy. The artificial polynucleotide sequence comprises: one or more features of naturally occurring eukaryotic chromosomes selected from the group consisting of gene loci, introns, exons, CpG islands, mobile elements, repetitive polynucleotide features, small scale genetic variation and large scale genetic variation, one or more features of naturally occurring prokaryotic chromosomes, or one or more features of naturally occurring viruses, phages or organelle sequences.In embodiments, any 21 contiguous nucleotides of the artificial polynucleotide sequence can have less than 100% sequence identity with any known naturally occurring genomic sequence of the same length. In another example, any 20 contiguous nucleotides of the artificial polynucleotide sequence can have less than 100% sequence identity with any known naturally occurring genomic sequence of the same length.

In another example, any 21 or more contiguous nucleotides of the artificial polynucleotide sequence can have less than 100% sequence identity with any known naturally occurring genomic sequence of the same length. In another example, any 20 or more contiguous nucleotides of the artificial polynucleotide sequence can have less than 100% sequence identity with any known naturally occurring genomic sequence of the same length.

The artificial polynucleotide sequence can comprise any one or more features of naturally occurring eukaryotic chromosomes selected from the group consisting of gene loci, CpG islands, mobile elements, repetitive polynucleotide features, small scale genetic variation and large scale genetic variation. The artificial polynucleotide sequence can comprise multiple gene loci; the repetitive polynucleotide features can comprise any one or more of terminal repeats, tandem repeats, inverted repeats and interspersed repeats; the gene loci can comprise immune receptor gene loci; the small scale genetic variation can comprise one or more SNPs, one or more insertions, one or more deletions, one or more microsatellites and/or multiple nucleotide polymorphisms; and/or the large scale genetic variation can comprise one or more deletions, one or more duplications, one or more copy-number variants, one or more insertions, one or more inversions and/or one or more translocations.

Alternatively or in addition, the artificial polynucleotide sequence can comprise one or more features of naturally occurring prokaryotic chromosomes. For example, the artificial chromosome may comprise any one or more features of naturally occurring prokaryote chromosomes selected from the group consisting of gene loci, DNA repeats, mobile elements, and operons.

Also disclosed but not part of the claims is a fragment of the artificial chromosome disclosed herein, which comprises from 20 to 10,000,000 contiguous nucleotides of the artificial polynucleotide sequence. The fragment may be an RNA fragment or a DNA fragment.

Also disclosed but not part of the claims is an artificial polynucleotide sequence comprising two or more fragments of the present disclosure conjoined to form a contiguous polynucleotide sequence. The artificial polynucleotide sequence may be an RNA or a DNA polynucleotide sequence.

Also disclosed but not claimed is a vector comprising a DNA fragment of the artificial chromosome disclosed herein, which fragment comprises from 20 to 10,000,000 contiguous nucleotides of the artificial polynucleotide sequence.

Also disclosed but not claimed is a vector comprising the artificial polynucleotide sequence disclosed herein, which artificial polynucleotide sequence is a DNA polynucleotide sequence.

Making the one or more artificial polynucleotide seuqences can comprise excising the fragment from a vector disclosed herein by endonuclease digestion, amplification or transcribing the DNA fragment comprised within the vector disclosed herein. In one example, the amplification may be polymerase-chain amplification.

Also described but not claimed is a fragment of an artificial chromosome made by a method disclosed herein, for example a fragment of an artificial chromosome made by a method comprising excising the fragment from the vector of the present disclosure by endonuclease digestion, or transcribing a DNA fragment comprised within the vector of the present disclosure.

Also described but not claimed is a method of making the artificial polynucleotide sequence disclosed herein, the method comprising excising the artificial polynucleotide sequence from the vector disclosed herein by endonuclease digestion, amplification, or transcribing the artificial polynucleotide sequence comprised within the vector disclosed herein. In one example, the amplification may be polymerase-chain amplification. Also described but not claimed is a method of making the artificial polynucleotide sequence disclosed herein, the method comprising producing the artificial polynucleotide sequence by DNA synthesis.

Also described but not claimed is an artificial polynucleotide sequence made by a method disclosed herein, such as an artificial polynucleotide sequence made by a method comprising excising the artificial polynucleotide sequence from the vector of the present disclosure by endonuclease digestion, or transcribing a DNA of an artificial polynucleotide sequence comprised within the vector of the present disclosure.

Also described but not claimed is the use of the artificial chromosome disclosed herein and/or the fragment disclosed herein and/or the artificial polynucleotide sequence disclosed herein to calibrate a polynucleotide sequencing process. A wide variety of sequencing processes may be calibrated in this regard.

Also described but not claimed is the use of the artificial chromosome disclosed herein and/or the fragment disclosed herein and/or the artificial polynucleotide sequence disclosed herein to calibrate a polynucleotide quantitation process.

The present disclosure also provides a method of calibrating a polynucleotide quantitation process, comprising:

i) making one or more artificial polynucleotide sequences comprising: retrieving a natural nucleotide sequence, and reversing the natural nucleotide sequence, wherein the reversed sequence has the same nucleotide bases in the same order as the natural nucleotide sequence, wherein the order of the nucleotide bases is in the 5' to 3' direction in the natural nucleotide sequence and in the 3' to 5' direction in the reversed sequence, wherein the artificial polynucleotide sequence comprises at least 100 nucleotides, wherein any 100 contiguous nucleotides of the artificial polynucleotide sequence have less than 100% sequence identity with any known naturally occurring genomic sequence of the same length, and wherein the artificial polynucleotide sequence is a DNA sequence;

ii) adding a known amount of the one or more artificial polynucleotide sequence to a sample comprising a target polynucleotide sequence to be determined;

iii) determining the quantity of the target polynucleotide;

iv) determining the quantity of the one or more artificial polynucleotide sequence disclosed herein; and

v) comparing the quantity of the one or more artificial polynucleotide sequence determined in iv) to the known amount of the one or artificial polynucleotide sequence in i);

vi) determining the accuracy of the quantity determination in iii) based on the comparing in iv); and

vii) calibrating the quantity determination in iii) using the accuracy determined in v). The one or more artificial polynucleotide sequence comprises: (a) one or more features of naturally occurring eukaryotic chromosomes selected from the group consisting of gene loci, introns, exons, CpG islands, mobile elements, repetitive polynucleotide features, small scale genetic variation and large scale genetic variation, (b) one or more features of naturally occurring prokaryotic chromosomes, or (c) one or more features of naturally occurring viruses, phages or organelle sequences. Also described but not claimed is the use of the artificial chromosome disclosed herein and/or the fragment disclosed herein and/or the artificial polynucleotide sequence disclosed herein to calibrate a polynucleotide amplification process.

Also described but not claimed is a method of calibrating a polynucleotide amplification process, comprising:

i) adding a known amount of one or more fragment disclosed herein and/or one or more artificial polynucleotide sequence disclosed herein to a sample comprising a target polynucleotide sequence to be determined;

ii) amplifying the target polynucleotide;

iii) amplifying the one or more fragment disclosed herein and/or the one or more artificial polynucleotide sequence disclosed herein; and

iv) comparing amplified regions of the one or more fragment and/or the one or more artificial polynucleotide sequence amplified in iii) to amplified regions of the target polynucleotide amplified in ii);

wherein the amplification in iii) is used to calibrate the amplification in ii).

In any of the methods disclosed herein, two or more fragments (or standards) disclosed herein may be added to a sample at the same or different concentrations. This has the advantage of permitting the replication of natural states of homozygosity or heterozygosity, or heterogeneity (e.g., replicating the rare mutant allele frequency of impure samples that contain both normal and tumour cells; e.g. replicating complex allele frequencies resulting from chromosomal polyploidy; e.g. replicating a fetal genotype against a background of maternal genotype in circulating DNA).

Also described but not claimed is a kit comprising one or more artificial chromosome disclosed herein and one or more fragment as disclosed herein or one or more artificial polynucleotide sequence disclosed herein.

Also described but not claimed is a computer programmable medium containing one or more artificial chromosome disclosed herein stored thereon.

Also described but not claimed is a computer implemented method for generating an artificial chromosome comprising an artificial polynucleotide sequence, the computer implemented method comprising:

generating initial data indicative of an initial polynucleotide sequence;

determining a matching value indicative of a similarity between the initial polynucleotide sequence and one or more known naturally occurring polynucleotide sequence;

modifying the initial data based on the matching value to determine modified data indicative of a modified polynucleotide sequence such that the modified polynucleotide sequence is distinguishable from any known naturally occurring genomic sequence; and

storing the modified data on a data store.

In the computer implemented method disclosed herein, modifying the initial data may comprise shuffling the initial data.

Also described but not claimed is a computer implemented method of calibrating a polynucleotide sequencing process, the computer implemented method comprising:

receiving first data relating to a target polynucleotide sequence;

receiving second data indicative of one or more fragment of an artificial chromosome as disclosed herein and/or one or more artificial polynucleotide sequence disclosed herein; determining based on the second data a quantitative value related to a property of the one or more fragment or the one or more artificial polynucleotide sequence relative to a property of the artificial chromosome, which quantitative value is indicative of an accuracy of determining the property of the one or more fragment and/or the one or more artificial polynucleotide sequence; and

adjusting a property related to the first data based on the quantitative value to determine a calibrated property of the target polynucleotide sequence.

The computer implemented method may further comprise generating the first and/or second data; and storing the first and/or second data on a data store.

Also described but not claimed is a computer system for calibrating a polynucleotide sequencing process, the computer system comprising:

a data port to receive

first data relating to a target polynucleotide sequence,

second data indicative of one or more fragment of an artificial chromosome as disclosed herein and/or one or more artificial polynucleotide sequence disclosed herein; and

a processor to

determine based on the second data a first quantitative value related to a property of the one or more fragment and/or the one or more artificial polynucleotide sequence relative to a property of the artificial chromosome, which quantitative value is indicative of an accuracy of determining the property of the one or more fragment and/or the artificial polynucleotide sequence, and

adjust the first data based on the quantitative value to determine a calibrated property of the target polynucleotide sequence.

Each feature of any particular aspect or embodiment or example of the present disclosure may be applied *mutatis mutandis* to any other aspect or embodiment or example of the present disclosure.

**Brief Description of Drawings**

The following figures further demonstrate certain aspects of the present disclosure. The disclosure may be better understood by reference to one or more of these figures in combination with the detailed description of specific embodiments presented herein.

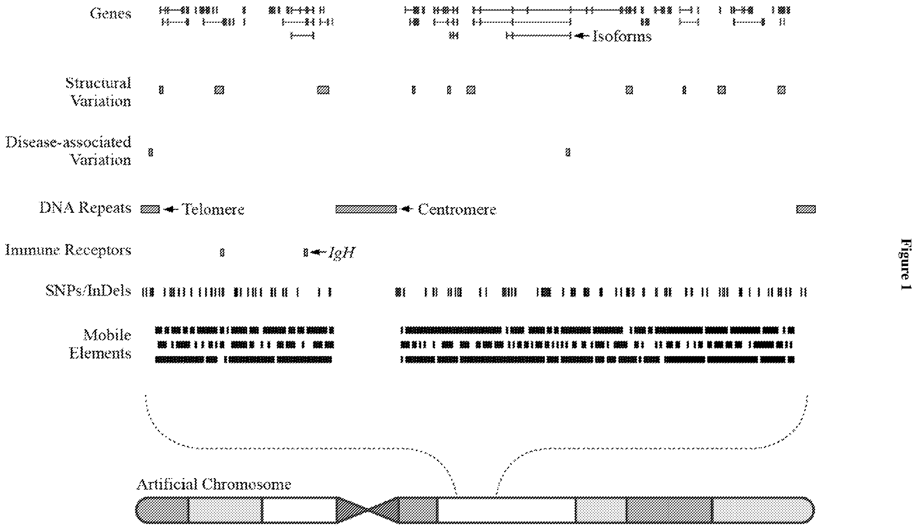

**Figure 1** illustrates potential structural features of an artificial chromosome of the present disclosure. The exemplified artificial chromosome contains features including (from top to bottom) genes, large-scale structural variation, disease-associated variation events, DNA repeat elements (including centromeres and telomeres), immune receptor loci, small-scale variation (e.g., <50nt) such as single nucleotide polymorphisms (SNPs), insertions or deletions (InDels); and mobile elements-derived sequences.

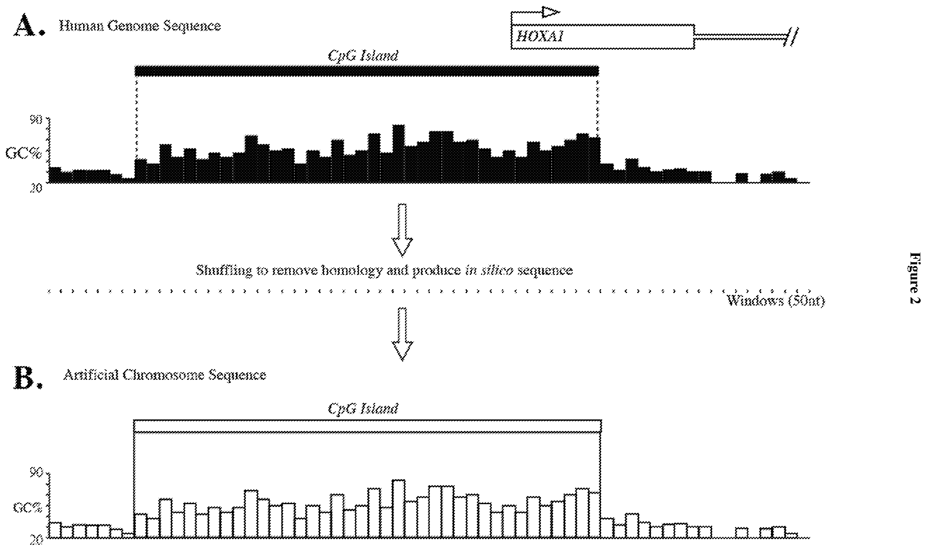

**Figure 2** illustrates the creation of an artificial chromosome by shuffling sequence to remove homology to any known natural sequence. The known DNA sequence (panel A) overlapping the CpG island (black box shown in panel A) in the promoter of the *HOXA1* gene was shuffled with a window size of 50nt. This removed homology with known or natural sequence (panel B), whilst maintaining high CpG dinucleotide content that defined the CpG Island (white box in panel B) at resolution of 50nt.

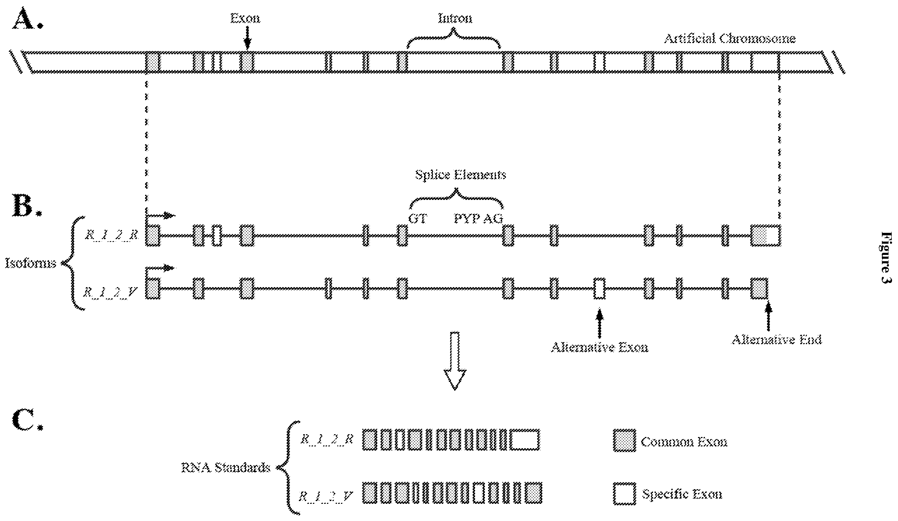

**Figure 3** illustrates a gene locus (panel A) comprising intervening exon and intron sequences within the artificial chromosome. (B) Alternative inclusion of exons can generate a number of different isoforms from a single gene locus. The lower panel (C) shows RNA standards generated to include the contiguous exonic sequences (with the intervening introns removed). RNA standards can be generated to represent different isoforms, with consensus exons (shaded) and alternative exons (white) indicated. By combining RNA standards representing alternative isoforms together at a range of concentrations, the biological process of alternative splicing is emulated.

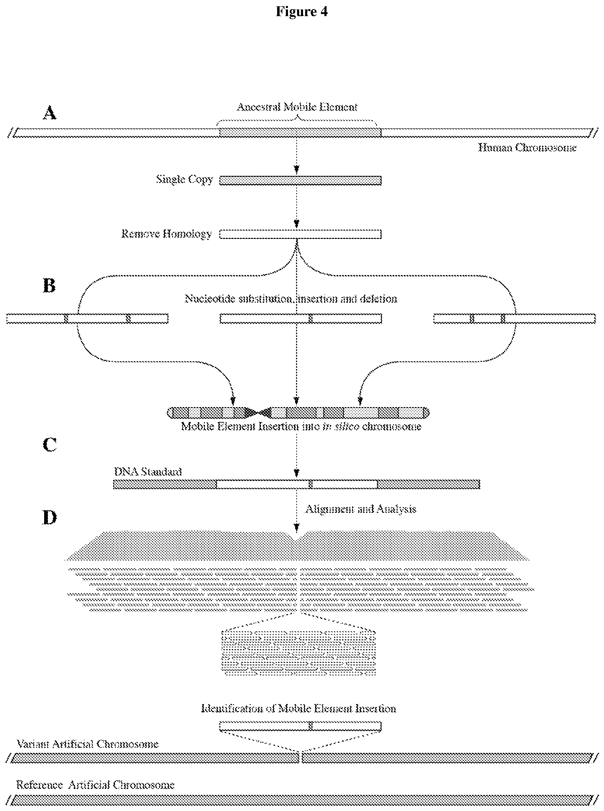

**Figure 4** illustrates the generation of a mobile element for inclusion in the artificial chromosome of the present disclosure. (A) Initially the sequence corresponding to a single copy of a mobile element (grey box) is retrieved from the human genome. Homology is removed to form an artificial, ancient mobile element (white box). (B) Multiple artificial mobile elements undergo further nucleotide substitution, insertion or deletion in parallel to model individual sequence divergence. Multiple artificial mobile elements are then assembled with the artificial chromosome. (C) DNA standards can be produced to represent the mobile element insertion. (D) Sequencing, alignment to the artificial chromosome (indicated by sequenced reads and histogram of sequence coverage) and analysis is able identify this mobile element.

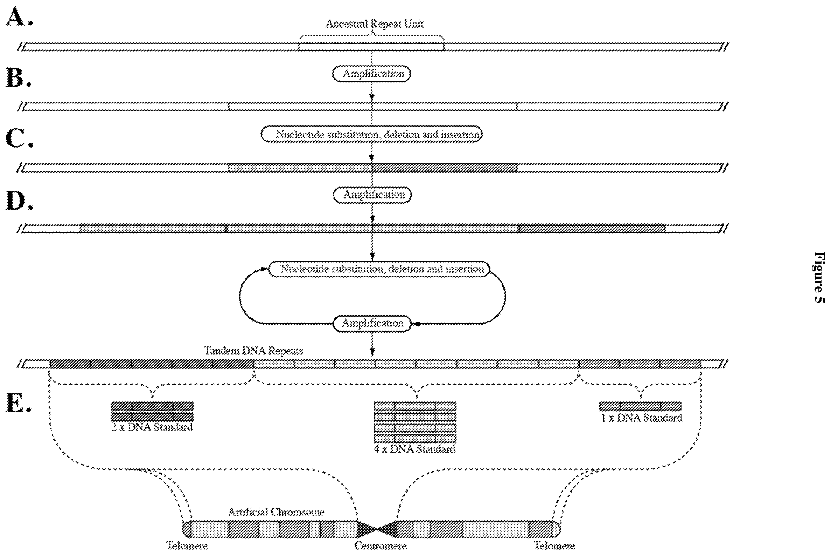

**Figure 5** illustrates the generation of particular examples of artificial DNA repeats which can be included in the artificial chromosome of the present disclosure. (A) Initially the sequence corresponding to a single copy of a DNA repeat of interest (such as a microsatellite, telomere or centromere repeat unit) is retrieved from the human genome. Homology is removed to form an artificial ("ancestral") mobile repeat element (white box). (B) The artificial mobile element is amplified. (C) Amplified artificial mobile elements undergo multiple nucleotide changes in parallel to model individual sequence divergence. (D) The artificial mobile element can be asymmetrically amplified. (E) The artificial sequence undergoes multiple amplification and nucleotide modification cycles to form large tandem DNA duplications with multiple subsets of repeats with varying copy number. (E) DNA standards can be produced that represent the different repeat subsets, with DNA standard abundance being proportional to repeat copy number.

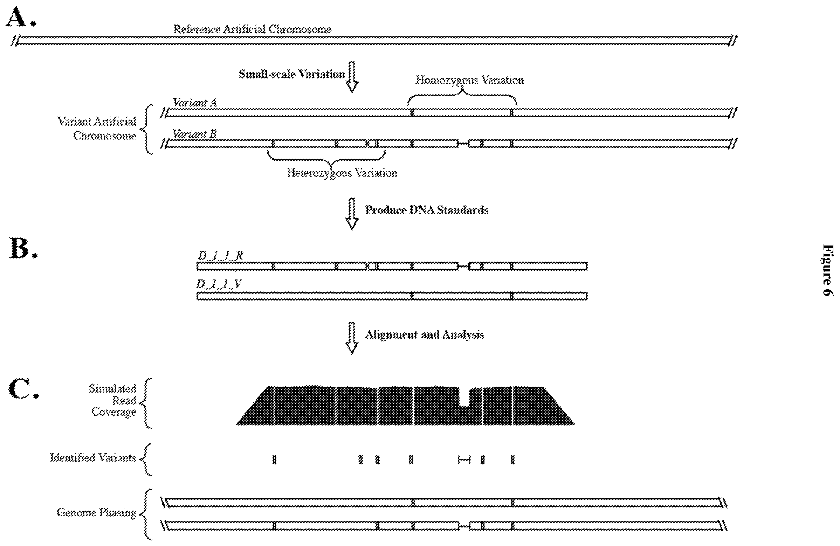

**Figure 6** illustrates the generation of artificial small-scale genetic variation which can be included in the artificial chromosomes of the present disclosure. (A) Small-scale genetic variation, including single-nucleotide polymorphisms, insertions, deletions etc., can be introduced into an artificial chromosome to form variant artificial chromosomes harbouring small-scale nucleotide variation. (B) Multiple DNA standards can be produced matching each variant artificial chromosome sequence, thereby emulating heterozygous or homozygous allele frequency. (C) Illustrates sequencing of the DNA standard, alignment to the reference artificial chromosome and analysis to identify the small-scale variation.

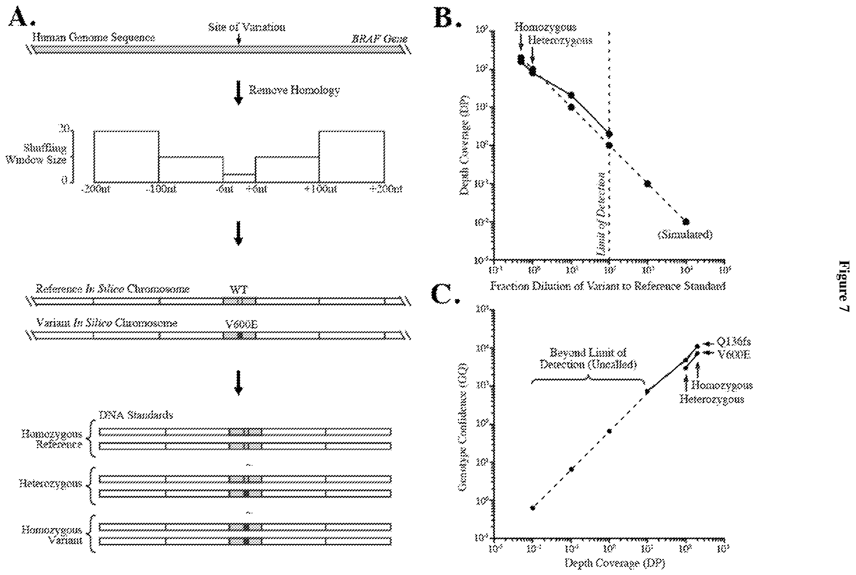

**Figure 7** illustrates the generation of artificial disease associated genetic variation in the artificial chromosomes of the present disclosure. (A) The sequence overlapping the site of the BRAF mutation V600E was retrieved from the human genome. The surrounding sequence was shuffled with increasing window size with increasing distance from the site of BRAF V600E mutation. The 12 nucleotide sequence surrounding the site of the BRAF V600E mutation was not shuffled. The shuffled sequence was assembled within the artificial chromosomes, producing a variant artificial chromosome sequence. DNA standards matching both the wild-type and disease associated *BRAF* V600E mutation were produced and combined to emulate homozygous or heterozygous genotype. (B) Scatter-plot illustrates the relationship between depth of sequence read coverage over variation compared to the relative dilution of the variant DNA standards to the reference DNA standard. (C) Scatter-plot illustrates the confidence associated with the assigned genotype (homozygous and heterozygous genotypes indicated) compared to the relative dilution of the variant DNA standard to the reference DNA standard.

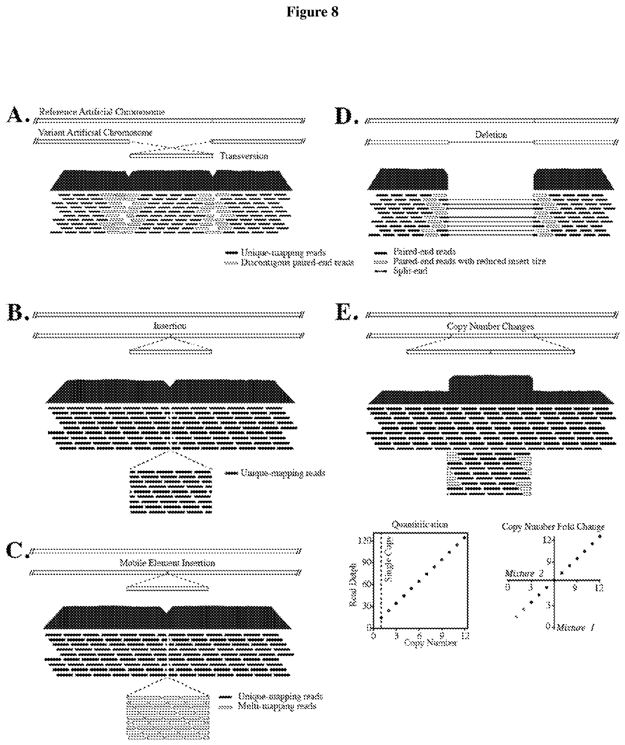

**Figure 8** illustrates the artificial large-scale genetic variation which can be incorporated in the artificial chromosomes of the present disclosure. Illustrated are examples of DNA standards that enable the measurement of different types of large-scale variation including (A) insertions, (B) deletions, (C) inversions, (D) tandem duplications and (E) mobile element insertions where the relative abundance of DNA standards can emulate features such as copy number variation in and between artificial chromosomes.

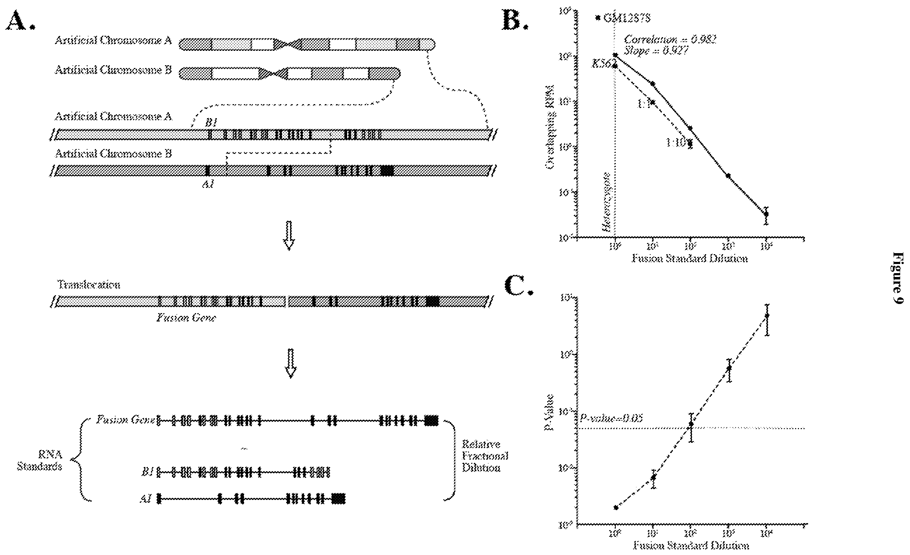

**Figure 9** illustrates a translocation which can be incorporated in the artificial chromosomes of the present disclosure. (A) The sequence between two different artificial chromosomes can be rearranged during a translocation. In the illustrated example, a fusion gene is generated when the translocation breakpoint occurs within two artificial genes (*A1* and *B1*). Three RNA standards can be produced that represent the two normal genes and the fusion gene sequence and combined at different relative concentrations to emulate homozygous and heterozygous genotypes. (B) Scatter-plot illustrates the abundance of the fusion gene RNA standard (measured as reads per million (RPM) overlapping the fusion intron junction) compared to the fractional dilution of the fusion gene RNA standard relative to the two normal gene isoforms RNA standards. This scatter-plot indicates the quantitative accuracy of the accompanying library and limits of sensitivity. Also indicated (dashed line) is the abundance of the endogenous human *BCR-ABL* fusion gene from the accompanying K562 RNA sample. The K562 RNA sample is titrated at increasingly dilutions with GM12878 RNA sample that does not contain the endogenous human *BCR-ABL* fusion gene. (C) Scatter-plot illustrates the significance (P-value) associated with the identification of the fusion junction at increasing dilutions of the fusion gene RNA standard relative to the two normal gene isoforms RNA standards.

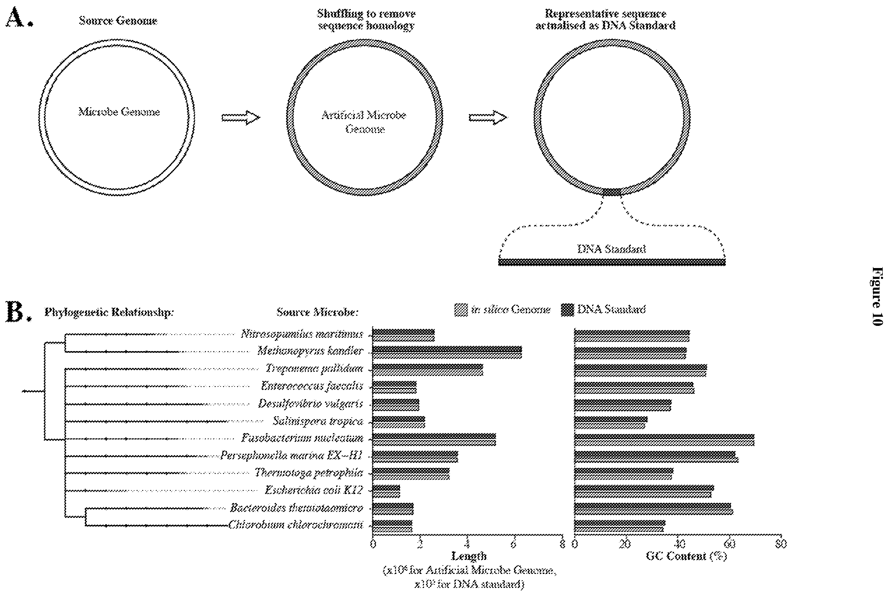

**Figure 10** illustrates an artificial chromosome simulating a microbe community. (A) In the generation of such an artificial chromosome, any one or more of a wide range of microbe genomes ranging in size, GC%, and taxa, are retrieved and shuffled to remove homology to natural sequences. (B) DNA standards can be generated that match reprentative subsequences within the artificial chromosomes. By combining these DNA standards at a range of concentrations, a heterogenous microbe community can be simulated.

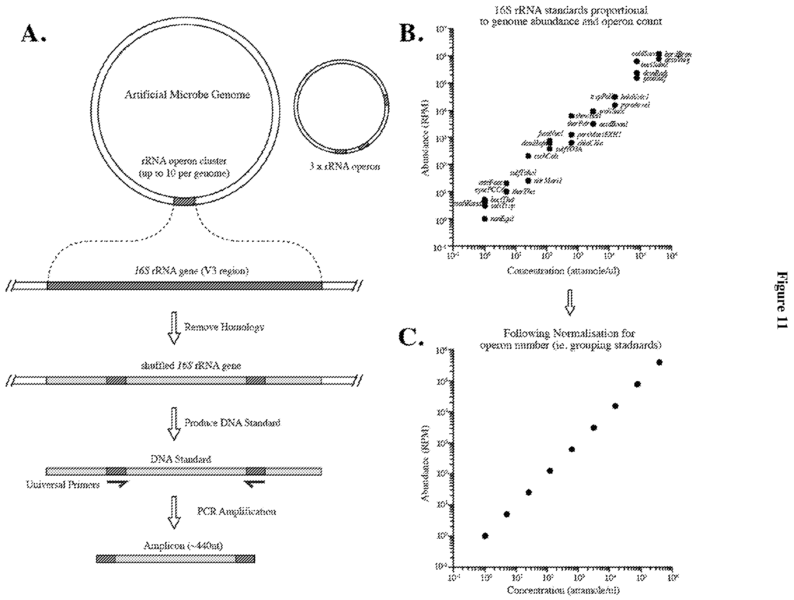

**Figure 11** illustrates one example of a method for generating artificial 16S rRNA markers. The 16S rRNA sequence can be used as a marker for metagneomic phylogenetic analysis. A DNA standard matching the 16s rRNA sequence, including the flanking universal primer sequences, in the artificial microbe genome is produced. This DNA standard can act as a template for PCR amplification and sequencing during metagneomic analysis. (B) Scatterplot illustrates the abundance of simulated reads from sequencing 16S DNA standards corresponding to a wide range of different microbe genomes (indicated). (C) Scatter-plot illustrates the normalsiation of 16S DNA standard abundance according to the rRNA operon count in corresponding microbe genome.

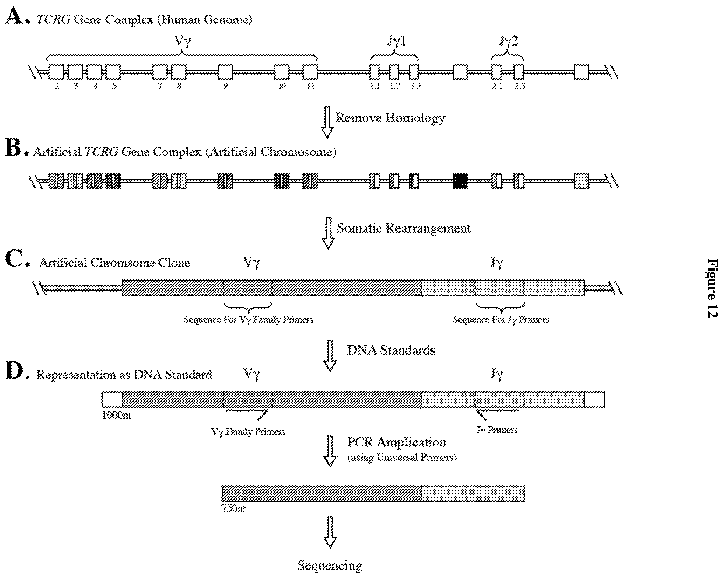

**Figure 12** illustrates one example method for producing an artifical TCRγ loci. (A) The TCRγ loci comprises 14 Vγ segments and 5 Jγ segments. (B) Sequences are shuffled to remove homology to natural sequences. (C) Segments are joined together with a process modeled on the biological processes of VJ recombination and somatic hypermutation to generate numerous artificial TCRγ clonotypes. (D) DNA standards can be produced to represent individual artificial TCRβ clonotypes that maintain sequences complementary to universal primers. DNA standards can be used as a target DNA mocelecule for PCR amplification with universal primers simultaneous to PCR amplification of natural TCRγ loci in accompanying human DNA sample. Each DNA standard thereby amplifies a distinct amplicon whose abundance is proportional to primer binding efficiency and abundance of DNA standard.

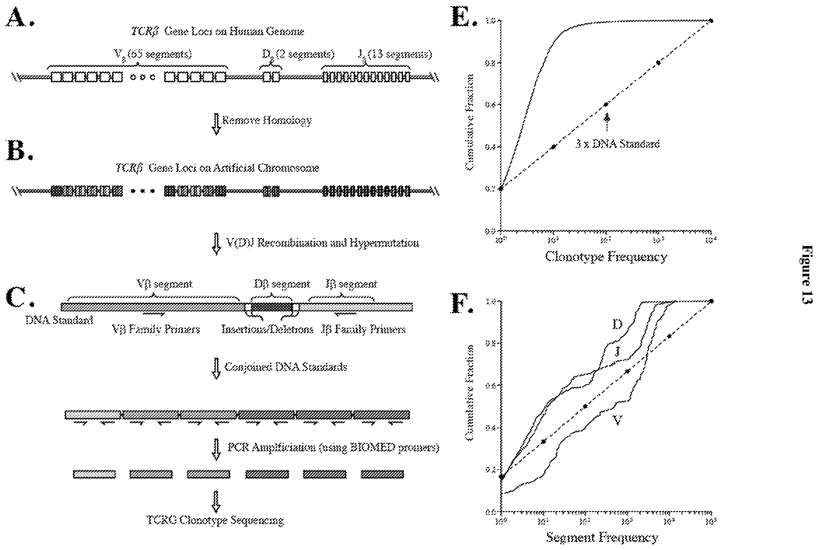

**Figure 13** illustrates one example of artificial *TCRβ* loci. (A) The *TCRβ* loci comprise 65 Vβ segments, 2 Dβ segments and 13 Jβ. (B) Segments are joined together with a process modelled on the biological processes of V(D)J recombination and somatic hypermutation as measured in healthy adult samples to generate numerous artificial *TCRβ* clonotypes. (C) DNA standards can be produced to represent individual artificial *TCRβ* clonotypes. DNA standards can retain the sequences complementary to primers used in PCR amplification of the loci during immune repertoire sequencing. DNA standards can be conjoined to form single continuous template before PCR amplification with universal primers (D) Cumulative frequency distribution of clonotypes identified within healthy adult subjects and, for comparison, the relative abundance of DNA standards measuring artificial clonotypes. The artificial clonotypes provide a quantitative scale that extends across the dynamic range of the natural clonotypes, and can be used to ascribe abundance and determine limit of detection. (E) Cumulative frequency distribution of individual V, J and D segments that are found within a healthy adult subject (shown with black line), and frequency distribution of individual V, J and D segments represented with DNA standards (shown with dashed line).

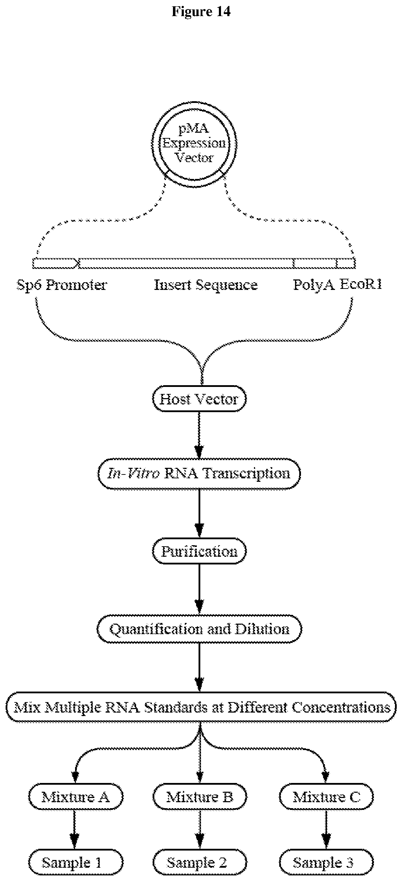

**Figure 14** illustrates an overview of a method by which RNA standards can be produced. The artificial chromosome sequence of interest is synthesized and inserted into an expression vector that is used for *in vitro* transcription to produce an RNA standard. The RNA standard is purified and quantified and diluted to appropriate concentration before being combined with other RNA standards to form a mixture. Different final mixtures can be added to different samples for analysis.

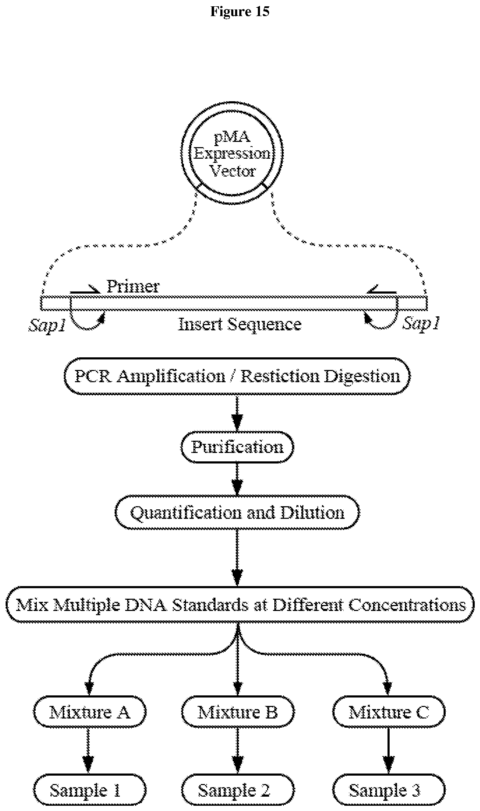

**Figure 15** illustrates an overview of a method by which DNA standards can be produced. The artificial chromosome sequence of interest is synthesized and inserted into a vector that is used as template for either (i) PCR amplification with flanking primers; or (ii) restriction endonuclease digestion at flanking sites. Excised DNA standard is purified and quantified and diluted to appropriate concentration before being combined with other DNA standards to form a mixture. Different final mixtures can be added to different samples for analysis.

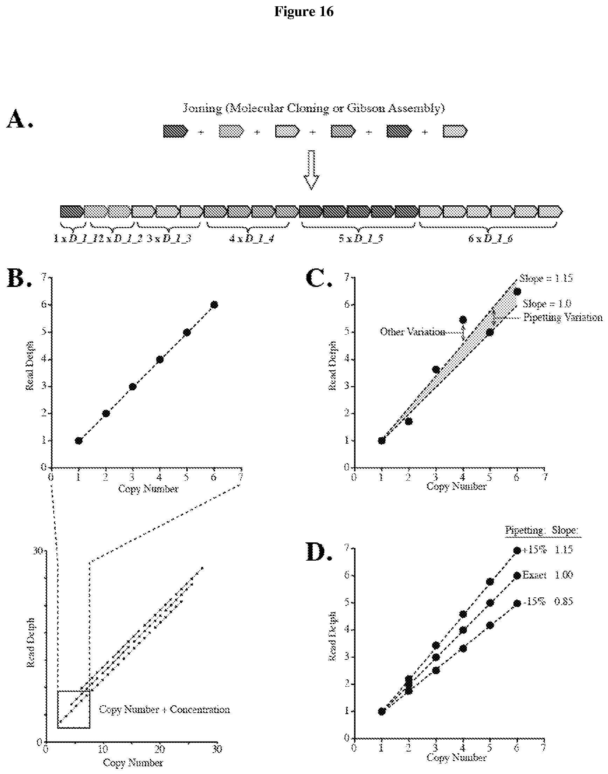

**Figure 16** illustrates one example method for the generation of conjoined DNA standards. (A) Schematic diagram indicates the ligation of multiple individual DNA standards into larger conjoined DNA standards. (B) By combining individual DNA standards at different copy number enables us to emulate differential abundance between individual standards comprising a single conjoined DNA standard. (C) Because the fold change in abundance is dependent between individual standards, we can distinguish variation that results from pipetting from other sources of variation. In this case, plotting the slope of the measured versus known abundance of individual DNA standards within the conjoined standard indicates the magnitude of pipetting error. (D) Normalizing the individual DNA standard abundance according to this slope can normalize and minimize this error.

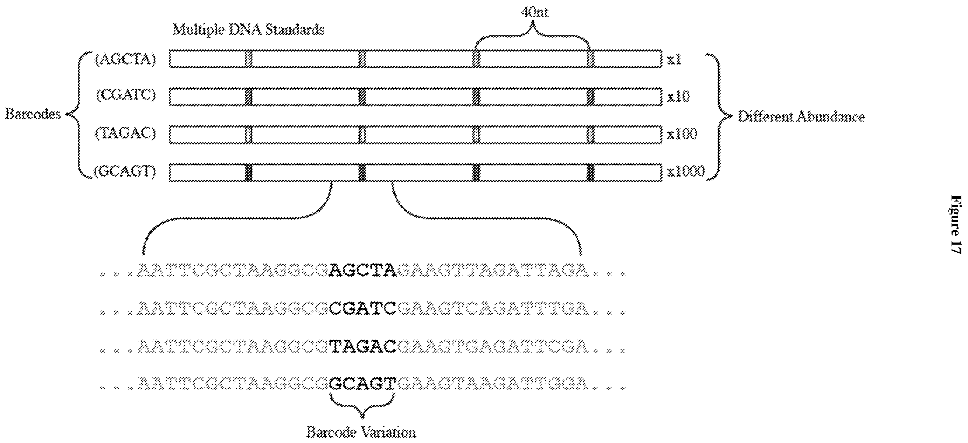

**Figure 17** illustrates one example method for producing of barcode variation. Contiguous or non-contiguous nucleotide sequences can be substituted into the sequence of RNA or DNA standards. Following sequencing, the barcodes can be used to distinguish between multiple identical DNA or RNA standards or derivative sequenced reads.

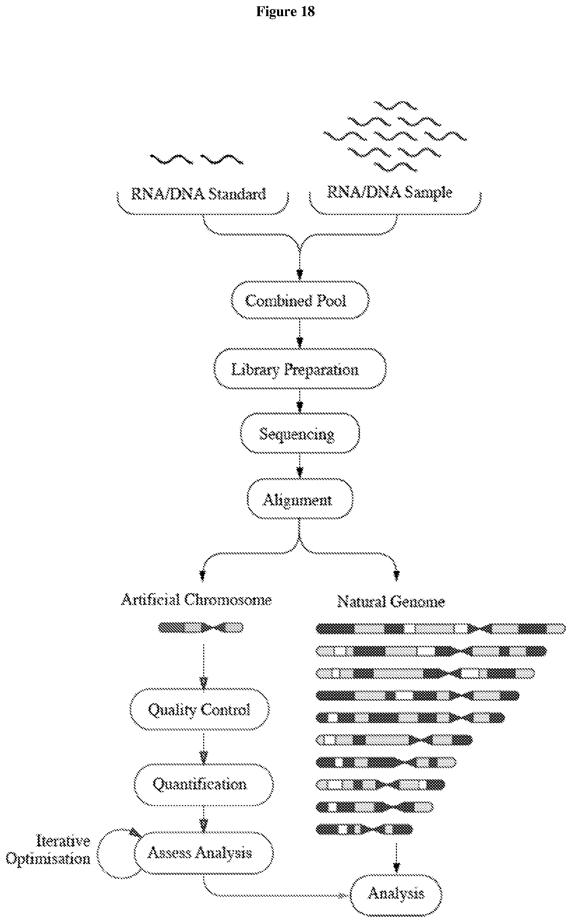

**Figure 18** illustrates a schematic overview of an example of the use of the artificial chromosomes and accompanying RNA/DNA standards during a next generation sequencing experiment. The RNA/DNA standards are added to the RNA/DNA sample of interest prior to library preparation and sequencing. The sequenced reads are simultaneously aligned to the reference genome of interest as well as the artificial chromosome. The alignment and assembly of sequenced reads to the artificial chromosome can be used to calibrate analysis of the accompanying reference genome.

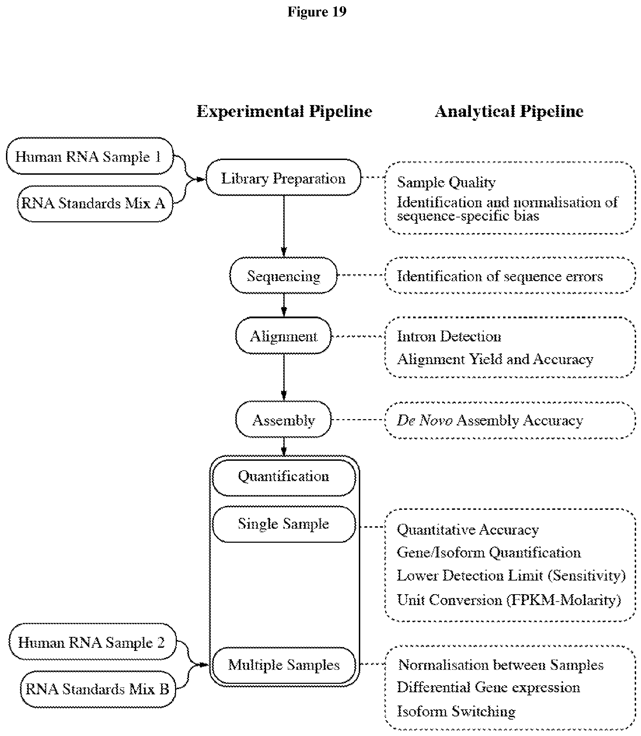

**Figure 19** illustrates a schematic overview of the use of RNA standards within a RNA sequencing experiment. Indicated (dashed boxes) are analytical aspects that can be assessed using DNA standards.

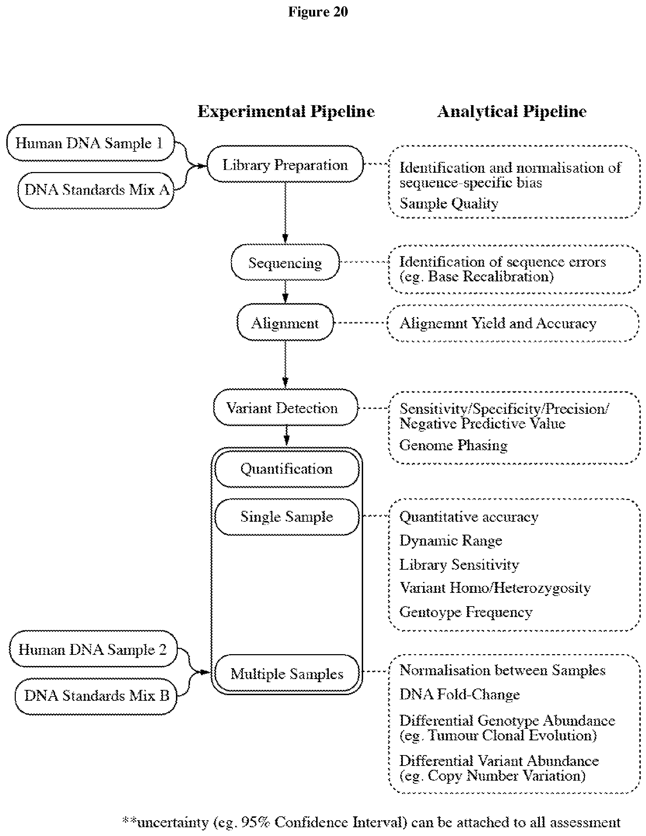

**Figure 20** illustrates a schematic overview of the use of DNA standards within a genome sequencing experiment. Indicated (dashed boxes) are analytical aspects that can be assessed using DNA standards.

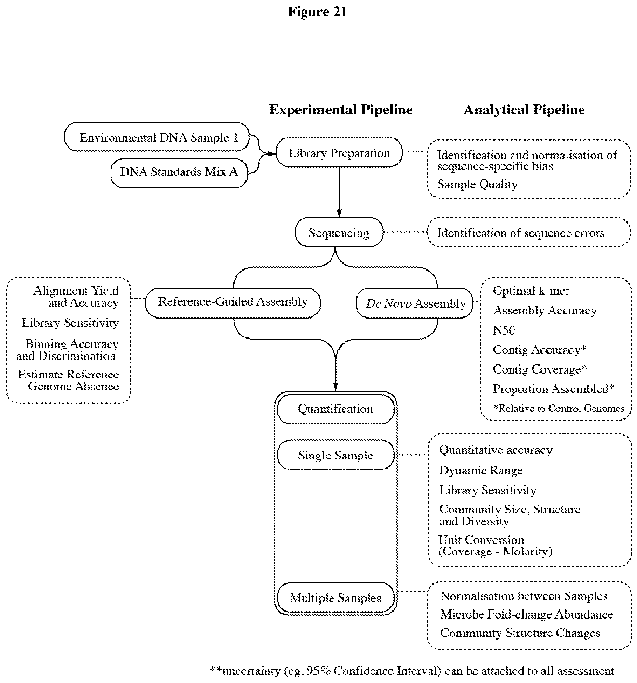

**Figure 21** illustrates a schematic overview of the use of DNA standards within a metagenomic sequencing experiment. Indicated (dashed boxes) are analytical aspects that can be assessed using DNA standards.

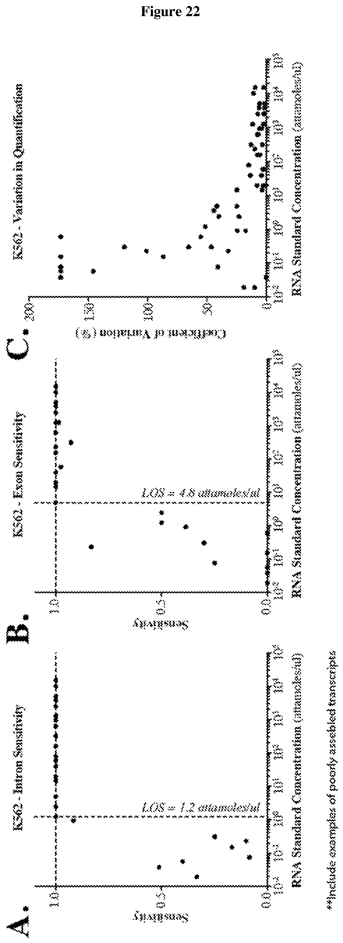

**Figure 22** illustrates one example of an RNA sequencing analysis using RNA standards and K562 total cell RNA. Scatterplot indicates the sensitivity of (A) intron and (B) exon discovery relative to abundance of RNA standard. This indicates limit of detection below which transcripts have insufficient coverage to enable robust assembly. (C) Scatterplot indicates the confidence associated with the observed quantitative measurement of the RNA standard relative to known abundance of the RNA standard.

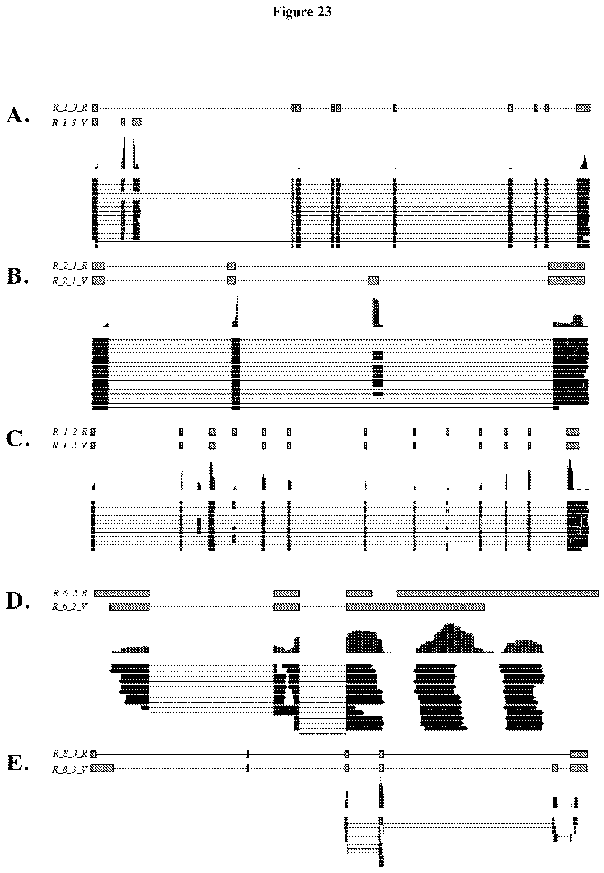

**Figure 23** illustrates the alignment of reads from the RNA sequencing analysis using RNA standards and K562 total cell RNA. (A-E) Five examples of gene loci comprising multiple isoforms encoded on the artificial chromosome are illustrated. Reads produced from sequencing from RNA standards are aligned to the artificial chromosome. Continuous alignments are shown as black bars and regions where alignment is split are shown as thin lines. Overlapping reads alignments are then used to assemble the full-length gene loci structure, including introns and exons and alternative splicing events. Histogram indicates sequence coverage from cumulative read alignments.

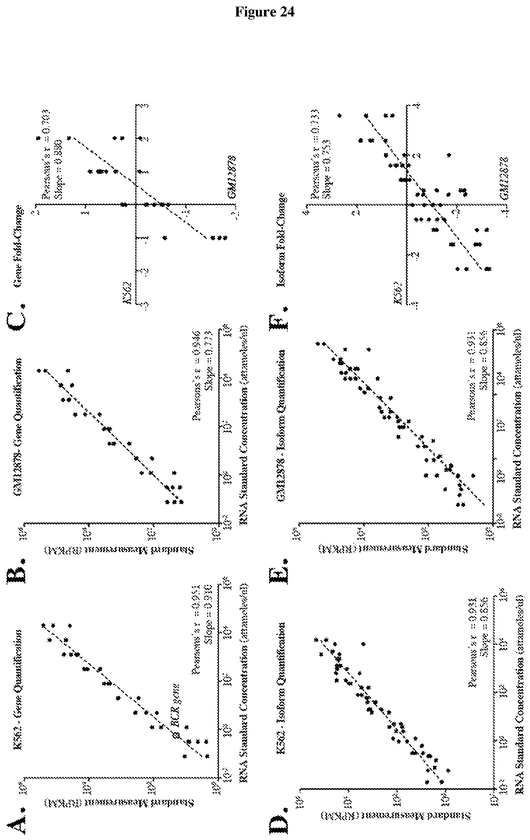

**Figure 24** illustrates the quantitative analysis from the RNA sequencing analysis of RNA standard with human cell RNA samples. (A, B) Scatter-plots indicate the observed abundance (measured in RPKM) relative to the known abundance of RNA standards representing genes when combined as (A) Mixture A with K562 human cell RNA sample or (B) Mixture B with GM12878 human cell RNA sample. The linear correlation and slope indicates quantitative accuracy of each RNA sequencing library. (C) Scatter-plot illustrating the observed fold-change in gene RNA standard abundance relative to the expected fold-change in abundance between Mixtures A (added to K562 RNA) and Mixture B (added to GM12878 RNA). (D, E) Scatterplot indicates the observed abundance of individual isoforms represented by each RNA standard when combined as (D) Mixture A added to K562 RNA sample or (E) Mixture B added to GM12878 RNA sample. (F) Scatter-plot illustrating the observed fold-change in isoform RNA standard abundance relative to the expected fold-change in abundance between Mixtures A and Mixture B. Fold change between individual isoforms emulates alternative splicing.

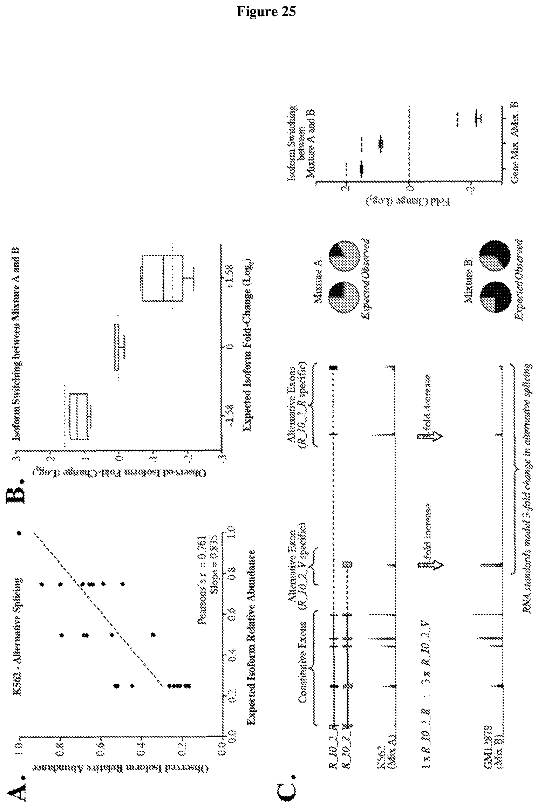

**Figure 25** illustrates one example use of spliced RNA standards. (A) Scatter-plot indicates the observed relative abundance of variant and reference isoform for each gene represented by RNA standards. (B) Box-whisker plot (min-max) indicates the observed fold change between isoforms in Mixture A (added to K562 RNA sample) and Mixture B (added to GM128787 RNA sample) relative to the expected isoform fold-change. (B) In this example, a single gene loci on the artificial chromosome encodes two distinct isoforms (*R_10_2_R* and *R_10_2_V*) that share constitutive exons but differ for the 3' alternative exons and termination site. We produced RNA standards representing each isoform at different conventions (3:1 ratio) for Mixture A and inverted (1:3 ratio) for Mixture B. (B) Plot indicates the observed (box-whisker plot showing min to max; n=3) relative to expected (dashed) expression of the *R_10_2* gene and *R_10_2_R* and *R_10_2_V* isoforms in Mixture A and Mixture B.

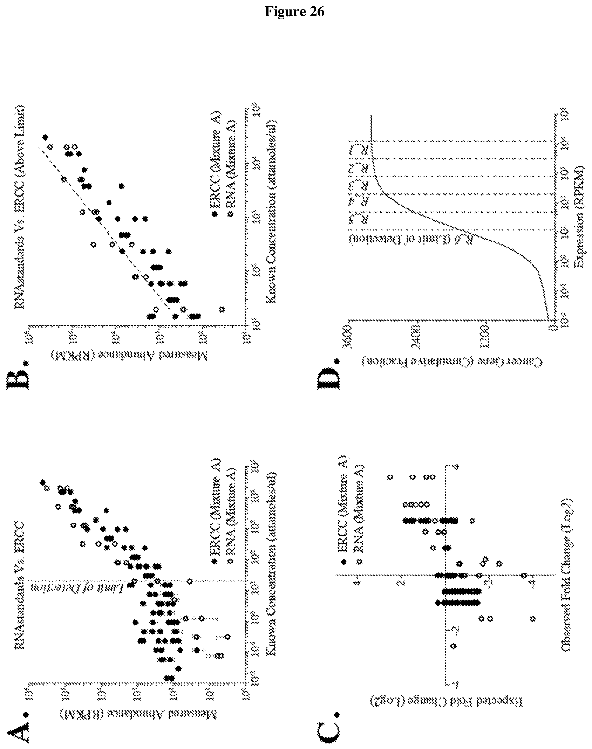

**Figure 26** illustrates a quantitative comparison of RNA standards and ERCC RNA Spike-ins. (A) Scatter-plot indicates comparison of observed abundance (measured in RPKM) to known concentration of ERCC RNA Spike-Ins (black) relative to RNA standards (grey). Based on three replicates with error bars indicating standard deviation. Limit of detection indicates known concentration of RNA Standards below which sampling is infrequent and variable. (B) ERCC RNA Spike-Ins (black) relative to RNA standards (grey) exhibit similar linear profile and correlation above limit of detection. (C) Scatter-plot indicates the observed fold-change relative to expected fold-change for ERCC RNA Spike-Ins (black) and RNA Standard (grey) abundance between Mixture A (added to normal lung RNA sample) and Mixture B (added to matched lung adenocarcinoma RNA sample) (D) Cumulative frequency distribution of cancer genes expression (black line). The measured abundance of added RNA standards is indicated (dashed line) to provide an overlapping quantitative reference ladder against which to measure the concentration of endogenous cancer genes within the accompanying lung adenocarcinoma RNA sample.

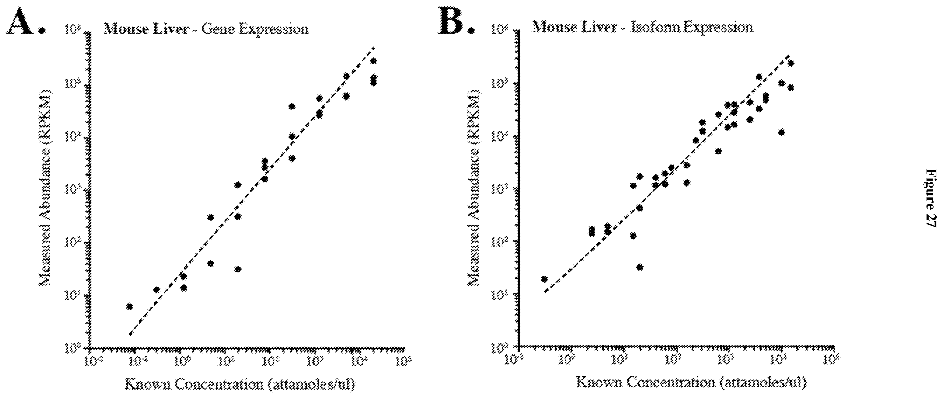

**Figure 27** shows a scatter plot indicating the observed abundance (measured in RPKM) relative to known abundance of RNA standards representing (A) genes or (B) individual isoforms when added to mouse liver RNA sample. Linear correlation and slope indicate quantitative accuracy of RNA sequencing libraries.

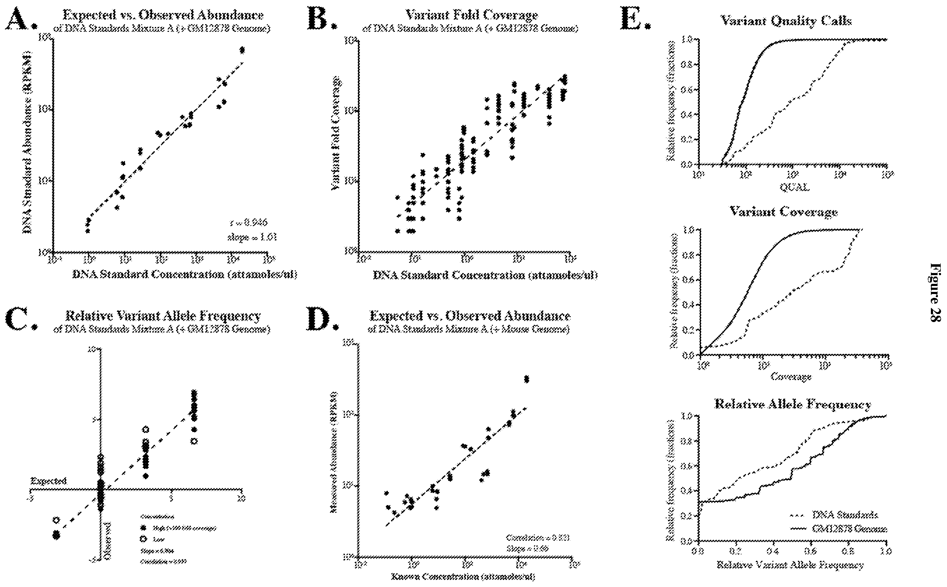

**Figure 28** illustrates an example DNA sequencing analysis using DNA standards and GM21878 genome DNA. (A) Scatter-plot compares the measured abundance (in RPKM) of DNA standards relative to the known abundance of DNA standards. (B) Scatter-plot indicates the alignment fold-coverage of genetic variants represented by DNA standards relative to the known concentration of the DNA standards. (C) Scatter-plot indicates the observed variant allele frequency compared to the known variant allele frequency. Variant allele frequency is indicated relative to the reference allele frequency. The linear correlation and slope indicates that quantitative accuracy with which allele frequency is observed. (D) Scatter-plot compares the measured abundance (in RPKM) of DNA standards relative to the known abundance of DNA standards when used in analysis with moue genome DNA. (E) Cumulative frequency distribution plots illustrate the total distribution of (upper panel) PHRED quality scores, (middle panel) fold coverage or (bottom panel) relative variant allele frequency of DNA standards (dashed line) relative to the accompanying GM12878 genome DNA sample (black line).

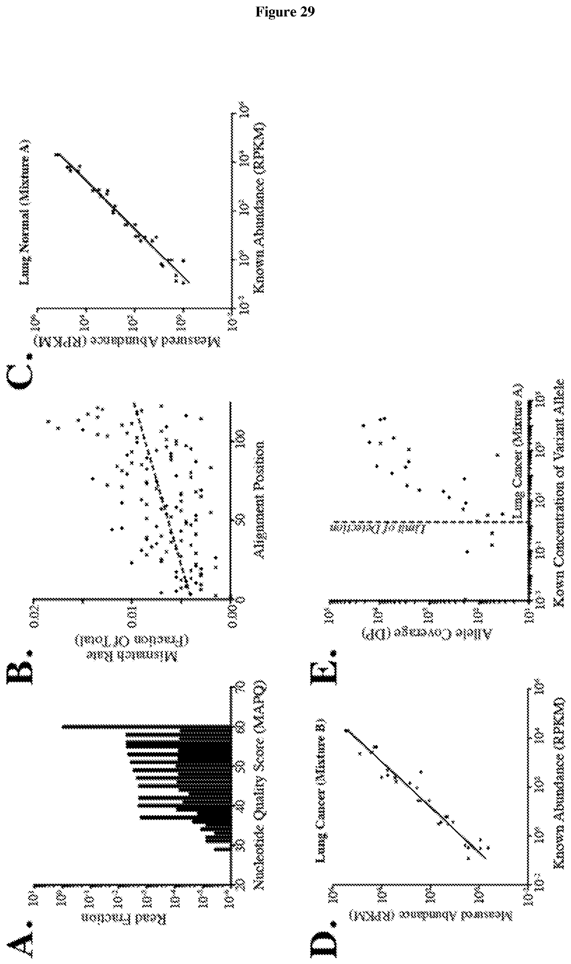

**Figure 29** illustrates an example DNA sequencing analysis using DNA standards and comparing matched lung adenocarcinoma and normal genome DNA. (A) Frequency distribution mapping quality (MAPQ) scores from read alignment to the artificial chromosome. (B) Relative distribution of nucleotide mismatches (between sequence read and artificial chromosome) across length of 125nt sequenced read from DNA standards. (C,D) Scatter-plots indicated the observed abundance relative to known abundance of DNA standards when combined as (C) Mixture A added to matched normal lung genome DNA sample or (D) Mixture B added to matched lung adenocarcinoma genome DNA sample. Linear correlation and slope indicate quantitative accuracy. (E) Scatter-plot indicates the sequencing coverage of genetic variants represented by DNA standards relative to the known concentration of DNA standard. A limit of detection (dashed line) indicates the lower bound concentration whereby genetic variation is not reliably detected.

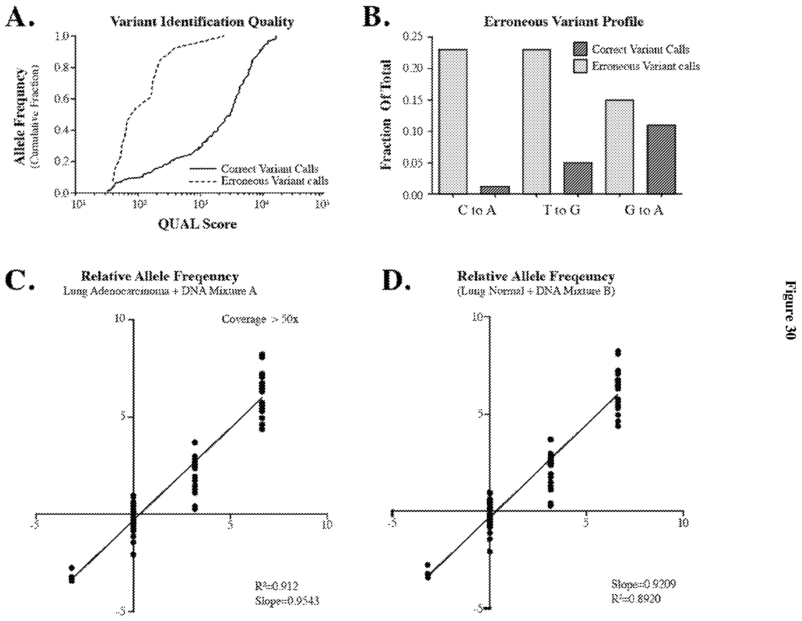

**Figure 30** illustrates an example DNA sequencing analysis to identify genetic variation using DNA standards and comparing matched lung adenocarcinoma and normal genome DNA. (A) Cumulative frequency distribution plot indicates the distribution of quality scores assigned to variants (black line) correctly identified or (dashed line) erroneously identified. The indicated difference in the quality score for correctly and incorrectly identified variation can be used to distinguish correctly and incorrectly identified variation in the accompanying lung adenocarcinoma genome DNA sample. (B) Histogram indicates the enrichment of specific nucleotide substitutions (C to A and T to G) in incorrectly identify variants compared to correctly identified variants. (C,D) Scatter-plots indicates the observed relative variant allele frequency (relative to reference allele frequency) compared to the known relative variant allele frequency of DNA standards combined as (C) Mixture A with lung adenocarcinoma genome DNA sample and (D) Mixture B with matched normal lung tissue genome DNA sample. The linear correlation and slope indicates that quantitative accuracy with which allele frequency is measured. Accurate and sensitive measurement of allele frequency is required to detect mutations that may be harboured by only a small subset of tumour cells within total lung adenocarcinoma sample.

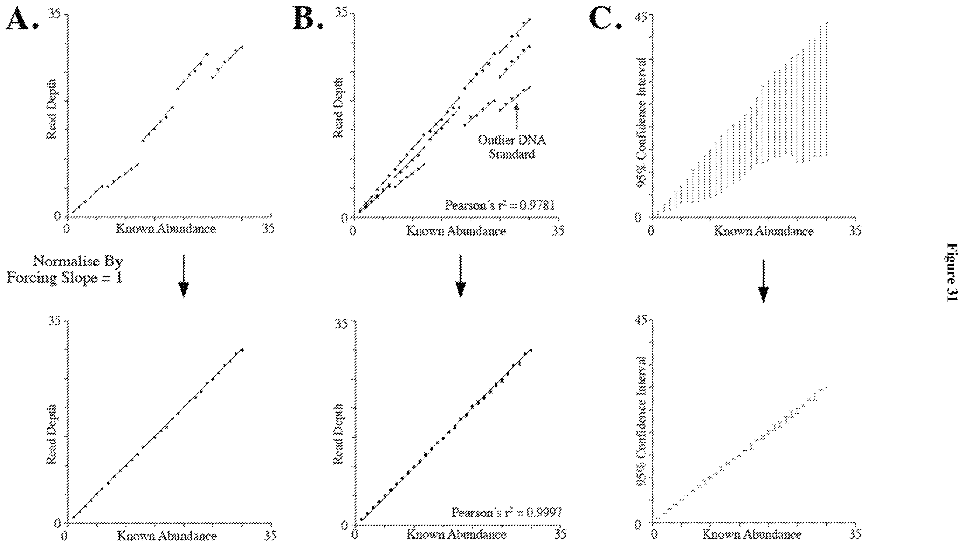

**Figure 31** illustrates an example DNA sequencing analysis using conjoined DNA standards. (A) Scatter-plot comparing the observed abundance of individual DNA standards compared to known abundance of DNA standards shown (upper panel) before normalisation for pipetting errors and (lower panel) following normalization by forcing conjoined DNA standards groups to exhibit a slope of 1. This enables the identification and removal of variation due to pipetting errors. (B) Multiple overlapping conjoined DNA standards are typically manufactured to provide at least three independent measurements at each known abundance point. Conjoined DNA standard group outliers (indicated) due to pipetting errors can be easily identified and removed. (C) Histogram (upper panel) indicates the 95% Confidence Interval determined for each known abundance point from three independent measurements. The 95% Confidence Interval is markedly smaller (lower panel) due to the higher quantitative accuracy following normalisation of DNA standard abundance to remove pipetting error.

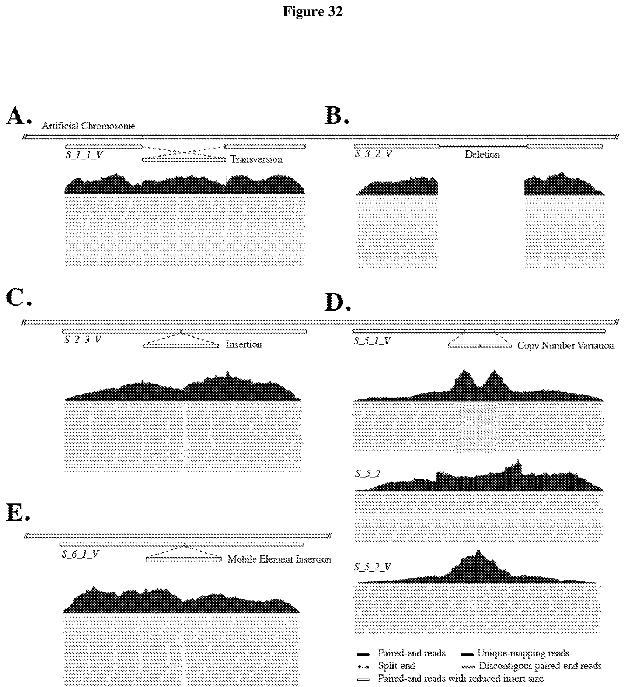

**Figure 32** illustrates examples of DNA standards representing large-scale structural variation. DNA standards were produced that represented (A) Inversions, (B) Deletions, (C) Insertions, (D) Copy-Number Variation and (E) Mobile Element Insertions. DNA Standards were combined with GM12878 human cell genome DNA for library preparation and sequencing. Alignment coverage from each example DNA standards are shown (black histogram) along with examples of individual sequence read alignments (grey bars).

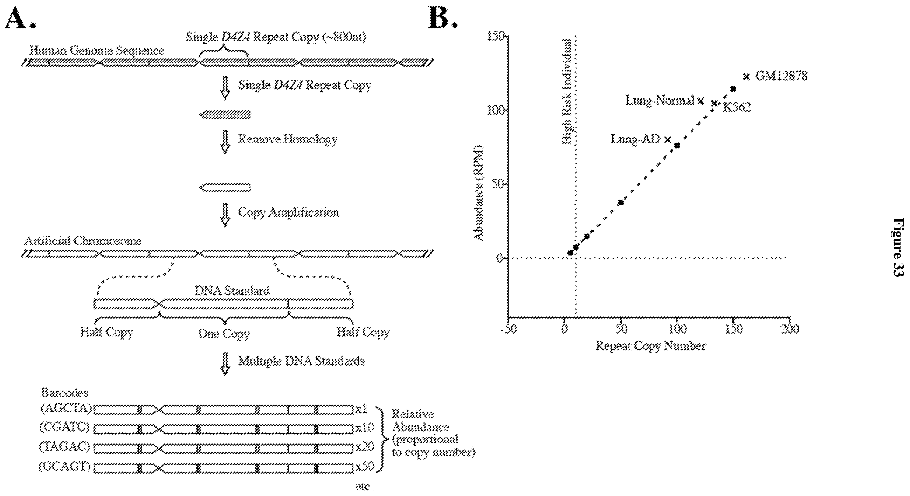

**Figure 33** illustrates one example of a method for producing an artificial *D4Z4* Repeat. (A) A single *D4Z4* Repeat copy (grey, arrow indicates relative direction) is retrieved from the human genome. The homology is removed (white box) and amplified to form head-to-tail repeat array. Multiple DNA standards matching repeat copy and flanking upstream and downstream half repeat copies, but distinguished by barcode variation, are produced. The relative abundance of DNA standards is proportional to the expected repeat copy number. (B) Scatter-plot illustrated the observed abundance of each DNA standard (in reads per million) relative to the expected copy-number. Also indicated are the *D4Z4* repeat unit copy number determined by comparison to DNA standards for the lung normal, adenocarcinoma, K562 and GM12878 genome DNA samples.

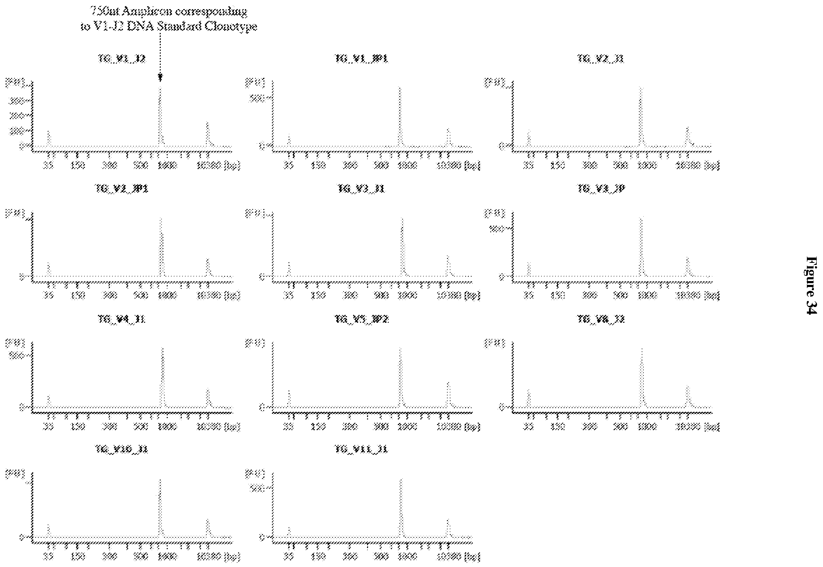

**Figure 34** illustrates BioAnalyser (2100 High Sensitivity DNA Assay; Agilent) traces that confirm the size and purity of 15 amplicons produced by successful PCR amplification of artificial TCRγ clonotypes DNA standards using BIOMED2 universal primers (TCRγ Tube A and B) primers.

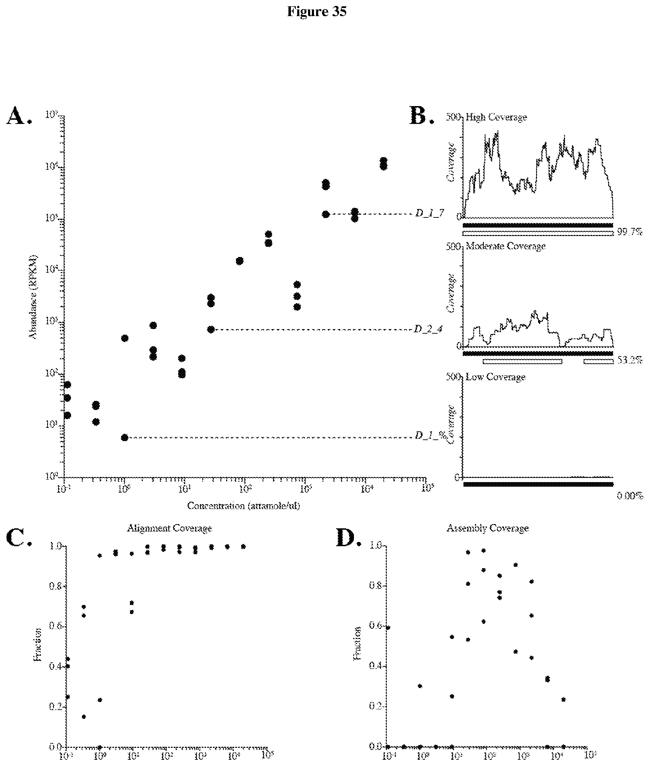

**Figure 35** illustrates an Analysis of Metagenome DNA Standards. (A) Scatterplot illustrates the observed abundance (measured in RPKM) of assembled DNA standard contigs relative to the expected concentration of the DNA standards. (B) Three examples illustrate the impact of DNA standard abundance on contig assembly and coverage. Whilst DNA standards at high concentration (upper panel) exhibit high sequence read coverage and full contig assembly, by contrast, DNA standards at low abundance (bottom panel) exhibit low sequence read coverage and are poorly assembled. (C,D) Scatterplot illustrates the known concentration of DNA standards relative to the fractional coverage of the DNA standard with (C) sequenced reads alignments or (D) *de novo* assembled contigs.

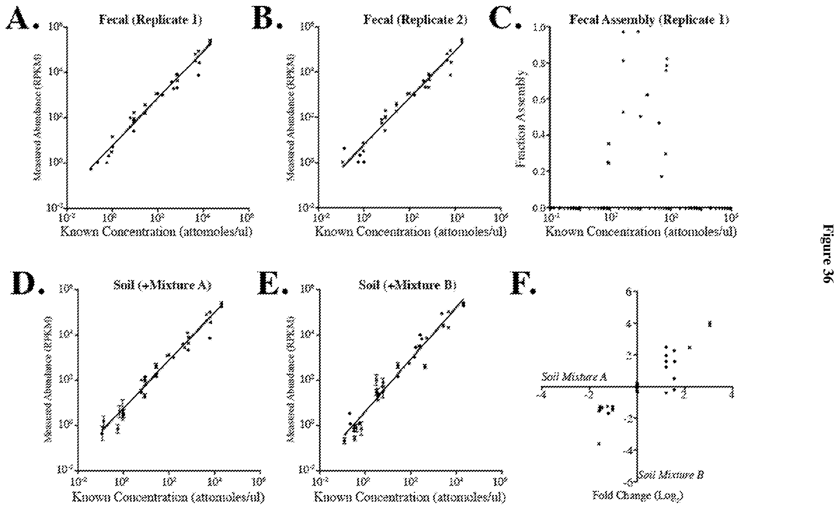

**Figure 36** illustrates an example metagenome analysis of DNA standards used with fecal or soil microbe DNA. (A,B) Scatterplot illustrates the observed abundance (measured in RPKM) compared to the expected abundance of DNA standards used with (A) Fecal Sample Replicate 1 (B) and Fecal Sample Replicate 2. (C) Scatter-plot indicates the fraction of DNA standard that is correctly assembled *de novo* compared to the known abundance of the DNA standard. (D,E) Scatterplot illustrates the observed abundance (measured in RPKM) compared to the expected abundance of DNA standards used with soil samples from Watsons Creek (D) Replicate 1-3 (Mixture A) and (E) Replicate 4-6 (Mixture B). (F) Scatterplot indicating the observed fold-change compared to expected fold-changes in abundance of DNA Standards between Mixture A (Soil Sample Replicates 1-3) and Mixture B (Soil Sample Replicates 4-6). Linear correlation and slope indicate quantitative accuracy with which DNA abundance fold-change is measured between samples.

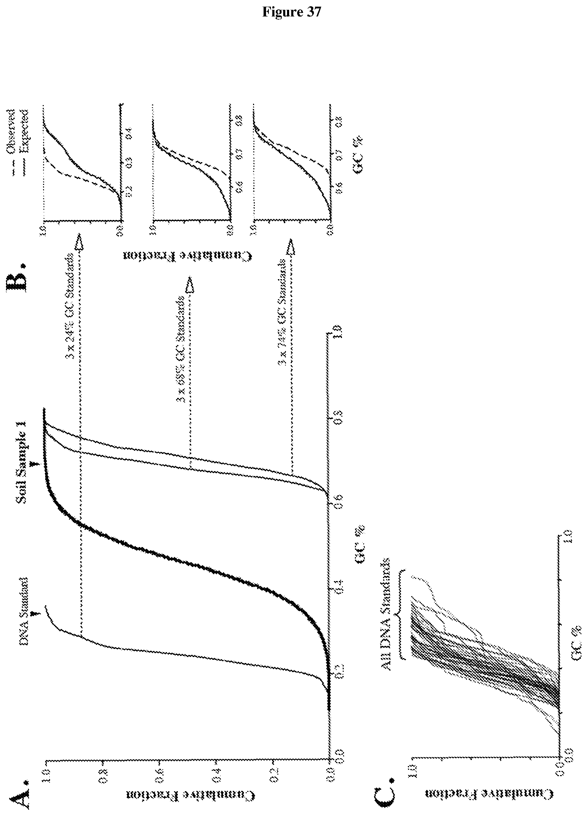

**Figure 37** illustrates one example method of producing DNA standards produced to measure GC bias. (A) Cumulative frequency distribution plot for GC content of sequenced reads from GC metagenome DNA standards (thin black line) and in accompanying Soil sample (Replicate 1; heavy black line). (B) Cumulative frequency distribution of experimentally-derived sequenced reads from selected DNA standards with extreme GC content (black line) compared to cumulative distribution of simulated reads (dashed line) from DNA standards. We observe an under-representation of experimentally-derived sequenced reads with extreme GC content relative to simulation. This indicates the quantitative impact of GC content on library preparation and sequencing procedures. (C) Cumulative frequency distribution of GC content of DNA standards added during sequencing of Soil Sample 1.

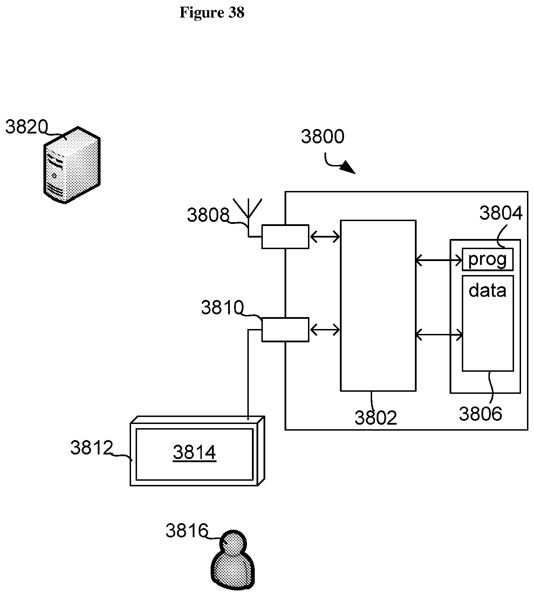

**Figure 38** illustrates a suitable computer system 3800 for calibrating a polynucleotide sequencing process. The computer system 3800 comprises a processor 3802 connected to a program memory 3804, a data memory 3806, a communication port 3808 and a user port 3810.

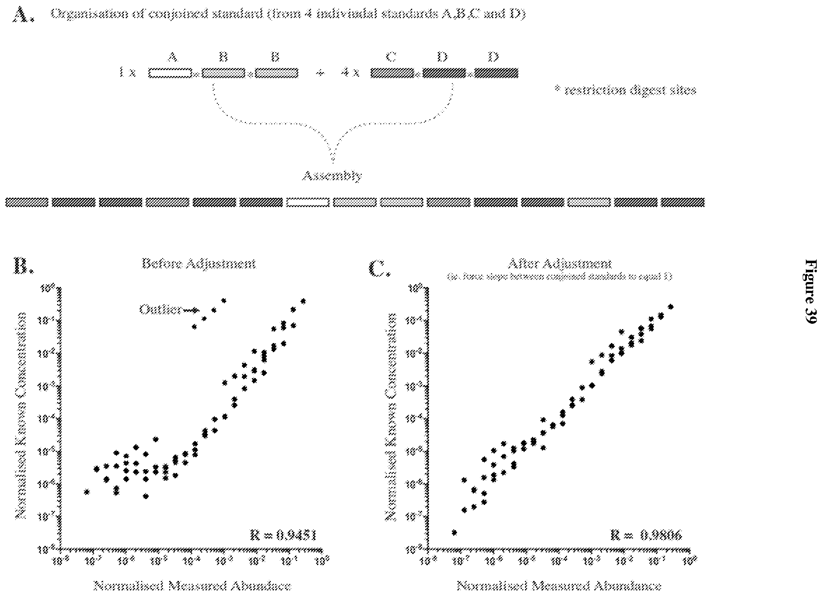

**Figure 39** illustrates one example method of producing conjoined synthetic standards to adjust for pipetting error in NGS methods. (A) Schematic illustrating possible construction of conjoined standards. (B) Illustrates a plot of the weighted normalized known concentration of each individual standard (derived from both the concentration of the hosting conjoined standard and the copy number within the conjoined standard) compared to the weighted-normalized measured abundance. (C) Illustrates the adjustment made after calibrating for known individual standard concentrations.

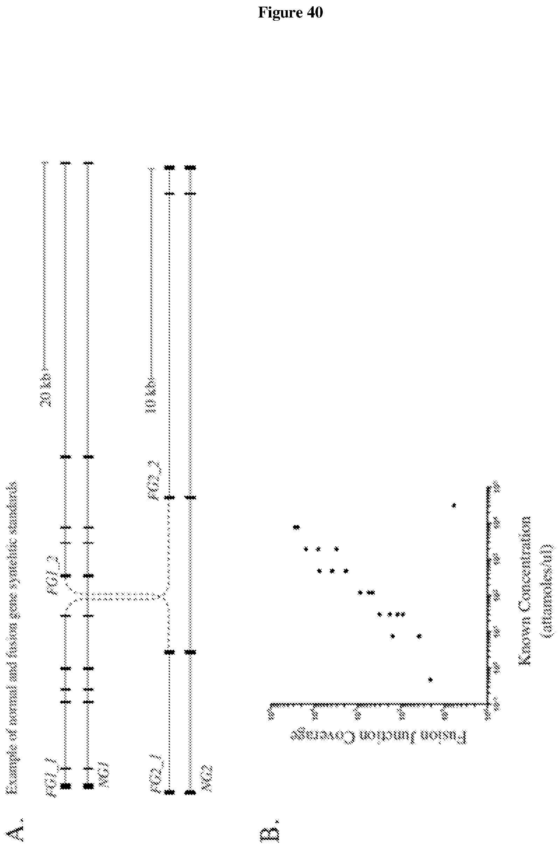

**Figure 40** (A) Illustrates the generation of normal gene and fusion gene synthetic standards. (B) Illustrates a plot of synthetic fusion gene coverage at a location across the fusion junction relative to the known concentration of the synthetic fusion genes within an experimental mixture.

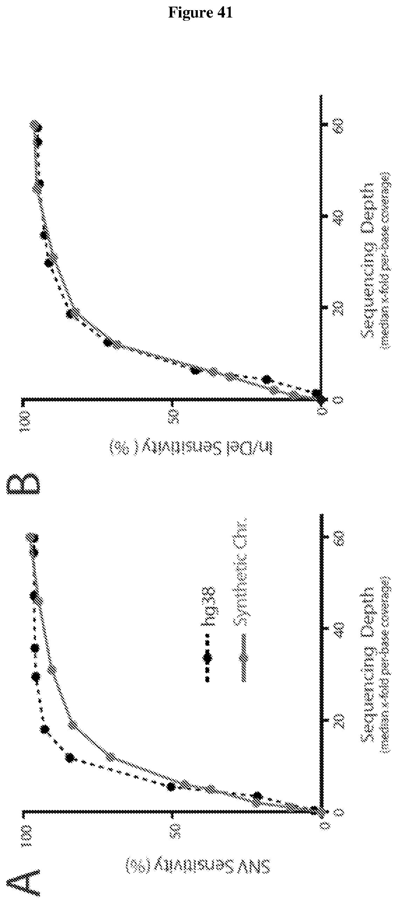

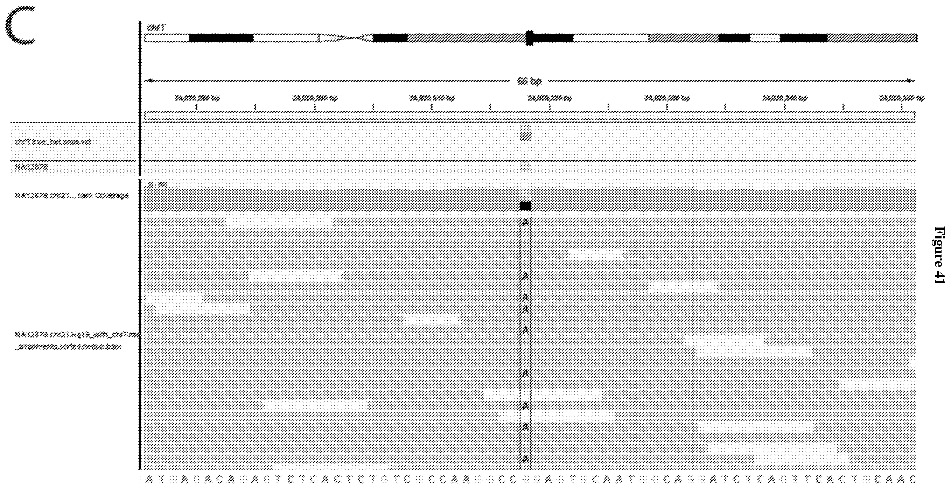

**Figure 41** (A) is a cumulative distribution plot indicating the sensitivity with which single nucleotide variants in both the NA12878 genome (dashed line) and synthetic chromosome (grey line) are identified. (B) A cumulative distribution plot indicating the sensitivity with which small insertions or deletions (indels) in both the NA12878 genome (dashed line) and synthetic chromosome (grey line) are identified. (C) A screenshot from Integrated Genome Viewer (IGV) showing a heterozygous variant in read alignments to the synthetic chromosome.

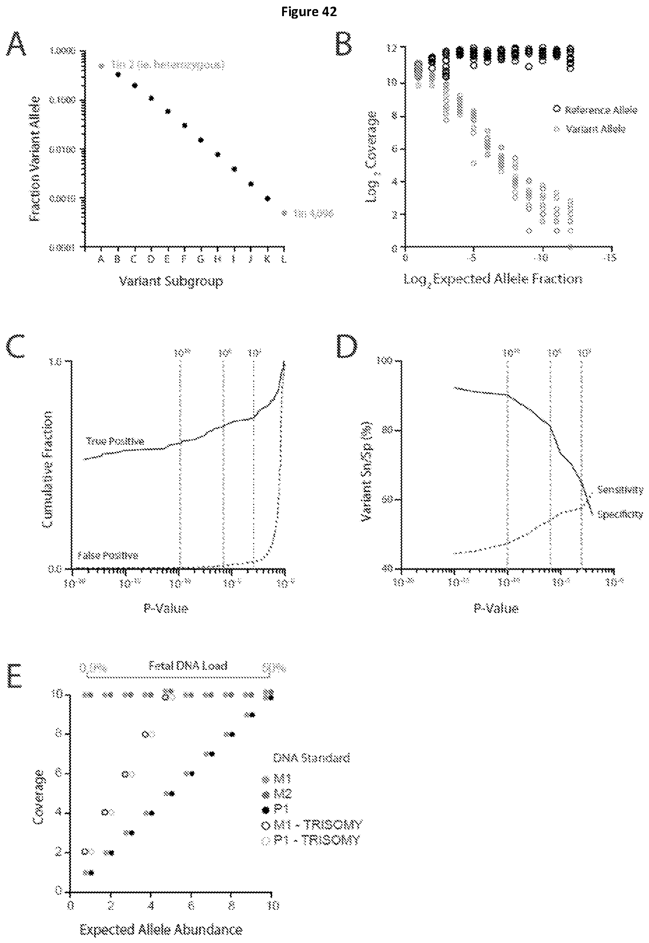

**Figure 42** (A) is a schematic plot indicating the range of variant allele frequencies present within the mixture. (B) A scatter-plot showing the expected variant allele fraction relative to the observed sequence coverage for both reference (black circle) and variant (grey circle outline). (C) A cumulative distribution of both true and false variant alleles identified according to the p-value threshold ascribed by VarScan2 (calculated by Fishers exact test of reference to variant allele coverage). (D) Illustrates the ratio of sensitivity and specificity with which variant alleles are detected relative to the p-value thresholds ascribed by VarScan2. (E) A schematic plot indicating the expected allele abundance of fetal and maternal variants across a range of fetal DNA loads. Also indicated (circle outline) is expected abundance for variants that represent trisomy events.

**Detailed Description**

General

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

As used herein, the singular forms of "a", "and" and "the" include plural forms of these words, unless the context clearly dictates otherwise.

The term "and/or", e.g., "X and/or Y" shall be understood to mean either "X and Y" or "X or Y" and shall be taken to provide explicit support for both meanings or for either meaning.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The term "about" as used herein refers to a range of +/-10% of the specified value.

Artificial chromosome:

The artificial chromosome disclosed herein may be produced as a physical polynucleotide sequence or may be produced and stored in a computer (*in silico*). For many of the applications described herein, it is sufficient for the artificial chromosome to remain *in silico.* However, physical polynucleotide sequences of the artificial chromosome can be produced using standard, well-known methods of polynucleotide generation.

The artificial chromosome disclosed herein may comprise a DNA or RNA polynucleotide sequence. Thus, any reference herein to a polynucleotide sequence is to be understood as a reference to a DNA sequence or to an RNA sequence. An artificial polynucleotide sequence used in methods of the claims is a DNA sequence.

The precise length of the artificial chromosome can vary in accordance with the particular use for which the artificial chromosome is designed. For example, the length of the artificial chromosome can range from about $10^3$ to $10^9$ nucleotides long. In one example, the artificial chromosome comprises or consists of a polynucleotide sequence which is at least 1,800 nucleotides in length. In another example, the artificial chromosome comprises or consists of a polynucleotide sequence which is less than 20 megabases (Mb; wherein 1 Mb is equal to 1,000,000 nucleotides) long. Thus, the artificial chromosome may, for example, be from 1,800 nucleotides long to 20Mb long.

The artificial chromosome comprises an artificial polynucleotide sequence, wherein any fragment of the artificial polynucleotide sequence is distinguishable from any known naturally occurring genomic sequence. One advantage of the artificial polynucleotide sequence is that such a fragment can be added directly to samples containing a natural polynucleotide target of interest, whilst still being distinguishable from any natural polynucleotides present in the sample. It will be appreciated that the artificial chromosome may comprise additional sequences which share some homology (or sequence identity) with known, natural genomic sequences. Any such additional sequences are not comprised within the artificial polynucleotide sequence of the artificial chromosome.

The artificial polynucleotide sequence can form any proportion of the artificial chromosome. Thus, the artificial polynucleotide sequence can comprise from 1% to 100% of the artificial chromosome. For example, the artificial polynucleotide sequence can comprise about 10%, 20%, 30%, 40%, 50%, 60%, 70%, 80%, 90% or 95% of the artificial chromosome. In one example, the artificial polynucleotide sequence forms the majority of the artificial chromosome. Thus, the artificial polynucleotide sequence may form 50% or more, 60% or more, 70% or more, 80% or more, 90% or more, 95% or more, 99% or more of the artificial chromosome. In another particular example, the artificial polynucleotide sequence forms 100% of the artificial chromosome.

The length of the artificial polynucleotide sequence can vary. The length of the artificial polynucleotide sequence may be the entire length of the artificial chromosome. Accordingly, the length of the artificial polynucleotide sequence can range from about $10^3$ to $10^9$ nucleotides long. In one example, the artificial polynucleotide sequence is at least 1,800 nucleotides in length. In another example, the artificial polynucleotide sequence is less than 20 Mb long. Thus, the artificial polynucleotide sequence may be, for example, from 1,800 nucleotides long to 20Mb long. In another example, the length of the artificial polynucleotide sequence may be the same as the length of the fragment disclosed herein. For example, the length of the artificial polynucleotide sequence may be, for example, from 100 nucleotides to 10,000,000 nucleotides in length.

The artificial polynucleotide sequence of the artificial chromosome has little or no homology with any known, naturally occurring sequence (i.e., with any polynucleotide sequence isolated from any living organism). Accordingly, the chromosome disclosed herein is described as an "artificial" chromosome. The extent of homology may be determined by a comparison of the artificial chromosome's artificial polynucleotide sequence with any known, naturally occurring polynucleotide sequence, using any suitable sequence comparison method known in the art. Little or no shared sequence identity between the artificial chromosome's artificial polynucleotide sequence and any known, naturally occurring polynucleotide sequence indicates that the artificial polynucleotide sequence has little or no homology to any known, naturally occurring sequence.

The artificial polynucleotide sequence of the artificial chromosome may be entirely artificial and may not have any homology to any known, naturally occurring sequence. Thus, the artificial chromosome sequence may share no sequence identity with any known, naturally occurring nucleotide sequence.

The artificial polynucleotide sequence comprises at least 100 nucleotides, wherein any 100 contiguous nucleotides of the artificial polynucleotide sequence have less than 100% sequence identity with any known naturally occurring genomic sequence of the same length. In other examples, any 50 contiguous nucleotides of the artificial polynucleotide sequence have less than 100% sequence identity with any known naturally occurring genomic sequence of the same length. In any of the artificial polynucleotide sequences disclosed herein, any 50, any 25, any 21 or any 20 contiguous nucleotides of the artificial polynucleotide sequence may have less than 100%, less than 95%, less than 90%, less than 80%, less than 70%, less than 60%, less than 50%, less than 40%, less than 30%, less than 20%, less than 10%, less than 5%, or less than 1% sequence identity with any known naturally occurring genomic sequence of the same length, in any combination or permutation. Thus, for example, any 21 contiguous nucleotides of the artificial polynucleotide sequence may have less than 50%, less than 40%, less than 30%, less than 20%, less than 10%, less than 5%, or less than 1% sequence identity with any known naturally occurring genomic sequence of the same length. In one particular example, any 21 contiguous nucleotides of the artificial polynucleotide sequence has less than 50% sequence identity with any known naturally occurring genomic sequence of the same length.

Small portions (e.g., 8, 9, 10, 11, 12, 13, 14 or 15 contiguous nucleotides) of the artificial chromosome may be homologous with any known, naturally occurring nucleotide sequences of the same length. For example, such small portions of the artificial chromosome may replicate a small portion of a known, naturally occurring nucleotide sequence which comprises a sequence variant of interest. For example, a small portion (e.g., 8, 9, 10, 11, 12, 13, 14 or 15 contiguous nucleotides) of the artificial chromosome may be 100% identical over its length to a known, naturally occurring nucleotide sequence which comprises a sequence variant of interest, such as a mutation in a particular gene. Whilst the majority of the artificial chromosome sequence may share little or no homology with any known, naturally occurring nucleotide sequence (and therefore, may be an artificial polynucleotide sequence), the artificial chromosome may additionally contain one or more such small portions or particular sequences of interest.

When the artificial chromosome comprises or consists of a polynucleotide sequence which shares some sequence identity with a known, naturally occurring nucleotide sequence, the artificial chromosome may not encode a functional mRNA, rRNA, tRNA, lncRNA, snRNA, snoRNA or functional polypeptide or protein.

The artificial polynucleotide sequence of the artificial chromosome disclosed herein can contain one or more general features of naturally occurring polynucleotide sequences (e.g., of naturally occurring chromosomes), despite having no shared primary nucleotide sequence identity with any known, naturally occurring polynucleotide sequence. Thus, the fragment of the artificial chromosome disclosed herein can contain one or more general features of naturally occurring polynucleotide sequences. For example, the artificial polynucleotide sequence can encode genetic features typically observed in eukaryotic and/or prokaryotic chromosomes or genomes including (but not limited to) genes, repeat elements, mobile elements, small-scale genetic variation, large-scale genetic variation, etc. Figure 1 provides an illustration of such exemplary features, any one or more of which may be included in the artificial polynucleotide sequence disclosed herein, in any combination.

Generating an artificial chromosome:

The present disclosure describes a method of making (or "constructing") the artificial chromosome or fragment thereof disclosed herein. In addition, the present describes an artificial chromosome or fragment thereof made (or "constructed") by any one or more of the methods disclosed herein. The artificial chromosome disclosed herein may be constructed by a number of suitable methods, as described herein. According to the claimed invention, an artificial polynucleotide sequence is made by a method comprising retrieving a natural nucleotide sequence, and reversing the natural nucleotide sequence , wherein the reversed sequence has the same nucleotide bases in the same order as the natural nucleotide sequence, wherein the order of the nucleotide bases is in the 5' to 3' direction in the natural nucleotide sequence and in the 3' to 5' direction in the reversed sequence. In embodiments not part of the claims, the artificial chromosome may be constructed by generating a contiguous polynucleotide sequence *in silico* having little or no sequence identity to other known, naturally occurring sequences, by the random addition of nucleotides to form an extended contiguous polynucleotide sequence. Suitable software programs which can be used to generate an artificial chromosome sequence include (for example and without limitation): software to produce random DNA sequences such as FaBox (Villesen 2007) or RANDNA(Piva and Principato 2006); software to shuffle DNA sequences such as uShuffle (Jiang, Anderson et al. 2008) and Shufflet (Coward 1999).

Alternatively, the artificial chromosome may be constructed by retrieving a known or natural nucleotide sequence identified from a natural source (which may be referred to herein as a "template" sequence) and then shuffling (or "rearranging") the nucleotides to remove or reduce the shared sequence identity of the template sequence with any known, naturally occurring polynucleotide sequence. In one example, all nucleotides of the artificial chromosome can be shuffled together to change nucleotide order. **In** one example, contiguous nucleotides within the template nucleotide sequence can be partitioned into windows of discrete nucleotide lengths along the template sequence and only those nucleotides within a single window can be shuffled together. This allows the primary nucleotide sequence within the window to be rearranged so that the shuffled (or "rearranged") sequence shares little or no sequence identity with any known, naturally occurring sequence, whilst retaining broader characteristics of nucleotide composition that are typical of the original known or natural sequence. For example, any nucleotide biasing within a window (such as high guanine or cytosine content) can be retained across the length of the shuffled window by ensuring that the same nucleotides present in the window applied to the template sequence are retained in the shuffled sequence within the same window (as exemplified by the illustration in Figure 2). Thus, the "shuffling" referred to herein reorders the same nucleotides within a fixed length of polynucleotide sequence, and does not involve an alteration of the numbers of each particular nucleotide present within that fixed length of polynucleotide sequence.

Retaining high level nucleotide composition characteristics of a template sequence can be advantageous because sequence-specific features can bias the representation of natural genetic features in next-generation sequencing and analysis. For example, sequences with high or low guanine or cytosine content (GC%) may be poorly amplified by PCR during library preparation, resulting in poor representation within sequencing libraries. Alternatively, it can be difficult to unambiguously align sequences with a repetitive sequence structure, resulting in poor representation during analysis. Since the artificial chromosome and standards disclosed herein can be designed to emulate natural genetic features, the synthetic primary sequence of the artificial chromosome or standards can be made to reflect the same sequence-specific bias as the template sequence. Thus, the artificial chromosome or standards disclosed herein can have an artificial primary sequence, whilst maintaining the nucleotide composition and/or repeat structure as the original template sequence.

The window size selected to perform any shuffling can correspond to a fixed polynucleotide length (e.g., 10, 15, 20, 30, 40, 50, 60, 70, 80, 90, 100, 150, 200, 250, 300, 400, 500, 600, 700, 800, 900, 1000 or more nucleotides). Alternatively, the window size selected can correspond to the boundaries of a higher-level genetic feature (e.g., introns, exons, CpG islands, and others) present in the template sequence. For example, the primary intron and exon sequences of a gene can be shuffled whilst still maintaining the organisation of exon and intron features. Thus, the structure and organisation of higher-level genetic features can be retained, despite the primary sequence of the artificial polynucleotide sequence within the artificial chromsome not matching known or natural sequences.

The artificial polynucleotide sequence is constructed by retrieving a known or natural nucleotide sequence identified from a natural source (a "template" sequence) and then reversing the template sequence. Naturally occurring nucleotide sequences (DNA or RNA sequences) have an intrinsic 5' to 3' directionality imposed by the phosphodiester bonds between the nucleotide bases. Reversing the sequence to the 3' to 5' direction violates this directionality and generates a sequence that no longer has homology (or sequence identity) to the original template sequence. One advantage of this method of making the artificial chromosome is that the nucleotide composition and repetitiveness of the original sequence is retained, even though sequence identity to the template sequence is removed. The reversed sequence is therefore "artificial" and can be distinguished from the original endogenous sequence (that has the correct directionality).

In embodiments, making the artificial polynucleotide sequence further comprises substituting nucleotides for alternative nucleotides within the sequence. For example, guanine nucleotides can be substituted for cytosine nucleotides, cytosine nucleotides can be substituted for guanine nucleotides, adenine nucleotides can be substituted for thymine nucleotides, and/or thymine nucleotides can be substituted for adenine nucleotides. By substituting nucleotides in a systematic manner, the repeat structure of the sequence can be maintained, the pyrimidine and purine composition can be maintained, and/or the GC content can be maintained, even though the individual nucleotides and the primary sequence may change.

It will be appreciated that the shuffling, substituting and reversing techniques can each be applied in any combination or permutation during construction of an artificial chromosome and/or fragment thereof. Thus, for example, a template sequence can be reversed and selected windows of the reversed sequence can then be shuffled in order to reduce or remove any residual homology in the reversed sequence to known natural sequences.

To confirm whether homology to known natural sequences exists within the artificial chromosome nucleotide sequence, known nucleotide sequence databases (such as the NCBI Nucleotide collection (nr/nt) database) can be queried with software programs such as the BLASTn software program (Altschul, S.F., *et al.,* 1990). Other suitable software programs facilitating the alignment and comparison of multiple nucleotide sequences can also be used, for example FASTA (Pearson and Lipman 1988) or ENA Sequence Search (http://www.ebi.ac.uk/ena/search/). For complex sequences, homology typically corresponds to 21 or more contiguous nucleotide sequences matching a known sequence (e.g., having 100% sequence identity over the 21 or more nucleotide sequence length). For simple sequences (such as repetitive or mono-nucleotide compositions), homology corresponds to an expected (E) value less than or equal to 0.01 (as defined in NCB1 BLAST (Altschul, S.F., *et al.,* 1990)). Thus, any 21 or more contiguous nucleotides of the artificial polynucleotide sequence disclosed herein may have an E value less than or equal to 0.01 (as defined in NCB1 BLAST (Altschul, S.F., *et al.,* 1990)).

If the shuffling, substituting and/or reversing techniques do not remove or sufficiently reduce the shared sequence identity with other, known, naturally occurring sequences to the extent desired, individual nucleotide substitutions can be made to achieve the desired level of reduced sequence similarity. Thus, the shuffled, substituted or reversed sequence can be further edited (or "curated") by the specific insertion, deletion or substitution of nucleotides to remove any remaining shared sequence identity. Accordingly, the methods of generating the artificial chromosome disclosed herein may further comprise editing shuffled, substituted or reversed nucleotide sequences to reduce or remove any shared sequence identity with any known, naturally occurring sequence.

Any natural genome or chromosome sequence can be shuffled, substituted or reversed to remove homology, whilst retaining characteristic features of the nucleotide composition of the natural genome or chromosome sequence. Suitable natural nucleotide sequences can be identified from any one or more publically available nucleotide online databases. Examples of suitable nucleotide online databases include GenBank and Nucleotide collection (nr/nt) database (National Center for Biotechnology Information), DNA Data Bank of Japan (National Institute of Genetics) and EMBL-BANK (European Bioinformatics Institute). Alternatively, suitable natural nucleotide sequences may be obtained by isolating polynucleotides from a natural source and sequencing those polynucleotides using known sequencing techniques. In one example, the natural genome or chromosome sequence is a mammalian genome or chromosome sequence, such as a human or murine genome or chromosome sequence. For example, the natural nucleotide sequence may be selected from a reference human genome sequence (e.g., the latest annotated version hg19). Alternatively, the natural nucleotide sequence may be selected from any mammalian sequence (e.g., *M.musculus* mm10), any vertebrate genome (e.g., *D.rerio* danRer7), any animal sequence (e.g., *C.elegans* ce10, D.melanogastor dm3, and others), any plant sequence (e.g., *A.thalianis* tair9), any fungi sequence (e.g., *N. crassa*) or any eukaryote sequence (e.g., *S.cerevisae* SacCer6), or any bacterial sequence (e.g., *E.coli* eschColiK12), or any archaea sequence (*e.g., M.kandleri* methKand1), or any viruses, phages and organelle sequence (eg. Hepatitis delta virus).

The artificial polynucleotide sequence within the artificial chromosome disclosed herein may be distinguishable from any known naturally occurring genomic sequence derived from a single species, or from any known naturally occurring genomic sequence derived from multiple species. For example, the artificial polynucleotide sequence within the artificial chromosome disclosed herein may be distinguishable from any known naturally occurring human genomic sequence. In another example, the artificial polynucleotide sequence within the artificial chromosome disclosed herein may be distinguishable from all known naturally occurring genomic sequences of any organism.

In another illustrative example, the *Anaeromyxobacter dehalogens* genome, which has a high GC content (75%), can be used as a template sequence. Shuffling the *A.dehalogens* genome sequence can produce an artificial chromosome comprising a polynucleotide sequence with no homology (or no shared sequence identity) to the original *A.dehalogens* genome (or any other natural or known sequence), yet which retains the high GC content that is a feature of the *A.dehalogens* genome.

The processes described herein can be used to generate multiple contiguous nucleotide sequences without homology (or shared sequence identity) to any known or natural sequence. These multiple sequences can be rearranged and combined to form a single merged contiguous sequence. Thus, the artificial chromosome disclosed herein can be constructed in a modular fashion, which provides a great deal of flexibility in its design and construction. For example, multiple sequences, possibly encoding different genetic features, can be constructed independently before being collectively assembled into a single complex artificial chromosome. Assembling different sequence combinations also affords the construction of custom-built artificial chromosomes for specific research or diagnostic requirements.

In addition, multiple (i.e., two or more) artificial chromosomes can be generated and used together. Accordingly, the present disclosure also describes a library of two or more artificial chromosomes. The number of chromosomes chosen to populate the library can be chosen depending on the particular intended application of the library. In one example, the library of artificial chromosomes can emulate the organization of entire genomes, including polyploid genomes. For example, a library of artificial chromosomes can be created containing 46 artificial chromosomes, to emulate the organization of the human genome across 46 distinct chromosome sequences. Thus, individual artificial chromosome sequences can be duplicated to form a polyploid artificial genome. Sequence variation can be incorporated between duplicate artificial chromosomes, thereby simulating natural zygosity. In another example, a library of artificial chromosomes may emulate multiple microbe genomes being present as a collection or community of microbes (such as may be present in an environmental sample which is subjected to sequencing analysis). For example, such a collection may comprise more than 10, such as about 30 different artificial chromosomes.

Additional artificial chromosome features:

As stated above, an artificial chromosome (or a fragment thereof) can incorporate higher level features such as eukaryote gene loci, CpG islands, mobile elements, repetitive polynucleotide features, small scale genetic variation and large scale genetic variation or prokaryote gene loci, DNA repeats, and/or mobile elements, despite containing a primary nucleotide sequence that is not present in one or more (or any) natural organisms and which does not encode full-length or functional mRNA, rRNA, tRNA, microRNA, piRNA, lncRNA, snRNA, snoRNA, a functional translated reading frame, a polypeptide or a protein. These and other additional or alternative features of the artificial chromosome are described herein.

## Artificial Genes

The artificial polynucleotide sequence of the artificial chromosome can comprise one or more artificial genes. The one or more artificial genes can comprise one or more exons with intervening introns. The introns and/or exons can be of any suitable length. For example, the exons may be from 25 nucleotides to 10 kilobases (kb) in length. The introns may be from 50 nucleotides to 2 megabases (Mb) in length. The total gene size may range from 200 nucleotides to 4 Mb. The number of artificial genes present on the artificial chromosome may vary from 1 to 10,000. The number of isoforms produced of each artificial gene may vary from 1 to 200. The number of exons per artificial gene may vary from 1 to 300. The number of introns per artificial gene may vary from 1 to 300.

The artificial genes can be created by any suitable method described herein. For example, the artificial genes can be created using the shuffling techniques described herein, using shuffling windows corresponding to the naturally occurring intron and exon sequences of the naturally occurring template nucleotide sequence. Once shuffled (and further manually edited, if required), the artificial gene can then be reconstructed in the artificial chromosome with the intron and exon structure of the original naturally occurring gene, (as exemplified by the illustration of an artificial chromosome in Figure 3). In addition, small sequence elements less than 15 nucleotides, such as splicing and transcription start site and stop sequence elements can be populated around the artificial gene loci encoded within the artificial chromosome.

## Artificial Mobile elements

The artificial polynucleotide sequence of the artificial chromosome can comprise one or more mobile repeat elements. Mobile repeat elements are highly similar DNA sequences that are present as multiple copies interspersed throughout the artificial chromosome. Their length and abundance can be varied as required. For example, the repeat unit of the artificial mobile elements which can be incorporated into the artificial chromosome of the present disclosure can be 5, 6, 7, 8, 9, 10, 15, 20, 30, 40, 50, 60, 70, 80, 90, 100, 150, 200, 250, 300, 350, 400, 450, 500, 600, 700, 800, 900, 1000 or more nucleotides in length. For example, the size of the repeat unit of the artificial mobile elements can vary from 100 nucleotides to 10kb. The number of repeat elements present in an artificial chromosome disclosed herein may constitute from 0.1-90% of the total artificial chromosome length.

In one example, the length and abundance of the mobile elements is tailored so as to emulate natural mobile insertion elements. Again, the primary sequence of the mobile element is generated so as to share little or no sequence identity with any known, naturally occurring mobile element. An example of a suitable mobile element that may be included in the artificial chromosome of the present disclosure is a mobile element emulating the human *SINE* element. Such a mobile element is about 350 nucleotides in length. In one example, multiple mobile elements emulating the human *SINE* element can be incorporated into the artificial chromosome so that they comprise about 10% (e.g., 10.7%) of the artificial chromosome sequence.

Artificial mobile elements can be generated so as to emulate the hierarchy of mobile repeat elements that results from the accumulation of mutations from ancient to recent insertion events (Lander, E.S. *et al.,* 2001). For example, initially, the original, natural ("ancestral") repeat sequence of the mobile element can be shuffled to remove homology to known natural sequences. The shuffled mobile element sequence can then be duplicated to produce multiple copies. For example, the artificial chromosome may contain at least 2, at least 3, at least 4, at least 5, at least 10, at least 20, at least 30, at least 40, at least 50, at least 60, at least 70, at least 80, at least 90, at least 100, at least 500, at least 1,000 or at least 2,000 or more copies of an artificial mobile element. One or more of the copies (or each copy) can then be subjected to random nucleotide substitutions, insertions and deletions to replicate sequence degeneration of mobile repeat sequences from the ancestral sequence (as exemplified by the illustration in Figure 4). The mobile elements can also undergo multiple further cycles of nucleotide substitution and amplification to create a range of mobile elements.

## Repeat polynucleotide sequences

The artificial polynucleotide sequence of the artificial chromosome can comprise repetitive polynucleotide features, such as repetitive DNA features including, for example, terminal repeats, for example telomeres, inverted repeats, and tandem repeats, for example centromeres. Tandem, inverted and terminal repeat DNA can evolve through a series of repeat unit amplification events resulting in the spreading of new repeat subfamilies. This process of generating repeat DNA sequence can be emulated when designing artificial repeat DNA by using consecutive rounds of repeat-unit amplification followed by artificially replicated sequence divergence (e.g., by manipulation of the repeat units to insert random nucleotide substitutions, deletions and/or insertions; as exemplified by the illustration in Figure 5). This iterative process can generate repeat DNA tandem arrays that maintain a hierarchal relationship between subsets of repeat units.

Thus, the artificial polynucleotide sequence of the artificial chromosome can comprise artificial repeat DNA that emulates repetitive human genetic features, such as satellite DNA. In another example, the artificial chromosome can contain one or more centromeres. The centromeres can constitute large arrays of tandem repeat units with DNA sequences between 25-5,000 nucleotides long. Alternatively or in addition, the artificial chromosome can contain repetitive telomere sequences. The repetitive telomere sequences can be of any suitable length. For example, the repetitive telomere sequences can comprise repeat units of 5, 6, 7, 8, 9, 10, 11, 12, 13, 14, 15, 20 or more nucleotides. For example, the repetitive telomere sequence can be from 4-10 nucleotides in length. In one example, such telomere sequences can comprise a 6 nucleotide motif tandemly repeated up to 10kb at the sequence termini. Other suitable repeats can be designed as required. Any suitable number of repeats can be incorporated into the artificial chromosome disclosed herein. In one example, the copy number of the telomere repeats may be from 5,000 - 50,000.

**Small-scale genetic variation**

Small-scale genetic variation (including, for example, single-nucleotide polymorphisms, insertions, deletions, duplications, and multiple nucleotide polymorphisms that are all less than 50 contiguous nucleotides in length) can be incorporated into multiple artificial chromosomes disclosed herein. For example, nucleotide differences between a pair of artificial chromosomes can be generated in order to simulate genetic variation, wherein the two or more variants present on two or more artificial chromosomes represent two or more alleles (as exemplified by the illustration in Figure 6). Accordingly, multiple artificial chromosomes can represent multiple alleles. For example, two matching copies of an artificial chromosome emulating a portion of a diploid genome can be produced so as to contain two copies of one allele, (thereby simulating homozygosity). Alternatively, each of the two copies of an artificial chromosome can contain a different allele (thereby simulating heterozygosity). It will be appreciated that multiple alleles can be prepared on multiple artificial chromosomes, as desired. Accordingly, the present disclosure provides collections (or "libraries") of multiple artificial chromosomes, representing naturally occurring allelic variation. In one example, 2, 3 or 4 artificial alleles on 2,3 or 4 artificial chromsomes are provided.

During the generation of small-scale genetic variation for incorporation in the artificial chromosomes disclosed herein, the small-scale variation nucleotide sequence and flanking artificial sequences may be required to be edited to remove any homology to known natural sequences.

Polynucleotide sequences representing genetic variation that is associated with disease can also be incorporated in the artificial chromosome disclosed herein. For example, specific diagnostic genetic features, such as a particular SNP, can be inserted into the artificial chromosome to provide matching local sequence context for the mutation, whilst maintaining little or no homology to known natural sequences at a broader level.

Since the emulation of known genetic variation requires multiple artificial chromosomes, it is possible to generate a particular artificial chromosome to be regarded as a "consensus", or "reference" sequence (similar to consensus genome assemblies such as hg19 human genome assembly, mm 10 mouse genome assembly etc.) and one or more multiple, distinct artificial chromosomes (or "variant" artificial chromosomes) that differ from the reference chromosome at one or more sites of genetic variation. Accordingly, the library of artificial chromosomes disclosed herein can comprise a single reference artificial chromosome and one or more variant artificial chromosomes that differ from the reference chromosome at one or more sites of genetic variation.

**Large-scale genetic variation**

Large-scale genetic variation (including, for example, large deletions, duplications, copy-number variants, insertions, inversions and translocations, each concerning nucleotide sequences of 50 or more contiguous nucleotides) can also be incorporated into multiple artificial chromosomes disclosed herein. Naturally occurring large-scale genetic variation often affects nucleotide sequences that are larger than the typical shotgun short sequence read length, further complicating the detection and resolution of structural variation in naturally occurring, sample nucleotide sequences.

Shuffling of nucleotide sequences affected by transversions, copy number variation and/or mobile-element insertions can be performed with a window size that matches the structural unit size of the large-scale variation, as described herein. For example, a single repeat unit can be shuffled before duplication, so that resulting duplicated copies share the same shuffled sequence. In another example, the sequence can be shuffled before transversion, so that only the orientation and breakpoints differ to the template sequence. In another example, the sequence can be shuffled before insertion of mobile elements, so that the insertion retains sequence homology to other mobile elements in the same artificial chromosome.

One example of large-scale genetic variation which can be incorporated into multiple artificial chromosomes disclosed herein is a translocation. Translocations can occur by which a sequence is rearranged between two artificial chromosomes, generating two reciprocal fusion artificial chromosomes, (as exemplified by the illustration in Figure 9). Translocations between two non-homologous artificial chromosomes can result in the fusion of two different genes to produce a chimeric gene fusion. Thus, the artificial chromosome disclosed herein can comprise one or more artificial chimeric gene fusions.

Artificial microbe genomes

The artificial polynucleotide sequence of the artificial chromosome disclosed herein can be designed to simulate a microbe genome (which artificial chromosomes are also referred to herein as "artificial microbe genomes"). For example, artificial chromosomes can be generated by shuffling natural microbe genomes to remove primary sequence homology to natural sequences (as exemplified by the illustration in Figure 10), whilst still retaining particular features of the original microbe genome, (such as, but not limited to, size, rRNA operon number, GC%, repeat content, etc.).

Multiple artificial chromosomes can be generated to simulate an artificial microbe community for metagenome analysis. Thus, also described but not claimed is a library of two or more artificial microbe genomes, in which any shared sequence identity with the original, naturally occurring microbe genome sequence has been reduced or removed. The relative abundance of individual artificial microbe genomes can be selected so as to correspond to the different abundances of microbe populations within a metagenome sample. Accordingly, the library of artificial microbe genomes can be generated so as to emulate a heterogeneous microbe community typically profiled during metagenome analysis. Any suitable number of artificial microbe genomes disclosed herein can be combined into a library. In one example, the library may contain 3-3,000 artificial microbe genomes.

The artificial microbe genomes disclosed herein can encode one or more gene loci. Gene loci may comprise artificial *16S* rRNA genes that are commonly used in phylogenetic profiling of metagenome communities (see, e.g., Edwards, R.A. *et al.,* 2006). PCR amplification and sequencing of the variable regions within the *16S* rRNA gene has been the primary approach to assess abundance and taxonomic diversity of microbes within a sample. Whilst the artificial 16S rRNA sequence present in the artificial microbe genomes disclosed herein is typically shuffled to remove homology to known natural sequences, the sequence complementary to universal primers used in amplicon sequencing can be tailored to remain identical to natural sequences, (as exemplified by the illustration in Figure 11).

**Artificial immune receptor clonotypes**

The artificial polynucleotide sequence of the artificial chromosome disclosed herein can encode one or more immune cell receptor gene loci, including representations of any one or more of the *IgA, IgH, IgL, IgK, IgM, TCRA TCRB,* and *TCRG* receptors, or others. These immunoglobulins and T-cell receptor loci undergo V(D)J recombination and somatic hypermutation to generate a diverse range of sequences called clonotypes. These biological processes can be modelled using artificial chromosome sequences to generate a suite of artificial clonotypes.

Variable (V) segment, Joining (J) segment and Diversity (D) segment sequences (and flanking introns) from immunoglobulin and T-cell receptor sequences can be retrieved from a genome sequence such as the human genome and shuffled separately to reduce or remove homology. In some examples, it may be desired to retain a small (for example, 20 nucleotide long) sequence complementary to universal primer sequences commonly used for amplicon profiling of immune receptors (see, e.g., van Dongen, J.J. *et al.,* 2003). V(D)J recombination of the artificial immunoglobulin and T-cell receptor loci can then be performed by randomly selecting a Joining (J) segment that is first combined with a randomly selected Diversity (D) segment to form a D-J gene segment, with intervening sequence removed, followed by the joining of a randomly selected Variable (V) segment, resulting in a rearranged artificial VDJ gene segment, (as exemplified by the illustration in Figures 12 and 13). The random selection of different segments generates a huge repertoire of different segment combination. Additional diversity can be added by the substitution, addition or deletion of nucleotides at segment junctions or within segments. Each rearranged, artificial VDJ gene segment is referred to herein as a "clonotype". A large number of artificial clonotypes can be produced by this method, emulating the size, diversity, complexity and profile of natural immune receptor clonotypes typically observed during the immune-repertoire sequencing of a human white blood cells.

Computer readable medium:

The artificial chromosomes disclosed herein may be provided *in silico* and may therefore be provided on a computer readable medium. Thus, also described but not claimed is a computer readable medium containing data representing one or more artificial chromosomes disclosed herein. The computer readable medium may be non-transitory.

The computer readable medium may be provided together with a computer system adapted to analyse the artificial chromosome or chromosomes stored on the computer readable medium.

The present disclosure also describes software allowing the analysis of the artificial chromosome or chromosomes stored on the computer readable medium. For example, the software may allow sequence comparisons to be performed, comparing the sequence of a given input sequence to the artificial chromosome sequence. Any known software package capable of achieving this function can be used.

Polynucleotide standards:

Any part or whole of the artificial chromosome sequences disclosed herein can be physically created as an DNA polynucleotide. Thus, also described but not claimed is a fragment of the artificial chromosome disclosed herein, wherein the fragment comprises or consists of from 20 to 10,000,000 contiguous nucleotides of the artificial polynucleotide sequence of the artificial chromosome. For example, the fragment may comprise or consist of any 10,000,000, any 1,000,000, any 500,000, any 100,000, any 50,000, any 10,000, any 1,000, any 500, any 400, any 300, any 250, any 200, any 150, any 100, any 50, any 25, any 21 or any 20 contiguous nucleotides of the artificial polynucleotide sequence. Such a fragment is referred to herein as a "standard". The polynucleotide standard matches the corresponding artificial sequence of the artificial chromosome. Accordingly, the polynucleotide standard is capable of representing any one or more features of the artificial chromosome disclosed herein. It will be appreciated that the standards disclosed herein can be used independently of the artificial chromosome. For example, artificial standards can be used to calibrate polynucleotide quantitation processes without requiring reference to the artificial chromosome.

The generation of physical, tangible standards based on the artificial chromosome disclosed herein allows the calibration of a wide variety of sequencing methods (including PCR amplification and NGS sequencing methods). For example, this may be performed by adding a known quantity of one or more polynucleotide standards to a given RNA or DNA sample before the amplification and/or sequencing method is performed. Analysis of the sequencing of the known polynucleotide standard with reference to the artificial chromosome provides a powerful calibration of the particular amplification and/or sequencing method used.

**Production of RNA standards**

In embodiments not part of the claims, the standard may be an RNA standard. An RNA standard is an RNA molecule that matches and represents a feature of interest encoded by the artificial chromosome. For example, the RNA standard can represent an artificial gene or transcribed element or fragment thereof that is encoded by the artificial chromosome. In one example, the RNA standard does not include any homology to any known, natural sequence. The length of the RNA standard can therefore vary depending on the feature of interest. In one example, the RNA standard can vary in length from 200 nucleotides to 30 kb.

The sequence of interest from the artificial chromosome can be synthesized into a DNA sequence. The DNA sequence can be inserted in operable linkage with an active promoter into a vector. Thus, the present disclosure also provides a DNA molecule encoding a fragment of the artificial chromosome. The present disclosure also provides a polynucleotide vector (such as a DNA vector) comprising a DNA sequence encoding a fragment of the artificial chromosome. Any suitable vector can be used. In one example, the vector is an expression vector. The expression vector can contain any suitable promoter and/or enhancer capable of directing transcription of the standard disclosed herein.

The vector disclosed herein can be used as a template for an RNA synthesis reaction that produces an RNA molecule. Thus, the present disclosure also provides a method for producing a polynucleotide standard disclosed herein, comprising synthesising an RNA molecule from a vector disclosed herein. Suitable RNA synthesis methods are well known. For example, such synthesis methods may be performed in a cell free, *in vitro* expression system. Alternatively, such methods may be performed in an *in vivo* expression system, such as a host cell. Any suitable host cell can be used. The produced RNA molecule can then be purified by known methods in order to produce the final RNA polynucleotide standard.

Thus, the present disclosure describes methods that can be used to produce an RNA standard that matches part or whole of the artificial sequence of the artificial chromosome sequence. An overview of a suitable method for the production of RNA standards is illustrated in Figure 14.

**Mixtures of multiple RNA standards**

Multiple RNA standards can be used collectively as a mixture. Accordingly, the present disclosure describes a mixture of one or more RNA standards disclosed herein. The mixture can comprise any suitable buffer to maintain the structural integrity of the RNA standards.

Individual RNA standards can be diluted at a range of different concentrations and then combined into a mixture of RNA standards. This mixture of RNA standards across a range of different concentrations can therefore comprise a quantitative scale. The quantitative scale can comprise a ladder of RNA standards at different sequential abundance. This scale can be used as a reference to measure the abundance of natural RNA transcripts within the accompanying sample. Alternative mixtures can be produced that differ in the relative concentration of individual RNA standards. Comparison of RNA standards in alternative mixtures can thereby measure differential abundance of the RNA standards, thereby providing a reference scale that can be used to measure changes in RNA abundance, such as occurs during gene expression, between two or more samples.

The number of RNA standards provided per mixture can vary from 3-3000, such as from 3-300 per mixture prepared. For example, a mixture may be provided containing about 90 RNA standards. The RNA standards may be added to a sample of interest so as to constitute from 0.001-50%, such as about 1% of the total RNA present in the sample.

**RNA standards representing artificial genes**

RNA standards can be designed to match any artificial gene of interest encoded within the artificial polynucleotide sequence of the artificial chromosome. The contiguous RNA standard sequence matches the artificial exon sequences whilst the intervening intron sequences are excluded (as exemplified in the illustration in Figure 3). Thus, an RNA standard can comprise or consist of a contiguous nucleotide sequence that corresponds to only the exon sequences of an artificial gene encoded by the artificial chromosome. This emulates the natural process of gene splicing, whereby intron sequences are removed and exons sequences are joined together.

RNA standards can be designed to emulate the biological process of alternative splicing, where particular exons are included or excluded to form multiple isoforms of a gene loci. In addition, multiple RNA standards matching each of the multiple isoforms generated from a single gene locus can be produced. By combining multiple RNA standards matching multiple alternative mRNA isoforms at different concentrations, alternative splicing events can be simulated, including, for example, intron retention, cassette exons, alternative transcription initiation and termination, non-canonical splicing, and others. The relative abundance of the RNA standards representing each isoform can be varied to correspond to the frequency of the alternative splicing event being represented.

**RNA standards representing artificial fusion genes**

A translocation between two artificial chromosomes can join two different artificial genes into a single fusion gene (or "chimera"). RNA standards can be produced so as to match fusion genes generated by translocation between artificial chromosomes.

Translocations usually affect only one chromosome of a chromosome pair (or of multiple equivalent chromosomes in higher order polyploidy organisms), with the other chromosome within the pair remaining unaffected. Therefore, it can be advantageous to produce RNA standards representing two normal (i.e., non-fused) copies of the gene and a single copy of the fused gene, thereby emulating a heterozygous genotype (as exemplified in the illustration in Figure 9). The relative concentration of the RNA standard matching the fusion gene can be varied to emulate the likely concentration in a test sample being studied of the particular fusion gene being modelled. For example, in the case of minimal residual diseases, where only a fraction of cells within a tumor sample harbor a translocation allele and express a fusion gene, a low concentration of the artificial fusion gene may be used.

**Production of DNA standards**

The standard used in the claimed methods is a DNA standard. A DNA standard is a DNA molecule that matches and represents an artificial sequence of interest in the artificial chromosome. In one example, the DNA standard matches the sequences of a feature in the artificial chromosome. Part or whole of the artificial chromosome sequence can be physically generated as a DNA molecule using any suitable known method of DNA synthesis. Accordingly, the size and content of the DNA standard can vary depending on the particular fragment of the artificial chromosome chosen to form the DNA standard. In one example, the DNA standard can vary in length from 100 nucleotides to 20 Mb.

The DNA molecule matching the artificial chromosome sequence may be inserted into a vector. Any suitable vector may be used. For example, the vector may be a plasmid vector. The synthesised DNA molecule may be inserted into the vector between any two suitable restriction endonuclease consensus recognition sites. For example, the synthesised DNA molecule may be inserted into the vector between two Type III restriction endonuclease consensus recognition sites (exemplified in the illustration in Figure 15). This allows the generation of the DNA standard by excision from the vector using one or more restriction endonucleases. Accordingly, the present disclosure describes a method of generating a DNA standard, comprising synthesising a DNA fragment corresponding to a sequence of the artificial chromosome, inserting the DNA fragment into a vector (such as a plasmid vector) and subsequently excising the DNA fragment from the vector by restriction endonuclease digestion.

Alternative methods of generating DNA standards can be used. For example, the DNA standard (which may, for example, be present in a vector, such as a plasmid vector) may be produced by an amplification reaction. For example, PCR amplification can be used to produce multiple copies of the DNA standard, by using PCR primers that are complementary to the sequence at either end of the DNA standard. Any suitable amplification method known to generate multiple copies of a DNA molecule may be used. An overview of a suitable method for the production of DNA standards is illustrated in Figure 15.

**Mixtures of multiple DNA standards**

Multiple DNA standards can be used collectively as a mixture. The mixture can comprise any suitable buffer to maintain the structural integrity of the DNA standards.

Individual DNA standards can be diluted at a range of different concentrations and then combined into a mixture of DNA standards. This mixture of DNA standards across a range of different concentrations can therefore comprise a quantitative scale. The quantitative scale can comprise a ladder of DNA standards at different sequential abundance. This scale can be used as a reference to measure the abundance of natural DNA transcripts within the accompanying sample.

Alternative mixtures can be produced that differ in the relative concentration of individual DNA standards. Comparison of DNA standards in alternative mixtures can thereby measure differential abundance of the DNA standards, thereby providing a reference scale that can be used to measure changes in abundance of DNA molecules between two or more accompanying samples. For example, differences in the abundance of DNA standards between two mixtures can provide a scale with which to compare differences in the abundance of microbial genome DNA between two samples.

The number of DNA standards provided per mixture can vary from 3-3000, such as from 3-300 per mixture prepared. For example, a mixture may be provided containing about 90 DNA standards. The DNA standards may be added to a sample of interest so as to constitute from 0.001-50%, such as about 1% of the total DNA present in the sample.

**Conjoined DNA standards**

Multiple DNA standards can be ligated together (or "conjoined") into a single contiguous sequence using standard molecular biology techniques, such as restriction digestion and ligation or Gibson assembly (e.g., as illustrated in Figure 16). Thus, the present disclosure also describes a conjoined DNA standard. The present disclosure also describes a method of preparing a conjoined DNA standard, comprising ligating together two or more DNA standards disclosed herein into a single, contiguous sequence.

A single conjoined standard can contain an individual DNA standard repeated to multiple copy numbers. Accordingly, copy-number can be employed to establish differential abundance of DNA standards. The present disclosure also provides a method of preparing a conjoined DNA standard comprising multiple individual DNA standards, with each DNA standard being present as multiple copies in the conjoined DNA standard.

In addition, a single conjoined standard can contain multiple, different individual DNA standards, each copied to any desired copy number, in any combination.

Variation in the abundance of individual DNA standards can result from errors in pipetting or aliquoting. However, joining multiple individual DNA standards into a large conjoined DNA standard removes any between-individual variation due to the pipetting or aliquoting (because the conjoined DNA standard is aliquoted once).

The abundance of multiple individual DNA standards at different copy-numbers that comprise a conjoined DNA standard can be used to estimate the error due to pipetting. This is because errors in pipetting the conjoined standard are the same and dependent between the individual DNA standards that are combined together to a conjoined DNA standard. The slope of the line of best fit plotted between the observed to known abundance of individual DNA standards that are joined into a single conjoined DNA standard indicates the estimate of pipetting error for the conjoined DNA standard. Subsequent normalization of DNA standard abundance according to this estimate can minimize this source of variation. This internal normalization approach enables a more accurate measure of abundance,

Any suitable type and number of individual DNA standards can be joined to form a conjoined DNA standard. In one example, 6 individual DNA standards are joined to form a single conjoined DNA standard. Furthermore, multiple conjoined DNA standards at a range of concentrations can be combined to form a mixture. In another example, 30 conjoined DNA standards are combined to form a mixture.

**DNA standards representing artificial microbe genomes**

Metagenomics entails a study of multiple genomes from different organisms, and can be applied to profile a community of microbe genomes. For example, a metagenomic analysis can be used to determine the sequence and to measure the abundance of multiple microbe genomes within a single sample (such as an environmental sample). DNA standards can be prepared that match and represent artificial microbe genomes, thereby emulating a microbial community structure and diversity.

Thus, the present disclosure describes DNA standards that are based on artificial microbe genomes. Such DNA standards may match only a representative subsequence of the full artificial microbe genomes (e.g., as illustrated in Figure 10). For example, microbe genome size varies considerably (generally between 0.5 and 7 Mb for common taxa). Therefore, DNA standards may be of proportional length (for example, between 1% size of 0.5 and 7 Kb) to the full-length artificial microbe genomes.

Furthermore, microbes' genomes exhibit a broad range of percentage GC content (e.g., from 20% - 75%). The DNA standards disclosed herein may be of proportional GC content (for example, ranging from 20% - 75%) to the full-length artificial microbe genomes. Using DNA standards that match only representative subsequences within the artificial microbe genomes can reduce the sequencing depth required to profile the microbe community whilst maintaining a wide range in abundance between standards that is similar to microbe community structures typically present in natural samples.

## DNA standards representing small-scale genetic variation

Small-scale genetic variation distinguishes two or more variant alleles of an artificial chromosome sequence (e.g., as illustrated in Figure 6). DNA standards can be designed to represent such small-scale genetic variation between multiple artificial chromosomes. For example, an individual DNA standard can be generated that matches the sequence of an allele present in a "reference" artificial chromosome, and an individual DNA standard can be generated that matches the sequence of an allele present in a "variant" artificial chromosome.

The relative abundance of the DNA standard can match the relative frequency of the allele. For example, one DNA standard matching an alternative variant and one DNA standard matching a reference variant at the same abundance can emulate the heterozygous frequency of allele in a diploid genome. In another example, a single DNA standard matching an alternative variant can emulate homozygous variation in a diploid genome. In another example, one DNA standard matching an alternative variant and one DNA standard matching a reference variant at varying abundance can emulate heterogeneous frequency (present in non-bi-allelic ratios, such as when only a subset of the sample harbors a mutation). Accordingly, DNA standards can be prepared so as to emulate the existence and frequency of genetic variation between artificial chromosomes.

## DNA standards representing large-scale structural variation

Large-scale genetic variation can distinguish two or more variant alleles of an artificial chromosome sequence. DNA standards can be designed to match and represent such large-scale genetic variation between multiple artificial chromosomes (e.g., as illustrated in Figure 8). The relative abundance of the DNA standard can match the relative frequency of large-scale variation, and emulate zygosity.

DNA standards can be provided that match the one or more repeat units in a tandem repeat array (e.g., as illustrated in Figure 5). Variations in the concentration of DNA standards can also be selected so as to emulate repeat unit copy number. For example, abundant DNA repeat standards can be prepared to correspond to high copy number variants. Conversely, low abundance DNA repeat standards can be prepared to correspond to low copy number variants. In addition, the relative abundance of the DNA standards can also be calibrated to match the desired allele frequency.

## Sequence barcodes to distinguish DNA standards

To distinguish between DNA standards that match the same DNA sequence (such as the same repeat element), one or more 'barcode' nucleotide sequences can be incorporated into DNA standards (e.g., as illustrated in Figure 17). Barcode nucleotide sequences are typically small (e.g., 4, 5, 6, 7, 8, 9, or 10 nucleotide) contiguous or non-contiguous nucleotide sequences that make up only a small fraction of the total DNA standard sequence. For example, the one or more barcode nucleotide sequences may constitute less than 10%, such as less than 9%, such as less than 8%, such as less than 7%, such as less than 6%, such as less than 5%, such as less than 4%, such as less than 3%, such as less than 2%, such as less than 1% of the total nucleotide sequence of the DNA standard. The existence of a barcode nucleotide sequence can allow the identification of a DNA standard. For example, when multiple DNA standards match the same artificial chromosome sequences, 'barcode' nucleotide sequences allow the identification of particular DNA standards within all DNA standards that match the same artificial chromosome sequences. The barcode sequence can be removed or modified during analysis so it does not interfere with the alignment.

## DNA standards representing immune receptor clonotypes

The DNA standards disclosed herein can be designed so as to match and represent artificial clonotypes generated from the immunoglobulins and T-cell receptors gene loci encoded within the corresponding artificial chromosome (e.g., as illustrated in Figures 12 and 13). In one example, DNA standards encompass the clonotype sequence of the randomly selected V,D and J segments. The DNA standards disclosed herein may also retain small sequences complementary to universal primer sequences commonly used in immune repertoire sequencing. For example, DNA standards may retain primer sequences described in BIOMED-2 (van Dongen, Langerak et al. 2003) study for profiling natural clonotype diversity.

A large number of DNA standards, each representing artificial clonotypes can be produced by this method. These DNA standards can be combined into a mixture that emulates the size, diversity, complexity and profile of natural receptor clonotypes typically observed during the immune-repertoire sequencing of human white blood cells.

## DNA standards representing *16S* marker genes

DNA standards can represent artificial *16S* rRNA gene sequences from an artificial microbe genome (e.g., as illustrated in Figure 11). The artificial *16S* rRNA gene has no homology to known sequences, with the exception of retaining two complementary sequences to the universal *16S* primers commonly used in amplicon sequencing. This enables the DNA standards to act as a template for PCR amplification with the *16S* primers. Amplification of the DNA standards thereby provides a synthetic and quantitative measure of PCR amplification and sequencing of 16S rRNA marker genes commonly used to determine microbe community identity and structure.

Methods of use:

The polynucleotide standards disclosed herein can be used to calibrate a wide variety of sequencing methods. This can be achieved by adding the polynucleotide standards to a sample comprising a target DNA/RNA sequence to be determined. The source of target DNA/RNA can come from any known organism or environmental sample. For example, the polynucleotide standards can be added to a sample of natural RNA derived from animal (such as mammalian, human, or other), plant (such as corn, rice, or other), microbial (such as bacteria, archaea, or other) and environmental (such as soil samples, human stools, clinical samples such as infected wound fluid, and other) sources. It will be appreciated that the polynucleotide standards disclosed herein can be used to calibrate sequencing methods performed on any sample containing a target DNA/RNA sequence to be determined.

Because the polynucleotide standards disclosed herein have little or no homology (or sequence identity) to natural polynucleotide sequences, sequenced reads derived from the polynucleotide standards can be distinguished from sequenced reads derived from natural RNA/DNA present in a sample (e.g., as illustrated in Figure 18). Thus, the fragments (standards) disclosed herein may have a percentage identity relative to known, naturally occurring sequences selected to allow sequenced reads derived from the polynucleotide standards to be distinguished from sequenced reads derived from natural RNA/DNA present in a sample. This enables the polynucleotide standards to be added to the RNA/DNA sample, prior to sequencing, and therefore undergo the same library preparation, sequencing, alignment and analysis as for the DNA/RNA sample of interest. However, following sequencing, reads matching polynucleotide standards can be distinguished from reads matching DNA/RNA sample of interest.

Accordingly, the methods disclosed herein comprise a step of determining the sequence of a target polynucleotide (DNA or RNA) of interest in a sample. The methods disclosed herein also comprise a step of determining the sequence of one or more polynucleotide standards which have been added to the sample. The methods disclosed herein further comprise a step of comparing the sequence and/or quantity of a target polynucleotide (DNA or RNA) of interest in a sample with the sequence and/or quantity of one or more polynucleotide standards which have been added to the sample. Such a comparison allows the normalization of values derived from the measurement of the target polynucleotide in the sample against the values derived from the measurement of the one or more polynucleotide standards. Accordingly, the methods disclosed herein may further comprise a step of normalizing the values derived from the measurement of the target polynucleotide in the sample against the values derived from the measurement of the one or more polynucleotide standards. Any suitable mathematical algorithm capable of normalizing these values can be used.

In many cases, the polynucleotide standards combined with an RNA/DNA sample constitute only a fraction of the combined total amount of RNA/DNA in the sample. This fractional contribution (typically between 0.1 and 10% of the total amount of RNA/DNA in the sample, or typically less than 10%, such as less than 5%, such as less than 1%, such as less than 0.5% of the total amount of RNA/DNA in the sample) varies according to the type of library preparations used in the analysis (e.g., rRNA removal, polyA or total RNA purification preparations). The fractional contribution of the polynucleotide standards can be inversely proportional to the sequencing depth attributed to the RNA/DNA sample. Therefore, the fractional total can be selected as the minimum amount required to sufficiently enable analysis of the polynucleotide standards.

**Measuring sequencing errors in polynucleotide standards**

Sequencing errors occur when nucleotides are determined incorrectly, possibly resulting from errors or artefacts of the library preparation or of the sequencing process itself. Analysis of sequenced reads from the polynucleotide standards can identify and quantify nucleotide error differences. Suitable software facilitating the identification of sequencing errors includes Quake (Kelley, Schatz et al. 2010) and SysCall (Meacham, Boffelli et al. 2011). This analysis can then be used to provide a measure of sequence performance and quality. This analysis also then allows a researcher to normalize or correct systematic sequencing errors within reads from the sample DNA/RNA, providing a far more accurate (both qualitatively and quantitatively) measurement of the target DNA/RNA of interest in the sample. The sequencing error profile of the polynucleotide standards can also be employed to distinguish sequencing errors from genuine nucleotide differences (such as SNPS or nucleotide modifications).

**Assessing sequence alignments with polynucleotide standards**

During a sequencing operation, small sequenced reads are often first aligned to a reference genome. The alignment of reads to a large reference genome is a computationally intensive task that can be performed in numerous ways, providing differential outcomes for speed, sensitivity and accuracy. The polynucleotide standards disclosed herein can be used to assess the efficiency and accuracy with which sequenced reads are aligned to the artificial chromosome disclosed herein, thereby calibrating the alignment methods performed. Accordingly, the methods disclosed herein may further comprise a step of aligning sequenced reads derived from the polynucleotide standards to the artificial chromosome from which those standards were derived. Any suitable alignment methods can be used to perform this step.

Example of suitable software facilitating the alignment of sequence reads include BWA (Li and Durbin 2009, Kelley, Schatz et al. 2010) and Bowtie (Langmead, Trapnell et al. 2009)

Sequenced reads are preferably aligned to both the reference genome and artificial chromosome concurrently. In one example, the artificial chromosome sequence combined with the reference genome to make an index that facilitates rapid alignment. This enables sequenced reads to be simultaneously aligned to both the artificial chromosome and reference genome (e.g., as illustrated in Figure 18). By the assessing the accuracy and sensitivity with which reads align to the artificial chromosome, a parallel and empirical assessment of reads aligning to the natural genome can be performed simultaneously.

The alignment of reads derived from the polynucleotide standards disclosed herein to the artificial chromosome can be assessed according to a number of characteristics, such as (but not limited to): sensitivity and specificity of correct read alignments; and/or proportion of reads-pairs mapped concordantly discordantly, or with dovetail; and/or alignment mismatches and base-wise accuracy.

RNA sequenced reads that traverse introns are required to be aligned to the reference genome in a split or non-contiguous manner. Disclosed herein are RNA standards that are designed to emulate the splicing of introns and exons. Such RNA standards can therefore be used to assess the split alignment of reads across introns. Split reads derived from the RNA standards can be aligned to both the artificial and natural chromosome. Examples of suitable software facilitating the split alignment of sequence reads include Tophat2 (Kim, Pertea et al. 2013) and STAR (Dobin, Davis et al. 2013). Split alignments on the artificial chromosomes can then be compared to artificial gene annotations to assess the sensitivity and specificity with which reads align across introns.

Alternative splicing, transcription initiation and termination generate a range of isoforms from single gene loci. Also disclosed herein are RNA standards that can be used to assess the accuracy with which spliced and unspliced alignments are assembled into full-length transcript models. For example, full-length transcript isoforms can be assembled from overlapping read alignments on both the artificial and natural chromosomes. Example of suitable software facilitating the assembly of sequence reads include Cufflinks (Trapnell, Williams et al. 2010) and Trinity (Haas, Papanicolaou et al. 2013). The structure of RNA transcripts assembled on can then be compared to artificial gene annotations to assess the sensitivity and specificity with which transcript assembly has occurred (e.g., as illustrated in Figure 3). This assessment can then be used to inform the assembly of gene models in the accompanying natural sample.

**Assessing quantitative accuracy with polynucleotide standards**

Individual polynucleotide standards can be diluted to known concentrations, and collectively combined to form a mixture that provides a quantitative scale of such standards. The particular values chosen to define the scale can be determined based on the likely quantities of target RNA/DNA present in the sample to be analysed. Following sequencing, the number of reads aligning to the polynucleotide standards can provide a quantitative measure of abundance. Comparison between the known molar concentration and measured read abundance of the polynucleotide standards can be used to inform the quantitative analysis within and between samples in a number of ways, including (but not limited to):

(i) Comparison of a known concentration of the polynucleotide standards to measured abundance of the same polynucleotide standards indicates the quantitative accuracy of the DNA/RNA sequencing method.

(ii) Dynamic range (the difference between the highest and lowest abundance of the polynucleotide standards) indicates quantitative linearity (or parts thereof). Departure from these expectations may allow the performance of quantitative normalization.

(iii) Lower limit of detection (the lowest concentration of polynucleotide standard detected) indicates library size and sensitivity.

(iv) Quantified polynucleotide standards comprise an internal reference for quantifying genes at corresponding abundance.

(v) Enables conversion of sequencing units (R/FPKM) to molar or absolute (transcript copy number) units.

(vi) Quantitative range of RNA standards enables normalization between two or more samples and enables comparative analysis of gene expression.

## Measuring gene expression with RNA standards

Gene expression profiling measures the abundance of multiple genes using RNA sequencing reads. The RNA standards disclosed herein can be added at a range of concentrations to form a mixture and thereby emulate differential gene expression. The accuracy with which the abundance of RNA standards is measured can be assessed, thereby assessing the quantitative accuracy of gene expression analysis in the accompanying natural RNA sample (e.g., as illustrated in Figure 19).

Multiple RNA standards can be combined across a range of known concentrations and collectively combined to form different mixtures, emulating differential gene abundance, and fold changes in gene expression between samples. The abundance of RNA standards can be measured. Example of suitable software facilitating the quantification of RNA standards include EdgeR (Robinson, McCarthy et al. 2010) and DEseq (Anders, McCarthy et al. 2013). Comparing the measured abundance of RNA standards against their known molar concentration can indicate the accuracy of transcript quantification. Comparing the abundance of natural genes against RNA standards or the quantitative reference scale comprising multiple RNA standards can also inform measures of gene expression.

Similarly, alternative RNA standard isoforms can be included at different concentrations to emulate alternative splicing. The abundance of RNAs standard isoforms can be measured using suitable software, such Cufflinks (Trapnell, Williams et al. 2010) or MISO (Katz, Wang et al. 2010). The observed fold-change in RNA standard isoform abundance between mixtures can be determined to assess the accuracy with which isoform switching and alternative splicing is measured between samples, independent of changes in gene expression. Comparing the abundance of natural isoforms against RNA standards can also inform measures of alternative splicing.

## Detecting small-scale genetic variation represented by DNA standards

DNA standards disclosed herein can be generated that represent variant and reference alleles of small-scale genetic variation in the artificial chromosome (e.g., as illustrated in Figure 6). A range of variables can impact on variant identification and genotype assignment including (but not limited to): variant zygosity; read alignment, quality and/or coverage; variant type and complexity (eg. SNPs, indels, homopolymers); proximal sequence context; and software used to identify small-scale genetic variation. The DNA standards disclosed herein can be used to assess the sensitivity and specificity with which small-scale genetic variation is identified. Sequence determination of DNA standards can identify small-scale variation with respect to reference artificial chromosome sequence. Suitable software for identifying small-scale genetic variation include GATK (McKenna, Hanna et al. 2010) and SAMtools (Li, Handsaker et al. 2009). The accuracy and sensitivity with which small-scale genetic variation is detected within the DNA standards can be assessed with respect to the artificial chromosome (e.g., as illustrated in Figure 20). A value of uncertainty (such as a 95% confidence interval) can also be ascribed to estimates of accuracy. Comparing the confidence and sensitivity with which small-scale genetic variation is identified in the artificial chromosomes can also inform the identification of small-scale genetic variation in the accompanying DNA sample.

## Measuring the allele frequency represented by DNA standards

The accurate quantification of an allele's frequency is required to correctly assign a genotype or estimate the fraction of DNA within a sample carrying a variant (such as when a subset of cancer cells within a tumor sample carry a deleterious variant). The DNA standards disclosed herein can be used to emulate differential allele frequency, and thereby assess or calibrate the quantitative accuracy with which allele frequency is measured.

For example, DNA standards representing different alleles can be combined at varying concentrations into a mixture that is combined with the natural DNA sample for sequencing. Comparison between the known molar concentration and measured read abundance of each of the variant alleles (each represented by different DNA standards) then enables a quantitative assessment of allele frequency to be performed. Thus, the DNA standards disclosed herein can be used to determine the sensitivity, specificity and precision of variant detection at different relative concentrations and to establish a quantitative scale for comparison with the detection and/or quantification of natural, target variant alleles. Thus, the methods disclosed herein can comprise a step of preparing a mixture of DNA standards representing variant alleles, wherein each variant DNA standard is added at a predetermined concentration. The methods may also comprise determining the sequence and quantity of each of the variant DNA standards in the mixture. The methods disclosed herein may further comprise a step of providing a quantitative scale of measured variant DNA standard frequency, which scale can then be used to calibrate the quantitative measure of natural DNA alleles determined in a single DNA sample, or between multiple DNA samples.

## Resolving large-scale variation represented by DNA standards

Large-scale or structural genetic variation can be computationally difficult to resolve correctly as it is often larger than the length of sequenced reads. DNA standards disclosed herein can be generated that represent and emulate large-scale variation. For example, DNA standards representing structural variation can be used to: assess the ability of software programs to correctly resolve structure; and quantify the relative abundance and copy number of structural variants, and/or to assign a genotype to a sequence comprising structural variation. Suitable software for resolving large-scale variation include BreakDancer (Chen, Wallis et al. 2009) and Cortex (Iqbal, Caccamo et al. 2012). The DNA standards disclosed herein can also be used to model the re-distribution of sequence reads due to structural variation with respect to the reference artificial chromosome. The measurement of DNA standards can inform an assessment of the accuracy with which large-scale variation is identified and quantified within the accompanying natural genome DNA sample.

## *De novo* assembly of DNA standards

In cases where no naturally occurring reference genome is available, genome sequences must be assembled *de novo* from overlapping sequence reads. Parallel *de novo* assembly of DNA standards can be performed simultaneously with the accompanying target genome DNA sample. Suitable software for *de novo* assembly includes Velvet (Zerbino and Birney 2008) and ABySS (Simpson, Wong et al. 2009). Variables that affect genome assembly include (but are not limited to): genome complexity and repeat content; ploidy; sequencing depth, quality and error rate; read length and insert size; and software program and parameters (including k-mer length, alignment approach, read soft-clipping, and other parameters) used. The impact of these variables on the *de novo* assembly of DNA standard can be assessed.

The assembled sequence can be compared to the known DNA standards to assess the performance of *de novo* assembly and impact of variables described above. *De novo* assembly of the artificial chromosome can be assessed according to any one or more of: N50 value; median, maximum and/or combined contig sizes; coverage and gaps of contigs relative to the artificial chromosome; mismatch or base-wise accuracy of contigs relative to the artificial chromosome; and the identification of large or systematic assembly errors. The assessment of de novo assembly of DNA standards can inform an assessment of *de novo* assembly of the accompanying target natural DNA sample.

## Metagenome analysis with DNA standards

Metagenome analysis often comprises the assembly and quantification of multiple microbe genomes from an environmental sample. The DNA standards disclosed herein can be used to emulate a complex microbe community, constituting a heterogeneous collection of genomes at a range of different abundances (e.g., as illustrated in Figure 10). These DNA standards representing microbe genomes can be used to assess metagenome analysis. Variables that affect metagenome analysis include (but are not limited to): microbe community genome sizes, complexity, repeat and GC content, and user-defined variables such as sequencing depth and coverage, quality, read length and insert size, and software and parameters used. The impact of these variables on the metagenome analysis of DNA standard can be assessed.

The metagenome DNA standards disclosed herein can be used to assess the performance of *de novo* assembly and analysis (e.g., as illustrated in Figure 21). The assembly of DNA standards in relation to the artificial chromosome can be assessed according to a number of features including (but not limited to): N50 value; and median and maximum contig size; coverage; base-wise accuracy of assembled DNA standard contigs can be compared relative to the corresponding artificial chromosome. The assessment of metagenome analysis of DNA standards can inform an assessment of the metagenome analysis of the accompanying target natural DNA sample.

NGS sequencing can determine the abundance and diversity of microbes within a sampled community. The DNA standards disclosed herein can be combined at different relative concentrations to form a mixture that comprises a quantitative reference. The methods disclosed herein may further comprise a step of providing a quantitative scale of measured metagenome DNA standard frequency, which scale can then be used to calibrate the quantitative measure of natural microbe genomes determined in the accompanying environmental sample.

The DNA standards can also be used to assess metagenome analysis relative to quantitative abundance. For example, the DNA standards can be used to assess (without limitation): the minimum sequence coverage required for efficient assembly; the lower limit of detection (i.e. the lowest concentration at which metagenome DNA standards are detected); and measures of library sensitivity, size and/or diversity. The metagenome DNA standards disclosed herein can also be used for quantitative comparison between two or more samples, which enables a comparative analysis of microbe community structure and diversity to be performed between two or more samples.

**16S rRNA profiling with DNA standards**

The *16S rRNA* gene is often used as a phylogenetic marker for profiling large of complex microbe communities. DNA standards can be generated that represent and match a portion of the 16s rRNA genes from artificial microbe genomes (e.g., as illustrated in Figure 11). Furthermore, DNA standards representing artificial *16S rRNA* genes can be combined at different relative concentration to emulate a microbe community and to allow an assessment of *16S* profiling applications to be performed.

DNA standards matching the artificial *16S* rRNA genes can retain small sequences complementary to universal primers, and therefore amplify in parallel to natural *16S rRNA* genes. The resulting amplicons from the DNA standards can then be analyzed to assess any one or more of: (i) differential PCR amplification bias; and (ii) quantitative accuracy by comparing the measure abundance of DNA standard amplicons relative to the known initial concentration of those DNA standards. In addition, the resulting amplicons from the DNA standards can be used to establish a quantitative scale for comparison to quantify amplicons from the accompanying metagenome sample of interest.

**Identifying GC bias with DNA standards**

The impact of GC content on several reactions during library preparation and sequencing results in a skewed representation of microbe genomes that causes biases in assembly and quantification (Chen, Y.C., *et al.,* 2013). The DNA standards disclosed herein can be used to assess the impact of GC content on sequencing and analysis.

DNA standards can be produced that match the wide range of GC-contents observed in microbe genomes. DNA standards can be combined within environmental DNA samples prior to sequencing and analysis. Biases in the alignment, assembly and/or quantification of DNA standards that correlate with GC-content can be identified. For example, differences between the measured abundance and known concentration of DNA standards can identify bias associated with GC-content, which in turn can allow subsequent quantitative normalization to counter impact of GC-content. The DNA standards disclosed herein can also be employed as a training set to establish normalization parameters that minimize GC-content bias in DNA quantification.

**Using DNA standards with immune receptor sequencing**

Immune repertoire sequencing employs a common set of primers to amplify the suite of immune receptor sequences expressed by white blood cells. The DNA standards disclosed herein can be designed so as to represent artificial clonotypes on the artificial chromosome (examples illustrated in Figure 12 and 13). The range and complexity of clonotype DNA standards can be tailored to emulate the complex and diverse profile of natural clonotypes expressed by a sample of white blood cells.

The DNA standards disclosed herein may also retain small sequences complementary to each primer pair commonly used in immune repertoire sequencing. Therefore, PCR amplification can be used to amplify the natural clonotypes of interest within the sample, but also the clonotypes represented by the DNA standards. Therefore, DNA standards can act as templates for amplification using universal primers during immune repertoire sequencing. Following amplification and sequencing, reads derived from DNA standards can be analysed to assess the performance of immune repertoire sequencing and to quantify the relative abundance of different clonotypes. DNA standards can also be used to determine amplification bias of different universal primers that can be due to differences in hybridisation efficiency. Amplification biases can be determined by comparing the measured abundance of DNA standard amplicons relative to the known initial concentration of the DNA standards. Clonotype abundance can be subsequently normalised to count determined amplification bias. The DNA standards disclosed herein can also be used to assess the detection and quantification of artificial clonotypes that can inform an assessment of clonotype detection and quantification of the accompanying target natural DNA sample.

Any of the methods disclosed herein may comprise adding two or more fragments (or standards) disclosed herein to a sample at the same or different concentrations in order to replicate homozygosity, heterozygosity or heterogeneity. For example, two different fragments (or standards) may be added at the same concentrations to replicate heterozygosity. Thus, adding fragments (or standards) at different concentrations can replicated homozygosity, heterozygosity or heterogeneity.

Kits:

The present disclosure also describes kits comprising one or more polynucleotide standards disclosed herein. Alternatively or in addition, the kits may comprise one or more vectors disclosed herein, which vectors comprise one or more polynucleotide sequences encoding one or more standards disclosed herein. The kits may also comprise one or more components suitable for expressing the vectors in order to produce the polynucleotide standards. The kits may comprise both the polynucleotide standards disclosed herein and the vectors disclosed herein. The kits may also be provided with information describing the particular polynucleotide standard contained therein, such as (but not limited to) its sequence, concentration, structural genomic features of interest, etc. The kits may also comprise one or more artificial chromosomes disclosed herein.

The kits may comprise a mixture of any one or more of the polynucleotide standards and/or vectors disclosed herein, in any combination. The mixture of standards and/or vectors may be provided together, in a single buffer, which may be provided in one or more containers. Alternatively, the mixture of standards and/or vectors may be provided in the form of multiple, separate containers, each comprising a single standard and/or vector, or a single concentration of a standard and/or vector. The separate containers may be provided in association with each other as a kit.

The kits may further comprise the computer apparatus, computer programmable media, and/or the computer software disclosed herein. Thus, the kits may be provided as a package allowing the physical polynucleotide standards to be used experimentally and allowing the computer apparatus and software to be used to relate the experimentally derived sequencing information to the artificial chromosome.

Computer system and computer implemented method:

The present disclosure also describes a computer system and a computer implemented method. Figure 38 illustrates a suitable computer system 3800 for calibrating a polynucleotide sequencing process. The computer system 3800 comprises a processor 3802 connected to a program memory 3804, a data memory 3806, a communication port 3808 and a user port 3810. The program memory 3804 is a non-transitory computer readable medium, such as a hard drive, a solid state disk or CD-ROM. Software, that is, an executable program stored on program memory 3804 causes the processor 3802 to perform the method disclosed herein.

The processor 3802 may then store the calibrated results on data store 3806, such as on RAM or a processor register. Processor 3802 may also send the calibrated results via communication port 3808 to a server, such as sample sequence database or computer system that manages a polynucleotide sequencing experiment.

The processor 3802 may receive data, such as data indicative of a polynucleotide sequence, fragments of an artificial chromosome or sequences of the sample, from data memory 3806 as well as from the communications port 3808 and the user port 3810, which is connected to a display 3812 that shows a visual representation 3814 of the sequencing result to a user 3816. In one example, the processor 3802 receives sequence data from a sequencing device via communications port 3808, such as by using a Wi-Fi network according to IEEE 802.11. The Wi-Fi network may be a decentralised ad-hoc network, such that no dedicated management infrastructure, such as a router, is required or a centralised network with a router or access point managing the network.

Although communications port 3808 and user port 3810 are shown as distinct entities, it is to be understood that any kind of data port may be used to receive data, such as a network connection, a memory interface, a pin of the chip package of processor 3802, or logical ports, such as IP sockets or parameters of functions stored on program memory 3804 and executed by processor 3802. These parameters may be stored on data memory 3806 and may be handled by-value or by-reference, that is, as a pointer, in the source code.

The processor 3802 may receive data through all these interfaces, which includes memory access of volatile memory, such as cache or RAM, or non-volatile memory, such as an optical disk drive, hard disk drive, storage server or cloud storage. The computer system 3800 may further be implemented within a cloud computing environment, such as a managed group of interconnected servers hosting a dynamic number of virtual machines.

It is to be understood that any receiving step may be preceded by the processor 3802 determining or computing the data that is later received. For example, the processor 3802 may determine the sequence data of the artificial chromosome and may store the sequence data in data memory 3806, such as RAM or a processor register. The processor 3802 may then request the data from the data memory 3806, such as by providing a read signal together with a memory address. The data memory 3806 may provide the data as a voltage signal on a physical bit line and the processor 3802 may receive the sequence data of the artificial chromosome via a memory interface.

It is to be understood that throughout this disclosure unless stated otherwise, data may be represented by data structures, such as ["G","A","T","C"] strings or list of binary tuples encoding the nucleotides. The data structures can be physically stored on data memory 3806 or processed by processor 3802.

It should be understood that the techniques of the present disclosure might be implemented using a variety of technologies. For example, the methods described herein may be implemented by a series of computer executable instructions residing on a suitable computer readable medium. Suitable computer readable media may include volatile (e.g. RAM) and/or non-volatile (e.g. ROM, disk) memory, carrier waves and transmission media. Exemplary carrier waves may take the form of electrical, electromagnetic or optical signals conveying digital data steams along a local network or a publically accessible network such as the internet.

It should also be understood that, unless specifically stated otherwise as apparent from the following discussion, it is appreciated that throughout the description, discussions utilizing terms such as "processing" or "computing" or "calculating", or "determining" or "displaying" or "calibrating" or "normalizing" or the like, can refer to the action and processes of a computer system, or similar electronic computing device, that processes and transforms data represented as physical (electronic) quantities within the computer system's registers and memories into other data similarly represented as physical quantities within the computer system memories or registers or other such information storage, transmission or display devices.

The present disclosure is now described further in the following non-limiting examples.

**Example 1:**

One example of an artificial chromosome was prepared as follows. We retrieved a 5,000nt sequence from human chr7: 271,335,00 - 271,385,00 (hg19). This sequence overlaps a CpG island (a sequence containing a density of CpG dinucleotides) in the promoter of the *HOXA1* gene. To remove homology we shuffled the 5,000nt sequence whilst maintaining CG dinucleotide pairings with a shuffling window size of 50nt. This process is illustrated in Figure 2. Shuffling the primary DNA sequence within windows rearranges the sequence to remove homology, whilst maintaining genetic features at a resolution larger than the window size. Where required, additional nucleotide substitutions, insertions and deletions were manually created to remove homology to known natural sequences. The resultant shuffled sequence was compared to the Nucleotide collection (nr/nt) database using the BLASTn software program (Altschul, S.F. et al., J Mol Biol 215, 403-10 (1990)) to confirm the absence of any sequence with greater than 21nt contiguous homology with any known or natural sequence. This example method produced a 5,000nt sequence that has no homology to known or natural sequences, but retains the higher-order CpG island genetic feature at resolution of 50nt within the *HOXA1* promoter.

**Example 2:**

One example of an artificial gene sequence in an artificial chromosome was prepared as follows. We first retrieved a gene sequence from the human genome (hg19) that comprises 12 exons and 11 introns. Individual exon and intron sequences as well as upstream/downstream 1,000nt sequences were retrieved. Each gene exon and intron sequence was individually shuffled with a 20nt window size to remove homology as described in Example 1. Shuffled exon and intron sequences were then assembled within the artificial chromosome in the correct order, with the orientation and distribution retained as for the original gene within the human genome. This artificial gene is denoted *R_1_2_R* as shown in Figure 3. The nucleotides immediately flanking inserted exons was manually edited to insert canonical dinucleotide AG-CT splice sites and poly-pyrimidine track nucleotides. Thus, the artificial gene retains the higher-level genetic features of gene loci that are present in natural human genes, but retains no primary sequence homology with the original human gene or with any other known nucleotide sequence.

**Example 3:**

One example of the inclusion of multiple genes, with each gene comprising multiple isoforms, into an artificial chromosome was performed as follows. We first retrieved human mRNA isoform sequences from the GENCODE v19 basic gene assembly (Harrow, Denoeud et al. 2006). Isoforms were ranked by combined exon length, exon number and isoform number. Thirty genes comprising two or more alternate isoforms were systematically sampled from this list. These isoforms were curated to include different examples of alternative gene splicing, including exon exclusion, exon inclusion, alternative transcription initiation, alternative transcription termination, intron retention and alternative 3' and 5' splice site usage. Each gene exon and intron sequence from the human genome (hg19) was retrieved and individually shuffled as described above in Example 1 to remove homology. Each shuffled sequence was then reassembled in the artificial chromosome to maintain exon-intron structure but remove homology to natural sequences. Distance between inserted gene loci in the artificial chromosome was maintained as similar as possible to distances typically observed between genes in the human genome. By this process we incorporated 30 artificial gene loci in the artificial chromosome as illustrated in Figure 1.

**Example 4:**

One example of a mobile element for inclusion in an artificial chromosome was prepared as follows. We retrieved natural human DNA sequences for five instances of mobile elements from common repeat classes (*AluSx, MIRb, L2a etc.*) (A.F.A. Smit, R. Hubley & P. Green RepeatMasker at http://repeatmasker.org). Repeat sequences were shuffled and curated as described above in Example 1 to remove homology. Shuffled repeat sequences were duplicated to a sufficient number so as be inserted into an artificial chromosome at the same density as present in the human genome. For example, a 8Mb artificial chromosome sequence will have 788 *AluSx*, 534 *MIRb*, 433 *L2a*, 93 *MER5B* and 166 *L1M5* repeat mobile elements to match the density of analogous natural repeat elements in the human genome. Individual repeat elements were then subjected to random nucleotide substitutions, insertions, and deletions to cause sequence divergence of individual repeat mobile elements from ancestral sequence, as illustrated in Figure 4. Sequence and length divergence of shuffled repeat mobile elements can be designed to match the sequence and length divergence of analogous natural elements in the human genome. Shuffled repeat motifs were then inserted into an artificial chromosome sequence with same density and distribution as analogous natural mobile elements in the human genome, as illustrated in Figure 1.

One example of a centromere for inclusion in an artificial chromosome was prepared as follows. We retrieved a single 171nt tandem repeat DNA sequence from an individual *ALR/Alpha* centromere in the human genome (A.F.A. Smit, R. Hubley & P. Green RepeatMasker at http://repeatmasker.org). This natural 171nt tandem repeat DNA sequence was shuffled and curated to remove homology to natural sequences and forms the ancestral repeat. From this ancestral repeat we performed 4 consecutive rounds of 4-fold amplification followed by 14% sequence divergence by random nucleotide substitution, insertions, and deletion. This resulted in a formation of a 10,944 nucleotide long artificial centromere element with internal hierarchal repeat structure analogous to that of the original human sequence, but sharing no sequence identity with the original human sequence. The artificial centromere element was then inserted into a central region of a chromosome sequence, as illustrated in Figure 1.

One example of a telomere for inclusion in an artificial chromosome was prepared as follows. We manually generated an artificial 6-mer nucleotide ancestral repeat motif (ATTGGG), which we subjected to multiple rounds of amplification and simulated sequence divergence to generate two 10.9 and 8.3kb long artificial telomere sequences, which were then added to each terminal end of the artificial chromosome sequence, as illustrated in Figure 1.

**Example 5:**

One example of small-scale genetic variation for inclusion in an artificial chromosome was prepared as follows. A list of human small-scale variation, including SNPs, insertions, deletions, heterozygous, microsatellite and multiple nucleotide polymorphisms (Sherry, S.T. et al. Nucleic Acids Res 29, 308-11 (2001) was ranked according to mutation type, nucleotide content and size. A total of 512 small-scale variants were systematically sampled from this list. Selected small-scale variants was manually curated to ensure representation of a wide range of mutation type, nucleotide content and size. The DNA sequence of human small-scale variation along with upstream and downstream flanking 5 nucleotide sequences was retrieved from the human genome sequence (hg19). We then substituted 268 small-scale variations into two artificial chromosomes, thereby producing a pair of variant artificial chromosomes that incoporate homozygous variation relative to the original 'reference' artificial chromosome. We next substituted 289 small-scale variations into only one single artificial variant allele chromosome, thereby producing heterozygous variation relative to the original 'reference' artificial chromosome. By this process, we can represent homo- and heterozygous small-scale variation in artificial chromsomes.

**Example 6:**

One example of the incorporation of disease-specific, small-scale genetic variation into an artificial chromosome was performed as follows. The *BRAF* V600E mutation results in an amino acid substitution at position 600 in the *BRAF* protein from a valine (V) to a glutamic acid (E) and is found in ~85% of melanoma cases (Davies, H. et al. Nature 417, 949-54 (2002)). DNA sequences matching either the wild type (T) or disease-associated variant *BRAF* V600E mutation (A) and the flanking upstream and downstream 150 nucleotides were retrieved from the human genome (corresponding to chr7 : 140,452,986 - 140,453,286 in the hg19 assembly). The 6 upstream and downstream nucleotides to the *BRAF* V600E mutation were not shuffled. However, the remaining flanking sequence was shuffled in increasingly large window sizes with increasing distance from the site of the *BRAF* V600E variation, as illustrated in Figure 7. For example, the sequence was shuffled with 6nt window size when within 20nt distance of *BRAF* V600E variation, 10nt window size when within 100nt distance of *BRAF* V600E variation, and 20nt window size when greater than 100nt distance of *BRAF* V600E variation. This removed homology with known natural sequences across the entire gene sequence, but increased the window resolution of shuffling in close proximity to the variant. The shuffled sequence was then substituted into the 'reference' artificial chromosome to form an artificial variant chromosome carrying the *BRAF* V600E mutation.

In another example, the K562 cell line contains a frame shift nucleotide insertion at ch17 : 7578523 - 7578524 (hg19) in the *TP53* gene sequence (Law, J.C. et al., Leuk Res 17, 1045-50 (1993)). The DNA sequences matching either the reference (T) or disease-associated variant *TP53* Q136fs mutation (TG) and the flanking upstream and downstream 150 nucleotides were retrieved from the human genome (corresponding to chr17 : 7,578,374 - 7,578,674 in hg19 assembly). The 6 upstream and downstream nucleotides to the *TP53* Q136fs mutation were not shuffled, with the remaining sequence shuffled with increasing window size per distance from TP53 Q136fs as described above. This sequence was then substituted into the 'reference' artificial chromosome to form an artificial variant chromosome carrying the *TP53* Q136fs mutation.

**Example 7:**

One example of the incorporation of large-scale genetic variation (>50nt) into an artificial chromosome was performed as follows. A catalogue of human large-scale variation (Sherry, Ward et al. 2001, MacDonald, Ziman et al. 2014) was ranked according to mutation type, nucleotide content and size. A total of 12 examples of large-scale variation were systematically sampled from the list of human large-scale variation and manually curated to ensure full representation of the diverse range of different types of large-scale variation, including large deletions, insertions, inversions (transversions), copy number variation and mobile-element insertions. The sequence of the structural variation, with an additional 1,000 nucleotide of flanking upstream and downstream sequence, was shuffled and curated to remove homology to known natural sequences, as previously described for Example 1. Notably, where possible shuffling was performed with respect to any internal structure (such as repeat or inverted units) of the large-scale variation where possible to maintain the internal hierarchy, as previously described in Example 4. These instances of structural variation are then inserted into the artificial chromosome sequence to produce a variant artificial chromosome. In this manner, we inserted 12 examples of large-scale structural variation of four different types within the artificial chromosome, as illustrated in Figure 12. A range of genotypes (homozygous and heterozygous) for structural variation can be established by the use of multiple variant artificial chromosomes with respect to the 'reference' artificial chromosome as described by the method in Example 6 above.

In another example, we incorporated DNA repeats that vary in copy number between multiple artificial chromosome as follows. We retrieved the DNA sequence for a single *D4Z4* repeat copy from the human genome (hg19) and shuffled with a window size matching the repeat copy size to remove homology to known natural sequences, as illustrated in Figure 33. The shuffled *D4Z4* repeat copy is then replicated and organized in a head-to-tail orientation to form arrays of 10, 20, 50, 100 and 200 shuffled *D4Z4* repeat copies. These repeat copy numbers encompasses the majority (99%) of observed *D4Z4* copy number in human subjects (Schaap, Lemmers et al. 2013). This includes copy number at 10 copies (exhibited by 95% of FSMD patients, 20 copies (high-risk individuals), 50 copies (for related individuals) and more than 100 copies (for unaffected individuals) (van der Maarel and Frants 2005). Each repeat arrays was then incorporated into artificial chromosomes, thereby producing a range of different genotypes that vary in artificial *D4Z4* repeat copy numbers.

**Example 8:**

One example of the formation of a fusion gene by translocation between two artificial chromosomes was performed as follows. We first produced two artificial chromosomes encoding two artificial genes, *B1* and an *A1* gene, using methods previously described in Example 2. The exon/intron structure of A1 and B1 genes was derived from the human *ABL1* and *BCR* genes respectively. The *B1* gene comprises 23 exons / 21 introns on artificial chromosome A and sequences representing the *A1* gene comprising 11 exons were generated on artificial chromosome B, as illustrated in Figure 9. The exon/intron structure of the genes was maintained within each artificial chromosome, but DNA sequences were shuffled to remove homology by methods described in Example 1 above. The artificial chromosome A and B sequence was then rearranged by a translocation (i) after exon 4 in the *B1* gene and (ii) before exon 2 in the *A1* gene, thereby generated a fusion gene comprising *B1* exons 1 to 13 and *A1* exons 2 to 11 on artificial chromosome A and a fusion gene matching *A1* exons 1 and *B1* exons 14 to 22 on artificial chromosome B, as illustrated in Figure 9. By this process, we performed a translocation of two artificial chromosomes to form a fusion gene event.

**Example 9:**

One example of the use of the artificial chromosomes disclosed herein to simulate microbe genome communities was performed as follows. Environmental DNA samples often contain a complex community of multiple microbe genomes. Here, we simulated a complex community of multiple artificial chromosomes representing microbe genomes (referred to herein as "artificial microbe genomes") of differing types, sizes, and abundance. Firstly, we retrieved high quality draft genome sequences (Chan, P.P., et al., Nucleic Acids Res 40, D646-52 (2012)) for total of 30 microbes. Selected microbe genomes were manually curated to ensure representation of wide range of taxa (including both archeae and bacteria), size (0.5-10Mbp), GC content (27-70%), rRNA operon count (1-10), and isolation from a diverse range of environments (human body, aquatic, terrestrial and extreme physical or chemical conditions). The selection (shown in Table 9) is aimed to represent the phylogenetic and genomic heterogeneity often encountered in a complex microbial population within an environmental DNA sample. Genome sequences were shuffled and manipulated to remove sequences with any sequence homology to known natural sequences. By this process, we produced a library of 30 artificial microbe genomes.

Another example of incorporating 16S rRNA genes into microbe genomes was performed. We retrieved the *16S rRNA* sequences corresponding to the 30 microbe genome sequences, as indicated in Table 9, from which artificial microbe genomes were previously produced using methods described above. *16S rRNA* sequences were shuffled and manually edited to remove homology to known natural sequences as previously described in Example 1. However, sequences required for the universal 16S primers (forward primer: CTACGGGAGGCAGCAG and reverse primer: GA CTACCAGGGTATCTAATCC) are retained. These primer sequences flanking approximately 460nt of shuffled sequence corresponding to the V3 region within the 16S rRNA gene, as illustrated in Figure 11. This intervening shuffled V3 sequence comprises an artificial marker with no homology to known natural sequences that will be amplified using universal 16S primers in a polymerase chain reaction. The synthetic marker 16S rRNA genes are assembled into the artificial microbe genome sequence with a frequency respecting the operon count (1-10) of the original microbe from which the microbe genome sequence was derived.

**Example 10:**

One example of the simulation of mammalian immunoglobulin sequence diversity using the artificial chromosomes disclosed herein was performed. The generation of artificial immune repertoire sequences allows the use of nucleotide standards to assess the accuracy and quantification of clonotypes during immune repertoire sequencing. We produced a $TCR\beta$ locus on an artificial chromosome and modelled the process of V(D)J recombination to produce a suite of artificial $TCR\beta$ clonotypes. Firstly, we retrieved the $TCR\beta$ gene sequence (which comprises 65 V$\beta$ segments, 2 D$\beta$ segments and 13 J$\beta$ segments) from the human genome (hg19). Each segment or intronic sequence was separately shuffled to remove homology to known natural sequences, with the exception of sequences complementary to primer sequences used in the BIOMED-2 study (van Dongen, J.J. et al. Leukemia 17, 2257-317 (2003)). Shuffled segments and flanking intronic sequences were then re-assembled to incorporate a $TCR\beta$ loci on the artificial chromosome, as illustrated in Figure 13.

The artificial $TCR\beta$ loci then underwent a simplified simulation of the biological processes that occur during T-cell differentiation of V(D)J recombination and somatic hypermutation to produce a $TCR\beta$ clone as follows. V(D)J recombination was simulated by the selection and joining of the $V\beta$, $D\beta$ and $J\beta$ segments corresponded to randomly selected $TCR\beta$ clonotypes previously identified within adult healthy males (Zvyagin, I.V. et al. Proc Natl Acad Sci U S A 111, 5980-5 (2014)). Somatic hypermutation was simulated by the insertion or deletion of nucleotides at junctions at a frequency based on randomly selected insertions and deletions in TCRβ clonotypes observed in adult healthy males (Zvyagin, I.V. et al. Proc Natl Acad Sci U S A 111, 5980-5 (2014)). Following this procedure, we produced 15 artificial $TCR\beta$ clonotypes.

In another example, we generated a $TCR\gamma$ locus on an artificial chromosome and modelled the VJ recombination to produce a suite of artificial $TCR\gamma$ clonotypes. We firstly retrieved 10 Vγ segments, 5 Jγ segments and 2 Cγ segments and flanking intronic sequence from human genome (hg19). Each segment or intronic sequence was separately shuffled to remove homology to known natural sequences with the exception of sequences complementary to primer sequences used in the BIOMED-2 study (van Dongen, Langerak et al. 2003). Shuffled sequences and flanking intronic sequences were re-assembled to form an artificial $TCR\gamma$ loci, as illustrated in Figure 12. We next modelled the diversification processes of VγJγ somatic recombination that occurs during T-cell differentiation.by randomly selecting and joining artificial Vγ segment and a Jγ segment to generate a range of TCRγ clonotypes. For example, we joined Vγ4 segment to Jγ1 segment to form a Vγ4Jγ1 clone (SEQ ID NO: 203). Following this procedure, we generated 15 artificial $TCRG$ VγJγ clones (SEQ ID NOs: 203-219).

**Example 11:**

One example of an RNA standard sequence that represents the *R_1_2_R* gene in the artificial chromosome was performed. The *R_1_2_R* gene locus was incorporated into the artificial chromosome using methods described in Example 2. The 13-exon sequences of the *R_1_2_R* gene was then joined together to form a continuous 1,310nt sequence (SEQ ID NO: 3), whilst the intervening 12 intronic sequences were removed, as illustrated in Figure 3. An additional ~100 nucleotide poly-adenine tract was added to the 3' end of the *R_1_2_R* mRNA sequence. The performance of RNA standards representing *R_1_2_R* standard using simulated sequenced reads was assessed. The Sherman software was used to simulate 1,000 paired-end 125-nt reads from the *R_1_2_R* sequence (SEQ ID NO: 3). We then aligned simulated reads to the artificial chromosome using the Tophat2 software (Kim, Pertea et al. 2013) with the following parameters:

>tophat2 cht_index simulated_reads.R1.fq simulated_reads.R1.fq

We found that all 1,000 reads aligned uniquely and correctly to the *R_1_2_R* gene. We found that simulated reads were correctly split and aligned across all 12 introns and 13, confirming the utility of the *R_1_2_R* standard.

**Example 12:**

One example of an RNA standard that represents an alternatively spliced mRNA isoform of the artificial *R 1_2* gene was performed. The *R_1_2_V* sequence comprises an alternatively spliced isoform to the *R_1_2_R* sequence included in the artificial chromosome, and described in Example 11 above. The *R_1_2_V* isoform sequence comprises the 12 exons that form a contiguous 1,310 nt sequence (SEQ ID NO: 4), whilst the intervening 11 intronic sequences are removed. Note that the *R_1_2 V* standard sequence has 11 exons in common with the alternative isoform *R_1_2_R* standard, as illustrated in Figure 3. However, it is missing an exon (4) and contains and additional two exons (5 and 6). Therefore, comparing the *R_1_2_R* and *R_1_2_V* RNA standards models the exclusion of exon 4 and inclusion of exon 5 and 6 by alternative splicing of the *R_1_2* artificial gene.

**Example 13:**

One example of the manufacture of an RNA standard was performed in order to produce an RNA standard representing the mature mRNA sequence of the *R_1_2_R* gene. The *R_1_2_R* sequence (SEQ ID NO: 3) was first synthesized as a DNA molecule using a commercially available service (ThermoFisher GeneArt). The sequence was inserted into a pMA expression plasmid in the following order of elements: (i) a SP6 promoter (ii) *R 1_2_R* gene sequence (iii) ~50 nucleotide poly-adenine sequence and (iv) *EcoR1* restriction site, as illustrated in Figure 14. The plasmid was transformed and cultured with *E.coli.* The plasmid was purified using QIAprep Spin Midiprep (Cat#12945). Plasmid clones were Sanger sequenced to confirm the accuracy, insertion and orientation of the above sequence elements. The plasmid was then linearized by digestion with *EcoR1* restriction endonuclease. Next, the plasmid was used as a template for an *in vitro* RNA synthesis reaction to generate a synthesized RNA polynucleotide standard that was then purified with a QIAquick column (QIAGEN). An aliquot of RNA standards was analyzed using BioAnalyzer RNA Chip (Agilent) to confirm the expected full-length transcription and concentration. The purified RNA standard was then diluted to a required concentration.

**Example 14:**

One example method to produce different mixtures of multiple RNA standards was performed. We firstly manufactured RNA standards representing the 30 genes encoded in the artificial chromosome as described in Example 11 and 13 above. We divided 30 RNA standards into 10 groups (with each group consisted of 3 RNA standards) as indicated in Table 1. We performed a 3-fold serial titration between the 10 groups, covering a $10^6$-fold range in abundance between lowest and highest group. The 30 RNA standards at different relative abundance were then combined to form a mixture. Therefore, the mixture comprises 30 different RNA standards at a sequential range of different concentrations that comprise a quantitative scale or ladder of RNA abundance. This collection of RNA standards was called Mixture A.

We next assembled the same 30 RNA Standards with a different range of abundances to form a different mixture we call Mixture B, as indicated in Table 1. The abundance of the RNA standards in Mixture B is such that a pairwise comparison between the abundance of RNA standards indicates 0, 2-fold or 4-fold increases or decreases in the abundance of RNA standards between Mixture A and Mixture B. This differential change in RNA standard abundance is similar to a natural gene population, and can be used to emulate changes in gene expression.

**Example 15:**

One example method to produce different mixtures of multiple alternatively spliced RNA standards was performed. We firstly manufactured 60 RNA standards (SEQ ID NOs: 1-62) using methods described in Example 13. RNA standards were organised as pairs comprising two alternative isoforms that share and differ in exon sequence content to each other, as described in Example 12 above.

We combined the 30 pairs of RNA standards into two alternative 3-fold serial dilutions to form Mixture A and B, such that pairwise comparison of abundance between alternative isoform RNA standards corresponded to a 1-, 2- and 3-fold change (indicated in Table 1). For example, we added *R_1_2_R* at 15,000 attomoles/ul and *R_1_2_V* at 5,000 attomoles/ul in Mixture A, and we added *R_1_2_R* at 1,250 attomoles/ul and R_1_2_V at 3,750 attomoles/ul in Mixture B. This corresponds to a 4-fold change in *R 1_2* gene expression between Mixture A and B, and also a 3-fold change in the relative concentration between individual *R_1_2_R* and *R_1_2_V* isoforms, thereby emulating the alternative splicing of the *R 1_2* gene. Differences in isoform abundance between mixtures can be compared to the alternative splicing of natural gene populations.

**Example 16:**

One example of RNA standards to represent a fusion gene was performed as follows. RNA standards were the manufactrured to match the (i) *B1* gene sequence (SEQ ID NO: 136) (ii) *A1* gene sequence (SEQ ID NO: 135) and (iii) *B1fA1* gene matching *B1* exons 1 to 13 sequence and *A1* exons 2 to 11 sequence (SEQ ID NO: 137). RNA standards were manufactured using methods previously described in Example 13.

**Example 17:**

One example of the manufacture of a DNA standard was performed in order to represent the artificial chromosome sequence between 6,974,486 - 6,975,593 nucleotides. The 1,122nt DNA standard sequence (SEQ ID NO: 63) and two flanking *Sap1* restriction sites (GCTCTTC) was first synthesized into a DNA molecule with commercially available service (ThermoFisher GeneArt). The sequence was then cloned into a high copy plasmid (pMA), as illustrated in Figure 14. Each plasmid is grown in *E.coli* culture and prepared using QIAprep Spin Midiprep (Cat#12945). DNA plasmids are purified using QIAquick column (QIAGEN) and diluted to a standard concentration to comprise stock. Plasmid clones are Sangersequenced to confirm the correct sequence and insertion into plasmid. The stock plasmid was used as a template for either DNA standard synthesis by PCR (using primers pairs at the termini of the *D_1_1_R* sequence are used to amplify the DNA standard) or restriction digest (*Sap1* restriction endonuclease cleaves 5/6nt downstream to the flanking *Sap1* site and can be used to excise the *D_1_1_R* standard DNA molecule without leaving addition nucleotides at terminus after cleavage). Following synthesis, an aliquot of the *D_1_1_R* standard is analyzed on an Agilent 21000 Bioanalyser to confirm the expected full-length size and concentration of the standard. Purified DNA standard is then diluted to required concentration.

**Example 18:**

One example method to produce different mixtures of multiple DNA standards was performed. We manufactured 30 DNA standards matching the artificial chromosome sequence, using the methods described in Example 17 above. The DNA standards were divided into 10 groups, each consisting of 3 DNA standards. We assembled a 3-fold serial dilution for each group (ie. three DNA standards have the same concentration), thereby covering a $10^6$-fold range in concentration between lowest and highest group of DNA standards (indicated in Table 5). The combination of DNA standards across this range of concentrations is termed Mixture A. This mixture thereby provides a quantitative scale or ladder of DNA abundance. We next assembled the same 30 DNA Standards at a different range of concentrations to form an alternative Mixture B, as indicated in Table 5. The abundance of each DNA standards in Mixture B is such that a pairwise comparison between the abundance of DNA standards indicates 0, 2-fold or 4-fold increases or decreases in the abundance of DNA standards between Mixture A and Mixture B. This change in DNA standard abundance between mixtures is similar to a natural DNA sequences and comprises a quantitative scale or ladder by which to measure fold changes in DNA abundance.

**Example 19:**

One example method of joining multiple DNA standards to produce a single, larger or 'conjoined' DNA standard was performed. A conjoined DNA standard is comprised of multiple individual DNA standards produced using methods described in Example 17 above. For example, a conjoined DNA standard A is comprised of 1 copy *D_1_1_R*; 2 copies *D_1_2_R*; 3 copies of *D_1_3_R*, 4 copies of *D_1_4_R*; 5 copies of *D_1_5 R*; 6 copies of *D_1_6_R*. Also note that by varying the copy number between 1 (*D_1_1_R*) and 6 (*D_1_6_R*) corresponds to a 6-fold increase in abundance between individual *D_1_1_R* and *D_1_6_R* standards, as illustrated in Figure 16. We organised 15 conjoined DNA standards (A-O) assembled from total 90 individual DNA standards using this approach, as indicated in Table 7. Therefore, each conjoined DNA standard comprises 6 individual DNA standards at 1- to 6-fold relative copy number.

Individual DNA standards were assembled into conjoined DNA standards at different copy numbers (1 copy *D_1_1_R*; 2 copies *D_1_2_R*; 3 copies of *D_1_3_R* ) as follows. Individual DNA standards were first cloned into a pUC19 vector. PCR amplification was performed using oligonucleotide primers with a 20-bp overlap at the junctions regions. Resultant PCR amplicons were ligated together using the Gibson Assembly Master Mix (New England BioLabs, Ipswich, MA) according to manufacturer's instructions. Briefly, a 6-fragment Gibson assembly was set up with 0.062pmol of vector fragment, 0.187 pmol of five of the insert fragments and 10ul of Gibson Assembly Master Mix (2x) to a final volume of 20ul. The final Gibson assembly was incubated at 50°C for 2hrs. Following incubation, samples were stored at -20°C for subsequent transformation and plasmid purification. Sanger-sequencing was used to confirm conjoined DNA standard insert sequence.

Conjoined DNA standards are titrated at increasing relative concentrations and combined to produce a Mixture C which encompasses a 15-fold increase in abundance, as indicated in Table 7.

## Example 20:

One example of DNA standards that represent genetic variation between artificial chromosomes was performed. Genetic variation can be incorporated between artificial chromosomes, as previously described in Example 5. We manufactured 32 pairs of DNA standards (SEQ ID NOs: 63-134) that match regions of the artificial chromosome sequences of equal length (1000nt), by the methods described in Example 17 above. Each pair comprises two DNA standards that match either 'reference' chromosomes (denoted_R) or variant artificial chromosomes (denoted _V). For example, we produced a DNA standard pairs; one DNA standard matching the variant allele (termed _D_1_1_V_ ; SEQ ID NO: 64) and the other DNA standard matching the reference _D_1_1_R_ standard (SEQ ID NO: 63) described in Example 20 above. The _D_1_1_V_ standard sequence differs from the _D_1_1_R_ standard sequence at 7 sites comprising 4 SNPs, a 12nt deletion, a 6nt insertion and a 33nt deletion, as illustrated in Figure 6. Where possible, 200nt sequence flanking upstream and downstream to sites of variation was also in the DNA sequence to minimize the impact of sequencing edge effects. In total, 30 DNA standards pairs contain 252 SNPS, insertions or deletions less than 50nt (between 5-8 SNPS, insertions or deletions per DNA standard) were manufactured using the methods described in as described in Example 17 above.

## Example 21:

One example method to produce different mixtures of DNA standards representing genetic variation. We can represent different polyploid genotypes by varying the relative abundance of DNA standard pairs that represent genetic variation, as described in Example 20. First the 30 DNA standard pairs are added at different abundances to form Mixture A, as indicated in **Table 5,** such that a pairwise comparison between DNA standard pairs indicates an total variant, equal, 3-fold, 9-fold, and 30-fold change in relative abundance between variant and reference DNA standards. This varying relative abundance between variant and reference DNA standards enables modelling of homozygous, heterozygous, and heterogeneous variation in a polyploid genome. For example, equal concentrations of DNA standards representing the reference and variant artificial chromosomes would represent a heterozygous genotype in a diploid organism such as human. The different relative concentration of DNA standards can establish a scale or ladder for measuring quantitative differences. We next assembled the same 30 DNA Standards pairs with a different range of abundances to form a different mixture we call Mixture B, as indicated in Table 5. The abundance of the DNA standards in Mixture B is such that a pairwise comparison between the relative abundance of reference and variant DNA standards indicates a range of fold-changes in the abundance of genetic variation between Mixture A and Mixture B. This differential change in the variant abundance is similar to changing allele frequencies between DNA samples.

## Example 22:

One example of DNA standards to represent specific disease-associated genetic variation was performed. We produced two DNA standards corresponding to the reference and variant artificial chromosomes previously described in Example 6. Therefore, the reference DNA standard matched the reference sequence (T for Q139fs and T for V600E; SEQ ID NO: 138) and the variant DNA standard matched disease-associated genetic variation (TG for Q139fs and A for V600E; SEQ ID NO: 139). DNA standards were manufactured as previously described in Example 17.

DNA standards were combined with equal abundance to thereby emulate a heterozygous genotype carrying single _TP53_ Q136fs and _BRAF_ V600E mutation and single wildtype alleles. We generated a serial dilution of variant DNA standards by 10-fold serial dilution in relation to the reference DNA standards as described in the Example 21 above. This can emulate a heterogeneous allele frequency where an increasingly small sub-population of DNA sample harbors a variant allele.

We performed next-generation sequencing (Illumina HiSeq 4000) on libraries containing different mixtures of reference and variant (containing mutations) DNA standards. We then analysed sequenced reads as follows: 1. We aligned sequenced reads to the human genome using BWA; 2. We processed the alignment using Picard tools; 3. We identified variants using the Genome Analysis Tool Kit (GATK). We identified both mutations (results taken from example output .vcf file from heterozygous mixture):

<u>p53 Frameshift Mutation</u>

B5_R 300 . T TG 962.73 . \

AC=1;AF=0.500;AN=2;BaseQRankSum=1.780;ClippingRankSum=0.008; \

DP=60;FS=2.250;MLEAC=1;MLEAF=0.500;MQ=60.00;MQ0=0; \

MQRankSum=0.472;QD=16.05;ReadPosRankSum=-0.008;SOR=0.430 \

GT:AD:DP:GQ:PL 0/1:24,32:56:99:1000,0,677 (GT 0/1 indicating a heterozygous allele, the 0 being the reference allele and the 1 being the variant allele)

BRAF V600E Mutation

B5_R 602 . T A 130.77 . \

AC=1;AF=0.500;AN=2;BaseQRankSum=0.306;ClippingRankSum=0.184; \

DP=15;FS=0.000;MLEAC=1;MLEAF=0.500;MQ=60.00;MQ0=0; \

MQRankSum=-0.429;QD=8.72;ReadPosRankSum=0.184;SOR=1.022 \

GT:AD:DP:GQ:PL 0/1:10,5:15:99:159,0,364

This example demonstrates the identification of clinically-important mutations represented on synthetic DNA standards at different homozygous, heterozygous and lower mutant allele frequencies. This provides an example whereby the mixture of the standards has been used to represent a heterozygous allele in a diploid human genome. The mutation modelled here (the BRAF V600E mutation) is of significant clinical relevance, demonstrating the value of the present calibration methods to the field of clinical diagnostics.

**Example 23:**

One example of DNA standards to represent large-scale genetic variation was performed. We manufactured DNA standards overlapping 12 examples of structural variation previously incorporated into the artificial chromosome, as described in Example 7. For each DNA standard, at least 600nt of upstream and downstream flanking sequence was included to prevent end-effects that may impact sequencing and assembly. DNA standards pairs are manufactured as previously described in Example 17, and can be combined at different relative abundance to from a mixture that models different genotypes using the method described in Example 21.

**Example 23.1:**

One example of DNA standards to represent copy-number variation was performed. We produced six DNA standards (SEQ ID NO: 167-172) overlapping the artificial *D4Z4* repeat array incorporated into artificial chromosomes in Example 7 above. Each DNA standards is a total 1,600nt in length and comprises (i) a single D4Z4 repeat copy approximately 800nt long (ii) 400nt upstream sequence matching half repeat copy (iii) 400nt downstream sequence matching half repeat copy, as illustrated in Figure 33. To distinguish between each DNA standard, we included one of six 'barcode' nucleotide sequences (AGCTA, CGATC, CACTG, TCAGC, TAGAC, and GCAGT) into the DNA sequence. Note that each sequence is only present on one DNA standard, and not on the other 5 DNA standards. Barcode nucleotides have an intervening distance of 40nt within the DNA standard sequence, so that each 100nt window will always contain at least 2 instances of the barcode sequences, as illustrated in Figure 17.

Each DNA standard was manufactured using the method described in Example 17, and DNA standards were titrated at the following relative concentrations; 10-fold, 13-fold, 50-fold and 150-fold as illustrated in Figure 33. This encompasses the majority of observed *D4Z4* copy number in human subjects(Schaap, Lemmers et al. 2013), from 10 copies exhibited by 95% of FSMD patients, to more than 100 copies for unaffected individuals(van der Maarel and Frants 2005). This process produced a mixture of DNA standards that represent different copy-numbers for a repetitive DNA sequence.

**Example 24:**

One example of DNA standards to represent microbe genome communities was performed. We produced 12 DNA standards (SEQ ID NO: 149-160) that match selected sequences within the artificial microbe genomes assembled in Example 9. Microbe genome sequences were selected such that the length and GC% of the DNA standards is proportional to the length and GC% of the artificial microbe genome, and therefore representative. This is indicated in Table 9 and illustrated in Figure 10. For example, the artificial *'Enterococcus faecal*-like' genome is 3.2Mb and has an average 38% GC content. By comparison the representative DNA standard MG 1 (SEQ ID NO: 149) matching the *'E.faecalis*-like' genome has a 2.2kb length (6.875% of the full genome length) and 38% GC content, thereby proportionately representing the length and GC content of the *'Efaecalis*-like' genome. DNA standards were manufactured as described previously in Example 17. The 12 DNA standards were organised into 4 groups, with each group combined at a 10-fold serial dilution of concentrations to form a mixture that that encompasses a $10^4$ fold-range in concentration.

**Example 25:**

One example of DNA standards to represent mammalian immunoglobulin sequence diversity was performed. We produced 15 DNA standards of 750nt length that matched the artificial $TCR\beta$ VDJ clonotypes sequences, produced using methods described in Example 10. DNA standards overlap the sequences complementary the BIOMED-2 primers, as well as the intervening V, J and D segments, as illustrated in Figure 13. DNA standards were manufactured as previously described in Example 17. DNA standards are organised into 5 groups (i.e., 3 standards per group), with each group combined at a 10-fold serial dilution of concentrations to form a mixture that that encompasses a $10^5$ fold-range in concentrations. This dynamic range spans human clonotype distribution profiles observed in healthy samples(Zvyagin, Pogorelyy et al. 2014) and also disease conditions such as minimal residual disease(Logan, Gao et al. 2011).

In another example, DNA standards were produced to represent the artificial *TCRG* VJ clonotype sequences described in Example 10. We produced 15 DNA standards (SEQ ID NOS: 186-202) of 750nt length that matched the artificial *TCRG* VyJy clonotype sequences produced in Example 10. DNA standards overlap the sequences complementary to the BIOMED-2 primers, as well as the intervening V and J segments, as illustrated in Figure 12. DNA standards were manufactured as previously described in Example 17, and combined to form a mixture as described above.

**Example 26:**

One example method of adding RNA standards to natural RNA sample for sequencing was performed. Firstly, K562 cells were cultured according to Coriell Cell Repositories growth protocols and standards. Briefly, K562 cells were cultured in RPMI 1640 medium (Gibco®) supplemented with 10% fetal bovine serum (FBS) at 37°C under 5% CO2. Total RNA was extracted from K562 cells using TRIzol (Invitrogen) according to the manufacturer's instruction. DNase treatment was subsequently performed on each sample with TURBO DNase (Life Technologies) followed by a clean-up with the RNA Clean and Concentrator Kit (Zymo Research). Total RNA was run on a BioAnalyzer to check for integrity and to determine the concentration. Only RNA with a RNA integrity number (RIN) >9.5 were used for library preparation.

RNA Standards were combined as Mixture A as previously described in Example 14 and Table 1. RNA Mixture A was then added to ~1% total volume with K562 total RNA (as measured with NanoDrop, ThermoScientific). The TruSeq Stranded Total RNA Sample Prep Kit (Illumina) was used to prepare RNA libraries according to manufacturer's instructions. Prepared libraries were quantified on Qubit (Invitrogen) and verified on Agilent 2100 Bioanalyzer (Agilent Technologies) before samples were pooled for sequencing. Sequencing is performed using a HiSeq 2500 insutrment (Illumine) with 125nt paired-end sequence reads.

**Example 27:**

One example method of assessing the alignment and assembly of RNA standards was performed. We produced RNA standards matching 30 genes comprising 2 alternative isoforms (60 RNA standards in total) using methods as described in Example 11 and 13 above. We diluted RNA standards to equal abundance and combined in equal proportion to form equal parts of Mixture C. The TruSeq Stranded Total RNA Sample Prep Kit (Illumina) was then used to prepare libraries directly from the RNA standards Mixture C according to manufacturer's instructions. Prepared libraries were quantified on Qubit (Invitrogen) and verified on Agilent 2100 Bioanalyzer (Agilent Technologies) before samples were sequenced with 125nt paired-end reads on a HiSeq 2500 (Illumina) instrument. The sequence read (.fastq) file was processed using methods described in Example 28. We then aligned sequence reads to the artificial chromosome (chrT) using Tophat2 with the following parameters:

>tophat2 chrT _index MixtureC.R1.fq MixtureC.R2.fq

From the resultant alignment (.bam) file, we determined the alignment statistics (for both total and split alignments) using methods described in Example 28. Notably, all RNA standards were of sufficient abundance such that they achieved full sequence read fold coverage, and this therefore enables an assessment of alignment when sequence fold-coverage is non-limiting. These results are summarised in Table 2. Specifically, we determine 98% sensitivity for total read alignments, and 0.99% sensitivity for spliced read alignments from RNA standards Mixture C. Furthermore, we assembled all gene structures with the exception of 18 introns and 16 exons missed, thereby confirming the performance of RNA standards matching gene loci (and isoforms) encoded on the artificial chromosome.

For comparison, we also simulated sequenced reads that would be generated from sequencing the same 60 RNA standards described above. Comparison of simulated reads to those experimentally-derived reads produced from the RNA standards as described above can distinguish the impact of variables due to alignment and assembly (that will influence both simulated and experimentally-derived reads) from variables due to library preparation and sequencing (that will influence only experimentally-derived reads, and not simulated reads).

We used RNASeqReadSimulator (http://alumni.cs.ucr.edu/~liw/rnaseqreadsimulator.html) software to simulate 125-nt paired-end reads generated from RNA standards that incorporate a 1% error rate that has been typically reported for Illumina sequencing technology (Bolotin, Mamedov et al. 2012). This generates a .fastq file as per standard sequencing on the HiSeq 2500 instrument. Sequence read file was processed and aligned as above and alignment statistics (for both total and split alignments) were determined using methods described in Example 28. Results are summarised in Table 2. Specifically, we observe a 98% sensitivity for alignment, and 99% sensitivity for spliced alignments, while missing 6 introns and 8 exons from final assembly.

Comparison of alignment and assembly outcomes for gene loci with simulated and experimentally-derived sequenced reads validate the use of RNA standards in sequencing experiments. Notably, simulated reads sufficiently recapitulate the performance of experimentally-derived sequenced reads for the alignment and assembly of RNA standards, indicating their utility in designing, modelling and analysing RNA standards matching transcribed features of artificial chromosomes.

## Example 28:

One example method of aligning reads constituting RNA standards and natural RNA sample library to artificial chromosome and natural reference genome was performed. Sequence files (.fastq) produced using method described in Example 26 were subject to de-multiplexing. Low-quality reads and sequences or adaptor contaminant sequences were removed from sequence files using trim_galore according to manufacturer's instructions: (http://www.bioinformatics.babraham.ac.uk/projects/trim_galore/).

The human genome (hg19) sequence was concatenated with the artificial chromosome (chrT) sequence to form a single file (.fasta). We then used bowtie-build to generate an index file (hg19_chrT_index.*) from the combined sequence file according to manufacturer's instructions (Langmead and Salzberg 2012). We next aligned sequenced reads (.fastq) to the index file (hg19_chrT_index.*) using Tophat2 (Kim, Pertea et al. 2013) with the following parameters:
>tophat2 hg19_chrT_index ./K562.R1.fq ./K562.R2.fq

This approach does not incorporate previous gene annotations to guide alignment, and is often required for discovery of new genes and *de novo* assembly of transcripts. We next assessed the alignments of sequenced reads to the artificial chromosome and natural genome according to a number of metrics described below and summarised in Table 2. Reads to Genome/Artificial Chromosome is determined by the number of reads that align to the artificial chromosome (Reads To ChrT) and the human genome (Reads to Hg19). For K562, we aligned 1,091,683 reads to the *artificial* chromosome and 65,778,796 reads to the human genome sequence.

Fraction Dilution is calculated from the fraction of reads aligning to the *artificial* chromosome relative to the genome indicates the dilution of the standards relative to the sample library. For K562 sample, 1.63% of library aligns to the *artificial* chromosome, indicating a 61-fold dilution factor.

Alignment Sensitivity is defined as the number of artificial gene bases of the gene loci encoded on the artificial chromosome with alignments (true positive) divided by the total number of artificial gene bases. For K562 sample 1, we observe an alignment sensitivity of 0.81
Alignment Specificity is defined as the number of artificial gene bases with alignments divided by the total number of bases with alignments. For K562 sample 1, we observe an alignment specificity of 0.83.

Spliced Alignment Sensitivity is defined as the number of artificial gene introns with correct split alignments divided by the total number of artificial gene introns. For K562 samples, the alignment sensitivity of 0.86, and is illustrated in Figure 22A.

Spliced Alignment Specificity is defined as the number of artificial gene introns matching split alignments divided by the number unique split alignments. For K562 samples, we observe an alignment specificity of 0.85.

Detection Limit corresponds to the highest abundance RNA standard that is not reliably detected within the sequenced library and is without overlapping alignments, and is illustrated in Figure 24D. We determine a lower limit of detection at 0.005 attamoles/ul (the highest abundance RNA standard *R_8_2* (SEQ ID NOs: 47, 48) not detected multiplied by dilution factor). Isoforms within the corresponding K562 RNA sample that are below this concentration may not be represented or detected within the sequencing library, and library sequencing has not proceeded to total saturation.

**Example 29:**

One example method of assembling reads from RNA standards into artificial genes was performed. Alignment files (.bam) generated by method described in from Example 28 were assembled into full-length transcript structures using Cufflink2 (Trapnell, Williams et al. 2010) according to default parameters:
>cufflinks K562_1 _mixA.bam

We assembled 108 transcript structures on the artificial chromosome, with an example illustrated in Figure 23. Note that this is higher than the number of RNA standards (60) due to the partial assembly of some RNA standards as multiple fragmented structures.

To assess assembly performance, we used Cuffcompare (Trapnell, Williams et al. 2010) according to default parameters to compare assembled transcripts relative to known transcript annotations on the artificial chromosome. We assessed transcript assembly according to the sensitivity and specificity of assembly relative to artificial gene structure at all levels (nucleotide, exon, intron, transcript, gene) and the fraction of artificial exons, introns and genes missing from the assembly. Further detail on the measures of sensitivity and specificity in relation to gene structures are described previously (Burset and Guigo 1996). The results for the assembly of RNA standards when combined with the K562 RNA sample in the present example are summarized, in Table 2. Notably, these measures based on gene assembly on artificial chromosome inform an assessment of matched *de novo* assembly of transcripts in accompanying K562 RNA sample.

Failure to assemble isoforms correctly can result from insufficient sequence coverage of RNA standards with low abundance. The most abundant RNA standard that fails to assemble correctly thereby indicates a lower limit of transcript assembly. This is illustrated in Figure 22A and Figure 22B by plotting the known concentration of each isoform relative to the sensitivity with which the exons, introns and full isoform structure are assembled. Transcripts from the accompanying K562 RNA sample that are present below this concentration will be expected to be poorly or only partially assembled.

**Example 30:**

One example method of quantifying RNA standards abundance was performed. We first added RNA standards, as previously prepared as Mixture A in Example 15, to three biological replicate K562 RNA samples for library preparation and sequencing using methods described in Example 26.

We first aligned sequenced reads (.fastq) to the index file (hg19_chrT_index.*) using Tophat2 (Kim, Pertea et al. 2013) with the following parameters:
>tophat2 -G annotations.gtf hg19_chrT_index ./K562.R1.fq ./K562.R2.fq

This approach uses gene annotations to guide alignment. The annotation file (annotations.gtf) comprises annotations of gene loci on the artificial chromosome, and natural genes annotations from GENCODE v19 (Harrow, Frankish et al. 2012) for the human genome. Alignment files (.bam) were quantified against RNA standard and human gene annotations using Cufflink2 (Trapnell, Williams et al. 2010) according to default parameters:
>cufflinks -G annotations.gtf K562_1_mixA.bam

Abundance can be quantified at two levels; abundance for each artificial gene (i.e., both DNA standard pair combined) and each isoform (i.e., each DNA standard isoform) was measured. To illustrate the quantification of RNA standards in Figure 24A, we plotted the measured gene abundance (in RPKM) relative to the known gene concentration (in attamoles/ul) of each artificial gene. The quantitative accuracy can be measured by correlation (Pearson's r) between the observed abundance of RNA standards (as measured by NG sequencing) to their expected abundance (that corresponds their known concentration when combined into Mixture A). For this example (RNA standards Mixture A combined with 3 replicate K562 RNA samples), the correlation is 0.95. The slope, illustrated in Figure 24A measures proportionality of increase (determined from non-linear regression fitting with a straight line and $1/Y^2$ weighting). This indicates the linear proportionality of observed compared to expected abundance across the dynamic range of the RNA standards. For this example, the slope is 0.91. These results are summarised in Table 2.

The accuracy with which an RNA standard is quantified is dependent on sequencing coverage, and quantification of low abundance RNA standards with low sequencing coverage is more variable than high abundance RNA standards. To illustrate this, we plotted the coefficient of variation (COV%) in quantitative measurement for each RNA Standard relative to the known concentration of each RNA standard in Figure 22C. This indicates that the RNA standards at 0.153 attamoles/ul have variation of high variation 97.07 (CV%) while genes at 1,250 attamoles/ul exhibit a low variation of 3.24 (CV%). This demonstrates the use of RNA standards to assess the confidence with which gene abundance is measured.

We can use RNA standards to convert the abundance of natural genes (in the accompanying RNA sample) that is measured by NG sequencing in reads per kilobase per million (RPKM) into concentration in molar units (attamoles/ul), as illustrated in Figure 24A. For example, in the accompanying K562 RNA sample we measure the expression of the *breakpoint cluster region gene* (*BCR*) to be at 20.9063 RPKM. This corresponds to a concentration of 0.019 attamoles/ul by comparison to similarly abundant RNA standards.

**Example 31:**

One example method using RNA standards to measure alternative splicing was performed. The accurate quantification of an individual isoforms is complicated by varying levels of sequence shared with other alternatively spliced isoforms from the same gene loci. Therefore, to assess the accuracy of isoform quantification, we plotted the measured isoform abundance (in RPKM) relative to the known isoform abundance (in attamoles/ul) of RNA standards in Mixture A (prepared in Example 15), as illustrated in Figure 24D. We next determined the correlation of 0.93 (Pearson's r) and slope of 0.86 for isoform RNA standards added with the K562 RNA sample, thereby providing an assessment of isoform quantification. These results are summarised in Table 2.

We next measured the relative abundance between the multiple individual isoform RNA standards that are generated from a single shared artificial gene loci in a process emulating alternative splicing. We plotted the observed relative abundance of paired isoforms compared to the known relative abundance of paired isoforms, as illustrated in Figure 25A, to indicate of the quantitative accuracy with which alternative splicing events are measured. For this sample, we observe a correlation of 0.76 (Pearson's r) and slope of 0.84 between RNA isoform pairs in Mixture A that were added to the K562 RNA sample. This assessment informs the analysis of alternatively splicing of natural genes in the accompanying K562 RNA sample.

**Example 32:**

One example method of using RNA standards to measure differences between multiple RNA samples was performed. Firstly, GM12878 cells were cultured according to Coriell Cell Repositories growth protocols and standards. Briefly, GM12878 were cultured in RPMI 1640 medium (Gibco) supplemented with 10% fetal bovine serum (FBS) at 37°C under 5% CO2. RNA was extracted from GM12878 cells using TRIzol (Invitrogen) according to the manufacturer's instruction. RNA Standards prepared as Mixture A and Mixture B as previously described in Example 14, and as indicated in Table 1. RNA Mixture A was added to K562 RNA samples and RNA Mixture B was added to GM12878 RNA samples to final volume of 1% of final sample (as measured by NanoDrop, ThermoScientific). Libraries were prepared, sequences as described above in Example 26. Sequenced read files (.fastq) for RNA standards Mixture B with accompanying GM12878 RNA sample were analysed with the artificial chromosome and reference human genome using the method described above in Examples 28-30. Results are summarised in Table 2 and illustrated in Figure 24B,F.

We next compared differences in the abundance of RNA standards between Mixture A (with K562 cell samples) and Mixture B (with GM12878 cell samples). We plotted the observed fold change between Mixture A and B compare to the expected fold-change, as illustrated in Figure 24C and indicated in Table 3. We observe a correlation of 0.70 (Pearson's r) and slope of 0.88 between expected and observed fold-change, indicating the accuracy with which differential RNA abundance is measured between accompanying RNA samples.

We next measured differences in the relative isoform abundance of RNA standards between samples. We plotted the observed versus expected fold change in isoform abundance between Mixture A and Mixture B as illustrated in Figure 24F and 25B. For this sample, the observed to expected isoform fold-change has a correlation of 0.73 (Pearson's r) and slope of 0.75 (summarised in Table 3), indicating the accuracy with which differential alternative splicing is measured between accompanying RNA samples.

Fold-changes in isoform abundance emulate quantitative alternative splicing events. We use the *R_10_2* gene to illustrate in Figure 25C how the standards can emulate fold-changes in alternative splicing. The *R_10_2* gene comprises two different isoforms that result from the alternative splicing of the 5$^{th}$ exon to generate a longer isoform (*_R*) or shorter version (*_V*). Coverage by simulated sequence reads, generated by methods previously described in Example 27, indicates that the *R_10_2* isoforms can be faithfully assembled. Standards representing the *R_10_2* genes were added to the Mixtures A and B such that the (i) gene expression decreases 5-fold and (ii) Isoform expression changes with a relative 3-fold increase of the *R_10_2_V* isoforms with concomitant 3-decrease in *R_10_R* isoform. This emulates 3-fold change in alternative splicing at exon 5 as illustrated in Figure 25C. We next quantified the fold-change in *R_10_2* isoform abundance between K562 cells with Mixture A and GM12878 cells with Mixture B, observing a 4-fold decrease in gene expression (which is an underestimation of 5-fold expected fold-change change in gene abundance) and a 3-fold change in relative isoform abundance, as illustrated in Figure 25C. This example demonstrates how varying abundance of isoform RNA standards can emulate alternative splicing differences between RNA samples.

We can restrict and of the above analysis to specific subsets of RNA standards. For example, we can determine the accuracy of alternative splicing of RNAs standards above a user-defined threshold abundance limit of assembly at 4.8 attamoles/ul, as illustrated in Figure 26B. Because this subset of RNA standards has higher sequence coverage than the average for all RNA standards, we observe more accurate measures (correlation, slope) of isoform quantification.

**Example 33:**

One example method of using RNA standards to calibrate differences between disease and normal RNA samples was performed. Total RNA samples from 3 normal human lung samples and 3 lung adenocarcinoma samples were purchased from Origene (Sample IDs: *CR560142, CR559185, CR560128, CR560083, CR560135, CR561324;* Rockville, MD). RNA standards Miture A was added at 1% total volume to each lung adenocarcinoma samples and RNA Mixture B is added at 1% volume to each lung normal RNA, using methods previously described in Example 26. To enable a comparison with previous published ERCC RNA Spike-Ins(Consortium 2005), we also added ERCC Spike-In Mixture 1 to each lung adenocarcinoma sample and ERCC Spike-In Mixture 2 to each lung normal sample according to manufacturer's instructions (tools.lifetechnologies.com/content/sfs/manuals/cms_086340.pdf). Combined RNA samples were prepared as libraries for sequencing, and analysed using methods described in Example 28-30 above. Results are summarised in Table 2.

We next compared the performance of RNA standards described herein with ERCC Spike-In sequences. We determined the alignment and expression fold-change for the ERCC Spike-Ins according to manufacturer's instructions, and measured alignment specificity and sensitivity, fraction dilution, detection limit and dynamic range, and quantitive accuracy (correlation and slope) as previously described (in Example 28-30) for both RNA standards and ERCC Spike-Ins. The comparison between ERCC Spike-Ins and RNA standards is summarized in Table 2.

We plotted the expected relative to known abundance of both RNA standards and ERCC Spike-Ins in Figure 26A,B. We also compare the fold-change between mixtures for both RNA standards and ERCC Spike-Ins as illustrated in Figure 26C.

ERCC standards exhibit similar alignment sensitivity (0.84) compared to RNA standards (0.81) but higher specificity (0.99) compared to RNA standards. This higher specificity of ERCC alignments is a result of ERCC Spike-Ins comprising only a single RNA sequence. Unlike RNA standards descried herein, and endogenous human genes, ERCC Spike-Ins are not comprised of multiple exons and intron sequences, and it is therefore only possible to align non-split reads to ERCC Spike-In sequences.

We next quantified the expression of human genes causatively associated with cancer (as curated by the Wellcome Trust Sanger Cancer Census(Futreal, Coin et al. 2004)) within the normal lungs RNA samples or lung adenocarcinoma RNA samples. We concatanated the genome coordinates (from GENCODE v19 annotations (Harrow, Denoeud et al. 2006)) of 464 genes coordinates of genes on the artificial chromosomes to form a single annotation file (CancerGenes_RNAstandards.gtf). We then measured expression of cancer genes and RNA standards using Cuffdiff (Trapnell, Williams et al. 2010) with the following parameters:

```
>Cuffdiff -g CancerGenes_RNAstandards.gtf \
LungCancer1.sam,LungCancer2.sam,LungCancer3.sam \
LungNormal1.sam,LungNormal2 sam,LungNormal3.sam
```

We then performed a comparative analysis to assess the quantitative accuracy of differential gene expression and alternative splicing of RNA Standards in Mixture A (with Lung Normal) and Mixture B (Lung Adenocarcinoma) using methods previously described in Example 28-30. Results are summarized in Table 3.

We plotted the measured abundance of cancer genes relative to the measured abundance of RNAs standards to illustrate in Figure 26D how the observed abudnace (in RPKM) of the RNA standards can be used to infer the concentration (in attamoles/ul) of corresponding cancer genes.

To illustrate how RNA standards can inform the analysis of individual genes in the accompanying RNA samples, we considered expression of the *mini-chromosome maintenance 2 (MCM2)* gene. *MCM2* is a marker of cell proliferation (Yang, Ramnath et al. 2006, Simon and Schwacha 2014) and enriched *MCM2* expression has been previously reported in lung adenocarcinomas samples(Zhang, Gong et al. 2014). Therefore, it is important to accurately measure fold-changes in *MCM2* expression between normal and matched tumor samples. *MCM2* has a complex spliced structure (comprising 16 exons) and is therefore well modeled using the RNA standards. We observed *MCM2* exhibits a mean expression of ~63.0 RPKM in Lung Normal Samples, but is enriched 2.07-fold (to mean 170.1 RPKM) in Lung Adenocarcinoma Samples. By comparison to RNA standards, we determine *MCM2* expression corresponds to a concentration of 19.53 attamoles/ul. Notably, RNA standards at a similar concentration (such as *R_6_1* and *R_6_2*) are poorly assembly and quantified. This suggests the measurement of *MCM2* expression between the accompanying Lung Normal and Lung Adenocarcinoma RNA sequencing should be interpreted cautiously.

The plot of measured RNA standard abundance illustrated in Figure 26D suggests a limit of detection at ~0.005615 attamoles/ul. We observe that 42.7% of cancer genes are above this limit of detection and are suitable for further analysis. Note that because this library has not been sequenced to saturation, additional cancer genes may be present at concentrations below this limit of detection, or undergo changes in gene expression that may not be accurately detected.

**Example 34:**

One example method of adding RNA standards to mouse RNA sample for sequencing was performed. We first obtained mouse liver tissue from a 4-month-old wild-type Swiss mouse. Total RNA was extracted from mouse liver sample using TRIzol (Invitrogen) according to the manufacturer's instruction. DNAse treatment was subsequently performed on each sample with TURBO DNase (Life Technologies) followed by a cleanup with the RNA Clean and Concentrator Kit (Zymo Research). Total RNA was run on a BioAnalyzer to check for integrity and to determine the concentration. Only RNA with a RNA integrity number (RIN) >9.5 was used for library preparation. RNA Standards, previously prepared as Mixture A in Example 15, was added to mouse liver RNA sample at 1% volume (as determined by NanoDrop, ThermoFischer). RNA samples were prepared and sequenced using methods described in Example 26.

We next concatenated the artificial chromosome (chrT) sequence with the mouse genome (mm10) sequence to form a single file (.fasta). We then generated an index file (mm10_chrT_index.*) from the combined sequence file using bowtie-build according to manufacturer's instructions (Langmead and Salzberg 2012). We next aligned sequenced reads (.fastq) to the index file (mm10_chrT_index.*) using Tophat2 (Kim, Pertea et al. 2013) with the following parameters:
>tophat2 mm10_chrT_index ./MouseLiver.R1.fq ./MouseLiver.R2.fq
to provide an alignment file (.bam). Analysis of alignment, assembly and quantification of RNAs standards accompanying the Mouse liver sample was performed using methods previously described in Example 28-30. The results are summarized in Table 2 and illustrated in Figure 27 and 28. Notably, the analysis of RNA standards in Mixture A that were added with mouse liver RNA sample exhibited a similar sensitivity (0.56) and specificity (0.97) as to RNA standards used with human RNA sample, as indicated in Table 2. This confirms that the performance of RNA standards is not affected by addition to the mouse RNA sample, nor the concomitant alignment of sequenced reads to the mouse genome.

**Example 35:**

One example method of analysing sequenced reads from RNA standards with non-human genomes was performed. We determined whether RNA standards perform comparably well as described in the previous Example 28-30 and 34 when used with different natural genomes from a range of different organism clades. We first downloaded genome sequences for the following organisms: *H.sapiens* (hg19), *M.musculus* (mm10), *C.elegans* (ce10), *D.melanogastor* (dm3), *A.thalianis* (tair9) *E.coli* (eschColiK12) and *M.kandleri* (methKand1) and *S.cerevisae* (SacCer6). Each individual genome sequence was concatenated with the artificial chromosome sequence (chrT) to form a single sequence (.fasta) file. Bowtie2-build was then used to build indexes corresponding to the combined sequence files according to manufacturer's instructions.

We next aligned sequenced reads from the library prepared from RNA standards combined in equal concentration to form Mixture C as described in Example 27. Sequenced reads were aligned to each individual index comprising artificial chromosome with an organism genome (denoted by *) using the following parameters:
>tophat2 *_chrT_index MixtureC.R1.fq MixtureC.R2.fq
where * corresponds to organism genome (e.g. Dm3,hg19 etc.)

For each resultant alignment (.bam), we determined the alignment statistics (for both total and split alignments) using methods described in Example 28 above. We observed that the number of reads aligning to the genome, and the specificity and sensitivity of total and spliced reads was largely invariant regardless of the accompanying genome. These results are summarised in Table 4 and indicate that RNA standards perform comparably well regardless of accompanying genome and that RNA standards can be used in conjunction with RNA samples from a wide range of organisms.

**Example 36:**

One example method of using RNA standards to measure fusion gene expression was performed. We simulated read libraries using methods previously described in Example 27 for the RNA standards representing normal (*A1* and *B1*) genes and fusion genes (*B1fA1*) resulting from the translocation of artificial chromosomes as described in Example 8. Read abundance is apportioned according to a 10-fold serial dilution of the fusion RNA standards relative to the two normal RNA standards (*A1* and *B1* gene) to encompass a $10^4$ fold range, as illustrated in Figure 9B. This results in the representation of the fusion RNA standard with in a increasingly small proportion of reads. We concatenated the RNA standard sequence reads to a final concentration of 1% with the experimentally derived RNA sequencing libraries generated from K562, GM12878, Lung Normal and Lung Cancer RNA samples described in detail above. The produced a library file (.fastq) for further analysis.

We next aligned sequenced reads (.fastq) to the index file (hg19_chrT_index.*) using Tophat2-fusion (Kim, Pertea et al. 2013) with the following parameters:

>tophat2-fusion hg19_chrT_index ./K562.R1.fq ./K562.R2.fq

to generate an alignment file (.bam) and fusion file (fusions.out) that indicated the number of reads (per million; RPM) overlapping the fusion intron generated by the translocation. We plotted the known concentration of each fusion RNA standard dilution relative to read coverage as illustrated in Figure 9B. We assessed the quantitative accuracy of fusion gene RNA Standard is using the correlation (0.982) and slope (0.927), indicating a relatively high accuracy for quantifying fusion gene expression relative to normal genes. In addition, we also plotted the confidence ascribed to the identification of the fusion RNA standard compared to the relative abundance of the RNA fusion gene, as illustrated in Figure 9C. This analysis indicates the accuracy, sensitivity and confidence with which fusion genes at corresponding coverage can be detected and quantified within the accompanying natural RNA sample.

The accompanying K562 RNA sample is heterozygous for the *BCR-ABL* gene fusion between chromosome 9 and 22(Grosveld, Verwoerd et al. 1986). We next used the RNA standards to inform the measurement of the relative abundance of endogenous *BCR-ABL1 (p210)* fusion gene in the K562 RNA sample. We titrated genome DNA from K562 cells with a 10-fold serial dilution against GM12878 genome DNA to emulate an increasingly small sub-population of cells (K562) harboring the *BCR-ABL1* fusion gene against a wild-type cell (GM12878) background. We plotted read (per million) abundance of the *BCR-ABL1 (p210)* fusion gene at serial dilutions of K562 cell fractions, as illustrated in Figure 9B. RNA standards corresponding to the abundance of the *BCR-ABL1 (p210)* fusion gene indicates a relative shallow limit of fusion gene detection sensitivity (corresponding to ~1:10 dilution) that is insufficient to monitor minimal residual disease. Therefore, the use of RNA standards representing fusion genes enables us to assess the sensitivity and accuracy of detecting fusion genes in an RNA sequencing library, and may be useful in monitoring minimal residual disease(Mitterbauer, Nemeth et al. 1999).

### Example 37:

One example method of adding DNA standards to a natural DNA sample for sequencing was performed. Human GM12878 cell line (Coriell Cell Repositories) were cultured in RPMI 1640 medium (Gibco®) supplemented with 10% fetal bovine serum (FBS) at 37°C under 5% CO2. DNA was extracted from GM12878 using TRIzol (Invitrogen) according to the manufacturer's instruction. The extracted DNA samples were treated with RNase A followed by a cleanup with Genomic DNA Clean & Concentrator kit (Zymo Research). Purified DNA was quantified on the Nanodrop (Thermo Scientific). DNA standards were combined as Mixture A as previously described in Example 18 and Table 5. DNA Mixture A is then added to ~1% total volume with GM12878 genome DNA (as measured with NanoDrop, ThermoScientific).

The TruSeq Stranded DNA Sample Prep Kit (Illumina) was used to prepare DNA libraries according to manufacturer's instructions. Prepared libraries were quantified on Qubit (Invitrogen) and verified on Agilent 2100 Bioanalyzer (Agilent Technologies) before samples were pooled for sequencing. Sequencing is performed using a HiSeq 2500 instrument (Illumine) with 125nt paired-end sequence reads.

### Example 38:

One example method of assessing the alignment and assembly of DNA standards was performed. We produced DNA standards matching 30 regions of the artificial chromosome with two alleles (reference and variant) using methods as described in Example 17 and 20 above. We diluted DNA standards standards to equal abundance and combined in equal proportion to form equal parts of Mixture C. The TruSeq Stranded DNA Sample Prep Kit (Illumina) was used to prepare DNA libraries according to manufacturer's instructions. Prepared libraries were quantified on Qubit (Invitrogen) and verified on Agilent 2100 Bioanalyzer (Agilent Technologies) before samples were sequenced as 125nt paired-end reads with HiSeq 2500 insurtment (Illumina). The sequence read (.fastq) file was processed and aligned using methods described in Example 39. We assessed alignment from the alignment (.bam) file using methods described in Example 39. Notably, all DNA standards were of sufficient abundance as to achieve full sequence fold-coverage. Alignment measurements where sequence fold-coverage is non-limiting are summarised in Table 6. Specifically, we determine 99% sensitivity and 97% specificity for read alignments, thereby validating the utility of DNA standards to represent regions of the artificial chromosome.

For comparison, we also simulated reads expected to be generated from the same DNA standards. Comparison of simulated reads to experimentally-derived reads produced above can distinguish the impact of variables due to alignment and assembly (that will influence both simulated and experimentally-derived reads) from variables due to sequencing (that will influence only experimentally-derived reads, and not simulated reads).

We used Sherman (http://www.bioinformatics.babraham.ac.uk/projects/sherman/) according to manufacturer's instructions to simulate 125nt paired-end reads generated by DNA standards as a .fastq file as per sequencing on HiSeq instrumentation.. Sequenced reads incorporate a 1% error rate that has been typically reported for Illumina sequencing technology (Bolotin, Mamedov et al. 2012). We aligned simulated sequence reads to the artificial chromosome (with using bwa with the identical parameters as above, and assessed alignments as described above. Results are summarised in Table 6. Specifically, we observe 99% sensitivity and 100% specificity for alignment of reads from DNA standards, thereby validating the utility of DNA standard matching sequences from the artificial chromosome. Notably, simulated reads sufficiently recapitulate the performance of experimentally-derived sequenced reads for the alignment and assembly of DNA standards, indicating their utility in designing, modelling and analysing DNA standards that match features of artificial chromosomes.

**Example 39:**

One example method of aligning reads constituting DNA standards and a natural DNA sample library to artificial chromosome and natural reference genome was performed. Sequence files (.fastq) produced using method in Example 37 were subject to de-multiplexing. Low-quality reads and sequences or adaptor contaminant sequences were removed from sequence files using trim_galore according to manufacturer's instruction (http://www.bioinformatics.babraham.ac.uk/projects/trim_galore/).

The human genome (hg19) sequence was concatenated with the artificial chromosome (chrT) sequence to form a single file (.fasta). We then used bwa index according to manufacturer's instruction (Langmead and Salzberg 2012) to generate an index file (hg19_chrT_index.*) from the combined sequence file. We next aligned reads to the index file using bwa (Li and Durbin 2009):

>bwa mem -M hg19_chrt.bwa sequence.read1.fq sequence.read2.fa >alignments.sam

to generate an alignment (.bam) file.

Sequencing errors can produce base-wise mismatches between read alignments and the artificial chromosome sequence. We can analyse of sequence errors alignments to assess sequencing quality. For example, the Sequencing Error Rate indicates the mean number of sequencing errors per 100nt sequenced. In this example whereby DNA standards are added with the GM12878 DNA sample, we determine that 0.67% of reads contain an erroneous mismatches, as illustrated in Figure 29A. The Sequencing Error Distribution also describes distribution of sequence errors across to the read, as illustrated in Figure 29B.

We next assessed the alignments of sequenced reads to the artificial chromosome and natural human (hg19) genome according to a number of metrics described below and summarised in Table 6.

Reads to Genome/Artificial Chromosome is the number of reads that align to the artificial chromosome and the human genome. For example, for the GM12878 sample, we aligned 2,029,597 reads to the artificial chromosome and 458,521,347 reads to the human genome sequence.

Fraction Dilution is the fraction of reads aligning to the artificial chromosome relative to the genome indicates the dilution of the standards relative to the sample library (Fraction Dilution). For GM12878 sample, 0.4% of library aligns to the artificial chromosome, indicating a 250-fold dilution factor.

Alignment Sensitivity is defined as the size of artificial DNA standard bases with overlapping alignments (true positive) divided by the total number of artificial DNA standard bases (true positive and false negative). For GM12878 samples, we observe a base-wise alignment sensitivity of 0.849.

Alignment Specificity is defined as the number of artificial DNA standard bases with overlapping alignments (true positive) divided by the total number of bases with overlapping alignments (true and false positive). For GM12878 samples, we observe a base-wise alignment specificity of 0.961.

The Detection Limit corresponds to the highest abundance DNA standard that is without read alignments and not reliably detected within the sequenced library. For GM12878 we observe a detection limit of 0.0037 attamoles/ul.

**Example 40:**

One example method of calculating pipetting error from conjoined DNA standards was performed as follows. Here we illustrate how to calculate pipetting error with conjoined DNA standards, and demonstrate how accurate the calculation of pipetting error is. This requires a known level of variation due to pipetting and variation from other sources. To do this, we first simulated the amount of variation due to pipetting and other sources based on sequenced libraries from DNA standards combined in equal combinations as previously described in Example 38. Variation due to pipetting error was defined as the difference in the abundance of individual DNA standards to the mean abundance of all DNA standards.

This is termed the expected variation due to pipetting and is dependent and identical between the individual DNA standards that together comprise a single conjoined DNA standard. Variation due to other sources, such as library preparation and sequencing, was determined by analysis of technical replicate sequence libraries prepared from the same DNA standards Mixture C. Variation corresponds to the difference in normalized abundance between technical replicates of the DNA Flat mix. The expected variation due to other sources is independent and different between the individual DNA standards that together comprise a single conjoined DNA standard. We incorporated these two sources of variation into the observed abundance of DNA standards mixture according to:

$$\text{Observed Abundance} = \text{Expected Abundance x expected variation due to pipetting x expected}$$

$$\text{variation due to other sources}$$

For this example, reads derived from DNA standards were simulated as previously described in Example 38. Read abundance was apportioned according to the known abundance of conjoined DNA standards, as indicated in Table 7. We plotted the observed abundance relative to the expected abundance for each DNA standards, as illustrated in Figure 31A. This demonstrates the characteristic dependent linear slope distribution exhibited by the individual DNA standards that together comprise a single conjoined DNA standard. Notably, multiple DNA standards, conjoined together, that exhibit an irregular albeit dependent abundance, as illustrated in Figure 31B, enable easier identification and omission of outliers due to pipetting.

We calculated the pipetting variation from the observed abundance of DNA standards (illustrated in Figure 31B) as follows; for each conjoined DNA standard, we first plotted a line of best fit (non-linear regression with Y-intercept constrained to 0 and weighted to $1/Y^2$) though the 6 individual DNA standards. The deviation of the line slope from one is proportional to pipetting inaccuracy. For example, for conjoined DNA standard A, we observe a slope of 1.188, which estimates that an additional 18% of conjoined DNA standard A has been added due to pipetting error. Calculations for all conjoined DNA standards are summarised in Table 7. Comparison of the calculated pipetting variation to the expected pipetting variation indicates that using this approach we estimate the error due to pipetting within an average margin of 3%.

We can next minimise variation due to pipetting by normalizing each conjoined DNA standard measurements by this calculated variation as follows. We first force the linear distribution of conjoined DNA standards to exhibit a slope of 1, as illustrated in Figure 31A,B. This improves the correlation (Pearson's r) between the expected and observed abundance of DNA standards to 0.99 (compared to 0.987 if DNA standards are independently measured without normalization; Figure 31B). The improvement in quantitative accuracy by noramlising for pipetting error is illustrated by the reduction of the coefficient of variation between conjoined DNA standards by ~10-fold from 16.13 to 0.73 (illustrated in Figure 31C). This enables users to calculate the amount of variation and inaccuracy due to pipetting variation and amount of variation from other sources and improve measurement confidence.

### Example 41:

One example method of quantifying DNA standards abundance was performed. We first measured the frequency of alignments at each region of the artificial chromosome represented by a DNA standard. Following normalisation for length thereby assigned a observed of each DNA standards in reads per million per kilobase (RPKM). We plotted the measured DNA standard abundance compared to the known concentration (in attamoles/ul) of each DNA standard to assess quantitative accuracy as illustrated in Figure 28A. Accordingly, the DNA standard quantification can be measured with correlation (Pearson's r) to provides an indication of concordance between observed and expected DNA standard abundance. For example, we observe a correlation of 0.94 for DNA standards previously prepared with the GM12878 genome DNA sample in Example 37. The slope indicates the linear proportionality of observed relative to expected abundance across the dynamic range of the DNA standards. For DNA standards combined as Mixture A with the GM12878 sample, the slope is 1.01. Results are summarised in Table 6.

### Example 42:

One example method of identifying genetic variation in DNA standards was performed. Alignment (.sam) files prepared using methods described in Example 40 were first pre-processed using SAMtools (Li, Handsaker et al. 2009) and Picard tools as follows:

```
>java -jar CreateSequenceDictionary.jar R=hg19_chrT.fa O=hg19_chrT.dict
>samtools faidx hg19_chrT.fa >hg19_chrT.fai
>java -jar SortSam.jar INPUT=alignments.sam OUTPUT=alignments.sort.bam \
SORT_ORDER=coordinate
>java -jar ReorderSam.jar INPUT=alignments.sort.bam \
OUTPUT=alignments.sort.reorder.bam REFERENCE=hg19_chrT.fa
>java -jar BuildBamIndex.jar INPUT=alignments.sort.reorder.bam
```

We then used the GATK toolkit (McKenna, Hanna et al. 2010) according to published best practices (http://www.broadinstitute.org/gatk/guide/best-practices), including the Unified Genome Haplotype caller, to identify genetic variation using following default parameters:

```
>java -jar GenomeAnalysisTK.jar -T HaplotypeCaller -R hg19_chrTfa \
```

```
-I alignments.sort.reorder.bam --genotyping_mode DISCOVERY \
--defaultBaseQualities 30 -o variants.vcf
```

Note that the method described herein simultaneously identifies variation on the artificial chromosome, but also between the GM12878 genome DNA and the reference human genome. We can assess the performance of variant identification in the artificial chromosome using the as follows.

The Variants Covered corresponds to the proportion of genetic variation with alignment coverage. For example, alignments overlap 490 (88%) of variation instances in the DNA standards accompanying the GM12878 DNA sample.

Variant Sensitivity is defined as the number of variants correctly identified (true positive) divided by the total number of variants represented within the DNA standards (true + false negative). This depends both sequencing depth and variant detection. For example, for GM12878 sample, we achieve a variation sensitivity of 0.65.

Variant Detection is defined as the Variation Sensitivity divided by Variants Covered provides a measure of variant detection independent to sequencing depth or coverage. For example, for GM12878 sample, we achieve a variant efficiency of 0.73

Variant Specificity is the number of variants correctly identified (true positive) divided by the total number of variants detected (true positive + false negative). For example, for GM12878 sample, we achieve a variant specificity of 0.57.

Median Quality Score is defined as the PHRED scaled probability that a variant exists at this site, can be assigned to each identified variant. For the GM12878 sample, the median quality score for correct variant calls is 1,803, whilst the median quality score for erroneous variant calls is 61, as illustrated in Figure 28E.

These results are summarised in Table 6. Descriptive statistics can be restricted to specific subsets of the variation represented within the DNA standards. For example, we can determine the sensitivity for detecting insertions within the DNA standards.

Erroneous variant calls on the artificial chromosome exhibit lower quality score than correct calls, as illustrated in Figure 30A, indicating the utility of the quality score to distinguish erroneous variant identification in the accompanying variant identification in the GM12878 genome. Similarly, we observe that specific nucleotide substitutions (C to A and T to G) are particularly enriched in erroneously called variation, suggesting that these nucleotide variants should be interpreted with additional caution, as illustrated Figure 30B.

The failure to identify variation correctly can often result from insufficient sequence coverage. This limit of sensitivity for identifying variation is illustrated in Figure 28B,E by plotting the expected concentration of each DNA standard to the fraction of variation correctly assigned for each DNA standard. The highest concentration DNA standard for which variation is not detected indicates the lower limit at which variation can be reliably detected within the accompanying GM12878 genome sample.

We next analyzed the relative allele frequency generated by varying the relative concentration of reference and variant DNA standards. We plotted the expected relative allele frequency (ie. abundance ratio of reference to variant DNA standard) to the observed relative allele coverage (as indicated by DP in the GATK output .vcf file) for the 115 variants identified on the artificial chromosome. This plots, as illustrated in Figure 28C, indicates the minimum correctly identified allele frequency was 1% and correct variation detection was limited to DNA standards at abundance above for 0.088 attamoles/ul. Restriction of alleles to only those with coverage >8 attamoles/ul improves allele frequency quantification with a correlation of 0.9574 and slope 0.9043, reflecting the importance of sufficient sequencing coverage for accurately detecting and quantifying rare variants.

We can also compare variant identification in the accompanying GM12878 genome DNA to variant identification in DNA standards with similar sequence read coverage. For example, the 25th-75th percentile of genome DNA variants exhibit a sequence coverage of coverage between 3 to 6-fold. This sequence coverage corresponds to five DNA standards that have a mean abundance of 0.15 attamoles/ul. Restricting our analysis to this subset of DNA standards suggests a sensitivity of 0.846, and specificity of 0.93 for identifying variation in the GM12878 genome.

**Example 43:**

One example method of quantifying variation in DNA standards between disease and normal human DNA samples was performed. Commercial DNA from normal lungs and adenocarcinoma of lungs was purchased from Origene *(CD563993, CR563976;* Rockville, MD). DNA Mixture A, as prepared in Example 18, was added to 1% total volume to lung adenocarcinoma DNA sample and DNA Mixture B is added to 1% volume to lung normal DNA sample (as determined by NanoDrop). DNA samples and libraries were prepared and sequenced using methods previously described in Example 37. Reads were aligned and analysed using methods described in Example 41-42. Results are summarised in Table 6.

DNA samples may harbor mutations at heterogeneous frequencies (distinct from the homozygous/heterozygous allele frequencies discussed previously). For example, cancer cells harboring specific mutations may only comprise a small proportion of the sample sequenced. We plot observed allele frequency relative to expected allele frequency, as illustrated in Figure 30C,D to determine the accuracy and sensitivity of allele quantification. For example, the lung adenocarcinoma sample has a Correlation (Pearson's r) 0.91 and slope of 0.95. The Limit of Detection indicates the lower frequency limit at which an allele can be reliably identified. For example, in this example the lower limit of detection is 0.0019 attomoles/ul. Similarly, the allele frequency provides an estimate of the sample purity, and would enable us to estimate the proportion of cancer cells within the sampled lung adenocarcinoma tissue for which we can resolve 1:100 allele frequencies down to 13-fold coverage or 0.0082 attomoles/ul.

**Example 44:**

One example method of adding DNA standards with mouse DNA samples. Mouse Liver tissue was obtained from a 4-month-old wild type Swiss SWR/J mouse. Genomic DNA was extracted mouse liver sample using TRIzol (Invitrogen) according to the manufacturer's instruction. The extracted DNA samples were treated with RNase A followed by a cleanup with Genomic DNA Clean & Concentrator kit (Zymo Research). Purified DNA was quantified on the Nanodrop (Thermo Scientific). DNA Mixture A, as prepared in Example 18, was added to 1% total volume to mouse DNA sample (as determined by NanoDrop). DNA samples and libraries were prepared and sequenced using methods previously described in Example 37.

The mouse genome (mm10) sequence was concatenated with the artificial chromosome (chrT) sequence to form a single file (mm10_chrT.fa). We then generated an index file (mm10_chrT_index.*) from the combined sequence file using bwa index according to manufacturer's instruction (Langmead and Salzberg 2012). We aligned sequenced reads (.fastq) to the index file (mm10_chrT_index.*) using bwa (Kim, Pertea et al. 2013) using methods described in Example 39. We analysed the alignment, quantification and variant detection of the DNA standards using methods described in Example 41, and illustrated in Figure 28D. The results, summarised in Table 6, indicate similar levels of alignment specificity, sensitivity, and quantification with both human and mouse genome DNA, indicating the performance of DNA standards is not influenced by addition of mouse DNA samples or concomitant alignment with mouse genome.

**Example 45:**

One example method of analysing sequenced reads from DNA standards with non-human genomes was performed. We determined whether DNA standards perform comparably well as when used with different natural genomes from a range of different organism clades. Index builds for a range of organisms genomes with accompanying artificial chromosomes were generated by methods previously described in Example 35. We next aligned sequenced reads from the DNA standards prepared a Mixture C using methods as described in Example 38. Sequence reads were aligned to each organisms genome / artificial chromosome sequence using bowtie (Li and Durbin 2009) with the following default parameters:

>bowtie2 -x*_chrT_index -1 MixtureC.R1.fq -2 MixtureC.R2.fq

where * corresponds to organism genome (e.g. Dm3,hg19 etc.)

For each resultant alignment (.bam), we measured the alignment sensitivity and specificity using methods described in Example 40. These results, summarised in Table 4, indicate that DNA standard alignment is largely invariant regardless of the accompanying organism genomes, and that DNA standards perform comparably well when used with a range of different organism DNA samples.

**Example 46:**

One example method of identifying disease associated genetic variation in DNA standards was performed. To assess the performance of DNA standards that represent specific instances of variation associated with disease, produced by methods described in Example 22, we simulated sequenced reads using methods previously described in Example 38. Read abundance were apportioned according to genotype (eg. heterozygous or varying heterogeneous scale).

The K562 cell line harbors the *TP53* Q139fs mutation, but not the *BRAF* V600E mutation. We added sequenced read to library from K562 genome DNA, prepared in Example 37. The reads are added at 1% total volume so that the DNA Standard modelling heterozygosity achieves similar coverage to accompanying K562 genome (ie. 10.4-fold). Sequence reads (from K562 and DNA standards) was aligned to the genome with the following parameters:

>bwa mem -M hg19_chrAB K562.R1.fq K562.R2.fq >alignments.chrB5.sam

Alignments were prepared as for Example 42, and we used the Genome Analysis Toolkit (DePristo, Banks et al. 2011) with the following parameters:

```
>java -jar ~/1000G/GenomeAnalysisTK.jar -T HaplotypeCaller -R hg19_chrAB \
-I alignments.chrB5.sam --genotyping_mode DISCOVERY
--defaultBaseQualities 30 -o variants.vcf
```

We next plotted the depth coverage (as indicated by DP in the GATK output .vcf file) of each variant in the variant DNA standards and the accompany K562 genome DNA relative to variant coverage, as illustrated in Figure 7B. Additionally, we plot the confidence with which each genotype is assigned relative to known concentrations of each DNA standard, as illustrated in Figure 7C, thereby indicating the confidence with which SNPs are identified across a $10^4$ fold dynamic range.

To model an increasingly small sub-population of cells harboring a mutation against a wild-type cell population, we titrated the K562 cell line DNA library (containing *TP53* Q139fs mutation) against a background of GM12878 genome DNA library (that does not contain the *TP53* Q139fs mutation) to form a 10-fold serial dilution encompassing a $10^5$ dynamic range. We then aligned these diluted libraries to the human genome/artificial chromosome using methods described in previous Example 39. Comparison of disease-associated variants identified in the DNA Standards and accompanying genome DNA sample is illustrated in Figure 7B. We observed that the V600E and Q139fs mutations could be identified accurately when the variant and reference DNA standards were in equal abundance (ie. heterozygous genotype) and, similarly, we could robustly identify the Q139fs mutation in the accompanying K562 DNA sample. However, we were unable to detect the Q139fs mutation when the variant DNA standard was diluted 10-fold relative to the reference DNA standard or when the accompanying DNA sample comprises 10-fold or more dilution of the K562 DNA.

**Example 47:**

One example method of assembly of structural variants represented by DNA standards was performed. DNA standards representing structural variation on the artificial chromosome (as previously described in Example 23) was added to 1% total volume to K562 genome DNA sample. DNA samples and libraries were prepared and sequenced using methods previously described in Example 37, and aligned to the artificial chromosome / human genome using methods previously described in Example 39.

We profiled sequence coverage of the following structural variation on the artificial chromosome; Three DNA standards of length 1837, 1824 and 1899 (SEQ ID NO: 171-173) that contained an inverted DNA sequence of length 635, 624 and 699 nt relative to the reference artificial chromosome (illustrated in Figure 32A). Three DNA standards of length 1898,1865 and1896 (SEQ ID NO: 174-176) that contained large DNA sequence insertions of length 698,665 and 696 relative to the reference artificial chromosome (illustrated in Figure 32B). Three DNA standards of length 1200nt (SEQ ID NO: 177-179) that contained large DNA sequence deletions of length 651, 634 and 683nt relative to the reference artificial chromosome (illustrated in Figure 32C). Three DNA standards of length 1200nt (SEQ ID NO: 180-182) that contained large DNA sequence tandem duplications of 4 repeat copies x 96nt (380nt), 2 copies x 202 (438nt) copies and 2 copies x 621nt relative to the reference artificial chromosome (illustrated in Figure 32D). Three DNA standards of length 1988, 1580 or 1430nt (SEQ ID NO: 183-185) that contained a mobile element repeat insertion relative to the reference artificial chromosome. The inserted repeat sequence matched the ancient repeat unit of the *AluSx, MIRb, L2a* transposons as previously described (illustrated in Figure 32E).

**Example 48:**

One example method of using DNA standards to calibrate measurement of copy-number repeats was performed. To assess the performance of DNA standards that represent D4Z4 copy number variation, produced by methods described in Example 23, we simulated sequenced reads using methods previously described in Example 38. Read abundance were apportioned according to copy number (from 10 - 150 copies) as previously described in Example 23.

We added sequenced read to library from K562, GM12878, Lung Adenocarcinoma and Normal Lung DNA samples using methods described in Example 37. We aligned reads to the artificial chromosome and to the human (hg19) genome using bwa (Langmead and Salzberg 2012) as previously described in Example 39. The observed abundance (in reads per million) of the DNA standards was plotted against known repeat copy number, as illustrated in Figure 33B, enabling an assessment the quantification of repeat copy number. We compared DNA standard copy number to coverage of the *D4Z4* repeat sequence in the human genome from the accompanying human DNA sample. After normalizing for differences in the size of the *D4Z4* repeat unit (~3,301nt) and the DNA standards, we estimate the number of *D4Z4* repeat units in the accompanying patient genome by comparison to DNA standards. For example, we estimate 161 repeat copies in the GM12878 genome, as illustrated in Figure 33B.

**Example 49:**

One example method of adding DNA standards to environmental DNA samples. Soils was collected from Watsons Creek and mangrove patch sites in Queensland, Australia. Soils samples were stored at 4°C prior to both chemical and biological analysis. Genomic DNA from soil samples was extracted using PowerSoil™ DNA kit (MoBio Laboratories, Carlsbad, CA, USA) according to the manufacturer's protocol. All genomic DNA was quantified by Nanodrop (Thermo Scientific). DNA Mixture A, as prepared in Example 18, was added to 1% total volume to soil DNA sample (as determined by NanoDrop).

TruSeq DNA PCR-free Sample Prep Kit (Illumina) was used to prepare DNA libraries according to manufacturer's instructions. Prepared libraries were quantified on Qubit (Invitrogen) and verified on Agilent 2100 Bioanalyzer (Agilent Technologies) before samples were pooled. Sequencing is performed using a HiSeq 2500 instrument with 125nt paired-end reads (Illumina).

**Example 50:**

One example method of aligning DNA standard reads to microbe genomes was performed. Sequence (.fastq) files produced by HiSeq 2500 instrument were subject to de-multiplexing. Low-quality reads and sequences or adaptor contaminant sequences were removed using trim_galore according to manufacturer's instructions (http://www.bioinformatics.babraham.ac.uk/projects/trim_galore/)

We combined all artificial microbe genomes, produced by methods described in Example 9, to generate a single index build using methods previous described in Example 39. We aligned sequenced reads to artificial microbe genome using bwa (Li and Durbin 2009) with the following parameters:

>bwa mem -M ArtChr.bwa sequence.read1.fq sequence.read2.fa \ alignments.sam

We assessed alignments (.bam files) to artificial microbe genomes according to; Reads that align to artificial microbe genomes. For example, in Soil Sample 1 we aligned 4,317,629 reads to the artificial microbe genomes. The Fraction Dilution is the fraction of reads aligning to the artificial microbe genomes relative to total reads. For example, in Soil Sample 1, 5.6% of reads within the library align to the artificial microbe genomes, corresponding to a 17.1-fold dilution factor. The Detection Limit corresponds to the highest abundance DNA standard that is not reliably detected within the sequenced library and is without alignments. For Soil Sample 1 we observe a detection limit of 1.0093. Sensitivity is defined as the number of DNA standard bases with overlapping alignments, as illustrated in Figure 35C. This is dependent on sequencing depth and alignment. For example, in Soil Sample 1, 80.2% of DNA standard bases have overlapping alignments. Results are summarised in Table 10.

**Example 51:**

One example method of using DNA standard reads to calibrate assembly of microbe genome community was performed as follows. We performed *de novo* sequence assembly using Velvet(Zerbino and Birney 2008) according to manufacturer's instructions:

```
>velvet_1.2.10/velveth ./output 91 -sam soil.sam
>velvet_1.2.10/velvetg ./output -exp_cov auto -cov_cutoff 0 -scaffolding no
```

We assessed contig assemblies according to; Coverage is the proportion of DNA standard size that are overlapped by assembled contigs. This is dependent on both sequencing depth and assembly. For example, in Soil Sample 1 we assembled contigs that cover 31.9% of the DNA standards, as illustrated in Figure 35D. Nodes is the number of distinct contigs correctly assembled (that match the DNA standards). For example, in Soil Sample 1, we assembly 20 (out of 36) nodes. The N50 statistics refer to the median mass of contigs relative to the total assembly (N50). For example, in Soil Sample 1 we determined a N50 statistic of 508. The Maximum Contig Size the largest size correctly assembled contig. For example, in Soil Sample 1 we assembled contigs up to 904nt that corresponds to 92.1% of the DNA standard full-length. Total Bases in Assembly is the number of reads aligning to correctly assembled contigs relative to total number of reads aligning to DNA standards. For example, in Soil Sample 1 we align 22.1% reads to assembled contigs. These results are summarised in Table 10.

**Example 52:**

One example method of using DNA standards to calibrate quantification of microbe genomes was performed. To assess the accuracy of quantification, we plotted the observed abundance (in RPKM) relative to the known concentration (in attamoles/ul) of each assembled contig (as illustrated in Figure 36A,B). We first measured the frequency of alignments at each region of the artificial microbe genome represented by a DNA standard. Following normalisation for length, we assigned a observed of each DNA standards in reads per million per kilobase (RPKM). We plotted the measured DNA standard abundance compared to the known concentration (in attamoles/ul) of each DNA standard to assess quantitative accuracy as illustrated in Figure 35A. Accordingly, the DNA standard quantification can be measured with correlation (Pearson's r) to provides an indication of concordance between observed and expected DNA standard abundance. For example, for DNA standards prepared with Soil Sample 1, we observe a correlation of 0.96 and slope is 1.061. Results are summarised in Table 10.

Genome assembly is dependent on sufficient sequencing coverage, as illustrated in Figure 35A. We observe that DNA standards at high concentration exhibit full sequence coverage and assembly, while, by contrast DNA standards at low expected concentration show spare sequence coverage and poor assembly, as illustrated in Figure 35B. This enables us to determine the expected coverage and assembly of microbe genomes according to their relative abundance in the accompanying soil sample.

**Example 53:**

One example method of using DNA standards to measure differences between multiple environmental DNA samples was performed. We first extracted DNA from three soil samples with high organic content with soil samples for comparison to three soil samples with low organic content, using methods previously described in Example 49. DNA Mixture A, as prepared in Example 18, was added to 1% total volume to three soil samples with high organic content and DNA Mixture B is added to 1% volume to three soil samples with low organic content. DNA samples and libraries were prepared and sequenced using methods previously described in Example 49. Reads were aligned and analysed using methods described in Example 50-52. Results are summarised in Table 10 and illustrated in Figure 36A,B.

We plotted the observed abundance of DNA standards forming Mixture A in high-organic content soil samples relative to observed abundance of DNA standards forming Mixture B in low-organic content soil samples to illustrate the DNA standard fold-changes in Figure 36C. We observe a correlation of 0. 8328 (Pearson's r) and slope of 1.149, as summarised in Table 11, indicating the accuracy with which differential DNA abundance is measured.

**Example 54:**

One example method of using DNA standards to calibrate quantification of microbe genomes in environmental DNA samples was performed. Fecal samples were collected from a healthy male in a 50 mL polypropylene tube. DNA was extracted from the fecal samples (0.25 g) using the MoBio PowerFecal™ DNA Isolation Kit (MoBio Laboratories, Carlsbad, CA, USA) according to the manufacturer's protocol.

DNA Mixture A, as prepared in Example 18, was added to 1% total volume to two replicate fecal samples from healthy human subject. DNA samples and libraries were prepared and sequenced using methods previously described in Example 49. Reads were aligned and analysed using methods described in Example 50-52. Results are summarised in Table 10 and illustrated in Figure 36D-F.

We assessed the assembly of DNA standards, using methods described above in Example 51. For example, in fecal sample 1, DNA standards comprised 0.89% of the total reads (2 million from 225 million). Sequenced reads were assembled into 14 contigs that encompasses 53.2% coverage of the DNA standards. We measured the abundance of assembled DNA standard contigs using methods previously described in Example 52. This provides an internal reference ladder for the quantification of metagenomes to inform microbe community analysis (Singh, Behal et al. 2009) and results are summarized in Table 10. For example, for Fecal Sample 1 we observe a correlation of 0.97, and slope of 1.041, indicating high quantitative accuracy for assembled DNA standards.

**Example 55:**

One example method of using DNA standards as template for PCR amplification was performed. DNA standards can be used in methods of amplicon sequencing, such as immune-repertoire sequencing where mammalian immunoglobulin sequence diversity is amplified and sequenced. We previously manufactured DNA representing artificial TCRγ clonotypes, using methods described in Example 25. We subjected DNA standards to PCR amplification (KAPA Biosystems) using universal BIOMED2 primer sequences (van Dongen, Langerak et al. 2003) for the *TCRγ* loci (present in *Tube A* and *B*) according to manufacturer's instructions. Amplified products were analyzed using a BioAnalyser (2100 High Sensitivity DNA Assay; Agilent). BioAnalyser traces indicate the amplification of a correctly sized 750nt product from all 15 TCRγ clonotype DNA standards, as illustrated in Figure 34. This confirms the utility of DNA standards as templates for PCR amplification during immune-repertoire sequencing.

We next produced a genomic DNA mixture of 10% gDNA from clonal T-ALL cells and 90% gDNA from a healthy's adult's PBMC, to model a clonal population of TCRγ clonotypes. The clonal T-ALL cell line, KARPAS 45 (Catalog N. 06072602, Human T-cell Leukaemia) was purchased from Cell Bank Australia. KARPAS 45 cells were cultured according to European Collection of Cell Cultures growth protocols and standards. Briefly, KARPAS 45 cells were cultured in RPMI 1640 medium (Gibco®) supplemented with 15% fetal bovine serum (FBS) at 37°C under 5% $CO_2$. Genomic DNA was extracted from KARPAS using TRIzol (Invitrogen) according to the manufacturer's instruction. The extracted DNA samples were treated with RNase A followed by a cleanup with Genomic DNA Clean & Concentrator kit (Zymo Research). Purified DNA was quantified on the Nanodrop (Thermo Scientific). Genomic DNA from a healthy adult's PBMC was extracted using the MoBio UltraClean kit (Catalog No. 12334-250). gDNA was eluted in solution TD3 and analysed on the Nanodrop (Thermo Scientific).

The artificial TCRγ clonotype DNA standards were then added at 1% of the total genomic DNA concentration of the mixture. We performed PCR amplification (KAPA Biosystems) using universal BIOMED2 primer sequences (as described above) on combined clonotype DNA standards and T-ALL / PBMC genome DNA mix. PCR amplicons were purified using the Wizard® SV Gel and PCR Clean-Up System (Promega) and were quantified on the Nanodrop (Thermo Scientific) and verified on the Agilent 2100 Bioanalyzer (Agilent Technologies).

The Nextera XT Sample Prep Kit (Illumina) was used to prepare libraries from PCR amplicons according to manufacturer's instructions. Prepared libraries were quantified on Qubit (Invitrogen) and verified on Agilent 2100 Bioanalyzer (Agilent Technologies) before samples were pooled. Sequencing is performed using a HiSeq 2500 instrument with 125nt paired-end reads (Illumina).

### Example 56:

One example method of using DNA standards in analysis of mammalian immunoglobulin sequence diversity was performed. To assess the performance of DNA standards that represent artificial *TCRβ* clonotypes, produced by methods described in Example 25, we first performed *in silico* PCR amplification (http://insilico.ehu.es/PCR/) of DNA standards with the BIOMED-2 *TCRβ* multiplex primer sequences (Tubes A-C)(van Dongen, Langerak et al. 2003) to produce a ~750nt amplicon sequence. Primer binding sites were required to have exact complementarity and we assumed no primerspecific amplification bias. We next simulated sequenced reads from the amplicon sequences using methods previously described in Example 38. Read abundance were apportioned according to the relative concentration of the DNA standards as described in Example 25. Reads are added at 1% fraction to previously published experimental amplicon sequencing libraries (.fastq) of the *TCRβ* loci in 3 healthy human subjects (Zvyagin, Pogorelyy et al. 2014). This data was retrieved from the NCBI Short Read Archive (SRA) with the Accession ID: SRP028752. These three libraries represent a *TCRβ* clonotypes profile in healthy adult human subjects. The human library files are analyzed using MiTCR according to manufacturer recommendations (Bolotin, Mamedov et al. 2012).

For each library, we determined the following metrics as summarised in Table 8. Number of Reads aligning to the human genome / artificial *TCRβ* clonotypes and the number of reads aligning to the DNA standards. In this example for Human Subject A we observe 25,191 reads that align to artificial *TCRβ* clonotypes. Fraction of Reads aligning to the artificial *TCRβ* clonotypes indicates the dilution factor of 1% for Human Subject A. The Limit of Detection indicates the highest abundance DNA standard that is not detected by sequenced reads in the library and the Dynamic Range indicates the fold difference between the highest and lowest abundance DNA standard detected by sequenced reads in the library. The Clone Sensitivity indicates the proportion of DNA standard for which the *artificial* TCRβ clonotype is correctly assigned. This can also include accuracy of Vβ,Dβ,Jβ segment assignment and detection of insertion/deletions.

We plot the observed frequency of artificial *TCRβ* clonotype relative to known concentration, to ascertain the accuracy of *TCRβ* clonotype abundance measurements by correlation and slope (results summarized in Table 8). The abundance of artificial *TCRβ* clonotype relative to natural *TCRβ* clonotypes in healthy human subjects is illustrated in Figure 13E. The abundance of artificial *TCRβ* V,J and D segments usage relative to natural *TCRβ* V,J and D segments in healthy human subjects is illustrated in Figure 13F.

### Example 57:

One example method of using DNA standards in analysis of *16S rRNA* phylogenetic profiling was performed. We produced 6 DNA standards (SEQ ID NO: 161-166) of length 1018nt that match 16S rRNA genes from 6 different artificial microbe genome representing a range of taxa, size, GC content and rRNA operon count as indicated Table 9. The DNA standards are designed to overlap the two universal 16S primers in V3 region of the 16S rRNA gene, with additional flanking 250nt sequence. The 16S DNA standards form a template for the PCR amplification to generate unique amplicon sequence. We performed *in silico* PCR amplification (http://insilico.ehu.es/PCR/) with the universal 16S primer sequences. This generated a unique and distinct amplicon from each of the DNA standards. The abundance of each amplicon was apportioned according to (i) initial abundance of the microbe genome within the artificial community and (ii) rRNA operon copy number within artificial microbe genome, as indicated in Figure 11. Amplicon abundance can also be influenced by primer binding efficiency, with the differential primer binding efficiency able to be identified and normalized using the 16S DNA standards. However, for this analysis we have assumed no bias in PCR amplification. We next generated a sequenced read library from 16S DNA standards using methods previously described in Example 38. Read abundance was apportioned according to the intended amplicon concentration and sequenced read library was combined with sequenced read library generated from the 16S profiling of the artificial microbe community. We plotted the observed abundance of 16S DNA standards relative to the intended concentration as illustrated Figure 11B. Note that rRNA operon count is required to fully normalize abundance of artificial microbe genome, as illustrated in Figure 11C. This indicates the limit of detection below which any microbe genomes in the companying sample may not be reliably detected.

**Example 58:**

One example method of using DNA standards to calibrate GC bias in sequencing was performed as follows. We designed and manufactured 9 DNA standards that were distinguished into 3 different groups corresponding to ~27%, 68% and 74% GC content (SEQ ID NO: 140-148). All DNA standards are of similar length (1,000nt) to minimize length-specific biases between GC-Meta standards. We combined 9 DNA Standards at equal concentration to form a single mixture using methods previously described in Example 38. This mixture was added to 1% total volume to DNA harvested from soils collected from Watsons Creek and mangrove patch sites in Queensland. Combined DNA samples were prepared as libraries and sequenced using methods previously described in Example 49.

We first aligned sequenced reads to artificial microbe genomes using bwa (Li and Durbin 2009):

>bwa mem -M chrt.bwa sequence.read1.fq sequence.read2.fa / >alignments.sam

We next plotted the abundance aligned reads relative to their GC content, as illustrated in Figure 37. For comparison, we generated simulated reads with a matched length and frequency from the DNA standards. Comparison of sequenced and simulated reads indicates under-sampling of both high GC- and AT-rich standards, as illustrated Figure 37A-C. This difference in observed and expected abundance can inform normalisation to minimise the impact of GC-dependent bias in DNA quantification.

**Example 59:**

One example method of using synthetic DNA standards mimicking TCRγ clonotypes to calibrate immune-repertoire sequencing was performed as follows. TCRγ (TCRG) is a preferential target for clonality analyses due to the relatively restricted suite of clonotypes it generates. In this example we designed, manufactured and used a synthetic TCRG standard during multiplex PCR and immune-receptor sequencing.

We retrieved 10 Vy segments, 5 Jy segments and 2 Cy segments and flanking intronic sequence from TCRG loci in the reference human genome (hg19; Figure 12). Each segment or intronic sequence was separately inverted and shuffled to remove homology to known natural sequences with the exception of sequences complementary to the forward and reverse primer sequences as described in Carlson et. al. 2013. We then combined the synthetic TCRG segments in all forward and reverse primer combinations. Segments were joined together with each interspersed with a single GC rich hairpin sequence designed to retard read-through PCR amplification. The sequences were then combined into 4 larger sequences that were synthesized (SEQ ID NOs: 203-206). Sequences were synthesized in four parts GeneArt (Life Technologies) and inserted into pMA-RQ vector. The four parts of the TCRG standards were ligated into one contiguous sequence into pUC19 using NEBuilder® HiFi DNA Assembly Master Mix (New England Biolabs). The final 14.4kb plasmid was grown up in a 50 mL culture, purified and used for DNA sequence verification. For TCRG standards synthesis, the final plasmid was digested with SapI and the 12kb fragment was gel extracted with Zymoclean™ Gel DNA Recovery Kit (Zymo Research).

The clonal T-ALL cell line, KARPAS 45 (Catalog N. 06072602, Human T-cell Leukaemia) was cultured according to European Collection of Cell Cultures growth protocols and standards. Briefly, KARPAS 45 were cultured in RPMI 1640 medium (Gibco®) supplemented with 15% fetal bovine serum (FBS) at 37°C under 5% $CO_2$. Genomic DNA (gDNA) was extracted from KARPAS 45 using TRIzol (Invitrogen) according to the manufacturer's instruction. The extracted DNA samples were treated with RNase A followed by a cleanup with Genomic DNA Clean & Concentrator kit (Zymo Research). Purified DNA was quantified using the BR dsDNA Qubit Assay on a Qubit 2.0 Fluorometer (Life Technologies). gDNA from a healthy adult's PBMC used as background. Briefly, gDNA was extracted using the MoBio UltraClean kit (Catalog No. 12334-250) according to manufacturer's instructions and eluted in solution TD3. The purified gDNA was analyzed on the Nanodrop (Thermo Scientific) and quantified using the BR dsDNA Qubit Assay on a Qubit 2.0 Fluorometer (Life Technologies).

In order to test the sensitivity, reproducibility and quantitative accuracy of the synthetic TCRG standards in a biological background, a mixture of gDNA from clonal T-ALL cells (KARPAS 45) was diluted to a 10, 1 and 0.1% final concentration with gDNA from a healthy adult's PBMC gDNA (that comprises a complex background of TCRG gentoypes) and 10% synthetic TCRG standards were created as described in Table 12. The individually prepared mixture was used as a template in a multiplex PCR reaction containing equimolar ratios of the VF and JR primer pool, KAPA HiFi HotStart Ready Mix (KAPA Biosystems) according to the manufacturer's recommendations. The PCR product from the multiplex PCR reaction was purified using the DNA Clean & Concentrator™-5 (Zymo Research). The purified PCR product was quantified using the BR dsDNA Qubit Assay on a Qubit 2.0 Fluorometer (Life Technologies) and verified on the Agilent 2100 Bioanalyzer with an Agilent High Sensitivity DNA Kit (Agilent Technologies).

The Nextera XT Sample Prep Kit (Illumina®) was used to prepare DNA libraries according to manufacturer's instructions. Prepared libraries were quantified on Qubit (Invitrogen) and verified on Agilent 2100 Bioanalyzer with a

Agilent High Sensitivity DNA Kit (Agilent Technologies). Libraries were sequenced on a HiSeq 2500 (Illumina®) at the Kinghorn Centre for Clinical Genomics.

Upon receipt of sequencing files, reads were aligned to an index comprising all possible real and synthetic TCRG using the following parameters:

bowtie2 -p 12 -x tcrg_combs -1 10TALL_TCRGstds1.1.fq -2 10TALL_TCRGstds1.2.fq -S 10TALL_TCRGstds1.combs.sam

We first analysed the synthetic TCRG standards. We first determined the relative abundance of each synthetic standard according to alignment frequency. We first noted that products were generated and sequenced from all primer combinations, providing positive control indication of their function.

We can also use the relative abundance of sequenced amplicons to assess the quantitative efficiency of primer combinations. Since all amplicon templates derive from a single sequence, the initial template abundance is uniform, and therefore differences will reflect differences in either primer efficiency and primer abundance in multiplex mixture. Therefore, we assembled a matrix of the relative abundance of each synthetic standard according to alignment frequency (Table 12). This matrix indicates relative performance of each primer pair within the PCR reaction. For example, the V11 forward primer in combination with the J1 reverse primer performs poorly, less than 4.1 times than average, whilst the V9 forward primer in combination with the JP1 reverse primer performs more than 2.15-fold better than average. This provides a normalization factor that can be used to adjust the quantification of the TCRG clonotypes in the accompanying sample.

Notably, this normalisation factor is calculated from internal synthetic controls that are subject to the same conditions; including temperature that defines primer hybridization and the relative primer concentrations in the multiplex primer mixtures. Therefore, we next determined the relative abundance of TCRG clontoypes in the accompanying mixture. Whilst some clonotpyes were absent from the library, we could conclude that they were not in the RNA sample (since we have previously validated each primer with the synthetic standards above). We then adjusted the relative concentration of each TCRG clonotype according to the normalization factor calculated from the synthetic standards above. Thus, the synthetic DNA standards described herein provide a useful calibration of NGS methods directed towards analysis of immune repertoire sequences.

**Example 60:**

One example method of using conjoined synthetic standards as quantitative DNA ladders was performed as follows. As explained above, errors in pipetting can cause variation between the abundance of multiple standards. To remove pipetting errors, individual DNA standards can be joined together. In such a case, differential copy number achieves differential abundance. Dependent variation between individual standards can be used to calculate the error due to variation in pipetting and ensure exact frequencies between alternative standards.

We designed conjoined standards in the following format (summarized in Figure 39). We designed multiple individual DNA standards (A, B, C and D) each of 600nt. These DNA standards were then organized into an ABB or CDD format that could then be joined together into a single contiguous sequence comprising 1 copy A; 2 copies B; 4 copies of C and 8 copies of D (SEQ ID NOs: 207-290). In addition, we added a further small linker sequence that hosts a I-Sce I restriction digestion site between individual DNA standards. This enabled us to liberate individual standards from the multiple standard after pipetting by restriction digestion and thereby generate mixtures of individual standards without variation due to pipetting.

Sequences comprising the combined repeats in the ABB and CDD organization were synthesized individually by Gene Art (Life Technologies). Each conjoint standard consists of one ABB and four CDD's. The five fragments were ligated into pUC19-FAFB (pUC19 with a FAFB filler sequence) using NEBuilder® HiFi DNA Assembly Master Mix according to manufacturer's protocol. The final plasmid of each conjoint standard, e.g., pUC19-FAFB-GA98 is digested with EcoRI and BamHI and subsequently gel extracted with Zymoclean™ Gel DNA Recovery Kit (Zymo Research) to obtain the 10.4kb conjoint DNA standard.

The concentration of all 21 conjoint DNA standards was measured using the BR dsDNA Qubit Assay on a Qubit 2.0 Fluorometer (Life Technologies). The conjoint DNA standards mixtures were combined to form a mixture spanning a $10^6$-fold concentration range using an epMotion 5070 epBlue™ software program to make the final mixtures robotically.

The mixture A was then added to final concentration of 10% with total gDNA extracted from the GM12878 cell line. GM12878 was provided by Madhavi Maddugoda (Epigenetics Research Group, Garvan Institute of Medical Research). GM12878 cells were cultured according to Coriell Cell Repositories growth protocols and standards. Briefly, GM12878 were cultured in RPMI 1640 medium (Gibco®) supplemented with 10% fetal bovine serum (FBS) at 37°C under 5% CO2. DNA was extracted from GM12878 and mouse using TRIzol (Invitrogen) according to the manufacturer's instruction. The extracted DNA samples were treated with RNase A followed by a cleanup with Genomic DNA Clean & Concentrator kit (Zymo Research). Purified DNA was quantified on the Nanodrop (Thermo Scientific).

The Nextera XT Sample Prep Kit (Illumina®) was used to prepare DNA libraries according to the manufacturer's instructions. Prepared libraries were quantified on Qubit (Invitrogen) and verified on Agilent 2100 Bioanalyzer with a Agilent High Sensitivity DNA Kit (Agilent Technologies). Libraries were sequenced on a HiSeq 2500 (Illumina®) at the Kinghorn Centre for Clinical Genomics.

We analysed the sequenced reads from the conjoined synthetic standards as follows. We first aligned sequenced reads to an index (comprising of each individual standard) with the following parameters:

bowtie2 -x conjoined_sequences -1 NGSreads.1.fq -2 NGSreads.2.fq -S output.sam

We next determined the abundance of each individual standard according to the alignment frequency. We then plotted the weighted normalized known concentration of each individual standard (derived from both the concentration of the hosting conjoined standard and the copy number within the conjoined standard) compared to the weighted-normalized measured abundance (Figure 39). This indicated a degree of variation in pipetting. For example, we observe a notable outlier conjoined standard that had been combined in the mixture at greater concentration than expected (indicated in Figure 39B). Given that this outlier equally affects all standards within the conjoined standard indicates that the outlier is due to pipetting, rather than an alternative technical variable and could therefore be removed prior to further analysis.

We determined a correlation of 0.9451 between the known concentration and the measured abundance of standards. We next applied the adjustment to force all individual standards within a conjoined standard to exhibit a slope of 1 (described in detail above). Adjustment improved the distribution of standards, adjusted for outliers, and improved the correlation to 0.9806 (Figure 39C), indicating the improved quantitative accuracy of the DNA standards.

**Example 61:**

One example method of using synthetic standards mimicking fusion gene events was performed as follows. Fusion gene events contribute to many human cancers, however, they can be difficult to identify using RNA sequencing methods. Synthetic RNA standards can be used to emulate fusion genes, and thereby assess the ability to detect fusion genes. In this example we designed, manufactured and used synthetic fusion-gene standards to calibrate an RNA sequencing method.

We selected 24 normal genes (from the list of RNA standards described in Example 36 above). We then assigned a fusion site within the intron of each gene, and paired sites to emulate 12 reciprocal translocation events. These 12 events then generated the sequence for 24 fusion genes (each translocation forms two reciprocal fusion genes; see SEQ ID NOs: 291-314 and Figure 40).

To generate fusion gene sequences hosted in an expression vector, we employed NEBuilder® HiFi DNA Assembly Master Mix (New England Biolabs) according to the manufacturer's protocol. Briefly, 40 μL aliquots of α-Select Silver Efficiency Chemically Competent *E. coli* (Bioline) were thawed on ice and transformed with 2 μL of diluted NEBuilder® HiFi DNA Assembled product per the manufacturer's suggested protocols. Transformed cells were plated on prewarmed 100 μg/mL ampicillin plates and incubated at 37°C overnight (18 hours). One colony from each plate was used to inoculate 5 mL LB broth containing 100 μg/mL ampicillin. Inoculated tubes were incubated overnight on a shaker at 37°C. Plasmids were isolated using the Qiagen Spin Miniprep Kit. The sequence of the purified plasmids was validated with Sanger sequencing.

To generate synthetic RNA standards, we employed an *in vitro* transcription reaction. For RNA synthesis, each plasmid was linearized with EcoRI-HF (New England Biolabs), followed by a Proteinase K treatment. The linearized plasmid was cleaned up using the Zymo ChIP DCC columns (Zymo Research). An *in vitro* transcription reaction was performed to synthesize the RNA transcripts. Full-length RNA transcripts were synthesized using the MEGAscript® Sp6 kit (Life Technologies) according to the manufacturer's instructions. The RNA was purified using a RNA Clean & Concentrator-25 column (Zymo Research) using the manufacturer's >200nt protocol. Purified RNA transcripts were verified on the Agilent 2100 Bioanalyzer with the RNA Nano kit (Agilent Technologies) and comprised stock inventory.

Synthetic fusion-gene standards were diluted to form a mixture spanning $10^6$ fold concentration, including a dynamic range in expression between each other and with the normal parent gene. All RNA Fusion transcripts' concentrations were measured on a Qubit 2.0 Fluorometer (Life Technologies, Carlsbad, CA, USA). The RNA fusion transcripts were pooled using an epMotion 5070 epBlue™ software program to assemble the final mixtures robotically spanning a $10^6$ -fold concentration range. This formed the final mixture stock.

The fusion gene synthetic standard mixtures were spiked into natural RNA samples derived from two human cell-types. K562 and GM12878. K562 and GM12878 cells were cultured according to Coriell Cell Repositories growth protocols and standards. Briefly, K562 and GM12878 were cultured in RPMI 1640 medium (Gibco®) supplemented with 10% fetal bovine serum (FBS) at 37°C under 5% $CO_2$. Total RNA was extracted from K562 and GM12878 using TRIzol (Invitrogen) according to the manufacturer's instructions. DNAse treatment was subsequently performed on each sample with TURBO DNase (Life Technologies) followed by a cleanup with the RNA Clean and Concentrator-25 Kit (Zymo Research). Total RNA was run on an Agilent Bioanalyzer 2100 to assess intactness and both the Nanodrop (Thermo Scientific) and Qubit (Life Technologies) were used to determine the concentration. Only RNA with a RNA integrity number (RIN) > 8.0 was used for library preparation.

K562 RNA contains the known BCR-ABL fusion gene. We generated a serial dilution K562 to GM12878 RNA at a 1:1, 1:10 and 1:100 fold ratio. 1 μg of combined RNA was used in each library preparation.

The RNA Fusion standards were added at 10% of the total RNA concentration of mixtures of K562 and GM12878 before library preparation. The RNA mixture was ribo-depleted using Ribo-Zero™ Magnetic Kit (Human/Mouse/Rat) (Epicentre). The ribo-depleted RNA was used to prepare libraries using KAPA Stranded RNA-Seq Library Preparation Kit for Illumina® platforms (KAPA Biosystems) according to the manufacturer's protocol. Prepared libraries were quantified using the HS dsDNA Qubit Assay on a Qubit 2.0 Fluorometer (Life Technologies, Carlsbad, CA, USA) and verified on Agilent 2100 Bioanalyzer (Agilent Technologies) before samples were pooled for sequencing.

We analysed sequenced reads as follows. First, sequenced reads were aligned to an index comprising both the synthetic chromosome and the human genome sequence (hg38) using Tophat2 aligner with the fusion-search option enabled as follows:

tophat --fusion-search -G gencode.v23.annotation.chrT _rna.gtf hg38.chrT 100K _RFMXA.1.fq 100K_RFMXA.2.fq

We then processed the resulting alignment file (accepted_hits.bam) and fusion.out files to assess synthetic gene performance. We correctly identified 19 (out of 24) fusion genes, whilst the remaining 5 unidentified fusion genes exhibited an abundance below 7.557 attamoles/μl, indicating the limit of sensitivity for fusion-gene discovery in this experiment.

We next plotted the coverage across the fusion junction relative to the known concentration of the fusion genes within the Mixture. We observed a linear relationship, with a Pearson's correlation of 0.9652 and a slope of 1.166, indicating that the fusion gene coverage provides a suitable measure of fusion gene expression (see Figure 40). Using the synthetic fusion genes as a measure, we found that ~21 reads aligns to *FG1_12_P2* fusion gene, which is similar to ~16 reads that align to BCR-ABL gene in the K562 RNA sample, indicating expression of this fusion gene to be low in the accompanying sample (where the K562 RNA is diluted at ~10%) to be ~1.6 attamoles/μl.

**Example 62:**

One example method of using synthetic standards mimicking germline variation was performed as follows. Germline variation in the diploid human genome occurs at largely homozygous and heterozygous allele frequencies. Homozygous genotypes can be represented by a single DNA standard, whilst heterozygous variation, that comprises two alleles at equal frequency, requires two DNA standards. More than two alleles may exist in a population, and a new DNA standard is required to represent each allele. However, because the human genome is diploid (i.e. there are two copies of each autosomal chromosome), only two standards will be required at any one time to mimic the diploid genome of an individual human.

To demonstrate this, we combined DNA standards representing 138 alternative single nucleotide variants (SNVs) at equal (i.e. heterozygous) or single (i.e. homozygous) concentration. The DNA standards were pooled using an epMotion 5070 epBlue™ software program to make the final mixtures robotically. We then added the DNA standards to genomic DNA extracted from the GM12878 human cell line. DNA was extracted from GM12878 and mouse using TRIzol (Invitrogen) according to the manufacturer's instructions. The Nextera XT Sample Prep Kit (illumina®) was used to prepare DNA libraries according to manufacturer's instructions. Prepared libraries were quantified on Qubit (Invitrogen) and verified on Agilent 2100 Bioanalyzer with an Agilent High Sensitivity DNA Kit (Agilent Technologies). Libraries were sequenced on a HiSeq 2500 (illumina®) at the Kinghorn Centre for Clinical Genomics. We then aligned sequenced reads to both the human genome (hg38) and the synthetic chromosome using BWA MEM (Li and Durbin 2009) with default parameters. Resultant alignments were then analyzed using the Genome Analysis Toolkit (GATK) according to best practices. At 30-fold coverage, we identified 89% of homozygous and 71% of heterozygous SNPs in the synthetic chromosome (Figure 41A). Note that this sensitivity of variant detection was similar to the accompanying NA12878 genome, for which we identified 86% of homozygous and 63% of heterozygous SNPs by comparison to previously described variant annotations (Zook, J.M. *et al.,* 2014).

**Example 63:**

One example method of using synthetic standards mimicking somatic mutations was performed as follows. Somatic mutations can underpin numerous conditions, with tumorigenic mutations in cancer being foremost among them. Unlike germ-line mutations, which are either homozygous or heterozygous and exist in all cells of a given individual, somatic mutations may be present in just a fraction of cells (a sub-clonal population) within a tumor sample and may also be confounded by frequent rearrangements and copy number variations in tumor genomes. For example, a tumor may be comprised of multiple clonal cell populations that have distinct genotypes according to their lineage. As a result, somatic mutations can be present across a wide range of different frequencies.

To demonstrate the use of DNA standards representing 138 somatic mutations across a range of frequencies, we combined DNA standards across a two-fold serial dilution relative to reference alleles to establish a scale of allele frequencies from 1:2 (i.e. heterozygous) to 1:4096 (Figure 42A). DNA standards were prepared, mixed and added to the NA12878 genome DNA and sequenced using methods described in Example 62. Libraries were sequenced on a HiSeq 2500 (illumina®) at the Kinghorn Centre for Clinical Genomics. We then aligned sequenced reads to both the human genome (hg38) and the synthetic chromosome using BWA MEM (Li and Durbin 2009) with default parameters. Resultant alignments were then analyzed using VarScan2 (Koboldt *et al.* 2009) with default parameters to identify genetic variation represented by the DNA standards, and quantify their relative frequency (i.e. the variant allele frequency).

We plotted the known concentration of the variants, relative to their measured frequency (Figure 42B). This indicated the accuracy with which variants are identified at different allele frequencies, with the correlation between the expected concentration and the measured abundance indicating the quantitative accuracy with which we measure variant allele frequency, and the limit of sensitivity with which we can identify variants and measure their frequency with accuracy. The scale of allele frequencies provides a reference against which the relative size of clonal sub-populations within an accompanying sample can be assessed.

At a high 25,000-fold coverage, we were able to identify at least one supporting read for all except 2 variants, both of which belong to the rarest allelic fraction (1/4096; Figure 42B). However, at this coverage, we also find >2000 potential false-positive variant calls in the DNA standards, created by sequencing and alignment errors, indicating a requirement to further filter variant candidates. Therefore, we next used the DNA standards to empirically determine the p-value (comprising a Fisher's Exact Test on the read counts supporting reference and variant alleles as performed by VarScan2) threshold according to requisite sensitivity and specificity. For example, a $1 \times 10^{-6}$ p-value threshold provides a sensitivity of 54% and specificity of 82% for identifying somatic variants. However, applying this stringency restricts the sensitivity of the assay to an allele frequency of 1/128 (i.e. a less than 1% frequency; Figure 42C,D).

**Example 64:**

One example method of using synthetic standards mimicking complex genotypes was performed as follows. More complex genotypes can be encountered in cases of chromosomal aneuploidy or when multiple individual genotypes are simultaneously sampled. For example, if we consider DNA circulating in the pregnant mother's blood we detect two overlapping genotypes, the fetus (that constitutes both maternal and paternal alleles) and the mother (that constitute two maternal alleles). Fetal alleles can be observed across a range of concentrations according to both the homozygous and heterozygous allele frequency in conjunction with the fraction of the circulating DNA that derives from the fetus (this can vary from about 1-40% of maternal circulating DNA during gestation). Allele frequencies can be further complicated by chromosomal aneuploidy, where autosomal chromosomes exist at non-diploid frequencies, such as using trisomy 21, the most common genetic congenital abnormality. For example, DNA standards that represent variants on chromosome 21 are added at a 1.5-fold higher frequency than DNA standards that represent variation on other autosomal chromosomes to emulate trisomy 21. Therefore, the allele frequency represented by the DNA standards reflects the combined (i) genotype frequency (i.e. heterozygous or homozygous) (ii) the relative abundance of fetal and maternal DNA in circulation and (iii) copy-number variation (such as chromosomal aneuploidy) in the fetal genome.

We designed 120 DNA standards that represent the constellation of fetal and maternal genotypes (both reference and variant; SEQ ID NOS: 315-434). Each standard is ~160nt long corresponding to the DNA fragment size typically observed in circulation. DNA standards were then combined at a range of concentrations to emulate the relative abundance of fetal and maternal DNA circulating within the pregnant mother's blood (Figure 42E). For example, we combined the two fetal DNA standards at equal concentration to represent a heterozygous genotype, before combining these two standards at a 10% fractional concentration to the maternal DNA standards that thereby represent the remaining 90% of circulating DNA retrieved from the blood.

To further demonstrate this, we generated a simulated library (using methods described in this Example above) from the mixture of DNA standards that represented 120 different variant events. The mixture encompassed the range of 4 different genotype combinations (fetal and maternal homozygous and heterozygous) across a range of different fetal DNA loads (0, 1, 10, 25 and 50%) with the subset of DNA standards representing variation from the human chromosome 21 added at an additional 1.5-fold enrichment to emulate trisomy 21. We aligned sequenced reads to the synthetic chromosome using BWA MEM (Li and Durbin 2009) with default parameters. Resultant alignments were then analyzed using VarScan2 (Koboldt *et al.* 2009) with default parameters to identify genetic variation represented by the DNA standards, and quantify their relative frequency (i.e. the variant allele frequency). Plotting the expected relative to observed genotype frequencies provides a reference scale against which the fetal variants in an accompanying sample can be measured, and inform determination of the fetal genotype and chromosomal aneuploidy.

**Example 65:**

One example method of generating a standard by reversing a template sequence was performed as follows. In particular, the following example describes how a DNA standard was designed to emulate a substitution mutation (G>T) that occurs at 1,849nt in the JAK2 gene (COSM12600) that causes a missense substitution (V617E) in the encoded protein and that is associated with cancer.

To generate a DNA standard, we first retrieved both the reference and variant allele along with ~200nt flanking sequence. To prevent homology to the original loci within the human genome, we reversed the sequence. The reversed DNA sequence for DNA standards representing the COSM12600 reference allele is described in SEQ ID NO: 435 and the variant allele is described in SEQ ID NO: 436.

We next identified sub-sequences within the DNA standards that retain significant homology to the human genome due to chance. We identified a 35nt small region of the DNA standard sequence (TTCTGATTCCTTTTTTTTTT CATGTTTCTTAACA (SEQ ID NO: 437)) that has significant (E-value > 0.01) homology. This sequence was then modified by either (i) shuffling whereby nucleotides are shuffled into a new order to remove homology (for example CTTATTTT TTTCATTCTGTTCCTATATTTTCGAT (SEQ ID NO: 438)) (ii) substitution whereby all G are substituted to C, all C are substituted to G, all A are substituted to T and all T are substituted to A (for example GAATAAAAAAAGTAAGACAAG GATATAAAAGCTA (SEQ ID NO: 439)). In this case, shuffling maintains the same nucleotide content as the original sequence, but abolishes any sequence repetitiveness, whilst substitution maintains sequence repetitiveness, but modifies nucleotide composition (however, the relative pyrimidine and purine content is maintained). The final DNA sequence for DNA standards representing the COSM12600 reference allele is described in SEQ ID NO: 440 and the variant allele is described in SEQ ID NO: 441.

We can similarly use this method to design DNA standards for any mutations. As illustrative examples, we have generated DNA standards to represent a range of mutations with clinical importance, including mutations in *BRAF* (COSM476; SEQ ID NO: 442, SEQ ID NO: 443), *KRAS* (COSM521; SEQ ID NO: 444, SEQ ID NO: 445), *IDH1* (COSM28746; SEQ ID NO: 446, SEQ ID NO: 447), *EGFR* (COSM6224; SEQ ID NO: 448, SEQ ID NO: 449), *FGFR3* (COSM715; SEQ ID NO: 450, SEQ ID NO: 451), *PIK3CA* (COSM775; SEQ ID NO: 452, SEQ ID NO: 453), *MYD88* (COSM85940; SEQ ID NO: 454, SEQ ID NO: 455), *KIT* (COSM1314; SEQ ID NO: 456, SEQ ID NO: 457), *CTNNB1* (COSM5664; SEQ ID NO: 458, SEQ ID NO: 459), *NRAS* (COSM584; SEQ ID NO: 460, SEQ ID NO: 461), *DAMT3A* (COSM52944; SEQ ID NO: 462, SEQ ID NO: 463) and *FOXL2* (COSM33661; SEQ ID NO: 464, SEQ ID NO: 465).

**Example 66:**

One example method of generating a standard mimicking small or large scale genetic variation by reversing a template sequence was performed as follows. In representing a larger structural genetic event, such as a deletion or an insertion, it can be important to maintain the sequence repetitiveness and structure surrounding the mutation, since local read alignment can be highly important to allow resolution of the structure of the large variant. Therefore, the reversion and/or substitution of a template sequence to generate DNA standards presents a particularly advantageous method to represent a large structural variants and maintain the often complex architecture and repetitive sequence structure observed in natural large structural variants.

This example describes how a DNA standard was designed to emulate a 17nt deletion (GAATTAAGAGAAGCAA (SEQ ID NO: 466); COSM6223) in the *EGRF* gene. We first retrieved 200nt of sequence flanking the reference and the variant (i.e. with the 17nt deletion) *EGRF* sequence. We then reversed the sequence to 3' to 5' and secondly substituted any nucleotides that retained homology (despite sequence reversal) to the human genome by chance. The final DNA standard sequence that represents the *EGRF* deletion (COSM6223) is provided in SEQ ID NO: 467 (reference) and SEQ ID NO: 468 (variant).

Importantly, DNA standards that represent insertions events are required to reverse (from 3' to 5') not only the sequence flanking the insertion breakpoint site, but also reverse the sequence that is inserted into the breakpoint. To demonstrate this, we designed DNA standards that represent a 14nt insertion (COSM20959) that occurs in the *ERBB2* gene. In this case, we retrieved the 200nt sequence flanking the mutation as well as the variant insertion sequence (CATACGTGATGGC (SEQ ID NO: 469)). The reference sequence and the variant sequence (containing the insertion) were then reversed, with subsequent substitution of nucleotides to any subsequences that retained homology to the human genome by chance. The final DNA standard sequence that represents the *ERBB2* insertion is provided in SEQ ID NO: 470 (reference) and SEQ ID NO: 471 (variant).

As illustrative examples, we have generated DNA standard sequences to represent a range of structural variants with clinical importance, including insertions and deletions in the *EGFR* (COSM6223; SEQ ID NO: 472, SEQ ID NO: 473), *IL7R* (COSM214586; SEQ ID NO: 474, SEQ ID NO: 475), *IL6ST* (COSM251361; SEQ ID NO: 476, SEQ ID NO: 477), *KIT* (COSM1326; SEQ ID NO: 478, SEQ ID NO: 479) genes.

Those skilled in the art will appreciate that the disclosure described herein is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features. It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive. The invention is defined by the scope of the appended claims.

**Tables:**

**Table 1.** Concentration of individual RNA Standards added to form Mixture A and B. Quantitative difference between Mixtures also indicated.

| SEQ ID Internal Id | | Concentration In Mix A (Attomoles/ul) | Concentration In Mix B (Attomoles/ul) | Expected Fold-Change Ratio | Log2 (Mix A/Mix B) |
|---|---|---|---|---|---|
| 1 | R_1_1_R | 10000 | 2500 | 4.00 | 2.00 |
| 2 | R_1_1_V | 10000 | 2500 | 4.00 | 2.00 |
| 3 | R_L2_R | 15000 | 1250 | 12.00 | 3.58 |
| 4 | R_1_2_V | 5000 | 3750 | 1.33 | 0.42 |
| 5 | R_L3_R | 5000 | 3750 | 1.33 | 0.42 |
| 6 | R_1_3_V | 15000 | 1250 | 12.00 | 3.58 |
| 7 | R_1_4_V | 15000 | 15000 | 1.00 | 1.00 |
| 8 | R_2_1_R | 625 | 312.5 | 2.00 | 1.00 |
| 9 | R_2_1_V | 625 | 312.5 | 2.00 | 1.00 |
| 10 | R_2_2_R | 312.5 | 468.75 | 0.67 | -0.58 |
| 11 | R_2_2_V | 937.5 | 156.25 | 6.00 | 2.58 |
| 12 | R_2_3_R | 937.5 | 156.25 | 6.00 | 2.58 |
| 13 | R_2_3_V | 312.5 | 468.75 | 0.67 | -0.58 |
| 14 | R_2_4_R | 1250 | 625 | 2.00 | 1.00 |
| 15 | R_3_1_R | 156.25 | 78.125 | 2.00 | 1.00 |
| 16 | R_3_1_V | 156.25 | 78.125 | 2.00 | 1.00 |
| 17 | R_3_2_R | 78.125 | 117.1875 | 0.67 | -0.58 |
| 18 | R_3_2_V | 234.375 | 39.0625 | 6.00 | 2.58 |
| 19 | R_3_3_R | 234.375 | 39.0625 | 6.00 | 2.58 |
| 20 | R_3_3_V | 78.125 | 117.1875 | 0.67 | -0.58 |
| 21 | R_4_1_R | 58.59375 | 9.765625 | 6.00 | 2.58 |
| 22 | R_4_1_V | 19.53125 | 29.296875 | 0.67 | -0.58 |
| 23 | R_4_2_R | 39.0625 | 19.53125 | 2.00 | 1.00 |
| 24 | R_4_2_V | 39.0625 | 19.53125 | 2.00 | 1.00 |
| 25 | R_4_3_R | 19.53125 | 29.296875 | 0.67 | -0.58 |
| 26 | R_4_3_V | 58.59375 | 9.765625 | 6.00 | 2.58 |
| 27 | R_5_1_R | 4.8828125 | 14.6484375 | 0.33 | -1.58 |
| 28 | R_5_1_V | 14.6484375 | 4.8828125 | 3.00 | 1.58 |
| 29 | R_5_2_R | 14.6484375 | 4.8828125 | 3.00 | 1.58 |
| 30 | R_5_2_V | 4.8828125 | 14.6484375 | 0.33 | -1.58 |
| 31 | R_5_3_R | 19.53125 | 19.53125 | 1.00 | 0.00 |
| 32 | R_6_1_R | 2.44140625 | 2.44140625 | 1.00 | 0.00 |
| 33 | R_6_1_V | 2.44140625 | 2.44140625 | 1.00 | 0.00 |
| 34 | R_6_2_R | 1.220703125 | 3.662109375 | 0.33 | -1.58 |
| 35 | R_6_2_V | 3.662109375 | 1.220703125 | 3.00 | 1.58 |
| 36 | R_6_3_R | 3.662109375 | 1.220703125 | 3.00 | 1.58 |
| 37 | R_6_3_V | 1.220703125 | 3.662109375 | 0.33 | -1.58 |
| 38 | R_7_1_R | 0.915527344 | 0.305175781 | 3.00 | 1.58 |
| 39 | R_7_1_V | 0.305175781 | 0.915527344 | 0.33 | -1.58 |
| 40 | R_7_2_R | 0.610351563 | 0.610351563 | 1.00 | 0.00 |
| 41 | R_7_2_V | 0.610351563 | 0.610351563 | 1.00 | 0.00 |
| 42 | R_7_3_R | 0.305175781 | 0.915527344 | 0.33 | -1.58 |
| 43 | R_7_3_V | 0.915527344 | 0.305175781 | 3.00 | 1.58 |

(continued)

| | SEQ ID Internal Id | Concentration In Mix A (Attomoles/ul) | Concentration In Mix B (Attomoles/ul) | Expected Fold-Change Ratio | Log2 (Mix A/Mix B) |
|---|---|---|---|---|---|
| 44 | R_1_4_R | 0.305175781 | 0.610351563 | 0.50 | -1.00 |
| 45 | R_8_1_R | 0.152587891 | 0.305175781 | 0.50 | -1.00 |
| | SEQ ID Internal Id | Concentration In Mix A (Attomoles/ul) | Concentration In Mix B (Attomoles/ul) | Expected Fold-Change Ratio | Log2 )Mix A/MMix B) |
| 46 | R_8_1_V | 0.152587891 | 0.305175781 | 0.50 | -1.00 |
| 47 | R_8_2_R | 0.076293945 | 0.457763672 | 0.17 | -2.58 |
| 48 | R_8_2_V | 0.228881836 | 0.152587891 | 1.50 | 0.58 |
| 49 | R_8_3_R | 0.228881836 | 0.152587891 | 1.50 | 0.58 |
| 50 | R_8_3_V | 0.076293945 | 0.457763672 | 0.17 | -2.58 |
| 51 | R_9_1_R | 0.038146973 | 0.076293945 | 0.50 | -1.00 |
| 52 | R_9_1_V | 0.038146973 | 0.076293945 | 0.50 | -1.00 |
| 53 | R_9_2_R | 0.019073486 | 0.114440918 | 0.17 | -2.58 |
| 54 | R_9_2_V | 0.057220459 | 0.038146973 | 1.50 | 0.58 |
| 55 | R_9_3_R | 0.057220459 | 0.038146973 | 1.50 | 0.58 |
| 56 | R_9_3_V | 0.019073486 | 0.114440918 | 0.17 | -2.58 |
| 57 | R_10_1_R | 2500 | 625 | 4.00 | 2.00 |
| 58 | R_10_1_V | 2500 | 625 | 4.00 | 2.00 |
| 59 | R_10_2_R | 1250 | 937.5 | 1.33 | 0.42 |
| 60 | R_10_2_V | 3750 | 312.5 | 12.00 | 3.58 |
| 61 | R_10_3_R | 3750 | 312.5 | 12.00 | 3.58 |
| 62 | R_10_3_V | 1250 | 937.5 | 1.33 | 0.42 |

**Table 2.** Analysis of libraries comprising RNA Standards with accompanying RNA sample. Statistics determined from the alignment, assembly and quantification of both RNA standards and endogenous genes.

| Library / RNA Standards Mixture | Simulated Rep1 | | RNA Standards Flat | | K562 MixA | | GM12878 MixB | | Mouse Liver MixA | | Lung Normal MixB | | Lung Cancer MixA | | Lung Normal ERCC Mix 1 | | Lunc Cancer ERCC Mix 2 | |
|---|---|---|---|---|---|---|---|---|---|---|---|---|---|---|---|---|---|---|
| **Overview** | | | | | | | | | | | | | | | | | | |
| Reads to Genome | 1,000,000 | | 22,025,286 | | 2,183,367 | | 1,412,061 | | 225,787 | | 46,351 | | 189,582 | | 364,269 | | 200,078 | |
| Reads to Artificial | 9,000,000 | | 0 | | 131,557,592 | | 88,572,155 | | 14,983,308 | | 48,348,495 | | 81,862,028 | | 82,051,730 | | 48,394,966 | |
| Fraction Dilution | 0.1000 | | 1.0000 | | 0.0163 | | 0.0157 | | 0.0148 | | 0.0010 | | 0.0023 | | 0.0044 | | 0.0041 | |
| **Alignment** | Sn | Sp | Sn | Sp | Sn | Sp | Sn | Sp | Sn | Sp | Sn | Sp | Sn | Sp | Sn | Sp | Sn | Sp |
| All Alignments | 0.98 | 0.99 | 0.98 | 0.96 | 0.81 | 0.84 | 0.89 | 0.83 | 0.64 | 0.86 | 0.57 | 0.78 | 0.63 | 0.80 | 0.84 | 0.99 | 0.78 | 0.99 |
| Spliced Alignments | 0.99 | 0.99 | 0.99 | 0.93 | 0.86 | 0.85 | 0.92 | 0.82 | 0.72 | 0.94 | 0.61 | 0.84 | 0.70 | 0.84 | - | - | - | - |
| Detection Limit | - | - | - | - | 0.0796 | - | 0.0018 | - | 0.0181 | - | 0.0049 | - | 0.0112 | - | | | | |
| **Assembly** | | | | | | | | | | | | | | | | | | |
| Base level | 0.89 | 1.00 | 0.86 | 1.00 | 0.71 | 0.95 | 0.75 | 0.95 | 0.56 | 0.97 | 0.47 | 0.92 | 0.51 | 0.92 | - | - | - | - |
| Exon level | | 0.99 | 0.80 | 0.98 | 0.70 | 0.77 | 0.75 | 0.79 | 0.60 | 0.86 | 0.45 | 0.67 | 0.53 | 0.73 | - | - | - | - |
| Intron level | | 1.00 | 0.79 | 1.00 | 0.72 | 0.95 | 0.77 | 0.98 | 0.63 | 0.98 | 0.49 | 0.92 | 0.56 | 0.94 | - | - | - | - |
| Intron chain level | | 0.89 | 0.67 | 0.66 | 0.40 | 0.38 | 0.50 | 0.48 | 0.40 | 0.50 | 0.28 | 0.35 | 0.33 | 0.42 | - | - | - | - |
| Locus level | | 0.85 | 0.72 | 0.59 | 0.53 | 0.21 | 0.59 | 0.23 | 0.47 | 0.33 | 0.38 | 0.18 | 0.41 | 0.19 | - | - | - | - |
| Missed exons | 0.08 | - | 0.16 | - | 0.22 | - | 0.17 | - | 0.35 | - | 0.48 | - | 0.42 | - | - | - | - | - |
| Missed Introns | 0.06 | - | 0.18 | - | 0.19 | - | 0.17 | - | 0.33 | - | 0.43 | - | 0.22 | - | - | - | - | - |
| Missed Loci | 0.00 | - | 0.06 | - | 0.22 | - | 0.13 | - | 0.34 | - | 0.34 | - | 0.25 | - | - | - | - | - |
| **Expression** | | | | | | | | | | | | | | | | | | |
| **Gene** Correlation | - | - | - | - | 0.95 | - | 0.95 | - | 0.94 | - | 0.90 | - | 0.93 | - | 0.95 | - | 0.92 | - |
| Slope | - | - | - | - | 0.91 | - | 0.96 | - | 0.91 | - | 0.92 | - | 0.94 | - | 0.92 | - | 0.89 | - |
| Y-int | - | - | - | - | 1.53 | - | 1.66 | - | 1.45 | - | 1.73 | - | 1.38 | - | 1.42 | - | 1.45 | - |
| **Isoform** Correlation | - | - | - | - | 0.93 | - | 0.87 | - | 0.93 | - | 0.73 | - | 0.90 | - | - | - | - | - |
| Slope | - | - | - | - | 0.86 | - | 0.84 | - | 0.83 | - | 0.75 | - | 0.86 | - | - | - | - | - |
| Y-int | - | - | - | - | -1.08 | - | 2.16 | - | 1.63 | - | -1.08 | - | 1.53 | - | - | - | - | - |
| **Alt. Splicing** Correlation | - | - | - | - | 0.64 | - | 0.49 | - | 0.57 | - | 0.20 | - | 0.78 | - | - | - | - | - |
| Slope | - | - | - | - | 0.84 | - | 0.62 | - | 0.78 | - | 0.52 | - | 0.97 | - | - | - | - | - |

**Table 3.** Analysis of RNA Standards between Mixture A and Mixture B measures quantitative accuracy of fold changes in gene expression and alternative splicing.

| Sample (with Mixture A) vs. Sample (with Mixture B) | 3 x K562 vs. 3 x GM12878 | 3 x Lung Normal vs. 3 x Lung Adenocarcinoma |
|---|---|---|
| **Gene Fold Change** | | |
| Correlation | 0.703 | 0.611 |
| Slope | 0.881 | 0.786 |
| Y-int | 0.529 | -1.020 |
| **Isofrom Fold Change** | | |
| Correlation | 0.850 | 0.670 |
| Slope | 0.750 | 0.450 |
| Y-int | -1.080 | 0.352 |

**Table 4.** Using RNA and DNA Standards with genomes from a range of different organisms. Indicated statistics determined from the alignment of sequenced reads to the in silico chromosome in the presence of reference genomes that represent the majority of taxa. Sn - Senstivity; Sp - Specificity

| Taxa | Human | Mouse | Fish | Fly | Worm | Plant | Yeast | Bacteria | Archaea |
|---|---|---|---|---|---|---|---|---|---|
| Genome | *hg19* | *mm10* | *danRer7* | *dm3* | *ce10* | *tair9* | *Sacer6* | *eschColiK12* | *methKand1* |
| **RNA Standards** | | | | | | | | | |
| Chr Sp. | 0.999 | 0.995 | 0.995 | 0.992 | 0.996 | 1.000 | 0.999 | 0.999 | 0.999 |
| Base Sn. | 0.992 | 0.992 | 0.992 | 0.992 | 0.992 | 0.992 | 0.992 | 0.992 | 0.992 |
| Base Sp. | 0.981 | 0.983 | 0.982 | 0.979 | 0.972 | 0.977 | 0.971 | 0.983 | 0.982 |
| Splice Sn. | 0.997 | 0.997 | 0.997 | 0.997 | 0.997 | 0.997 | 0.997 | 0.997 | 0.997 |
| Splice Sp. | 0.942 | 0.942 | 0.942 | 0.942 | 0.942 | 0.942 | 0.942 | 0.942 | 0.942 |
| **DNA Standards** | | | | | | | | | |
| Chr Sp. | 0.999 | 0.999 | 0.999 | 0.999 | 0.999 | 0.999 | 0.999 | 0.999 | 0.999 |
| Base Sn. | 0.989 | 0.989 | 0.989 | 0.989 | 0.989 | 0.989 | 0.989 | 0.989 | 0.989 |
| Base Sp. | 0.987 | 0.986 | 0.984 | 0.987 | 0.982 | 0.0987 | 0.983 | 0.992 | 0.993 |

**Table 5.** Concentration of individual DNA Standards added to form Mixture A and B. Quantitative difference between Mixtures also indicated.

| SEQ ID | Internal Id | Concentration In Mix A | Concentration In Mix B | Expected Fold-Change Ratio | Log2 (Mix A/Mix B) |
|---|---|---|---|---|---|
| 63 | D_1_1_R | 1.52 | 4.57 | 0.30 | -1.60 |
| 64 | D_1_1_V | 1.52 | 4.57 | 0.30 | -1.60 |
| 65 | D_1_2_R | 123.46 | 370.37 | 0.30 | -1.60 |
| 66 | D_1_2_V | 123.46 | 370.37 | 0.30 | -1.60 |
| 67 | D_1_3_R | 41.15 | 123.46 | 0.30 | -1.60 |
| 68 | D_1_3_V | 41.15 | 123.46 | 0.30 | -1.60 |
| 69 | D_1_4_R | 13.72 | 13.72 | 1.00 | 0.00 |
| 70 | D_1_4_V | 13.72 | 13.72 | 1.00 | 0.00 |
| 71 | D_1_5_R | 4.57 | 1.52 | 3.00 | 1.60 |
| 72 | D_1_5_V | 4.57 | 1.52 | 3.00 | 1.60 |
| 73 | D_1_6_R | 10,000.00 | 10,000.00 | 1.00 | 0.00 |
| 74 | D_1_6_V | 10,000.00 | 10,000.00 | 1.00 | 0.00 |
| 75 | D_1_7_R | 0.51 | 0.06 | 9.00 | 3.20 |
| 76 | D_1_7_V | 0.51 | 0.06 | 9.00 | 3.20 |
| 77 | D_1_8_R | 0.17 | 0.51 | 0.30 | -1.60 |
| 78 | D_1_8_V | 0.17 | 0.51 | 0.30 | -1.60 |
| 79 | D_1_9_R | 74.07 | 24.69 | 3.00 | 1.60 |
| 80 | D_1_9_V | 8.23 | 222.22 | 0.00 | -4.80 |
| 81 | D_1_10_R | 3,333.33 | 1,111.11 | 3.00 | 1.60 |
| 82 | D_1_10_V | 3,333.33 | 1,111.11 | 3.00 | 1.60 |
| 83 | D_1_11_R | 1,111.11 | 3,333.33 | 0.30 | -1.60 |
| 84 | D_1_11_V | 1,111.11 | 3,333.33 | 0.30 | -1.60 |
| 85 | D_1_12_R | 370.37 | 41.15 | 9.00 | 3.20 |
| 86 | D_1_12_V | 370.37 | 41.15 | 9.00 | 3.20 |
| 87 | D_2_1_R | 0.30 | 8.23 | 0.00 | -4.80 |
| 88 | D_2_1_V | 2.74 | 0.91 | 3.00 | 1.60 |
| 89 | D_2_2_R | 24.69 | 666.67 | 0.00 | -4.80 |
| 90 | D_2_2_V | 222.22 | 74.07 | 3.00 | 1.60 |
| 91 | D_2_3_R | 0.06 | 0.17 | 0.30 | -1.60 |
| 92 | D_2_3_V | 0.06 | 0.17 | 0.30 | -1.60 |
| 93 | D_2_4_R | 2.74 | 24.69 | 0.10 | -3.20 |
| 94 | D_2_4_V | 24.69 | 2.74 | 9.00 | 3.20 |
| 95 | D_2_5_R | 8.23 | 0.30 | 27.00 | 4.80 |
| 96 | D_2_5_V | 0.91 | 2.74 | 0.30 | -1.60 |
| 97 | D_2_6_R | 2,000.00 | 18,000.00 | 0.10 | -3.20 |
| 98 | D_2_6_V | 18,000.00 | 2,000.00 | 9.00 | 3.20 |
| 99 | D_2_7_R | 0.91 | 0.01 | 81.00 | 6.30 |
| 100 | D_2_7_V | 0.10 | 0.10 | 1.00 | 0.00 |
| 101 | D_2_8_R | 0.03 | 0.91 | 0.00 | -4.80 |
| 102 | D_2_8_V | 0.30 | 0.10 | 3.00 | 1.60 |
| 103 | D_2_9_R | 2.47 | 733.33 | 0.00 | -8.20 |
| 104 | D_2_9_V | 244.44 | 7.41 | 33.00 | 5.00 |
| 105 | D_2_10_R | 6,000.00 | 222.22 | 27.00 | 4.80 |
| 106 | D_2_10_V | 666.67 | 2,000.00 | 0.30 | -1.60 |
| 107 | D_2_11_R | 222.22 | 6,000.00 | 0.00 | -4.80 |
| 108 | D 2 11 V | 2,000.00 | 666.67 | 3.00 | 1.60 |

(continued)

| SEQ ID | Internal Id | Mix A (Attomoles/ul) | Mix B (Attomoles/ul) | Expected Fold-Change Ratio | Log2 (Mix A/Mix B) |
|---|---|---|---|---|---|
| 109 | D_2_12_R | 666.67 | 8.23 | 81.00 | 6.30 |
| 110 | D_2_12_V | 74.07 | 74.07 | 1.00 | 0.00 |
| 111 | D_3_1_R | 0.03 | 9.05 | 0.00 | -8.20 |
| 112 | D_3_1_V | 3.02 | 0.09 | 33.00 | 5.00 |
| 113 | D_3_2_R | 0.10 | 0.03 | 3.00 | 1.60 |
| 114 | D_3_2_V | 0.01 | 0.30 | 0.00 | -4.80 |
| 115 | D_3_3_R | 81.48 | 2.47 | 33.00 | 5.00 |
| 116 | D_3_3_V | 0.82 | 244.44 | 0.00 | -8.20 |
| 117 | D_3_4_R | 0.27 | 27.16 | 0.00 | -6.60 |
| 118 | D_3_4_V | 27.16 | 0.27 | 99.00 | 6.60 |
| 119 | D_3_5_R | 9.05 | 0.03 | 297.00 | 8.20 |
| 120 | D_3_5_V | 0.09 | 3.02 | 0.00 | -5.00 |
| 121 | D_3_6_R | 200.00 | 19,800.00 | 0.00 | -6.60 |
| 122 | D_3_6_V | 19,800.00 | 200.00 | 99.00 | 6.60 |
| 123 | D_3_7_R | 1.01 | 0.00 | 891.00 | 9.80 |
| 124 | D_3_7_V | 0.01 | 0.11 | 0.10 | -3.50 |
| 125 | D_3_8_R | 0.00 | 1.01 | 0.00 | -8.20 |
| 126 | D_3_8_V | 0.34 | 0.01 | 33.00 | 5.00 |
| 127 | D_3_9_R | 6,600.00 | 22.22 | 297.00 | 8.20 |
| 128 | D_3_9_V | 66.67 | 2,200.00 | 0.00 | -5.00 |
| 129 | D_3_10_R | 0.11 | 0.00 | 33.00 | 5.00 |
| 130 | D_3_10_V | 0.00 | 0.34 | 0.00 | -8.20 |
| 131 | D_3_11_R | 22.22 | 6,600.00 | 0.00 | -8.20 |
| 132 | D_3_11_V | 2,200.00 | 66.67 | 33.00 | 5.00 |
| 133 | D_3_12_R | 733.33 | 0.82 | 891.00 | 9.80 |
| 134 | D_3_12_V | 7.41 | 81.48 | 0.10 | -3.50 |

**Table 6.** Analysis of libraries comprising DNA Standards with accompanying genome DNA sample. Statistics determined from the alignment, assembly and quantification of both DNA standards and endogenous genomes are indicated.

| DNA Standards Mixture | Library Simulated Flat | DNA Standards Flat | GM12878 MixA | Mouse Liver MixA | Lung Normal MixA | Lung Cancer MixB | Structural Variants Flat |
|---|---|---|---|---|---|---|---|
| **Overview** | | | | | | | |
| Reads to Genome | - | - | 458521347 | 11799567 | 221843673 | 233622706 | 458521347 |
| Reads to *Artificial* | 4000000 | 123408715 | 2029597 | 538524 | 1532753 | 2119754 | 22291561 |
| Fraction Dilution | - | - | 0.004 | 0.046 | 0.009 | 0.009 | 0.007 |
| **Alignment** | | | | | | | |
| Sensitivity | 0.994 | 0.997 | 0.849 | 0.789 | 0.823 | 0.854 | 0.916 |
| Specificity | 1.000 | 0.975 | 0.961 | 0.958 | 0.980 | 0.984 | 0.943 |
| **Variation** | | | | | | | |
| Covered | 1.000 | 1.000 | 0.880 | 0.720 | 0.890 | 0.910 | - |
| Detection Sensitivity | 1.000 | 1.000 | 0.650 | 0.570 | 0.730 | 0.780 | - |
| Efficiency | 1.000 | 1.000 | 0.730 | 0.680 | 0.780 | 0.810 | - |
| Specificity | 1.000 | 0.780 | 0.570 | 0.410 | 0.650 | 0.710 | - |
| **Aundance** | | | | | | | |
| Correlation | - | - | 0.949 | 0.967 | 0.980 | 0.990 | - |
| Slope | - | - | 0.910 | 0.950 | 1.030 | 1.010 | - |
| Detection Limit | - | - | 0.004 | 0.012 | 0.002 | 0.002 | - |

**Table 7.** Multiple individual DNA standards can be ligated together to form conjoined DNA standards. The analysis of dependent and independent variation between multiple individual DNA standards that are ligated together enables the discernment of pipetting error and other error sources. Indicated are the designs of conjoined DNA standards and the calculation of pipetting / other error.

| Cojoined DNA Standard Design | | | | | Analysis of Conjoined DNA Standards | | | | | Analysis of Individual DNA Standards | | |
|---|---|---|---|---|---|---|---|---|---|---|---|---|
| DNA ID | Conj. Group | Conc. | Copy No. | Exp. Abun. | Pip. Error | Other Error | Obs. Abun. | Conj. Slope | Norm. Abun. | Pip. Error | Other Error | Obs. Abun. |
| 1 | A | 1 | 1 | 1 | 1.19 | 1.00 | 1.18 | 1.19 | 0.99 | 1.19 | 1.00 | 1.17 |
| 2 | A | 1 | 2 | 2 | 1.19 | 1.00 | 2.38 | 1.19 | 2.00 | 0.88 | 1.00 | 1.77 |
| 3 | A | 1 | 3 | 3 | 1.19 | 1.02 | 3.62 | 1.19 | 3.05 | 1.15 | 1.02 | 3.56 |
| 4 | A | 1 | 4 | 4 | 1.19 | 0.98 | 4.67 | 1.19 | 3.93 | 0.98 | 0.98 | 3.79 |
| 5 | A | 1 | 5 | 5 | 1.19 | 1.00 | 5.94 | 1.19 | 5.00 | 0.88 | 1.00 | 4.42 |
| 6 | A | 1 | 6 | 6 | 1.19 | 1.01 | 7.21 | 1.19 | 6.07 | 1.19 | 1.01 | 7.29 |
| 7 | B | 2 | 1 | 2 | 0.98 | 1.00 | 1.96 | 0.98 | 1.99 | 1.19 | 1.00 | 2.36 |
| 8 | B | 2 | 2 | 4 | 0.98 | 1.02 | 3.99 | 0.98 | 4.06 | 1.19 | 1.02 | 4.91 |
| 9 | B | 2 | 3 | 6 | 0.98 | 1.00 | 5.91 | 0.98 | 6.02 | 0.84 | 1.00 | 5.08 |
| 10 | B | 2 | 4 | 8 | 0.98 | 0.99 | 7.73 | 0.98 | 7.87 | 1.12 | 0.99 | 8.67 |
| 11 | B | 2 | 5 | 10 | 0.98 | 1.00 | 9.81 | 0.98 | 9.99 | 0.88 | 1.00 | 8.82 |
| 12 | B | 2 | 6 | 12 | 0.98 | 1.00 | 11.81 | 0.98 | 12.03 | 1.14 | 1.00 | 13.73 |
| 13 | C | 3 | 1 | 3 | 1.03 | 0.98 | 3.04 | 1.02 | 2.97 | 1.12 | 0.98 | 3.27 |
| 14 | C | 3 | 2 | 6 | 1.03 | 0.98 | 6.06 | 1.02 | 5.93 | 0.84 | 0.98 | 4.90 |
| 15 | C | 3 | 3 | 9 | 1.03 | 0.99 | 9.13 | 1.02 | 8.93 | 0.88 | 0.99 | 7.78 |
| 16 | C | 3 | 4 | 12 | 1.03 | 1.01 | 12.41 | 1.02 | 12.13 | 0.88 | 1.01 | 10.76 |
| 17 | C | 3 | 5 | 15 | 1.03 | 1.01 | 15.65 | 1.02 | 15.30 | 1.12 | 1.01 | 17.36 |
| 18 | C | 3 | 6 | 18 | 1.03 | 0.99 | 18.42 | 1.02 | 18.00 | 1.12 | 0.99 | 20.02 |
| 19 | D | 4 | 1 | 4 | 0.84 | 0.98 | 3.31 | 0.85 | 3.92 | 1.06 | 0.98 | 4.07 |
| 20 | D | 4 | 2 | 8 | 0.84 | 1.02 | 6.83 | 0.85 | 8.09 | 0.98 | 1.02 | 8.05 |
| 21 | D | 4 | 3 | 12 | 0.84 | 1.01 | 10.20 | 0.85 | 12.07 | 1.12 | 1.01 | 13.65 |
| 22 | D | 4 | 4 | 16 | 0.84 | 1.00 | 13.42 | 0.85 | 15.88 | 1.12 | 1.00 | 17.71 |
| 23 | D | 4 | 5 | 20 | 0.84 | 1.02 | 17.12 | 0.85 | 20.27 | 1.14 | 1.02 | 23.45 |
| 24 | D | 4 | 6 | 24 | 0.84 | 1.00 | 20.23 | 0.85 | 23.95 | 1.06 | 1.00 | 25.31 |
| 25 | E | 5 | 1 | 5 | 1.14 | 1.02 | 5.78 | 1.15 | 5.05 | 0.88 | 1.02 | 4.53 |
| 26 | E | 5 | 2 | 10 | 1.14 | 1.02 | 11.55 | 1.15 | 10.09 | 1.15 | 1.02 | 11.82 |
| 27 | E | 5 | 3 | 15 | 1.14 | 1.02 | 17.35 | 1.15 | 15.15 | 0.98 | 1.02 | 15.12 |
| 28 | E | 5 | 4 | 20 | 1.14 | 0.98 | 22.35 | 1.15 | 19.52 | 0.98 | 0.98 | 18.83 |
| 29 | E | 5 | 5 | 25 | 1.14 | 1.01 | 28.73 | 1.15 | 25.09 | 1.19 | 1.01 | 30.20 |
| 30 | E | 5 | 6 | 30 | 1.14 | 1.00 | 34.02 | 1.15 | 29.71 | 1.03 | 1.00 | 30.49 |
| 31 | F | 6 | 1 | 6 | 1.19 | 0.98 | 7.02 | 1.20 | 5.87 | 0.77 | 0.98 | 4.43 |
| 32 | F | 6 | 2 | 12 | 1.19 | 1.02 | 14.54 | 1.20 | 12.17 | 0.98 | 1.02 | 12.14 |
| 33 | F | 6 | 3 | 18 | 1.19 | 1.00 | 21.44 | 1.20 | 17.94 | 1.19 | 1.00 | 21.37 |
| 34 | F | 6 | 4 | 24 | 1.19 | 0.99 | 28.41 | 1.20 | 23.77 | 0.98 | 0.99 | 23.18 |
| 35 | F | 6 | 5 | 30 | 1.19 | 1.02 | 36.25 | 1.20 | 30.34 | 0.84 | 1.02 | 25.90 |
| 36 | F | 6 | 6 | 36 | 1.19 | 1.02 | 43.51 | 1.20 | 36.41 | 0.77 | 1.02 | 28.37 |
| 37 | G | 7 | 1 | 7 | 1.06 | 1.02 | 7.50 | 1.06 | 7.09 | 1.03 | 1.02 | 7.41 |
| 38 | G | 7 | 2 | 14 | 1.06 | 1.02 | 15.01 | 1.06 | 14.18 | 0.88 | 1.02 | 12.73 |
| 39 | G | 7 | 3 | 21 | 1.06 | 0.99 | 21.81 | 1.06 | 20.61 | 1.12 | 0.99 | 22.70 |
| 40 | G | 7 | 4 | 28 | 1.06 | 1.01 | 29.95 | 1.06 | 28.31 | 0.84 | 1.01 | 24.14 |
| 41 | G | 7 | 5 | 35 | 1.06 | 1.00 | 37.03 | 1.06 | 35.00 | 1.19 | 1.00 | 41.80 |
| 42 | G | 7 | 6 | 42 | 1.06 | 0.98 | 43.59 | 1.06 | 41.20 | 1.03 | 0.98 | 41.71 |

| DNA ID | Conj. Group | Conc. | Copy No. | Exp. Abun. | Pip. Error | Other Error | Obs. Abun. | Conj. Slope | Norm. Abun. | Pip. Error | Other Error | Obs. Abun. |
|---|---|---|---|---|---|---|---|---|---|---|---|---|
| 43 | H | 8 | 1 | 8 | 1.12 | 0.99 | 8.83 | 1.12 | 7.92 | 1.12 | 0.99 | 8.74 |
| 44 | H | 8 | 2 | 16 | 1.12 | 0.98 | 17.60 | 1.12 | 15.78 | 1.03 | 0.98 | 15.96 |
| 45 | H | 8 | 3 | 24 | 1.12 | 1.01 | 27.17 | 1.12 | 24.37 | 1.12 | 1.01 | 27.60 |
| 46 | H | 8 | 4 | 32 | 1.12 | 1.00 | 35.85 | 1.12 | 32.15 | 1.15 | 1.00 | 37.10 |
| 47 | H | 8 | 5 | 40 | 1.12 | 1.00 | 44.84 | 1.12 | 40.22 | 1.15 | 1.00 | 46.44 |
| 48 | H | 8 | 6 | 48 | 1.12 | 1.00 | 53.56 | 1.12 | 48.03 | 1.14 | 1.00 | 54.47 |
| 49 | I | 9 | 1 | 9 | 1.15 | 1.02 | 10.54 | 1.15 | 9.15 | 0.84 | 1.02 | 7.83 |
| 50 | I | 9 | 2 | 18 | 1.15 | 0.98 | 20.40 | 1.15 | 17.71 | 1.15 | 0.98 | 20.08 |
| 51 | I | 9 | 3 | 27 | 1.15 | 1.00 | 31.22 | 1.15 | 27.10 | 0.98 | 1.00 | 26.68 |
| 52 | I | 9 | 4 | 36 | 1.15 | 1.00 | 41.38 | 1.15 | 35.92 | 0.88 | 1.00 | 31.63 |
| 53 | I | 9 | 5 | 45 | 1.15 | 1.01 | 52.10 | 1.15 | 45.23 | 1.15 | 1.01 | 52.37 |
| 54 | I | 9 | 6 | 54 | 1.15 | 1.00 | 61.90 | 1.15 | 53.74 | 0.88 | 1.00 | 47.20 |
| 55 | J | 10 | 1 | 10 | 0.88 | 1.00 | 8.83 | 0.89 | 9.97 | 1.12 | 1.00 | 11.15 |
| 56 | J | 10 | 2 | 20 | 0.88 | 0.98 | 17.36 | 0.89 | 19.60 | 1.15 | 0.98 | 22.20 |
| 57 | J | 10 | 3 | 30 | 0.88 | 1.01 | 26.78 | 0.89 | 30.24 | 1.06 | 1.01 | 32.24 |
| 58 | J | 10 | 4 | 40 | 0.88 | 1.02 | 35.92 | 0.89 | 40.56 | 1.19 | 1.02 | 48.96 |
| 59 | J | 10 | 5 | 50 | 0.88 | 1.00 | 44.01 | 0.89 | 49.70 | 1.03 | 1.00 | 50.98 |
| 60 | J | 10 | 6 | 60 | 0.88 | 1.01 | 53.53 | 0.89 | 60.45 | 1.15 | 1.01 | 70.39 |
| 61 | K | 11 | 1 | 11 | 0.88 | 1.00 | 9.66 | 0.88 | 10.93 | 1.19 | 1.00 | 12.91 |
| 62 | K | 11 | 2 | 22 | 0.88 | 0.98 | 19.08 | 0.88 | 21.59 | 0.77 | 0.98 | 16.27 |
| 63 | K | 11 | 3 | 33 | 0.88 | 1.02 | 29.58 | 0.88 | 33.47 | 0.88 | 1.02 | 30.00 |
| 64 | K | 11 | 4 | 44 | 0.88 | 1.01 | 39.27 | 0.88 | 44.44 | 1.15 | 1.01 | 51.76 |
| 65 | K | 11 | 5 | 55 | 0.88 | 0.99 | 47.90 | 0.88 | 54.20 | 0.98 | 0.99 | 52.41 |
| 66 | K | 11 | 6 | 66 | 0.88 | 1.02 | 59.22 | 0.88 | 67.01 | 1.03 | 1.02 | 70.07 |
| 67 | L | 12 | 1 | 12 | 0.77 | 0.98 | 9.06 | 0.76 | 11.89 | 1.06 | 0.98 | 12.33 |
| 68 | L | 12 | 2 | 24 | 0.77 | 0.99 | 18.32 | 0.76 | 24.04 | 1.12 | 0.99 | 26.74 |
| 69 | L | 12 | 3 | 36 | 0.77 | 1.01 | 27.92 | 0.76 | 36.64 | 1.15 | 1.01 | 42.67 |
| 70 | L | 12 | 4 | 48 | 0.77 | 0.98 | 36.25 | 0.76 | 47.57 | 0.88 | 0.98 | 41.33 |
| 71 | L | 12 | 5 | 60 | 0.77 | 0.98 | 45.27 | 0.76 | 59.41 | 0.77 | 0.98 | 44.82 |
| 72 | L | 12 | 6 | 72 | 0.77 | 1.00 | 55.34 | 0.76 | 72.63 | 1.14 | 1.00 | 82.69 |
| 73 | M | 13 | 1 | 13 | 1.12 | 1.02 | 14.79 | 1.13 | 13.09 | 1.14 | 1.02 | 15.13 |
| 74 | M | 13 | 2 | 26 | 1.12 | 1.02 | 29.60 | 1.13 | 26.19 | 0.98 | 1.02 | 26.16 |
| 75 | M | 13 | 3 | 39 | 1.12 | 1.00 | 43.77 | 1.13 | 38.73 | 0.98 | 1.00 | 38.13 |
| 76 | M | 13 | 4 | 52 | 1.12 | 1.02 | 59.09 | 1.13 | 52.29 | 0.88 | 1.02 | 46.90 |
| 77 | M | 13 | 5 | 65 | 1.12 | 1.01 | 73.60 | 1.13 | 65.13 | 1.15 | 1.01 | 75.97 |
| 78 | M | 13 | 6 | 78 | 1.12 | 1.00 | 87.03 | 1.13 | 77.02 | 1.14 | 1.00 | 87.34 |
| 79 | N | 14 | 1 | 14 | 1.15 | 1.02 | 16.41 | 1.16 | 14.17 | 0.77 | 1.02 | 11.06 |
| 80 | N | 14 | 2 | 28 | 1.15 | 1.00 | 32.18 | 1.16 | 27.79 | 0.84 | 1.00 | 23.31 |
| 81 | N | 14 | 3 | 42 | 1.15 | 1.00 | 48.25 | 1.16 | 41.67 | 1.06 | 1.00 | 43.81 |
| 82 | N | 14 | 4 | 56 | 1.15 | 1.00 | 64.67 | 1.16 | 55.84 | 1.19 | 1.00 | 66.62 |
| 83 | N | 14 | 5 | 70 | 1.15 | 1.00 | 80.92 | 1.16 | 69.88 | 1.19 | 1.00 | 83.21 |
| 84 | N | 14 | 6 | 84 | 1.15 | 1.02 | 98.27 | 1.16 | 84.86 | 1.06 | 1.02 | 90.86 |
| 85 | O | 15 | 1 | 15 | 0.98 | 1.00 | 14.70 | 0.98 | 15.03 | 0.77 | 1.00 | 11.54 |
| 86 | O | 15 | 2 | 30 | 0.98 | 1.01 | 29.55 | 0.98 | 30.21 | 0.98 | 1.01 | 29.78 |
| 87 | O | 15 | 3 | 45 | 0.98 | 1.01 | 44.74 | 0.98 | 45.73 | 0.98 | 1.01 | 45.47 |
| 88 | O | 15 | 4 | 60 | 0.98 | 1.00 | 58.85 | 0.98 | 60.16 | 1.12 | 1.00 | 67.32 |
| 89 | O | 15 | 5 | 75 | 0.98 | 0.99 | 72.40 | 0.98 | 74.00 | 1.15 | 0.99 | 83.96 |
| 90 | O | 15 | 6 | 90 | 0.98 | 0.98 | 86.74 | 0.98 | 88.67 | 1.19 | 0.98 | 103.76 |

**Table 8.** Analysis of libraries comprising RNA Standards with accompanying immune cell receptor clonotype sequencing. Statistics determined from the alignment, assembly and quantification of both DNA standards and endogenous clonotypes are indicated.

| Immune Receptor Sample | *TCRβ* Female A | *TCRβ* Female B | *TCRβ* Female C |
|---|---|---|---|
| **Overview** | | | |
| Genome Alignments | 2,519,121.00 | 2,921,540.00 | 3,075,138.00 |
| *In Silico* Alignments | 25,191 | 29,215 | 30,751 |
| Dilution | 0.01 | 0.01 | 0.01 |
| **Assembly** | | | |
| Limit of Detection | 0.01 | 0.01 | 0.1 |
| Dynamic Range | 10^6 | 10^6 | 10^5 |
| Clone Sensitivity | 1 | 1 | 1 |
| **Expression** | | | |
| Correlation | 1 | 1 | 1 |
| Slope | 1 | 1 | 1 |
| Y-intercept | 0 | 0 | 0 |
| Highest Clone | 81,565 | 104,654 | 178,359 |
| Fraciton | 0.032 | 0.036 | 0.058 |

EP 3 234 128 B1

Table 9. Design of *in silico* Microbe Genomes and DNA Standards for metagenomic analysis and 16S phylogenetic profiling applications. Indicated are the source genomes and statistics for length and GC% of *in silico* genomes and representative DNA standards.

| SEQ ID | Internal Id | Artificial Microbe Genome | | | DNA Standard | | | |
| | | Source Genome | Length | GC% | Start Coord. | Stop Coord. | Length | GC% |
|---|---|---|---|---|---|---|---|---|
| **Meatagenome Analysis** | | | | | | | | |
| 149 | M1_G | enteFaec | 3218030 | 0.375 | 319803 | 323021 | 3218 | 0.381 |
| 150 | M2_G | eschColi | 4639674 | 0.508 | 461950 | 466589 | 4639 | 0.510 |
| 151 | M3_G | therPetr | 1823510 | 0.461 | 180351 | 182175 | 1824 | 0.458 |
| 152 | M4_G | fusoNucl | 2174499 | 0.272 | 867848 | 870022 | 2174 | 0.281 |
| 153 | M5_G | trepPall | 1138010 | 0.528 | 111796 | 112934 | 1138 | 0.538 |
| 154 | M6_G | saliTrop | 5183330 | 0.695 | 1034653 | 1039836 | 5183 | 0.695 |
| 155 | M7_G | methKand1 | 1694968 | 0.612 | 337010 | 338705 | 1695 | 0.604 |
| 156 | M8_G | persMariEXH1 | 1930283 | 0.372 | 191034 | 192964 | 1930 | 0.374 |
| 157 | M9_G | chloChlo | 2572078 | 0.443 | 1541252 | 1543824 | 2572 | 0.444 |
| 158 | M10_G | bactThet | 6260360 | 0.428 | 5006316 | 5012576 | 6260 | 0.432 |
| 159 | M11_G | nitrMari1 | 1645258 | 0.342 | 162526 | 164171 | 1645 | 0.350 |
| 160 | M12_G | desuVulg | 3570857 | 0.631 | 712169 | 715740 | 3571 | 0.621 |
| **16S Phylogenetic Profiling** | | | | | | | | |
| 161 | M1_SR | enteFaec | 3218030 | 0.375 | 1018270 | 1019270 | 1000 | 0.539 |
| 162 | M2_SR | eschColi | 4639674 | 0.508 | 3246223 | 3247223 | 1000 | 0.540 |
| 163 | M3_SR | therPetr | 1823510 | 0.461 | 754164 | 755164 | 1000 | 0.586 |
| 164 | M4_SR | fusoNucl | 2174499 | 0.272 | 1072371 | 1073371 | 1000 | 0.520 |
| 165 | M5_SR | trepPall | 1138010 | 0.528 | 230163 | 231163 | 1000 | 0.534 |
| 166 | M6_SR | saliTrop | 5183330 | 0.695 | 202619 | 203619 | 1000 | 0.591 |

**Table 10.** Analysis of metagenome libraries comprising DNA Standards with accompanying environmental sample. Statistics determined from the alignment, assembly and quantification of both DNA standards and DNA sample indicated.

| | | | | Adult Male Fecal | | Watson's Creek Soil Samples | | | | | |
|---|---|---|---|---|---|---|---|---|---|---|---|
| Library Standards Mixture | Simulated Flat | Standard Flat | GC Flat | Fecal (Rep1) MixA | Fecal (Rep2) MixA | Soil 1 (Rep 1) MixA | Soil 1 (Rep2) MixA | Soil 1 (Rep3) MixA | Soil 2 (Rep 1) Mix B | Soil 2 (Rep 2) Mix B | Soil 2 (Rep 3) Mix B |
| **Overview** | | | | | | | | | | | |
| Reads to Genome | 0.00 | 0.00 | 0.00 | 225662643 | 229258092 | 76069281 | 64245984 | 80676540 | 70760900 | 70054850 | 74436488 |
| Reads to Artificial | 4000000 | 4000000 | 4000000 | 2014973 | 1979618 | 4317629 | 5151742 | 3712994 | 5165622 | 6617292 | 3486096 |
| Fraction Dilution | - | - | - | 0.009 | 0.009 | 0.057 | 0.080 | 0.046 | 0.073 | 0.094 | 0.047 |
| **Base** | | | | | | | | | | | |
| Sensitivity | 1.00 | 0.997 | 1.000 | 0.80 | 0.82645193 | 0.80 | 0.78 | 0.86 | 0.83 | 0.77 | 0.86 |
| Specificity | 1.00 | 0.975 | 1.000 | 1.00 | 0.999856307 | 1.00 | 1.00 | 1.00 | 1.00 | 1.00 | 1.00 |
| **Assembly** | | | | | | | | | | | |
| Fraction | 1.00 | 0.79 | 0.84 | 0.53 | 0.52 | 0.32 | 0.28 | 0.30 | 0.28 | 0.24 | 0.32 |
| Nodes | 36 | 50 | 10 | 14 | 17 | 20 | 23 | 16 | 18 | 20 | 32 |
| N50 | 1007 | 319 | 894 | 697 | 895 | 508 | 409 | 465 | 658 | 675 | 706 |
| Max. Contig Size | 1007 | 978 | 987 | 937 | 946 | 904 | 847 | 1054 | 999 | 910 | 963 |
| Total Bases | 1.00 | 0.35 | 0.67 | 0.15 | 0.21 | 0.22 | 0.21 | 0.16 | 0.29 | 0.20 | 0.27 |
| **Abundance** | | | | | | | | | | | |
| Correlation | - | - | - | 0.97 | 0.95 | 0.96 | 0.96 | 0.97 | 0.95 | 0.96 | 0.95 |
| Slope | - | - | - | 1.04 | 1.04 | 1.06 | 1.09 | 1.03 | 1.16 | 1.18 | 1.13 |

**Table 11.** Analysis of DNA Metagenome Standards between Mixture A and Mixture B measures quantitative accuracy of fold changes in microbe abundance.

| Sample (with Mixture A) vs. Sample (with Mixture B) | 3 x Soil (High Carbon Content) vs. 3 x Soil (Low Carbon content) |
|---|---|
| **Microbe Abundance** | |
| Correlation | 0.833 |
| Slope | 1.149 |

**Table 12**. Synthetic TCRG standards

| Alignment Number: | | Forward Primer | | | | | | | |
|---|---|---|---|---|---|---|---|---|---|
| | | *TRGVB* | *TRGVA* | *TRGV10* | *TRGV1* | *TRGV9* | *TRGV8* | *TRGV6* | *TRGV11* |
| **Reverse** | *TRGJ1* | 117385 | 138592 | 152191 | 174702 | 272588 | 313671 | 313975 | 62995 |
| **Primer** | *TRGJ2* | 138146 | 108388 | 115122 | 179881 | 238369 | 312534 | 330985 | 96303 |
| | *TRGJP1* | 177614 | 112225 | 236343 | 352427 | 549125 | 529199 | 606064 | 165804 |
| | *TRGJP* | 144436 | 320182 | 189878 | 224320 | 457219 | 452330 | 500545 | |
| | *TRGJP2* | *211121* | 253929 | 227857 | 316722 | 310073 | 368423 | 368662 | 103574 |
| | | | | | | | | | |
| **Normalisation Factor:** | | Forward Primer | | | | | | | |
| | | *TRGVB* | *TRGVA* | *TRGV10* | *TRGV1* | *TRGV9* | *TRGV8* | *TRGV6* | *TRGV11* |
| **Reverse** | *TRGJ1* | 0.45198665 | 0.53364342 | 0.58600588 | 0.67268366 | 1.04959012 | | | 0.24255994 |
| **Primer** | *TRGJ2* | 0.53192612 0.68389621 0.55614553 0.53192612 | 0.41734403 | 0.44327305 | 0.69262521 | 0.91783111 | 1.20777871 | 1.20894925 | 0.37021118 |
| | *TRGJP1* | | 0.43211826 | 0 91003007 | 1.35700727 0.86373595 1.21952647 | 2.11438572 | 1.20340073 2.03766138 | 1.27444563 2.33362725 | 0.63842223 |
| | *TRGJP* | | 1.23284907 | 0.73111829 | | 1.76050503 | 1.74168011 | 1.9273302 1.41951954 | 0.5562726 |
| | *TRGJP2* | | 0.97774432 | 0.87135504 | | 1.19392474 | 1.41859928 | | 0.3988079 |

**References:**

- Altschul, S.F., Gish, W., Miller, W., Myers, E.W. & Lipman, D.J. Basic local alignment search tool. J Mol Biol 215, 403-10 (1990).

- Anders, S., D. J. McCarthy, Y. Chen, M. Okoniewski, G. K. Smyth, W. Huber and M. D. Robinson (2013). "Count-based differential expression analysis of RNA sequencing data using R and Bioconductor." Nat Protoc 8(9): 1765-1786.

- Baker, S.C. et al. The External RNA Controls Consortium: a progress report. Nat Methods 2, 731-4 (2005).

- Bentley, D.R. et al. Accurate whole human genome sequencing using reversible terminator chemistry. Nature 456, 53-9 (2008).

- Bernstein, B.E. et al. Genomic maps and comparative analysis of histone modifications in human and mouse. Cell 120, 169-81 (2005).

- Bolotin, D. A., I. Z. Mamedov, O. V. Britanova, I. V. Zvyagin, D. Shagin, S. V. Ustyugova, M. A. Turchaninova, S. Lukyanov, Y. B. Lebedev and D. M. Chudakov "Next generation sequencing for TCR repertoire profiling: platform-specific features and correction algorithms." Eur J Immunol 42(11): 3073-3083 (2012).

- Burset, M. and R. Guigo "Evaluation of gene structure prediction programs." Genomics 34(3): 353-367 (1996).

- Carlson, C., O'Emerson, R., Sherwood, A., Desmarais, C., Chung, M-W., Parsons, J., Steen, M., A LaMadrid-Herrmannsfeldt, M., Williamson, D., Livingston, R., Wu, D., Wood, B, Rieder, M. & Robins, H. "Using synthetic templates to design an unbiased multiplex PCR assay." Nature Communications 4, Article number 2680 (2013).

- Chen, K., J. W. Wallis, M. D. McLellan, D. E. Larson, J. M. Kalicki, C. S. Pohl, S. D. McGrath, M. C. Wendl, Q. Zhang, D. P. Locke, X. Shi, R. S. Fulton, T. J. Ley, R. K. Wilson, L. Ding and E. R. Mardis (2009). "BreakDancer: an algorithm for high-resolution mapping of genomic structural variation." Nat Methods 6(9): 677-681.

- Chen, Y.C., Liu, T., Yu, C.H., Chiang, T.Y. & Hwang, C.C. Effects of GC bias in next-generation-sequencing data on de novo genome assembly. PLoS One 8, e62856 (2013).

- Clarke, J. et al. Continuous base identification for single-molecule nanopore DNA sequencing. Nat Nanotechnol 4, 265-70 (2009).

- Consortium, E. (2005). "Proposed methods for testing and selecting the ERCC external RNA controls." BMC Genomics 6: 150.

- Coward, E. (1999). "Shufflet: shuffling sequences while conserving the k-let counts." Bioinformatics 15(12): 1058-1059.

- Davies, H. et al. Mutations of the BRAF gene in human cancer. Nature 417, 949-54 (2002).

- DePristo, M. A., E. Banks, R. Poplin, K. V. Garimella, J. R. Maguire, C. Hartl, A. A. Philippakis, G. del Angel, M. A. Rivas, M. Hanna, A. McKenna, T. J. Fennell, A. M. Kernytsky, A. Y. Sivachenko, K. Cibulskis, S. B. Gabriel, D. Altshuler and M. J. Daly (2011). "A framework for variation discovery and genotyping using next-generation DNA sequencing data." Nat Genet 43(5): 491-498.

- Dobin, A., C. A. Davis, F. Schlesinger, J. Drenkow, C. Zaleski, S. Jha, P. Batut, M. Chaisson and T. R. Gingeras (2013). "STAR: ultrafast universal RNA-seq aligner." Bioinformatics 29(1): 15-21.

- Edwards, R.A. et al. Using pyrosequencing to shed light on deep mine microbial ecology. BMC Genomics 7, 57 (2006).

- Eid, J. et al. Real-time DNA sequencing from single polymerase molecules. Science 323, 133-8 (2009).

- Futreal, P. A., L. Coin, M. Marshall, T. Down, T. Hubbard, R. Wooster, N. Rahman and M. R. Stratton (2004). "A census of human cancer genes." Nat Rev Cancer 4(3): 177-183.

- Grosveld, G., T. Verwoerd, T. van Agthoven, A. de Klein, K. L. Ramachandran, N. Heisterkamp, K. Stam and J. Groffen (1986). "The chronic myelocytic cell line K562 contains a breakpoint in bcr and produces a chimeric bcr/c-abl transcript." Mol Cell Biol 6(2): 607-616.

- Haas, B. J., A. Papanicolaou, M. Yassour, M. Grabherr, P. D. Blood, J. Bowden, M. B. Couger, D. Eccles, B. Li, M. Lieber, M. D. Macmanes, M. Ott, J. Orvis, N. Pochet, F. Strozzi, N. Weeks, R. Westerman, T. William, C. N. Dewey, R. Henschel, R. D. Leduc, N. Friedman and A. Regev (2013). "De novo transcript sequence reconstruction from RNA-seq using the Trinity platform for reference generation and analysis." Nat Protoc 8(8): 1494-1512.

- Harrow, J., F. Denoeud, A. Frankish, A. Reymond, C. K. Chen, J. Chrast, J. Lagarde, J. G. Gilbert, R. Storey, D. Swarbreck, C. Rossier, C. Ucla, T. Hubbard, S. E. Antonarakis and R. Guigo (2006). "GENCODE: producing a reference annotation for ENCODE." Genome Biol 7 Suppl 1: S4 1-9.

- Harrow, J., A. Frankish, J. M. Gonzalez, E. Tapanari, M. Diekhans, F. Kokocinski, B. L. Aken, D. Barrell, A. Zadissa, S. Searle, I. Barnes, A. Bignell, V. Boychenko, T. Hunt, M. Kay, G. Mukherjee, J. Rajan, G. Despacio-Reyes, G. Saunders, C. Steward, R. Harte, M. Lin, C. Howald, A. Tanzer, T. Derrien, J. Chrast, N. Walters, S. Balasubramanian, B. Pei, M. Tress, J. M. Rodriguez, I. Ezkurdia, J. van Baren, M. Brent, D. Haussler, M. Kellis, A. Valencia, A. Reymond, M. Gerstein, R. Guigo and T. J. Hubbard (2012). "GENCODE: the reference human genome annotation for The ENCODE Project." Genome Res 22(9): 1760-1774.

- Iqbal, Z., M. Caccamo, I. Turner, P. Flicek and G. McVean (2012). "De novo assembly and genotyping of variants using colored de Bruijn graphs." Nat Genet 44(2): 226-232.

- Jiang, M., J. Anderson, J. Gillespie and M. Mayne (2008). "uShuffle: a useful tool for shuffling biological sequences while preserving the k-let counts." BMC Bioinformatics 9: 192.

- Jiang, L. et al. Synthetic spike-in standards for RNA-seq experiments. Genome Res 21, 1543-51 (2011).

- Johnson, D.S., Mortazavi, A., Myers, R.M. & Wold, B. Genome-wide mapping of in vivo protein-DNA interactions. Science 316, 1497-502 (2007).

- Katz, Y., E. T. Wang, E. M. Airoldi and C. B. Burge (2010). "Analysis and design of RNA sequencing experiments for identifying isoform regulation." Nat Methods 7(12): 1009-1015.

- Kelley, D. R., M. C. Schatz and S. L. Salzberg (2010). "Quake: quality-aware detection and correction of sequencing errors." Genome Biol 11(11): R116.

- Kim, D., G. Pertea, C. Trapnell, H. Pimentel, R. Kelley and S. L. Salzberg (2013). "TopHat2: accurate alignment of transcriptomes in the presence of insertions, deletions and gene fusions." Genome Biol 14(4): R36.

- Koboldt, D.C. et al. (2009) "VarScan: variant detection in massively parallel sequencing of individual and pooled samples." Bioinformatics 25: 2283-5.

- Lander, E.S. et al. Initial sequencing and analysis of the human genome. Nature 409, 860-921 (2001).

- Langmead, B. and S. L. Salzberg (2012). "Fast gapped-read alignment with Bowtie 2." Nat Methods 9(4): 357-359.

- Langmead, B., C. Trapnell, M. Pop and S. L. Salzberg (2009). "Ultrafast and memory-efficient alignment of short DNA sequences to the human genome." Genome Biol 10(3): R25.

- Law, J.C., Ritke, M.K., Yalowich, J.C., Leder, G.H. & Ferrell, R.E. Mutational inactivation of the p53 gene in the human erythroid leukemic K562 cell line. Leuk Res 17, 1045-50 (1993).

- Li, H. and R. Durbin (2009). "Fast and accurate short read alignment with Burrows-Wheeler transform." Bioinformatics 25(14): 1754-1760.

- Li, H., B. Handsaker, A. Wysoker, T. Fennell, J. Ruan, N. Homer, G. Marth, G. Abecasis and R. Durbin (2009). "The Sequence Alignment/Map format and SAMtools." Bioinformatics 25(16): 2078-2079.

- Li, H., B. Handsaker, A. Wysoker, T. Fennell, J. Ruan, N. Homer, G. Marth, G. Abecasis, R. Durbin and S. Genome Project Data Processing (2009). "The Sequence Alignment/Map format and SAMtools." Bioinformatics 25(16): 2078-2079.

- Lieberman-Aiden, E. et al. Comprehensive mapping of long-range interactions reveals folding principles of the human genome. Science 326, 289-93 (2009).

- Logan, A. C., H. Gao, C. Wang, B. Sahaf, C. D. Jones, E. L. Marshall, I. Buno, R. Armstrong, A. Z. Fire, K. I. Weinberg, M. Mindrinos, J. L. Zehnder, S. D. Boyd, W. Xiao, R. W. Davis and D. B. Miklos (2011). "High-throughput VDJ sequencing for quantification of minimal residual disease in chronic lymphocytic leukemia and immune reconstitution assessment." Proc Natl Acad Sci U S A 108(52): 21194-21199.

- MacDonald, J. R., R. Ziman, R. K. Yuen, L. Feuk and S. W. Scherer (2014). "The Database of Genomic Variants: a curated collection of structural variation in the human genome." Nucleic Acids Res 42(Database issue): D986-992.

- McKenna, A., M. Hanna, E. Banks, A. Sivachenko, K. Cibulskis, A. Kernytsky, K. Garimella, D. Altshuler, S. Gabriel, M. Daly and M. A. Depristo (2010). "The Genome Analysis Toolkit: A MapReduce framework for analyzing next-generation DNA sequencing data." Genome Res.

- Meacham, F., D. Boffelli, J. Dhahbi, D. I. Martin, M. Singer and L. Pachter (2011). "Identification and correction of systematic error in high-throughput sequence data." BMC Bioinformatics 12: 451.

- Mitterbauer, G., P. Nemeth, S. Wacha, N. C. Cross, I. Schwarzinger, U. Jaeger, K. Geissler, H. T. Greinix, P. Kalhs, K. Lechner and C. Mannhalter (1999). "Quantification of minimal residual disease in patients with BCR-ABL-positive acute lymphoblastic leukaemia using quantitative competitive polymerase chain reaction." Br J Haematol 106(3): 634-643.

- Mortazavi, A., Williams, B.A., McCue, K., Schaeffer, L. & Wold, B. Mapping and quantifying mammalian transcriptomes by RNA-Seq. Nat Methods 5, 621-8 (2008).

- Pearson, W. R. and D. J. Lipman (1988). "Improved tools for biological sequence comparison." Proc Natl Acad Sci U S A 85(8): 2444-2448.

- Piva, F. and G. Principato (2006). "RANDNA: a random DNA sequence generator." In Silico Biol 6(3): 253-258.

- Robinson, M. D., D. J. McCarthy and G. K. Smyth (2010). "edgeR: a Bioconductor package for differential expression analysis of digital gene expression data." Bioinformatics 26(1): 139-140.

- Ronaghi, M., Uhlen, M. & Nyren, P. A sequencing method based on real-time pyrophosphate. Science 281, 363, 365 (1998).

- Rothberg, J.M. et al. An integrated semiconductor device enabling non-optical genome sequencing. Nature 475, 348-52 (2011).

- Schaap, M., R. J. Lemmers, R. Maassen, P. J. van der Vliet, L. F. Hoogerheide, H. K. van Dijk, N. Basturk, P. de Knijff and S. M. van der Maarel (2013). "Genome-wide analysis of macrosatellite repeat copy number variation in worldwide populations: evidence for differences and commonalities in size distributions and size restrictions." BMC Genomics 14: 143.

- Sherry, S. T., M. H. Ward, M. Kholodov, J. Baker, L. Phan, E. M. Smigielski and K. Sirotkin (2001). "dbSNP: the NCBI database of genetic variation." Nucleic Acids Res 29(1): 308-311.

- Simon, N. E. and A. Schwacha (2014). "The Mcm2-7 Replicative Helicase: A Promising Chemotherapeutic Target." Biomed Res Int 2014: 549719.

- Simpson, J. T., K. Wong, S. D. Jackman, J. E. Schein, S. J. Jones and I. Birol (2009). "ABySS: a parallel assembler for short read sequence data." Genome Res 19(6): 1117-1123.

- Singh, J., A. Behal, N. Singla, A. Joshi, N. Birbian, S. Singh, V. Bali and N. Batra (2009). "Metagenomics: Concept, methodology, ecological inference and recent advances." Biotechnol J 4(4): 480-494.

- Trapnell, C., B. A. Williams, G. Pertea, A. Mortazavi, G. Kwan, M. J. van Baren, S. L. Salzberg, B. J. Wold and L. Pachter (2010). "Transcript assembly and quantification by RNA-Seq reveals unannotated transcripts and isoform switching during cell differentiation." Nat Biotechnol 28(5): 511-515.

- van der Maarel, S. M. and R. R. Frants (2005). "The D4Z4 repeat-mediated pathogenesis of facioscapulohumeral muscular dystrophy." Am J Hum Genet 76(3): 375-386.

- van Dongen, J. J., A. W. Langerak, M. Bruggemann, P. A. Evans, M. Hummel, F. L. Lavender, E. Delabesse, F. Davi, E. Schuuring, R. Garcia-Sanz, J. H. van Krieken, J. Droese, D. Gonzalez, C. Bastard, H. E. White, M. Spaargaren, M. Gonzalez, A. Parreira, J. L. Smith, G. J. Morgan, M. Kneba and E. A. Macintyre (2003). "Design and standardization of PCR primers and protocols for detection of clonal immunoglobulin and T-cell receptor gene recombinations in suspect lymphoproliferations: report of the BIOMED-2 Concerted Action BMH4-CT98-3936." Leukemia 17(12): 2257-2317.

- Villesen, P. (2007). "FaBox: an online toolbox for fasta sequences." Molecular Ecology Notes 7(6): 965-968.

- Yang, J., N. Ramnath, K. B. Moysich, H. L. Asch, H. Swede, S. J. Alrawi, J. Huberman, J. Geradts, J. S. Brooks and D. Tan (2006). "Prognostic significance of MCM2, Ki-67 and gelsolin in non-small cell lung cancer." BMC Cancer 6: 203.

- Zerbino, D. R. and E. Birney (2008). "Velvet: algorithms for de novo short read assembly using de Bruijn graphs." Genome Res 18(5): 821-829.

- Zhang, W., W. Gong, H. Ai, J. Tang and C. Shen (2014). "Gene expression analysis of lung adenocarcinoma and matched adjacent non-tumor lung tissue." Tumori 100(3): 338-345.

- Zook, J.M. et al. Integrating human sequence data sets provides a resource of benchmark SNP and indel genotype calls. Nat Biotechnol 32, 246-51 (2014).

- Zvyagin, I. V., M. V. Pogorelyy, M. E. Ivanova, E. A. Komech, M. Shugay, D. A. Bolotin, A. A. Shelenkov, A. A. Kurnosov, D. B. Staroverov, D. M. Chudakov, Y. B. Lebedev and I. Z. Mamedov (2014). "Distinctive properties of identical twins' TCR repertoires revealed by high-throughput sequencing." Proc Natl Acad Sci U S A 111(16): 5980-5985.

## Claims

1. A method of calibrating a polynucleotide sequencing process, comprising:

i) making one or more artificial polynucleotide sequence comprising:

retrieving a natural nucleotide sequence, and
reversing the natural nucleotide sequence, wherein the reversed sequence has the same nucleotide bases in the same order as the natural nucleotide sequence, wherein the order of the nucleotide bases is in the 5' to 3' direction in the natural nucleotide sequence and in the 3' to 5' direction in the reversed sequence, wherein the artificial polynucleotide sequence comprises at least 100 nucleotides, wherein any 100 contiguous nucleotides of the artificial polynucleotide sequence have less than 100% sequence identity with any known naturally occurring genomic sequence of the same length, and wherein the artificial polynucleotide sequence is a DNA sequence;

wherein the one or more artificial polynucleotide sequence comprises:

(a) one or more features of naturally occurring eukaryotic chromosomes selected from the group consisting of gene loci, introns, exons, CpG islands, mobile elements, repetitive polynucleotide features, small scale genetic variation and large scale genetic variation,

(b) one or more features of naturally occurring prokaryotic chromosomes, or
(c) one or more features of naturally occurring viruses, phages or organelle sequences;

ii) adding the one or more artificial polynucleotide sequence to a sample comprising a target polynucleotide sequence to be determined,
iii) determining the sequence of the target polynucleotide;
iv) determining the sequence of the one or more artificial polynucleotide sequence;
v) comparing the sequence determined in iv) to an original sequence of the artificial polynucleotide sequence;
vi) determining the accuracy of the sequence determination in iv) based on the comparing in iv); and
vii) calibrating the sequence determination in iii) using the accuracy determined in vi).

2. A method of calibrating a polynucleotide quantitation process, comprising:

i) making one or more artificial polynucleotide sequence comprising:

retrieving a natural nucleotide sequence, and
reversing the natural nucleotide sequence, wherein the reversed sequence has the same nucleotide bases in the same order as the natural nucleotide sequence, wherein the order of the nucleotide bases is in the 5' to 3' direction in the natural nucleotide sequence and in the 3' to 5' direction in the reversed sequence, wherein the artificial polynucleotide sequence comprises at least 100 nucleotides, wherein any 100 contiguous nucleotides of the artificial polynucleotide sequence have less than 100% sequence identity with any known naturally occurring genomic sequence of the same length, and wherein the artificial polynucleotide sequence is a DNA sequence;

wherein the one or more artificial polynucleotide sequence comprises:

(a) one or more features of naturally occurring eukaryotic chromosomes selected from the group consisting of gene loci, introns, exons, CpG islands, mobile elements, repetitive polynucleotide features, small scale genetic variation and large scale genetic variation,
(b) one or more features of naturally occurring prokaryotic chromosomes, or
(c) one or more features of naturally occurring viruses, phages or organelle sequences,

ii) adding a known amount of one or more artificial polynucleotide sequence to a sample comprising a target polynucleotide sequence to be determined,
iii) determining the quantity of the target polynucleotide;
iv) determining the quantity of the one or more artificial polynucleotide sequence; and
v) comparing the quantity of the one or more artificial polynucleotide sequence determined in iii) to the known amount of the one or more artificial polynucleotide sequence in i);
vi) determining the accuracy of the quantity determination in iii) based on the comparing in iv); and
vii) calibrating the quantity determination in ii) using the accuracy determined in v).

3. A method as claimed in claim 1 or claim 2, wherein any 50 contiguous nucleotides of the artificial polynucleotide sequence have less than 100% sequence identity with any known naturally occurring genomic sequence of the same length.

4. A method as claimed in claim 1 or claim 2, wherein any 21 contiguous nucleotides of the artificial polynucleotide sequence have less than 100% sequence identity with any known naturally occurring genomic sequence of the same length.

5. A method as claimed in claim 1 or claim 2, wherein any 20 contiguous nucleotides of the artificial polynucleotide sequence have less than 100% sequence identity with any known naturally occurring genomic sequence of the same length.

6. A method as claimed in any preceding claim, wherein:

i) the artificial polynucleotide sequence comprises multiple gene loci;
ii) the repetitive polynucleotide features comprise any one or more of terminal repeats, tandem repeats, inverted repeats and interspersed repeats;

iii) the gene loci comprise immune receptor gene loci;
iv) the small scale genetic variation comprises one or more SNPs, one or more insertions, one or more deletions, one or more microsatellites and/or multiple nucleotide polymorphisms; and/or
v) the large scale genetic variation comprises one or more deletions, one or more duplications, one or more copy-number variants, one or more insertions, one or more inversions and/or one or more translocations.

7. A method as claimed in any preceding claim, wherein making the one or more artificial polynucleotide sequence comprises excising the artificial polynucleotide sequence from a vector by endonuclease digestion, amplification or transcribing the artificial polynucleotide sequence comprised within the vector.

8. A method as claimed in any preceding claim, wherein making the artificial polynucleotide sequence comprises shuffling the nucleotides to remove or reduce the sequence identity with any naturally occurring polynucleotide sequence, wherein contiguous nucleotides within the natural or reversed nucleotide sequence are partitioned into windows of discrete nucleotide lengths along the natural or reversed nucleotide sequence and only those nucleotides within a single window are shuffled together.

9. A method as claimed in any one of claims 1-8, wherein making the artificial polynucleotide sequence comprises substituting nucleotides for alternative nucleotides within the sequence, wherein guanine nucleotides are substituted for cytosine nucleotides, cytosine nucleotides are substituted for guanine nucleotides, adenine nucleotides are substituted for thymine nucleotides and thymine nucleotides are substituted for adenine nucleotides.

10. A method as claimed in any one of claims 1-9, wherein making the artificial polynucleotide sequence further comprises one or both of:

i) shuffling the nucleotides to remove or reduce the sequence identity with any naturally occurring polynucleotide sequence, and
ii) substituting nucleotides for alternative nucleotides within the sequence.

11. A method as claimed in claim 10, wherein the natural nucleotide sequence is reversed and selected windows of the reversed sequence are shuffled to reduce or remove any residual homology in the reversed sequence to known natural sequences.

## Patentansprüche

1. Verfahren zum Kalibrieren eines Polynucleotidsequenzierungsvorgangs, das Folgendes umfasst:

i) Herstellen einer oder mehrerer künstlicher Polynucleotidsequenzen, umfassend:

das Erhalten einer natürlichen Nucleotidsequenz und
das Umkehren der natürlichen Nucleotidsequenz, wobei die umgekehrte Sequenz die gleichen Nucleotidbasen in der gleichen Reihenfolge wie die natürliche Nucleotidsequenz aufweist, wobei die Reihenfolge der Nucleotidbasen in der 5'-3'-Richtung in der natürlichen Nucleotidsequenz ist und in der 3'-5'-Richtung in der umgekehrten Sequenz ist, wobei die künstliche Polynucleotidsequenz zumindest 100 Nucleotide umfasst, wobei beliebige 100 zusammenhängende Nucleotide der künstlichen Polynucleotidsequenz weniger als 100 % Sequenzidentität mit irgendeiner bekannten, natürlich vorkommenden genomischen Sequenz derselben Länge aufweisen und wobei die künstliche Polynucleotidsequenz eine DNA-Sequenz ist;

wobei die eine oder mehreren künstlichen Polynucleotidsequenzen Folgendes umfassen:

(a) ein oder mehrere Merkmale natürlich vorkommender eukaryotischer Chromosomen, die aus der aus Genloci, Introns, Exons, CpG-Inseln, mobilen Elementen, repetitiven Polynucleotidmerkmalen, genetischen Variationen im kleinen Maßstab und genetischen Variationen im großen Maßstab bestehenden Gruppe ausgewählt sind,
(b) ein oder mehrere Merkmale natürlich vorkommender prokaryotischer Chromosomen; oder
(c) ein oder mehrere Merkmale natürlich vorkommender Viren-, Phagen- oder Organellensequenzen;

ii) Hinzufügen der einen oder mehreren künstlichen Polynucleotidsequenzen zu einer Probe, die eine zu bestimmende Ziel-Polynucleotidsequenz umfasst,
iii) Bestimmen der Sequenz des Ziel-Polynucleotids;
iv) Bestimmen der Sequenz der einen oder mehreren künstlichen Polynucleotidsequenzen;
v) Vergleichen der in iv) bestimmten Sequenz mit einer Originalsequenz der künstlichen Polynucleotidsequenz;
vi) Bestimmen der Genauigkeit der Sequenzbestimmung in iv) basierend auf dem Vergleich in iv); und
vii) Kalibrieren der Sequenzbestimmung in iii) unter Verwendung der in vi) bestimmten Genauigkeit.

2. Verfahren zum Kalibrieren eines Polynucleotidquantifizierungsvorgangs, das Folgendes umfasst:

   i) Herstellen einer oder mehrerer künstlicher Polynucleotidsequenzen, umfassend:

   das Erhalten einer natürlichen Nucleotidsequenz und
   das Umkehren der natürlichen Nucleotidsequenz, wobei die umgekehrte Sequenz die gleichen Nucleotidbasen in der gleichen Reihenfolge wie die natürliche Nucleotidsequenz aufweist, wobei die Reihenfolge der Nucleotidbasen in der 5'-3'-Richtung in der natürlichen Nucleotidsequenz ist und in der 3'-5'-Richtung in der umgekehrten Sequenz ist, wobei die künstliche Polynucleotidsequenz zumindest 100 Nucleotide umfasst, wobei beliebige 100 zusammenhängende Nucleotide der künstlichen Polynucleotidsequenz weniger als 100 % Sequenzidentität mit irgendeiner bekannten, natürlich vorkommenden genomischen Sequenz derselben Länge aufweisen und wobei die künstliche Polynucleotidsequenz eine DNA-Sequenz ist;

   wobei die eine oder mehreren künstlichen Polynucleotidsequenzen Folgendes umfassen:

   (a) ein oder mehrere Merkmale natürlich vorkommender eukaryotischer Chromosomen, die aus der aus Genloci, Introns, Exons, CpG-Inseln, mobilen Elementen, repetitiven Polynucleotidmerkmalen, genetischen Variationen im kleinen Maßstab und genetischen Variationen im großen Maßstab bestehenden Gruppe ausgewählt sind,
   (b) ein oder mehrere Merkmale natürlich vorkommender prokaryotischer Chromosomen; oder
   (c) ein oder mehrere Merkmale natürlich vorkommender Viren-, Phagen- oder Organellensequenzen;

   ii) Hinzufügen einer bekannten Menge einer oder mehrerer künstlicher Polynucleotidsequenzen zu einer Probe, die eine zu bestimmende Ziel-Polynucleotidsequenz umfasst,
   iii) Bestimmen der Menge des Ziel-Polynucleotids;
   iv) Bestimmen der Menge der einen oder mehreren künstlichen Polynucleotidsequenzen; und
   v) Vergleichen der in iii) bestimmten Menge der einen oder mehreren künstlichen Polynucleotidsequenzen mit der bekannten Menge der einen oder mehreren künstlichen Polynucleotidsequenzen in i);
   vi) Bestimmen der Genauigkeit der Mengenbestimmung in iii) basierend auf dem Vergleich in iv); und
   vii) Kalibrieren der Mengenbestimmung in ii) unter Verwendung der in v) bestimmten Genauigkeit.

3. Verfahren nach Anspruch 1 oder Anspruch 2, wobei beliebige 50 zusammenhängende Nucleotide der künstlichen Polynucleotidsequenz weniger als 100 % Sequenzidentität mit irgendeiner bekannten, natürlich vorkommenden genomischen Sequenz derselben Länge aufweisen.

4. Verfahren nach Anspruch 1 oder Anspruch 2, wobei beliebige 21 zusammenhängende Nucleotide der künstlichen Polynucleotidsequenz weniger als 100 % Sequenzidentität mit irgendeiner bekannten, natürlich vorkommenden genomischen Sequenz derselben Länge aufweisen.

5. Verfahren nach Anspruch 1 oder Anspruch 2, wobei beliebige 20 zusammenhängende Nucleotide der künstlichen Polynucleotidsequenz weniger als 100 % Sequenzidentität mit irgendeiner bekannten, natürlich vorkommenden genomischen Sequenz derselben Länge aufweisen.

6. Verfahren nach einem der vorangegangenen Ansprüche, wobei:

   i) die künstliche Polynucleotidsequenz mehrere Genloci umfasst;
   ii) die repetitiven Polynucleotidmerkmale ein oder mehrere beliebige terminale Wiederholungen, Tandemwiederholungen, umgekehrte Wiederholungen und verstreut liegende Wiederholungen umfassen;
   iii) die Genloci Immunrezeptor-Genloci umfassen;

iv) die genetische Variation im kleinen Maßstab ein oder mehr SNPs, eine oder mehrere Insertionen, eine oder mehrere Deletionen, eine oder mehrere Mikrosatelliten und/oder mehrere Nucleotidpolymorphismen umfasst; und/oder

v) die genetische Variation im großen Maßstab eine oder mehrere Deletionen, eine oder mehrere Duplikationen, eine oder mehrere Kopienzahlvarianten, eine oder mehrere Insertionen, eine oder mehrere Inversionen und/oder eine oder mehrere Translokationen umfasst.

7. Verfahren nach einem der vorangegangenen Ansprüche, wobei das Herstellen der einen oder mehreren künstlichen Polynucleotidsequenzen das Exzidieren der künstlichen Polynucleotidsequenz aus einem Vektor durch Endonuclease-Verdau, Amplifikation oder Transkription der künstlichen Polynucleotidsequenz, die im Vektor enthalten ist, umfasst.

8. Verfahren nach einem der vorangegangenen Ansprüche, wobei das Herstellen der künstlichen Polynucleotidsequenz das Shuffling der Nucleotide umfasst, um die Sequenzidentität mit irgendeiner natürlich vorkommenden Polynucleotidsequenz zu entfernen oder zu verringern, wobei zusammenhängende Nucleotide in der natürlichen oder umgekehrten Nucleotidsequenz in Fenster aus separaten Nucleotidlängen entlang der natürlichen oder umgekehrten Nucleotidsequenz geteilt werden und nur jene Nucleotide in einem einzelnen Fenster zusammen geshuffelt werden.

9. Verfahren nach einem der Ansprüche 1 bis 8, wobei das Herstellen der künstlichen Polynucleotidsequenz das Substituieren von Nucleotiden durch alternative Nucleotide innerhalb der Sequenz umfasst, wobei Cytosinnucleotide durch Guaninnucleotide substituiert werden, Guaninnucleotide durch Cytosinnucleotide substituiert werden, Thyminnucleotide durch Adeninnucleotide substituiert werden und Adeninnucleotide durch Thyminnucleotide substituiert werden.

10. Verfahren nach einem der Ansprüche 1 bis 9, wobei das Herstellen der künstlichen Polynucleotidsequenz weiters eines oder beide der Folgenden umfasst:

i) Shuffling der Nucleotide, um die Sequenzidentität mit irgendeiner natürlich vorkommenden Polynucleotidsequenz zu entfernen oder zu verringern, und
ii) Substituieren von Nucleotiden durch alternative Nucleotide innerhalb der Sequenz.

11. Verfahren nach Anspruch 10, wobei die natürliche Nucleotidsequenz umgekehrt wird und ausgewählte Fenster der umgekehrten Sequenz geshuffelt werden, um jegliche Resthomologie in der umgekehrten Sequenz mit bekannten natürlichen Sequenzen zu verringern oder zu entfernen.

**Revendications**

1. Procédé d'étalonnage d'un processus de séquençage de polynucléotide, comprenant les étapes consistant à :

i) fabriquer une ou plusieurs séquences de polynucléotide artificielles, comprenant les étapes consistant à :

récupérer une séquence nucléotidique naturelle, et
inverser la séquence nucléotidique naturelle, dans lequel la séquence inversée présente les mêmes bases nucléotidiques dans le même ordre que la séquence nucléotidique naturelle, dans lequel l'ordre des bases nucléotidiques est dans la direction 5' à 3' dans la séquence nucléotidique naturelle et dans la direction 3' à 5' dans la séquence inversée, dans lequel la séquence de polynucléotide artificielle comprend au moins 100 nucléotides, dans lequel 100 nucléotides contigus quelconques de la séquence de polynucléotide artificielle présentent moins de 100 % d'identité de séquence avec toute séquence génomique naturelle connue de même longueur, et dans lequel la séquence de polynucléotide artificielle est une séquence d'ADN ;

dans lequel les une ou plusieurs séquences de polynucléotide artificiels comprennent :

(a) une ou plusieurs caractéristiques de chromosomes eucaryotes naturels choisis dans le groupe comprenant des loci de gène, des introns, des exons, des îlots de CPG, des éléments mobiles, des caractéristiques de polynucléotide répétitives, une variation génétique à petite échelle et une variation génétique à grande échelle,

(b) une ou plusieurs caractéristiques de chromosomes procaryotes naturels, ou

(c) une ou plusieurs caractéristiques de virus, de phages ou de séquences d'organelle naturels ;

ii) ajouter les une ou plusieurs séquences de polynucléotide artificielles à un échantillon comprenant une séquence de polynucléotide cible à déterminer,

iii) déterminer la séquence du polynucléotide cible ;

iv) déterminer la séquence des une ou plusieurs séquences de polynucléotide artificielle ;

v) comparer la séquence déterminée en iv) à une séquence originale de la séquence de polynucléotide artificielle ;

vi) déterminer la précision de la détermination de la séquence en iv) sur la base de la comparaison en iv) ; et

vii) étalonner la détermination de séquence en iii) en utilisant la précision déterminée en vi).

2. Procédé d'étalonnage d'un processus de quantification de polynucléotides, comprenant les étapes consistant à :

i) fabriquer une ou plusieurs séquences de polynucléotide artificielles, comprenant les étapes consistant à :

récupérer une séquence nucléotidique naturelle, et

inverser la séquence nucléotidique naturelle, dans lequel la séquence inversée présente les mêmes bases nucléotidiques dans le même ordre que la séquence nucléotidique naturelle, dans lequel l'ordre des bases nucléotidiques est dans la direction 5' à 3' dans la séquence nucléotidique naturelle et dans la direction 3' à 5' dans la séquence inversée, dans lequel la séquence de polynucléotide artificielle comprend au moins 100 nucléotides, dans lequel 100 nucléotides contigus quelconques de la séquence de polynucléotide artificielle présentent moins de 100 % d'identité de séquence avec toute séquence génomique naturelle connue de même longueur, et dans lequel la séquence de polynucléotide artificielle est une séquence d'ADN ;

dans lequel les une ou plusieurs séquences de polynucléotide artificielles comprennent :

(a) une ou plusieurs caractéristiques de chromosomes eucaryotes naturels choisis dans le groupe comprenant des loci de gène, des introns, des exons, des îlots de CPG, des éléments mobiles, des caractéristiques de polynucléotide répétitives, une variation génétique à petite échelle et une variation génétique à grande échelle,

(b) une ou plusieurs caractéristiques de chromosomes procaryotes naturels, ou

(c) une ou plusieurs caractéristiques de virus, de phages ou de séquences d'organelle naturelles,

ii) ajouter une quantité connue d'une ou plusieurs séquences de polynucléotide artificielles à un échantillon comprenant une séquence de polynucléotide cible à déterminer,

iii) déterminer la quantité du polynucléotide cible ;

iv) déterminer la quantité des une ou plusieurs séquences de polynucléotide artificielles ; et

v) comparer la quantité des une ou plusieurs séquences de polynucléotide artificielles déterminées en iii) à la quantité connue des une ou plusieurs séquences de polynucléotide artificielles en i) ;

vi) déterminer la précision de la détermination de quantité en iii) sur la base de la comparaison en iv) ; et

vii) étalonner la détermination de la quantité en ii) en utilisant la précision déterminée en v).

3. Procédé selon la revendication 1 ou la revendication 2, dans lequel 50 nucléotides contigus quelconque de la séquence de polynucléotide artificielle présentent moins de 100% d'identité de séquence avec toute séquence génomique naturelle connue de même longueur.

4. Procédé selon la revendication 1 ou la revendication 2, dans lequel 21 nucléotides contigus quelconque de la séquence de polynucléotide artificielle présentent moins de 100% d'identité de séquence avec toute séquence génomique naturelle connue de même longueur.

5. Procédé selon la revendication 1 ou la revendication 2, dans lequel 20 nucléotides contigus quelconque de la séquence de polynucléotide artificielle présentent moins de 100% d'identité de séquence avec toute séquence génomique naturelle connue de même longueur.

6. Procédé selon l'une quelconque des revendications précédentes, dans lequel :

i) la séquence de polynucléotide artificielle comprend de multiples loci de gène ;

ii) les caractéristiques de polynucléotide répétitives comprennent une ou plusieurs répétitions terminales, répétitions en tandem, répétitions inversées et répétitions entremêlées ;

iii) les loci de gène comprennent des loci de gène de récepteur immunitaire ;

iv) la variation génétique à petite échelle comprend un ou plusieurs SNP, une ou plusieurs insertions, une ou plusieurs délétions, un ou plusieurs microsatellites et/ou plusieurs polymorphismes nucléotidiques ; et/ou

v) la variation génétique à grande échelle comprend une ou plusieurs délétions, une ou plusieurs duplications, une ou plusieurs variantes de nombre de copies, une ou plusieurs insertions, une ou plusieurs inversions et/ou une ou plusieurs translocations.

7. Procédé selon l'une quelconque des revendications précédentes, dans lequel la fabrication des une ou plusieurs séquences de polynucléotides artificielles comprend une excision de la séquence de polynucléotide artificielle d'un vecteur par digestion d'endonucléase, amplification ou transcription de la séquence de polynucléotide artificielle comprise dans le vecteur.

8. Procédé selon l'une quelconque des revendications précédentes, dans lequel la fabrication de la séquence de polynucléotide artificielle comprend le mélange des nucléotides pour éliminer ou réduire l'identité de séquence avec toute séquence de polynucléotide naturelle, dans lequel des nucléotides contigus à l'intérieur de la séquence nucléotidiques naturelle ou inversée sont divisés en fenêtres de longueurs nucléotidiques discrètes le long de la séquence nucléotidique naturelle ou inversée et seuls les nucléotides à l'intérieur d'une seule fenêtre sont mélangés ensemble.

9. Procédé selon l'une quelconque des revendications 1 à 8, dans lequel la fabrication de la séquence de polynucléotide artificielle comprend une substitution de nucléotides pour des nucléotides alternatifs dans la séquence, dans lequel des nucléotides de guanine sont substitués à des nucléotides de cytosine, des nucléotides de cytosine sont substitués à des nucléotides de guanine, des nucléotides d'adénine sont substitués à des nucléotides de thymine et des nucléotides de thymine sont substitués à des nucléotides d'adénine.

10. Procédé selon l'une quelconque des revendications 1 à 9, dans lequel la fabrication de la séquence de polynucléotide artificielles comprend en outre l'une des étapes suivantes ou les deux :

i) mélanger les nucléotides pour supprimer ou réduire l'identité de séquence avec toute séquence de polynucléotide naturelle, et

ii) substituer des nucléotides à des nucléotides alternatifs dans la séquence.

11. Procédé selon la revendication 10, dans lequel la séquence nucléotidique naturelle est inversée et des fenêtres sélectionnées de la séquence inversée sont mélangées pour réduire ou éliminer toute homologie résiduelle dans la séquence inversée avec des séquences naturelles connues.

Figure 2

A.

Exon

Intron

Artificial Chromosome

B.

Splice Elements

GT          PYP AG

Isoforms

R_1_2_R

R_1_2_V

Alternative Exon

Alternative End

C.

RNA Standards

R_1_2_R

R_1_2_V

Common Exon

Specific Exon

Figure 3

EP 3 234 128 B1

86

**Figure 4**

A — Ancestral Mobile Element — Human Chromosome

Single Copy

Remove Homology

B — Nucleotide substitution, insertion and deletion

Mobile Element Insertion into *in silico* chromosome

C — DNA Standard

Alignment and Analysis

D

Identification of Mobile Element Insertion

Variant Artificial Chromosome

Reference Artificial Chromosome

Figure 5

**A.**

Reference Artificial Chromosome

Small-scale Variation

Homozygous Variation

Variant Artificial Chromosome

Variant A

Variant B

Heterozygous Variation

Produce DNA Standards

**B.**

D_1_1_R

D_1_1_V

Alignment and Analysis

**C.**

Simulated Read Coverage

Identified Variants

Genome Phasing

Figure 6

**Figure 7**

Figure 8

Figure 9

Figure 10

Figure 11

**A.** *TCRG* Gene Complex (Human Genome)

**B.** Artificial *TCRG* Gene Complex (Artificial Chromosome)

**C.** Artificial Chromsome Clone

**D.** Representation as DNA Standard

Figure 12

**A.** TCRβ Gene Loci on Human Genome

Vβ (65 segments)   Dβ (2 segments)   Jβ (13 segments)

⇓ Remove Homology

**B.** TCRβ Gene Loci on Artificial Chromosome

⇓ V(D)J Recombination and Hypermutation

**C.**

Vβ segment   Dβ segment   Jβ segment

DNA Standard   Vβ Family Primers   Insertions/Deletions   Jβ Family Primers

⇓ Conjoined DNA Standards

⇓ PCR Amplificiation (using BIOMED primers)

⇓

TCRG Clonotype Sequencing

**E.**

Cumulative Fraction

3 x DNA Standard

Clonotype Frequency

**F.**

Cumulative Fraction

D

J

V

Segment Frequency

Figure 13

96

EP 3 234 128 B1

Figure 14

Figure 15

Figure 16

Figure 17

**Figure 18**

Figure 19

**Experimental Pipeline**          **Analytical Pipeline**

Figure 20

**Experimental Pipeline**          **Analytical Pipeline**

Human DNA Sample 1

DNA Standards Mix A

Library Preparation ---- Identification and normalisation of
sequence-specific bias

Sample Quality

Sequencing ---- Identification of sequence errors
(eg. Base Recalibration)

Alignment ---- Alignemnt Yield and Accuracy

Variant Detection ---- Sensitivity/Specificity/Precision/
Negative Predictive Value

Genome Phasing

Quantification

Single Sample ---- Quantitative accuracy

Dynamic Range

Library Sensitivity

Variant Homo/Heterozygosity

Gentoype Frequency

Human DNA Sample 2

DNA Standards Mix B

Multiple Samples ---- Normalisation between Samples

DNA Fold-Change

Differential Genotype Abundance
(eg. Tumour Clonal Evolution)

Differential Variant Abundance
(eg. Copy Number Variation)

**uncertainty (eg. 95% Confidence Interval) can be attached to all assessment

**Figure 21**

**Experimental Pipeline**     **Analytical Pipeline**

Environmental DNA Sample 1

DNA Standards Mix A

Library Preparation ········· Identification and normalisation of
sequence-specific bias

Sample Quality

Sequencing ·········· Identification of sequence errors

Alignment Yield
and Accuracy

Library Sensitivity

Binning Accuracy
and Discrimination

Estimate Reference
Genome Absence

Reference-Guided Assembly          *De Novo* Assembly

Optimal k-mer

Assembly Accuracy

N50

Contig Accuracy*

Contig Coverage*

Proportion Assembled*

*Relative to Control Genomes

Quantification

Single Sample ········· Quantitative accuracy

Dynamic Range

Library Sensitivity

Community Size, Structure
and Diversity

Unit Conversion
(Coverage - Molarity)

Multiple Samples ········ Normalisation between Samples

Microbe Fold-change Abundance

Community Structure Changes

**uncertainty (eg. 95% Confidence Interval) can be attached to all assessment

A. K562 - Intron Sensitivity

B. K562 - Exon Sensitivity

C. K562 - Variation in Quantification

**Include examples of poorly assebled transcripts

Figure 22

**Figure 23**

Figure 24

**A.**

K562 - Alternative Splicing

Observed Isoform Relative Abundance

Pearsons's r = 0.761
Slope = 0.835

Expected Isoform Relative Abundance

**B.**

Isoform Switching between Mixture A and B

Observed Isoform Fold-Change (Log$_2$)

-1.58    0    +1.58

Expected Isoform Fold-Change (Log$_2$)

**C.**

Constitutive Exons    Alternative Exon (R_10_2_V specific)    Alternative Exons (R_10_2_R specific)

R_10_2_R
R_10_2_V

K562 (Mix A)

1 x R_10_2_R : 3 x R_10_2_V    3-fold increase    3-fold decrease

GM12878 (Mix B)

RNA standards model 3-fold change in alternative splicing

Mixture A.

Expected Observed

Mixture B.

Expected Observed

Isoform Switching between Mixture A and B

Fold Change (Log$_2$)

Gene Mix. A Mix. B

Figure 25

Figure 26

**Figure 27**

Figure 28

Figure 29

Figure 30

Figure 31

**Figure 32**

**Figure 33**

Figure 34

Figure 35

Figure 36

Figure 37

**Figure 38**

3820

3800

3808

3804

prog

data

3810

3802

3806

3812

3814

3816

Figure 39

A. Organisation of conjoined standard (from 4 individual standards A,B,C and D)

**Figure 40**

A. Example of normal and fusion gene synthetic standards

B.

Figure 41

Figure 41

**Figure 42**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LUKAS PAUL**. Spike-in RNA variants: Design, Production and Application. *ERCC 2.0 Workshop*, 17 August 2014, https://de.slideshare.net/ERCC-Workshop/20140710-3-1paulercc20workshop
- **ALTSCHUL, S.F. et al.** *J Mol Biol*, 1990, vol. 215, 403-10
- **SHERRY, S.T. et al.** *Nucleic Acids Res*, 2001, vol. 29, 308-11
- **DAVIES, H. et al.** *Nature*, 2002, vol. 417, 949-54
- **LAW, J.C. et al.** *Leuk Res*, 1993, vol. 17, 1045-50
- **CHAN, P.P. et al.** *Nucleic Acids Res*, 2012, vol. 40, D646-52
- **VAN DONGEN, J.J. et al.** *Leukemia*, 2003, vol. 17, 2257-317
- **ZVYAGIN, I.V. et al.** *Proc Natl Acad Sci U S A*, 2014, vol. 111, 5980-5
- **ALTSCHUL, S.F.** ; **GISH, W.** ; **MILLER, W.** ; **MYERS, E.W.** ; **LIPMAN, D.J.** Basic local alignment search tool.. *J Mol Biol*, 1990, vol. 215, 403-10
- **ANDERS, S.** ; **D. J. MCCARTHY** ; **Y. CHEN** ; **M. OKONIEWSKI** ; **G. K. SMYTH** ; **W. HUBER** ; **M. D. ROBINSON**. Count-based differential expression analysis of RNA sequencing data using R and Bioconductor. *Nat Protoc*, 2013, vol. 8 (9), 1765-1786
- **BAKER, S.C. et al.** The External RNA Controls Consortium: a progress report.. *Nat Methods 2*, 2005, 731-4
- **BENTLEY, D.R. et al.** Accurate whole human genome sequencing using reversible terminator chemistry.. *Nature*, 2008, vol. 456, 53-9
- **BERNSTEIN, B.E. et al.** Genomic maps and comparative analysis of histone modifications in human and mouse.. *Cell*, 2005, vol. 120, 169-81
- **BOLOTIN, D. A.** ; **I. Z. MAMEDOV** ; **O. V. BRITANOVA** ; **I. V. ZVYAGIN** ; **D. SHAGIN** ; **S. V. USTYUGOVA** ; **M. A. TURCHANINOVA** ; **S. LU-KYANOV** ; **Y. B. LEBEDEV** ; **D. M. CHUDAKOV**. Next generation sequencing for TCR repertoire profiling: platform-specific features and correction algorithms. *Eur J Immunol*, 2012, vol. 42 (11), 3073-3083
- **BURSET, M.** ; **R. GUIGO**. Evaluation of gene structure prediction programs. *Genomics*, 1996, vol. 34 (3), 353-367
- **CARLSON, C.** ; **O'EMERSON, R.** ; **SHERWOOD, A.** ; **DESMARAIS, C.** ; **CHUNG, M-W.** ; **PARSONS, J.** ; **STEEN, M.** ; **A LAMADRID-HERRMANNSFELDT, M.** ; **WILLIAMSON, D.** ; **LIVINGSTON, R.** Using synthetic templates to design an unbiased multiplex PCR assay. *Nature Communications*, 2013, vol. 4
- **CHEN, K.** ; **J. W. WALLIS** ; **M. D. MCLELLAN** ; **D. E. LARSON** ; **J. M. KALICKI** ; **C. S. POHL** ; **S. D. MCGRATH** ; **M. C. WENDL** ; **Q. ZHANG** ; **D. P. LOCKE**. BreakDancer: an algorithm for high-resolution mapping of genomic structural variation.. *Nat Methods*, 2009, vol. 6 (9), 677-681
- **CHEN, Y.C.** ; **LIU, T.** ; **YU, C.H.** ; **CHIANG, T.Y.** ; **HWANG, C.C.** Effects of GC bias in next-generation-sequencing data on de novo genome assembly. *PLoS One*, 2013, vol. 8, e62856
- **CLARKE, J. et al.** Continuous base identification for single-molecule nanopore DNA sequencing.. *Nat Nanotechnol*, 2009, vol. 4, 265-70
- **CONSORTIUM, E.** Proposed methods for testing and selecting the ERCC external RNA controls. *BMC Genomics*, 2005, vol. 6, 150
- **COWARD, E.** Shufflet: shuffling sequences while conserving the k-let counts. *Bioinformatics*, 1999, vol. 15 (12), 1058-1059
- **DAVIES, H. et al.** Mutations of the BRAF gene in human cancer. *Nature*, 2002, vol. 417, 949-54
- **DEPRISTO, M. A.** ; **E. BANKS** ; **R. POPLIN** ; **K. V. GARIMELLA** ; **J. R. MAGUIRE** ; **C. HARTL** ; **A. A. PHILIPPAKIS** ; **G. DEL ANGEL** ; **M. A. RIVAS** ; **M. HANNA**. A framework for variation discovery and genotyping using next-generation DNA sequencing data. *Nat Genet*, 2011, vol. 43 (5), 491-498
- **DOBIN, A.** ; **C. A. DAVIS** ; **F. SCHLESINGER** ; **J. DRENKOW** ; **C. ZALESKI** ; **S. JHA** ; **P. BATUT** ; **M. CHAISSON** ; **T. R. GINGERAS**. STAR: ultrafast universal RNA-seq aligner. *Bioinformatics*, 2013, vol. 29 (1), 15-21
- **EDWARDS, R.A. et al.** Using pyrosequencing to shed light on deep mine microbial ecology.. *BMC Genomics*, 2006, vol. 7, 57
- **EID, J et al.** Real-time DNA sequencing from single polymerase molecules.. *Science*, 2009, vol. 323, 133-8

- **FUTREAL, P. A.** ; **L. COIN** ; **M. MARSHALL** ; **T. DOWN** ; **T. HUBBARD** ; **R. WOOSTER** ; **N. RAHMAN** ; **M. R. STRATTON**. A census of human cancer genes. *Nat Rev Cancer*, 2004, vol. 4 (3), 177-183
- **GROSVELD, G.** ; **T. VERWOERD** ; **T. VAN AGTHOVEN** ; **A. DE KLEIN** ; **K. L. RAMACHANDRAN** ; **N. HEISTERKAMP** ; **K. STAM** ; **J. GROFFEN**. The chronic myelocytic cell line K562 contains a breakpoint in bcr and produces a chimeric bcr/c-abl transcript. *Mol Cell Biol*, 1986, vol. 6 (2), 607-616
- **HAAS, B. J.** ; **A. PAPANICOLAOU** ; **M. YASSOUR** ; **M. GRABHERR** ; **P. D. BLOOD** ; **J. BOWDEN** ; **M. B. COUGER** ; **D. ECCLES** ; **B. LI** ; **M. LIEBER**. De novo transcript sequence reconstruction from RNA-seq using the Trinity platform for reference generation and analysis.. *Nat Protoc*, 2013, vol. 8 (8), 1494-1512
- **HARROW, J.** ; **F. DENOEUD** ; **A. FRANKISH** ; **A. REYMOND** ; **C. K. CHEN** ; **J. CHRAST** ; **J. LAGARDE** ; **J. G. GILBERT** ; **R. STOREY** ; **D. SWARBRECK**. GENCODE: producing a reference annotation for ENCODE. *Genome Biol*, 2006, vol. 7 (1), S4 1-9
- **HARROW, J.** ; **A. FRANKISH** ; **J. M. GONZALEZ** ; **E. TAPANARI** ; **M. DIEKHANS** ; **F. KOKOCINSKI** ; **B. L. AKEN** ; **D. BARRELL** ; **A. ZADISSA** ; **S. SEARLE**. GENCODE: the reference human genome annotation for The ENCODE Project. *Genome Res*, 2012, vol. 22 (9), 1760-1774
- **IQBAL, Z.** ; **M. CACCAMO** ; **I. TURNER** ; **P. FLICEK** ; **G. MCVEAN**. De novo assembly and genotyping of variants using colored de Bruijn graphs. *Nat Genet*, 2012, vol. 44 (2), 226-232
- **JIANG, M.** ; **J. ANDERSON** ; **J. GILLESPIE** ; **M. MAYNE**. uShuffle: a useful tool for shuffling biological sequences while preserving the k-let counts. *BMC Bioinformatics*, 2008, vol. 9, 192
- **JIANG, L. et al.** Synthetic spike-in standards for RNA-seq experiments. *Genome Res*, 2011, vol. 21, 1543-51
- **JOHNSON, D.S.** ; **MORTAZAVI, A.** ; **MYERS, R.M.** ; **WOLD, B.** Genome-wide mapping of in vivo protein-DNA interactions.. *Science*, 2007, vol. 316, 1497-502
- **KATZ, Y.** ; **E. T. WANG** ; **E. M. AIROLDI** ; **C. B. BURGE**. Analysis and design of RNA sequencing experiments for identifying isoform regulation. *Nat Methods*, 2010, vol. 7 (12), 1009-1015
- **KELLEY, D. R.** ; **M. C. SCHATZ** ; **S. L. SALZBERG**. Quake: quality-aware detection and correction of sequencing errors. *Genome Biol*, 2010, vol. 11 (11), R116
- **KIM, D.** ; **G. PERTEA** ; **C. TRAPNELL** ; **H. PIMENTEL** ; **R. KELLEY** ; **S. L. SALZBERG**. TopHat2: accurate alignment of transcriptomes in the presence of insertions, deletions and gene fusions. *Genome Biol*, 2013, vol. 14 (4), R36
- **KOBOLDT, D.C. et al.** VarScan: variant detection in massively parallel sequencing of individual and pooled samples. *Bioinformatics*, 2009, vol. 25, 2283-5
- **LANDER, E.S. et al.** Initial sequencing and analysis of the human genome.. *Nature*, 2001, vol. 409, 860-921
- **LANGMEAD, B.** ; **S. L. SALZBERG**. Fast gapped-read alignment with Bowtie 2. *Nat Methods*, 2012, vol. 9 (4), 357-359
- **LANGMEAD, B.** ; **C. TRAPNELL** ; **M. POP** ; **S. L. SALZBERG**. Ultrafast and memory-efficient alignment of short DNA sequences to the human genome. *Genome Biol*, 2009, vol. 10 (3), R25
- **LAW, J.C.** ; **RITKE, M.K.** ; **YALOWICH, J.C.** ; **LEDER, G.H.** ; **FERRELL, R.E**. Mutational inactivation of the p53 gene in the human erythroid leukemic K562 cell line. *Leuk Res*, 1993, vol. 17, 1045-50
- **LI, H.** ; **R. DURBIN**. Fast and accurate short read alignment with Burrows-Wheeler transform. *Bioinformatics*, 2009, vol. 25 (14), 1754-1760
- **LI, H.** ; **B. HANDSAKER** ; **A. WYSOKER** ; **T. FENNELL** ; **J. RUAN** ; **N. HOMER** ; **G. MARTH** ; **G. ABECASIS** ; **R. DURBIN**. The Sequence Alignment/Map format and SAMtools. *Bioinformatics*, 2009, vol. 25 (16), 2078-2079
- **LIEBERMAN-AIDEN, E. et al.** Comprehensive mapping of long-range interactions reveals folding principles of the human genome.. *Science*, 2009, vol. 326, 289-93
- **LOGAN, A. C.** ; **H. GAO** ; **C. WANG** ; **B. SAHAF** ; **C. D. JONES** ; **E. L. MARSHALL** ; **I. BUNO** ; **R. ARMSTRONG** ; **A. Z. FIRE** ; **K. I. WEINBERG**. High-throughput VDJ sequencing for quantification of minimal residual disease in chronic lymphocytic leukemia and immune reconstitution assessment. *Proc Natl Acad Sci U S A*, 2011, vol. 108 (52), 21194-21199
- **MACDONALD, J. R.** ; **R. ZIMAN** ; **R. K. YUEN** ; **L. FEUK** ; **S. W. SCHERER**. The Database of Genomic Variants: a curated collection of structural variation in the human genome. *Nucleic Acids Res*, 2014, vol. 42, D986-992
- **MCKENNA, A.** ; **M. HANNA** ; **E. BANKS** ; **A. SIVACHENKO** ; **K. CIBULSKIS** ; **A. KERNYTSKY** ; **K. GARIMELLA** ; **D. ALTSHULER** ; **S. GABRIEL** ; **M. DALY**. The Genome Analysis Toolkit: A MapReduce framework for analyzing next-generation DNA sequencing data. *Genome Res.*, 2010

- **MEACHAM, F.** ; **D. BOFFELLI** ; **J. DHAHBI** ; **D. I. MARTIN** ; **M. SINGER** ; **L. PACHTER**. Identification and correction of systematic error in high-throughput sequence data. *BMC Bioinformatics*, 2011, vol. 12, 451

- **MITTERBAUER, G.** ; **P. NEMETH** ; **S. WACHA** ; **N. C. CROSS** ; **I. SCHWARZINGER** ; **U. JAEGER** ; **K. GEISSLER** ; **H. T. GREINIX** ; **P. KALHS** ; **K. LECHNER**. Quantification of minimal residual disease in patients with BCR-ABL-positive acute lymphoblastic leukaemia using quantitative competitive polymerase chain reaction. *Br J Haematol*, 1999, vol. 106 (3), 634-643

- **MORTAZAVI, A.** ; **WILLIAMS, B.A.** ; **MCCUE, K.** ; **SCHAEFFER, L.** ; **WOLD, B.** Mapping and quantifying mammalian transcriptomes by RNA-Seq.. *Nat Methods*, 2008, vol. 5, 621-8

- **PEARSON, W. R.** ; **D. J. LIPMAN**. Improved tools for biological sequence comparison. *Proc Natl Acad Sci U S A*, 1988, vol. 85 (8), 2444-2448

- **PIVA, F.** ; **G. PRINCIPATO**. RANDNA: a random DNA sequence generator. *In Silico Biol*, 2006, vol. 6 (3), 253-258

- **ROBINSON, M. D.** ; **D. J. MCCARTHY** ; **G. K. SMYTH**. edgeR: a Bioconductor package for differential expression analysis of digital gene expression data. *Bioinformatics*, 2010, vol. 26 (1), 139-140

- **RONAGHI, M.** ; **UHLEN, M.** ; **NYREN, P.** A sequencing method based on real-time pyrophosphate.. *Science*, 1998, vol. 281, 363, 365

- **ROTHBERG, J.M. et al.** An integrated semiconductor device enabling non-optical genome sequencing.. *Nature*, 2011, vol. 475, 348-52

- **SCHAAP, M.** ; **R. J. LEMMERS** ; **R. MAASSEN** ; **P. J. VAN DER VLIET** ; **L. F. HOOGERHEIDE** ; **H. K. VAN DIJK** ; **N. BASTURK** ; **P. DE KNIJFF** ; **S. M. VAN DER MAAREL**. Genome-wide analysis of macrosatellite repeat copy number variation in worldwide populations: evidence for differences and commonalities in size distributions and size restrictions. *BMC Genomics*, 2013, vol. 14, 143

- **SHERRY, S. T.** ; **M. H. WARD** ; **M. KHOLODOV** ; **J. BAKER** ; **L. PHAN** ; **E. M. SMIGIELSKI** ; **K. SIROTKIN**. dbSNP: the NCBI database of genetic variation. *Nucleic Acids Res*, 2001, vol. 29 (1), 308-311

- **SIMON, N. E.** ; **A. SCHWACHA**. The Mcm2-7 Replicative Helicase: A Promising Chemotherapeutic Target. *Biomed Res Int*, 2014, vol. 2014, 549719

- **SIMPSON, J. T.** ; **K. WONG** ; **S. D. JACKMAN** ; **J. E. SCHEIN** ; **S. J. JONES** ; **I. BIROL**. ABySS: a parallel assembler for short read sequence data. *Genome Res*, 2009, vol. 19 (6), 1117-1123

- **SINGH, J.** ; **A. BEHAL** ; **N. SINGLA** ; **A. JOSHI** ; **N. BIRBIAN** ; **S. SINGH** ; **V. BALI** ; **N. BATRA**. Metagenomics: Concept, methodology, ecological inference and recent advances. *Biotechnol J*, 2009, vol. 4 (4), 480-494

- **TRAPNELL, C.** ; **B. A. WILLIAMS** ; **G. PERTEA** ; **A. MORTAZAVI** ; **G. KWAN** ; **M. J. VAN BAREN** ; **S. L. SALZBERG** ; **B. J. WOLD** ; **L. PACHTER**. Transcript assembly and quantification by RNA-Seq reveals unannotated transcripts and isoform switching during cell differentiation.. *Nat Biotechnol*, 2010, vol. 28 (5), 511-515

- **VAN DER MAAREL, S. M.** ; **R. R. FRANTS**. The D4Z4 repeat-mediated pathogenesis of facioscapulohumeral muscular dystrophy. *Am J Hum Genet*, 2005, vol. 76 (3), 375-386

- **VAN DONGEN, J. J.** ; **A. W. LANGERAK** ; **M. BRUGGEMANN** ; **P. A. EVANS** ; **M. HUMMEL** ; **F. L. LAVENDER** ; **E. DELABESSE** ; **F. DAVI** ; **E. SCHUURING** ; **R. GARCIA-SANZ**. Design and standardization of PCR primers and protocols for detection of clonal immunoglobulin and T-cell receptor gene recombinations in suspect lymphoproliferations: report of the BIOMED-2 Concerted Action BMH4-CT98-3936. *Leukemia*, 2003, vol. 17 (12), 2257-2317

- **VILLESEN, P.** FaBox: an online toolbox for fasta sequences. *Molecular Ecology Notes*, 2007, vol. 7 (6), 965-968

- **YANG, J.** ; **N. RAMNATH** ; **K. B. MOYSICH** ; **H. L. ASCH** ; **H. SWEDE** ; **S. J. ALRAWI** ; **J. HUBERMAN** ; **J. GERADTS** ; **J. S. BROOKS** ; **D. TAN**. Prognostic significance of MCM2, Ki-67 and gelsolin in non-small cell lung cancer. *BMC Cancer*, 2006, vol. 6, 203

- **ZERBINO, D. R.** ; **E. BIRNEY**. Velvet: algorithms for de novo short read assembly using de Bruijn graphs. *Genome Res*, 2008, vol. 18 (5), 821-829

- **ZHANG, W.** ; **W. GONG** ; **H. AI** ; **J. TANG** ; **C. SHEN**. Gene expression analysis of lung adenocarcinoma and matched adjacent non-tumor lung tissue. *Tumori*, 2014, vol. 100 (3), 338-345

- **ZOOK, J.M. et al.** Integrating human sequence data sets provides a resource of benchmark SNP and indel genotype calls.. *Nat Biotechnol*, 2014, vol. 32, 246-51

- **ZVYAGIN, I. V.** ; **M. V. POGORELYY** ; **M. E. IVANOVA** ; **E. A. KOMECH** ; **M. SHUGAY** ; **D. A. BOLOTIN** ; **A. A. SHELENKOV** ; **A. A. KURNOSOV** ; **D. B. STAROVEROV** ; **D. M. CHUDAKOV**. Distinctive properties of identical twins' TCR repertoires revealed by high-throughput sequencing. *Proc Natl Acad Sci U S A*, 2014, vol. 111 (16), 5980-5985